(19) Europäisches Patentamt
European Patent Office
Office européen des brevets

(11) Publication number: **0 209 897 A2**

(12) **EUROPEAN PATENT APPLICATION**

(21) Application number: 86110125.1

(22) Date of filing: 23.07.86

(51) Int. Cl.⁴: C 07 K 5/00
C 08 K 7/00, C 07 C 103/50
C 07 D 233/64, A 61 K 37/64
A 61 K 31/195, C 07 F 9/65
A 61 K 31/66

(30) Priority: 24.07.85 US 758625

(43) Date of publication of application:
28.01.87 Bulletin 87/5

(84) Designated Contracting States:
AT BE CH DE FR GB IT LI LU NL SE

(71) Applicant: MERCK & CO. INC.
126, East Lincoln Avenue P.O. Box 2000
Rahway New Jersey 07065(US)

(72) Inventor: Boger, Joshua S.
719 New Gulph Road
Bryn Mawr Pennsylvania 19010(US)

(72) Inventor: Bock, Mark G.
1603 Leon Drive
Hatfield Pennsylvania 19440(US)

(72) Inventor: Freidinger, Roger
2185 Rebecca Drive
Hatfield Pennsylvania 19440(US)

(72) Inventor: Veber, Daniel F.
290 Batleson Road
Ambler Pennsylvania 19002(US)

(72) Inventor: Patchett, Arthur A.
1090 Minisink Way
Westfield New Jersey 07090(US)

(72) Inventor: Greenlee, William J.
115 Herrick Avenue
Teaneck New Jersey 07666(US)

(72) Inventor: Parsons, William H.
2171 Oliver Street
Rahway New Jersey 07065(US)

(74) Representative: Abitz, Walter, Dr.-Ing. et al,
Abitz, Morf, Gritschneder, Freiherr von Wittgenstein
Postfach 86 01 09
D-8000 München 86(DE)

(54) **Peptide enzyme inhibitors.**

(57) Enzyme peptides of the formula

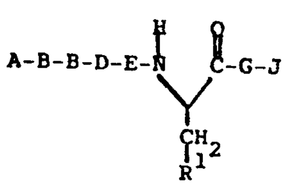

(I.)

and analogs thereof which inhibit renin and are useful for treating various forms of renin-associated hypertension and hyperaldosteronism.

EP 0 209 897 A2

41180/1213A
41190/1252A



TITLE OF THE INVENTION

PEPTIDE ENZYME INHIBITORS

BACKGROUND OF THE INVENTION

1. Field of The Invention

The present invention is concerned with novel peptides which inhibit renin, some peptides of which also inhibit angiotensin converting enzyme (A.C.E.).

The present invention is also concerned with pharmaceutical compositions containing the novel peptides of the present invention as active ingredients, with methods of treating renin-associated hypertension and hyperaldosteronism, with treating congestive heart failure, with diagnostic methods which utilize the novel peptides of the present invention, and with methods of preparing the novel peptides of the present invention.

Renin is a proteolytic enzyme of molecular weight about 40,000, produced and secreted by the juxtaglomerular cells of the kidney. Renin acts on the plasma substrate, angiotensinogen, to split off the decapeptide angiotensin I, which is converted to the potent pressor agent angiotensin II. Thus, the renin-angiotensin system plays an important role in normal cardiovascular homeostasis and in some forms of hypertension.

In the past, attempts to modulate or manipulate the renin-angiotensin system have met with success in the use of inhibitors of angiotensin I converting enzyme. In view of this success, it was concluded that a specific inhibitor of the limiting enzymatic step that ultimately regulates angiotensin II production, the action of renin on its substrate, would be at least equally successful. Thus, an effective inhibitor of renin has been long sought as both a therapeutic agent and as an investigative tool.

2. Brief Description of the Prior Art

There has been substantial interest in the synthesis of useful renin inhibitors for many decades; and the following table lists the major classes of renin inhibitors that have been studied, as well as their relative inhibition constants ($K_i$):

| Class | $K_i$ (M) |
|---|---|
| Renin antibody | probably $10^{-6}$ |
| Pepstatin | $10^{-6}$ - $10^{-7}$ |
| Phospholipids | $10^{-3}$ |
| Substrate analogs | |
| Tetrapeptides | $10^{-3}$ |
| Octa- to tridecapeptides | $10^{-5}$ - $10^{-6}$ |

Umezawa et al., in J. Antibiot. (Tokyo) 23: 259-262, 1970, reported the isolation of a peptide, known as pepstatin, from actinomyces that was an inhibitor of aspartyl proteases such as pepsin, cathepsin D, and renin. Pepstatin, the structure of which is

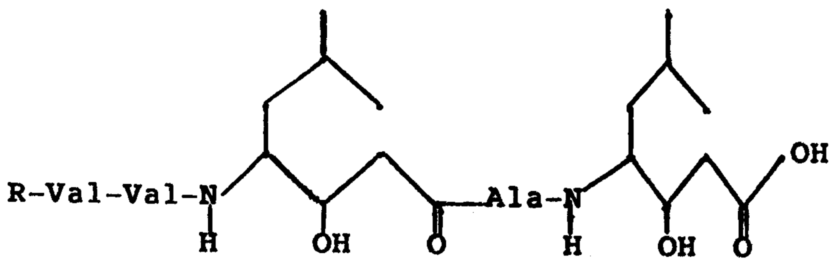

was subsequently found by Gross et al., in Science 175:656, 1971, to reduce blood pressure in vivo after the injection of hog renin into nephrectomized rats, bit it has not found wide application as an experimental agent because of its limited solubility and its additional inhibition of a variety of other acid proteases in addition to renin.

To date, many efforts have been made to prepare a specific renin inhibitor based on substrate analogy, with this analogy generally being based on the known pig renin substitute, since since the human renin substrate has only recently been elucidated (Tewksbury et al., Circulation 59, 60, Supp. II: 132, Oct. 1979), This substrate analogy based on pig renin has always been considered acceptable in the art as predictive of human renin inhibitory activity because of the closely related activity of the two renins, even though the human and pig substrates are not the same. Thus, while pig renin does not cleave the human

renin substrate, human renin, on the other hand, does cleave the pig renin substrate. See Poulsen et al., Biochim. Biophys. Acta 452:533-537, 1976; and Skeggs, Jr. et al., J. Exp. Med. 106:439-453, 1957. Moreover, the human renin inhibitory activity of the peptides of the present invention most active in inhibiting pig renin has been confirmed, thus providing further evidence of this accepted correlation between human and pig renin activity.

It has been found, for example, using pig renin substrate analogy, that the octapeptide sequence extending from histidine-6 through tyrosine-13 has kinetic parameters essentially the same as those of the full tetradecapeptide renin substrate. The amino acid sequence of the octapeptide in pig renin substrate is as follows:

```
 6   7   8   9   10  11  12  13
-His-Pro-Phe-His-Leu-Leu-Val-Tyr-
```

Renin cleaves this substrate between $Leu^{10}$ and $Leu^{11}$.

Kokubu et al., Biochem. Pharmacol. 22: 3217-3223, 1973, synthesized a number of analogs of the tetrapeptide found between residues 10 to 13, but while inhibition could be shown, inhibitory constants were only of the order of $10^{-3}$M.

Analogs of a larger segment of renin substrate were also synthesized: Burton et al., Biochemistry 14: 3892-3898, 1975, and Poulsen et al., Biochemistry 12: 3877-3882, 1973. Two of the major obstacles which had to be overcome to obtain an effective renin inhibitor useful in vivo were lack of

solubility and weak binding (large inhibitory constant). Modifications to increase solubility soon established that the inhibitory properties of the peptides are markedly dependent on the hydrophobicity of various amino acid residues, and that increasing solubility by replacing lipophilic amino acids with hydrophilic isosteric residues becomes counterproductive. Other approaches to increasing solubility have had limited success. Various modifications designed to increase binding to renin have also been made, but here too, with only limited success. For a more detailed description of past efforts to prepare an effective inhibitor of renin, see Haber and Burton, Fed. Proc. Fed. Am. Soc. Exp. Biol. 38: 2768-2773, 1979.

More recently, Szelke et al., in work described in European Patent Publication No. 45,665; Nature, 299, 555 (1982); Hypertension, 4, Supp. 2, 59, 1981; British Patent No. 1,587,809; and "Novel Transition-State Analogue Inhibitors of Renin", a presentation at the Eighth American Peptide Symposium, May 22-27, 1983, Tucson, Arizona, have replaced the Leu-Leu site of renin cleavage by isosteric substitution, and obtained compounds with excellent potency.

Powers et al., in Acid Proteases, Structure, Function and Biology, Plenum Press, 1977, 141-157 have suggested that in pepstatin, statine occupies the space of the two amino acids on either side of the cleavage site of a pepsin substrate, and Tang et al., in Trends in Biochem. Sci., 1:205-208 (1976) and J. Biol. Chem., 251:7088-94, 1976, have proposed that the statine residue of pepstatin resembles the transition

state for pepsin hydrolysis of peptide bonds. However, the applicability of these concepts to renin inhibitors is not taught in any of these references, and would be speculative due to the known high degree of specificity of the renin enzyme.

Kokubu et al., Biochem. Biophys. Res. Comm. 118:929-933, 1984; and Fehrentz et al., FEBS Letters 167: 273-276, 1984, have prepared a renin inhibitor in which a C-terminal aldehyde is used to mimic $Leu^{10}$ of the substrate. However, there is no suggestion of the renin inhibitors of the present invention in which statine and other moieties replace $Leu^{10}$-$Leu^{11}$ of the substrate.

Veber and Rich, in U.S. Patent No. 4,384,994 and published European Patent Application No. 0,077,029; Evans and Rittle, in U.S. Patent No. 4,397,786; Veber and Boger, in published European Patent Application No. 0,077,028; Boger et al, Nature, 303:81-84 (1983); have all described renin inhibitory peptides containing statine; and in Nature there is further described renin inhibitors having a shortened C-terminus, with a non-peptide ending after the 11-position. However, none of these references describe or suggest the renin inhibitors of the present invention and the significant increase in renin inhibitory activity obtainable therewith. Moreover, the Nature reference teaches away from renin inhibitors having non-peptide components after the 11-position, as with the inhibitors of the present invention.

For other articles describing previous efforts to devise renin inhibitors, see Marshall, Federation Proc. 35: 2494-2501, 1976; Burton et al.,

*Proc. Natl. Acad. Sci. USA* 77: 5476-5479, Sept. 1980; Suketa et al., *Biochemistry* 14: 3188, 1975; Swales, *Pharmac. Ther.* 7: 173-201, 1979; Kokubu et al., *Nature* 217: 456-457, Feb. 3, 1968; Matsushita et al., *J. Antibiotics* 28: 1016-1018, Dec. 1975; Lazar et al., *Biochem. Pharma.* 23: 2776-2778, 1974; Miller et al., *Biohem. Pharma.* 21: 2941-2944, 1972; Haber, *Clinical Science* 59:7s-19s, 1980; Rich et al., *J. Org. Chem.* 43: 3624, 1978, and *J. Med. Chem.* 23: 27, 1980; Burton et al., U.S. Pat. No. 4,269,827; Castro et al., U.S. Pat. No. 4,185,096; and Sankyo Jap. Pat. No. 76-067001.

## DETAILED DESCRIPTION OF THE INVENTION AND PREFERRED EMBODIMENTS

In accordance with the present invention there are provided renin inhibitory peptides of the formula:

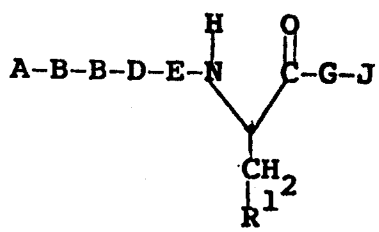

(I.)

wherein:

A is hydrogen; or 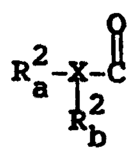

where

X is -O-; -O-CH-; -CH-O-; -CH-; -NH-CH-; or -S-CH-; and

$R_a^2$ and $R_b^2$ may be the same or different and are hydrogen; $W-(CH_2)_n-$ or $W-(CH_2)_m-CH=CH-(CH_2)_p$, where W is hydrogen; $C_{1-4}$alkyl; aryl; $C_{3-7}$cycloalkyl; or $C_{3-7}$cycloalkyl or aryl

substituted with up to five members independently selected from the group consisting of $C_{1-8}$alkyl, trifluoromethyl, hydroxy, $C_{1-4}$alkoxy, and halo; n is 0 to 5; m is 0 to 2; and p is 0 to 2; except that where X is -O-, only one of $R_a^2$ or $R_b^2$ is present;

B is absent; glycyl; sarcosyl; or 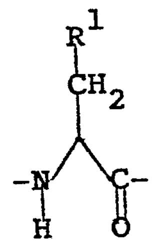,

where $R^1$ is as defined further below;

D is absent; or 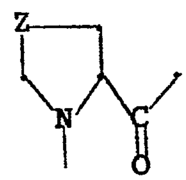, where Z is $-(CH_2)_l-$ and l is 1 or 2; or -S-;

E is absent; or 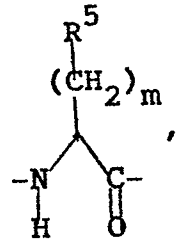, where m is 1 to 4; and

$R^5$ is hydrogen; $C_{1-4}$ alkyl; aryl; aryl-$C_{1-4}$ alkyl; aryl $C_{1-4}$ alkyl or aryl where the aryl portion is substituted with up to three members selected from the group consisting of $C_{1-4}$ alkyl, trifluoromethyl, hydroxy, $C_{1-4}$ alkoxy, and halo; or indolyl;

G is (1) 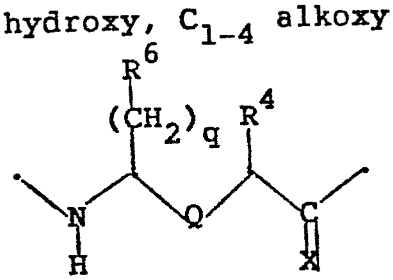 where q is 1 to 4; X is O, or H, H;

$R^4$ is hydrogen; or $\underset{R^3}{\mathrm{CH}}\text{-}R^9$,

where $R^9$ is hydrogen; $C_{1-4}$alkyl; hydroxy, or $C_{3-7}$cycloalkyl; and $R^3$ is hydrogen; $C_{1-4}$alkyl; aryl; aryl $C_{1-4}$alkyl; aryl $C_{1-4}$alkyl or aryl substituted with up to three members selected from the group consisting of $C_{1-4}$alkyl, trifluoromethyl, hydroxy, $C_{1-4}$alkoxy, and halo; or indolyl;

$R^6$ is $C_{3-6}$ alkyl; $C_{3-7}$ cycloalkyl; aryl; or $C_{3-7}$cycloalkyl or aryl substituted with up to three members selected from the group consisting of $C_{1-4}$alkyl, trifluoromethyl, hydroxy, $C_{1-4}$alkoxy, and halo; and

Q is 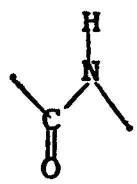 ; 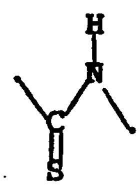 ; 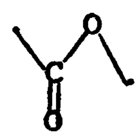 ; 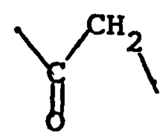 ;

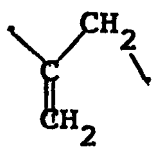 ; 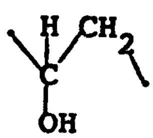 ; 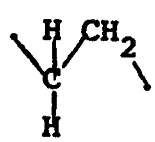 ; 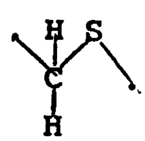 ;

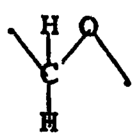 ; 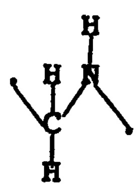 ; 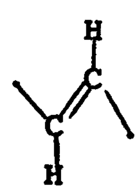 ; 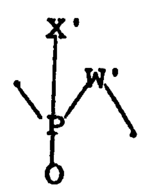 ;

wherein X' is hydroxy; $OR_4'$ wherein $R_4'$ is as defined below; amino; or mono- or di-$C_{1-4}$alkyl amino; and W' is absent; -O-; -NH-; or $-CH_2-$;

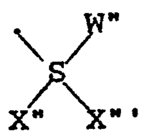

where X" and X"' are independently absent; or $\overset{O}{\uparrow}\!\!\!\!S$; and

W" is absent; $-CH_2-$; or $-\overset{R^8}{\overset{|}{C}}H-$, where $R^8$ is hydrogen or $C_{1-3}$ alkyl;

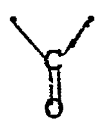 ; 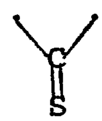 ; or

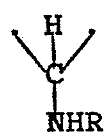 ; where R is hydrogen; $C_{1-4}$ alkyl; formyl; $C_{1-4}$ alkanoyl; aroyl; carboxy; $C_{1-4}$ alkoxycarbonyl; aryloxycarbonyl; or aryl $C_{1-4}$ alkoxycarbonyl; or

(2) 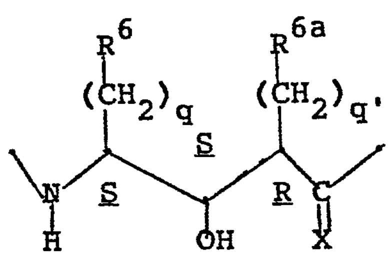 where q is 1 to 4; q' is 0 to 4; X is O or H, H;

$R^6$ is as defined above; and

$R^{6a}$ is hydrogen; $C_{1-8}$alkyl; $C_{2-8}$alkyl substituted with one or two members independently selected from the group consisting of hydroxy, carboxy, carboxy ester or amide, amino, mono-, di-, or tri-$C_{1-4}$alkylamino, and guanidyl; wherein said substitution occurs on the last 1 or 2 carbon atoms of the alkyl chain; aryl; $C_{3-7}$cycloalkyl; or aryl or $C_{3-7}$cycloalkyl substituted with up to three members selected from the group consisting of $C_{1-4}$alkyl, trifluoromethyl, hydroxy, $C_{1-4}$alkoxy, and halo; wherein the substituent of the above formula has 2*R*, 3*S*, 4*S* configuration;

J is (1) $-Y-(CH_2)_n-R^7$

where

Y is -NH-, -O- or $N(CH_2)_n-R^7$;

n is 0 to 5; and

$R^7$ is hydrogen, provided that where n is 0 and $R^7$ is hydrogen, that G is other than Sta and E is other than Phe; hydroxy; $C_{1-4}$alkyl; $C_{3-7}$cycloalkyl; aryl; aryl substituted with up to five members independently selected from the group consisting of $C_{1-6}$alkyl, trifluoromethyl, hydroxy, $C_{1-4}$alkoxy, amino, mono- or di- $C_{1-4}$ alkylamino, and halo; $N(R')_2$, where R' may be the same or different and is hydrogen, $C_{1-4}$alkyl, aryl, aryl $C_{1-4}$alkyl, heterocyclic, or

heterocyclic $C_{1-4}$alkyl;
$N(R')^{+}{}_{3}A^{-}$, where R' is as defined above, and $A^{-}$ is a counterion; guanidyl; heterocyclic; heterocyclic substituted with up to five members independently selected from the group consisting of $C_{1-6}$alkyl, hydroxy, trifluoromethyl, $C_{1-4}$alkoxy, halo, aryl, aryl $C_{1-4}$alkyl, amino, and mono- or di-$C_{1-4}$alkylamino; or heterocyclic substituted with another, the same or different, heterocyclic;

(2) 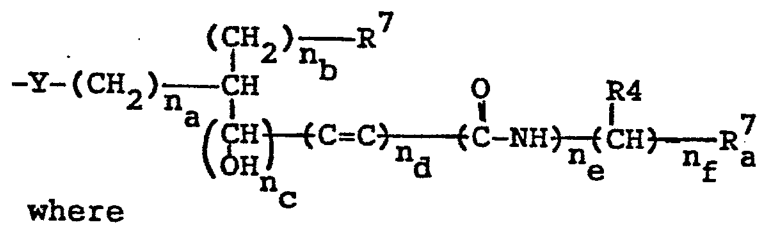

where

Y is as defined above;

$n_a$ is 0 or 1;

$n_b$ is 1 to 4;

$n_c$ is 0 or 1;

$n_d$ is 0 or 1;

$n_e$ is 0 or 1, provided that $n_e$ cannot be 1 when $n_d$ is 0;

$n_f$ is 1 to 4;

$R^4$ is hydrogen; or $-CH(R^3)-R^9$, where

$R^9$ is hydrogen; $C_{1-4}$alkyl; hydroxy; or $C_{3-7}$cycloalkyl; and $R^3$ is hydrogen; $C_{1-4}$alkyl; aryl;

aryl $C_{1-4}$alkyl;

aryl $C_{1-4}$alkyl or aryl substituted with up to three members selected from the group consisting of $C_{1-4}$alkyl, trifluoromethyl, hydroxy, $C_{1-4}$alkoxy, and halo; or indolyl; and

$R^7$ and $R^7_a$ may be the same or different and have the same meaning as $R^7$ above and $R^7_a$ may additionally be

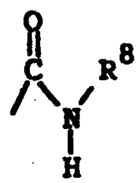 or 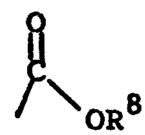,

where $R^8$ is hydrogen or $C_{1-3}$alkyl;

(3) 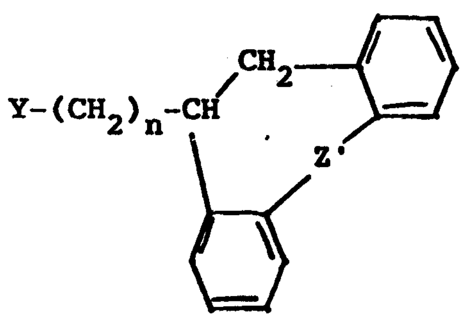

where

Y is as defined above;

n is 0 or 1; and

Z' is

(a) 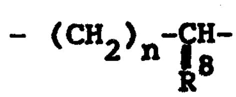

where

n is 0 or 1; and

$R^8$ is as defined above; or

(b) $-(CH_2)_n-\underset{\displaystyle CH_2}{\underset{|}{C}}-$

where

n is 0 or 1; or

(4) (a) $Y-(\overset{\displaystyle R^{10}}{\overset{|}{CH}})_q-R^{11}$; (b) $Y-(\overset{\displaystyle R^{12}}{\overset{|}{CH}})_{q'}-R^{13}$; or

(c) $Y-\underset{\displaystyle R^{14}}{\underset{|}{CH}}-R^{11}$

where

Y is -NH- or -O-;

q is 1-5;

q' is 0-5;

$R^{10}$ is hydrogen; hydroxy; $N(R")_2$, where R" may be the same or different and is hydrogen or $C_{1-4}$alkyl; guanidyl; or $N^{\oplus}(R")_3A^{\ominus}$, where R" is as defined above, and $A^{\ominus}$ is a counterion; provided that at least one $R^{10}$ is not hydrogen;

$R^{11}$ is $C_{1-4}$alkyl; $C_{3-7}$cycloalkyl; aryl; aryl substituted with up to three members independently selected from the group consisting of $C_{1-6}$alkyl, trifluoromethyl, hydroxy, $C_{1-4}$alkoxy, amino, mono- or di- $C_{1-4}$alkylamino, amino $C_{1-4}$alkyl, mono-, di-, or tri-$C_{1-4}$alkylamino-$C_{1-4}$alkyl, halo, carboxy, carboxy ester or amide, carboxy-$C_{1-4}$-alkoxy, carboxy-$C_{1-4}$-alkoxy ester or amide, α-aminocarboxy-

$C_{1-4}$alkyl, α-aminocarboxy-$C_{1-4}$-alkyl ester or amide, carboxy-$C_{1-4}$-alkyl, carboxy-$C_{1-4}$-alkyl ester or amide, guanidyl, and guanidyl-$C_{1-4}$-alkyl; carboxy, ester or amide; sulfo; heterocyclic; or heterocyclic substituted with up to five members independently selected from the group consisting of $C_{1-6}$alkyl, hydroxy, trifluoromethyl, $C_{1-4}$alkoxy, halo, aryl, aryl $C_{1-4}$alkyl, amino, and mono- or di-$C_{1-4}$alkylamino;

$R^{12}$ is hydrogen; or carboxy, ester or amide;

$R^{13}$ is carboxy, ester or amide; sulfo; or aryl substituted with up to three members selected from the group consisting of amino-$C_{1-4}$alkyl, mono-, di-, or tri-$C_{1-4}$-alkyl-amino-$C_{1-4}$-alkyl, halo, carboxy, carboxy ester or amide, carboxy-$C_{1-4}$alkoxy, carboxy-$C_{1-4}$alkoxy ester or amide, α-amino-carboxy-$C_{1-4}$alkyl, α-aminocarboxy-$C_{1-4}$-alkyl ester or amide, carboxy-$C_{1-4}$-alkyl, carboxy-$C_{1-4}$alkyl ester or amide, guanidyl, and guanidyl-$C_{1-4}$-alkyl; and

$R^{14}$ is carboxy, ester or amide;

(d) 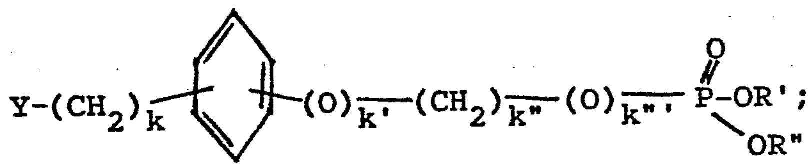

or

(e) 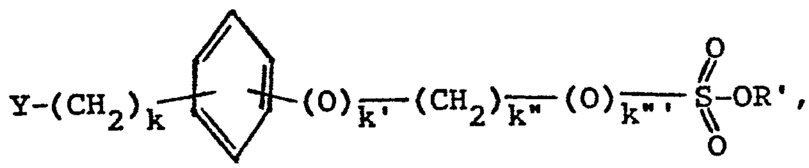

where

Y is -NH- or -O-;

k is 0-4;

k' is 0 or 1;

k" is 0-4;

k"'is 0 or 1;

R' is hydrogen or $C_{1-4}$alkyl; and

R" is hydrogen or $C_{1-4}$alkyl;

(5) 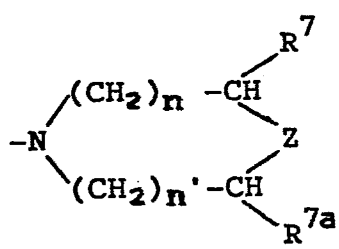

where Z is NH, N-$R^7$,O,S or $CHR^7$;

n' is 0 to 5; and

$R^{7a}$ is hydrogen, hydroxy, $C_{1-4}$-alkyl,$C_{3-7}$-cydoalkyl, aryl,ary 1 substituted with from one to five members independently selected from the group consisting of $C_{1-6}$-alkyl trifluoromethyl, hydroxy, $C_{1-4}$alkoxy, amino, mono- or di- $C_{1-4}$ alkylamino, and halo; $N(R')_2$, where R' may be the same or different and is hydrogen,

$C_{1-4}$alkyl, aryl, aryl $C_{1-4}$alkyl, heterocycli, or heterocyclic $C_{1-4}$alkyl; $N(R')_3^+A^\ominus$, where R' is as defined above, and $A^\ominus$ is a counterion; guanidyl; heterocyclic; heterocyclic substitutued with up to five members independently selected from the group consisting of $C_{1-6}$alkyl, hydroxy, trifluoromethyl, $C_{1-4}$alkoxy, halo, aryl, aryl $C_{1-4}$alkyl, amino, and mono- or di-$C_{1-4}$alkylamino; or heterocyclic substituted with another, the same or different, heterocyclic;

$R^1$ is hydrogen; $C_{1-4}$ alkyl; hydroxy $C_{1-4}$alkyl; aryl; aryl substituted with up to three members selected from the group consisting of $C_{1-4}$ alkyl, trifluoromethyl, hydroxy, $C_{1-4}$ alkoxy, and halo; indolyl; 4-imidazolyl; amino $C_{2-4}$ alkyl; acyl $C_{2-4}$ alkyl wherein the acyl is $R^9-\overset{O}{\overset{\|}{C}}-$ and $R^9$ is as defined above; guanidyl $C_{2-3}$ alkyl; or methylthiomethyl;

wherein all of the asymmetric carbon atoms have an S configuration, except for those in the B, D, and G substituents, which may have an S or R configuration;

and a pharmaceutically acceptable salt thereof.

While both the S and R chiralities for asymmetric carbon atoms in the B substituent are

included in the peptides of the present invention, preferred chiralities are indicated in the description which follows.

In the above definitions, the term "alkyl" is intended to include both branched and straight chain hydrocarbon groups having the indicated number of carbon atoms.

The term "halo" means fluoro, chloro, bromo and iodo.

The aryl substituent represents phenyl, and naphthyl.

The heterocyclic substituent recited above represents any 5- or 6-membered ring containing from one to three heteroatoms selected from the group consisting of nitrogen, oxygen, and sulfur; having various degrees of unsaturation; wherein the nitrogen and sulfur heteroatoms may optionally be oxidized; wherein the nitrogen heteroatom may optionally be quaternized; and including any bicyclic group in which any of the above heterocyclic rings is fused to a benzene ring. Heterocyclic substituents in which nitrogen is the heteroatom are preferred, and of these, those containing a single nitrogen atom are preferred. Fully saturated heterocyclic substituents are also preferred. Thus, piperidine is a preferred heterocyclic substituent. Other preferred heterocyclic substituents are: pyrryl, pyrrolinyl, pyrrolidinyl, pyrazolyl, pyrazolinyl, pyrazolidinyl, imidazolyl, imidazolinyl, imidazolidinyl, pyridyl, piperidinyl, pyrazinyl, piperazinyl, pyrimidinyl, pyridazinyl, oxazolyl, oxazolidinyl, isoxazolyl, isoxazolidinyl, morpholinyl, thiazolyl, thiazolidinyl, isothiazolyl, isothiazolidinyl, indolyl, quinolinyl,

isoquinolinyl, benzimidazolyl, benzothiazolyl, benzoxazolyl, furyl, thienyl and benzothienyl.

Where the heterocyclic substituent itself is substituted, it is preferred that the substituent be aryl$C_{1-4}$alkyl.

The novel renin inhibitory peptides of the present invention may also be described in terms of common amino acid components and closely related analogs thereof, in accordance with the following formula:

A-B-B-D-E-Y-G-J-

(II.)

The A, B, D, G, J and L components correspond to the same portions of Formula I.

The common amino acid components of Formula II are as follows:

| | |
|---|---|
| A has | the same meaning as above in Formula I; |
| B is | Ala, Leu, Ser, Thr, Phe, Tyr, Trp, His, Lys, Orn, Arg, or Met; |
| D is | Pro; |
| E is | Ala, Leu, Phe, HomoPhe, BisHomoPhe, Tyr, HomoTyr, Trp, or HomoTrp; |
| Y is | the same as B; |
| G has | the same meaning as above in Formula I; and |
| J has | the same meaning as above in Formula I; |

It will be understood that closely related analogs of the above common amino acids, for example, aliphatic amino acids in addition to Ala, Val, Leu, and Ile, such as α-aminobutyric acid (Abu), and substituted phenyl derivatives of Phe, are included in the broad description of the novel inhibitory peptides of the present invention represented by Formula I and its definitions. Thus, the peptides of Formula II and its definitions represent preferred peptides of the present invention.

Preferred inhibitory peptides of the present invention are the following:

BOC[1]-His-Pro-Phe-His-Sta-OEt

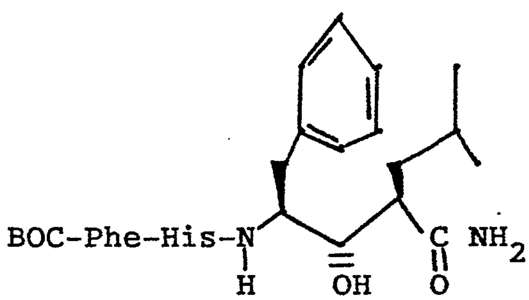

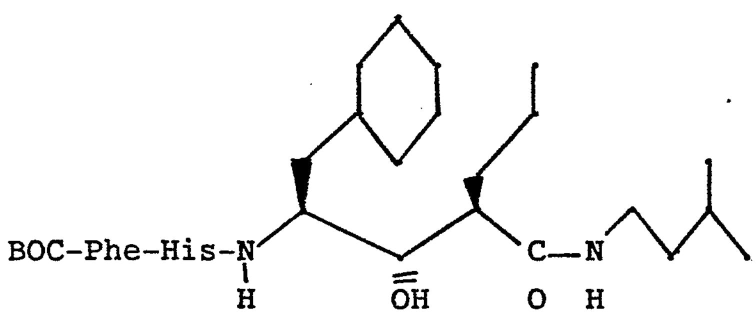

BOC-Phe-His-ACHPA[2]-$NH_2$

BOC-HomoPhe-His-Sta-$NH_2$

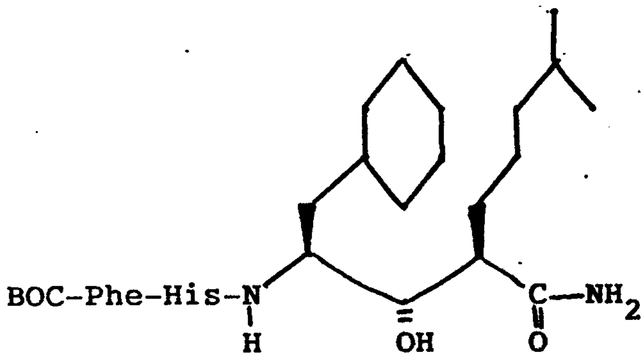

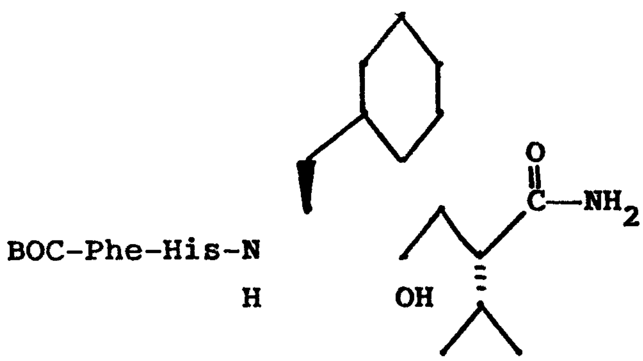

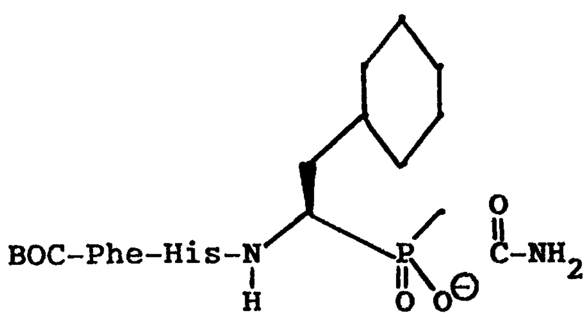

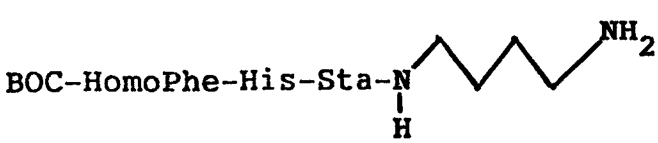

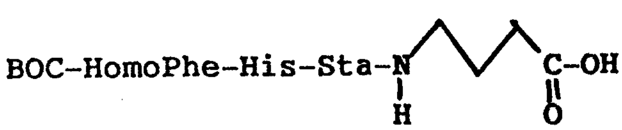

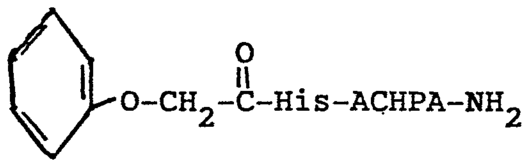

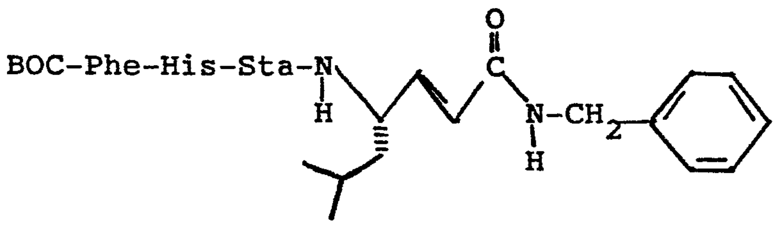

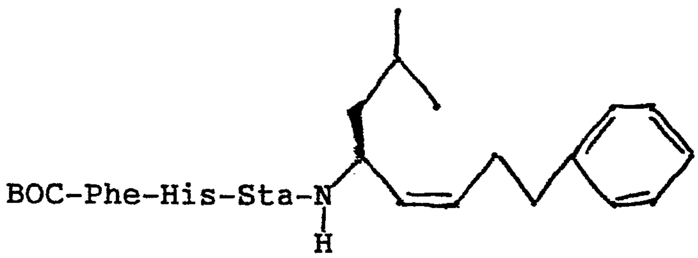

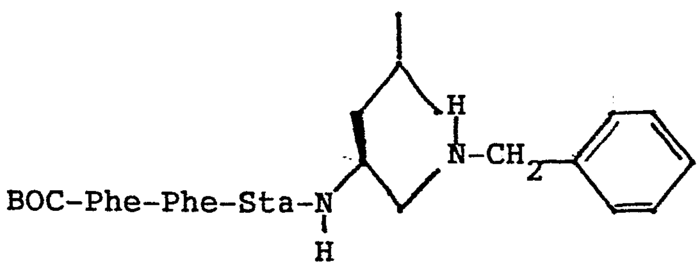

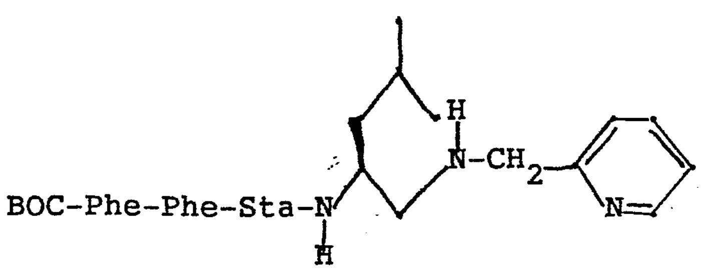

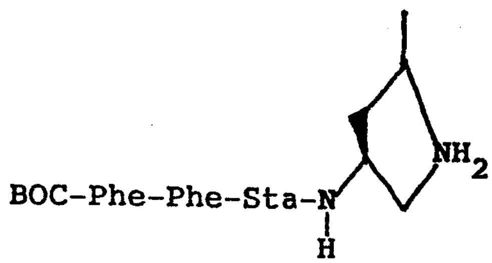

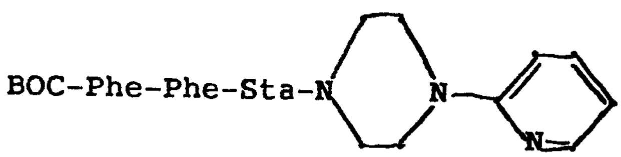

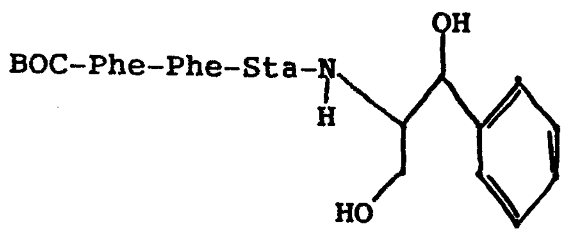

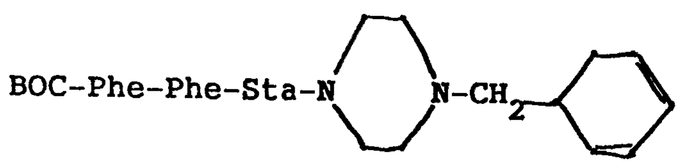

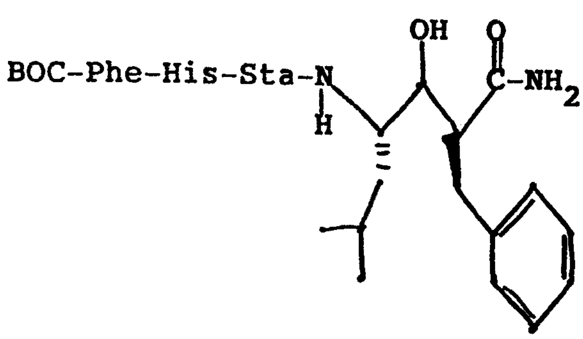

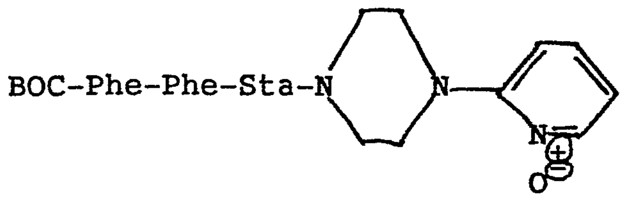

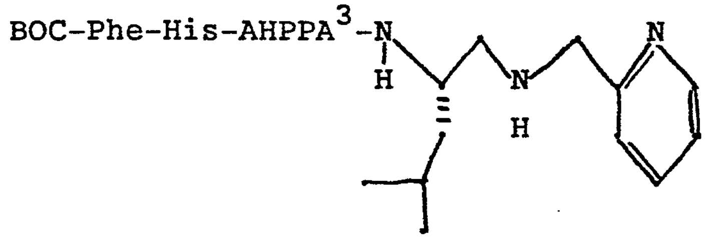

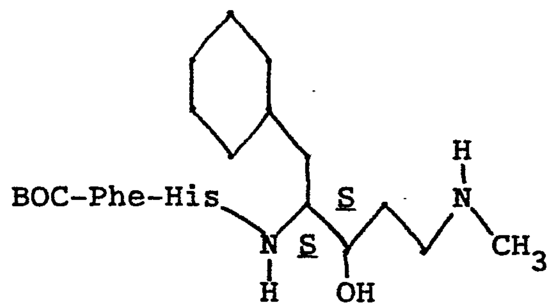

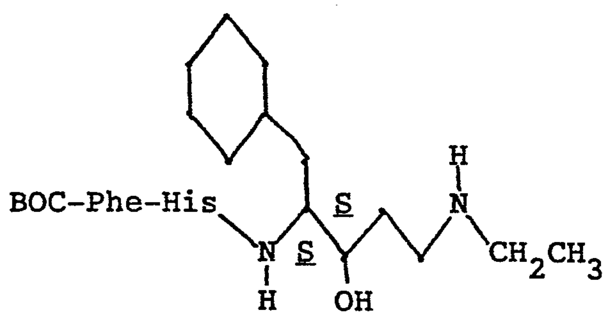

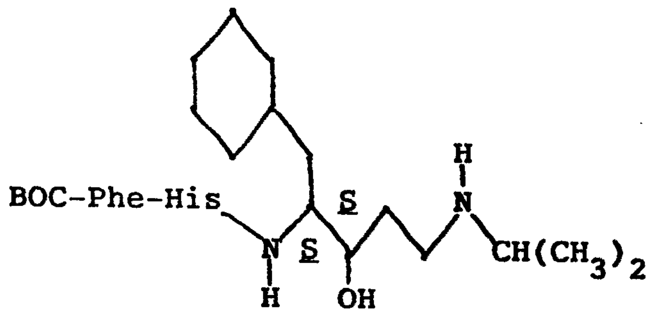

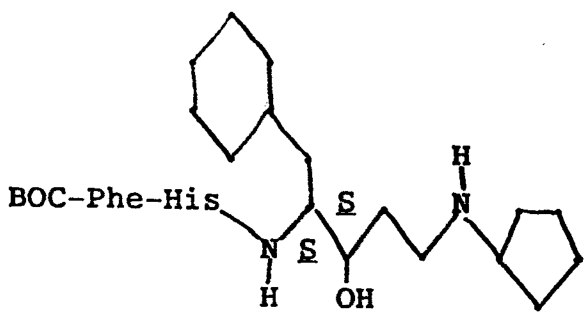

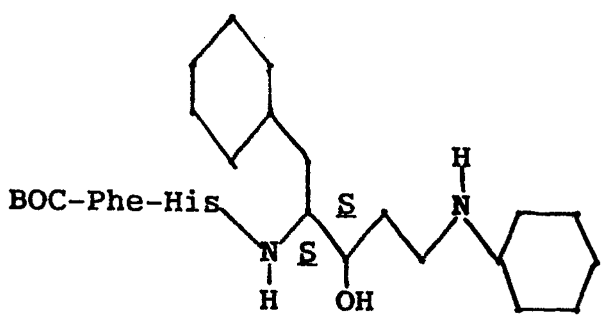

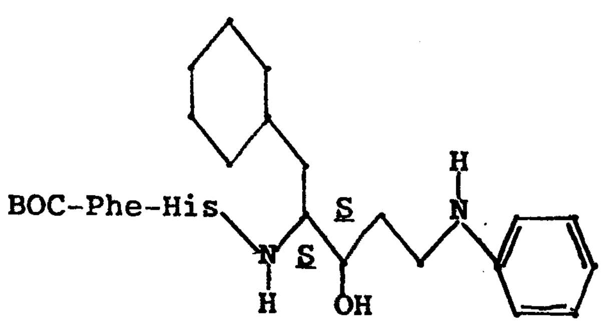

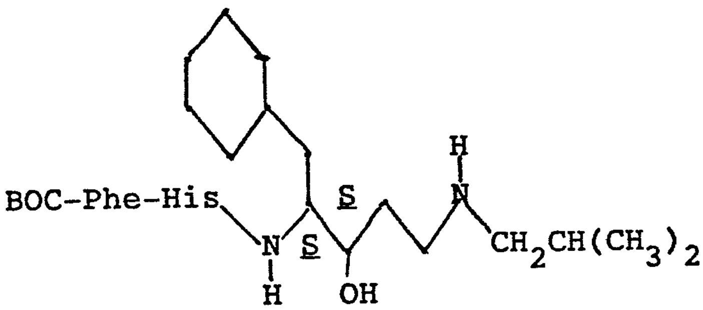

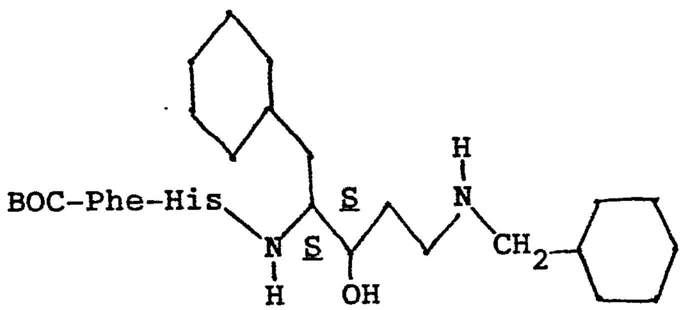

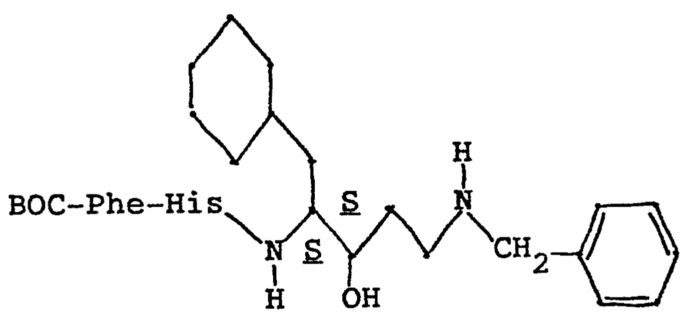

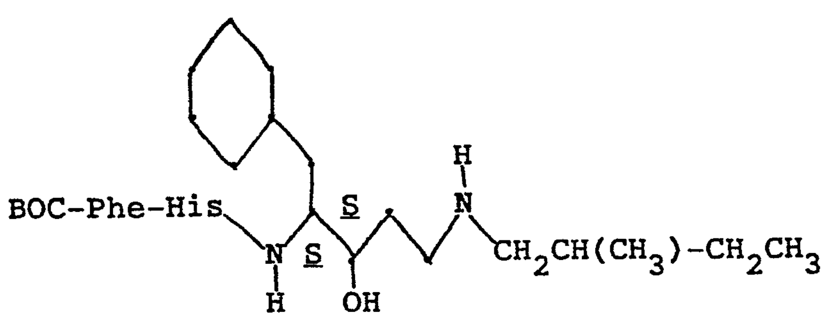

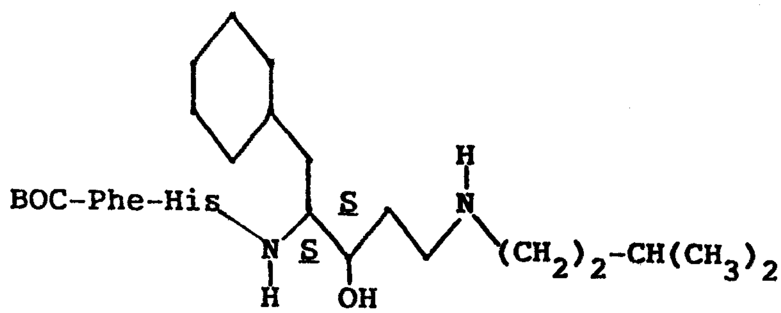

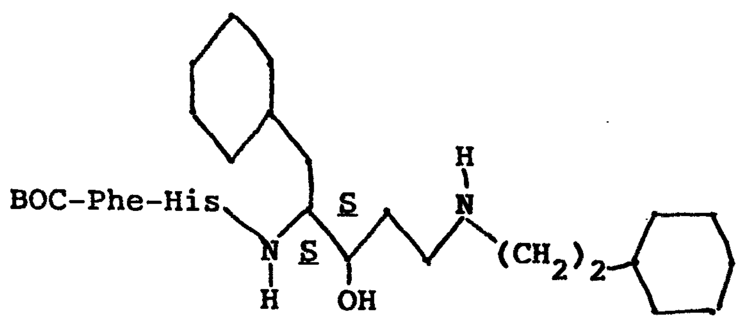

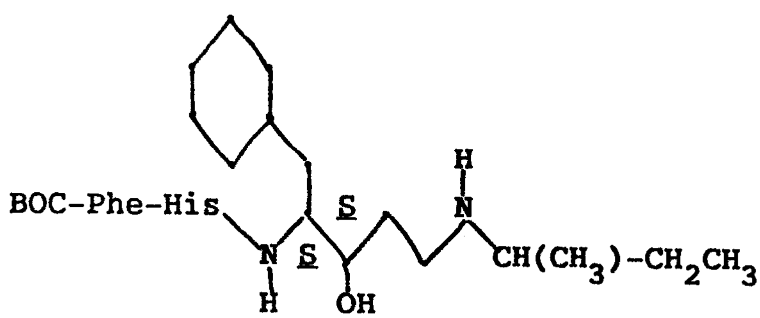

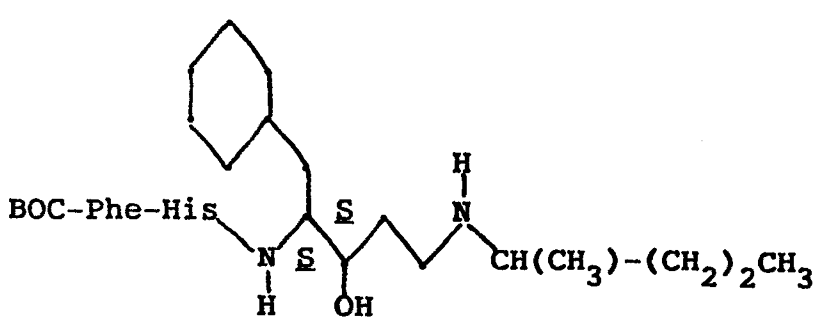

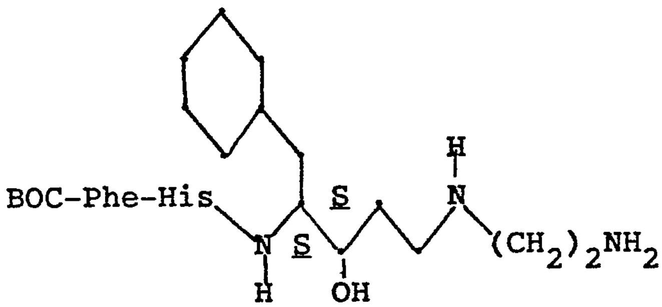

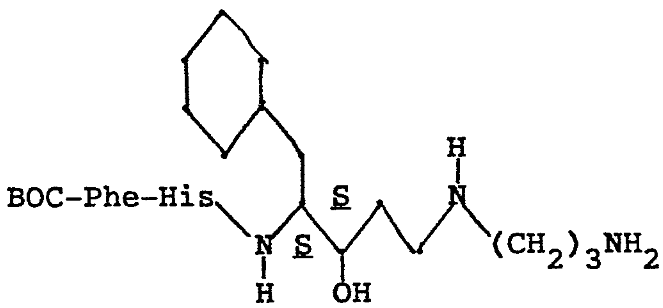

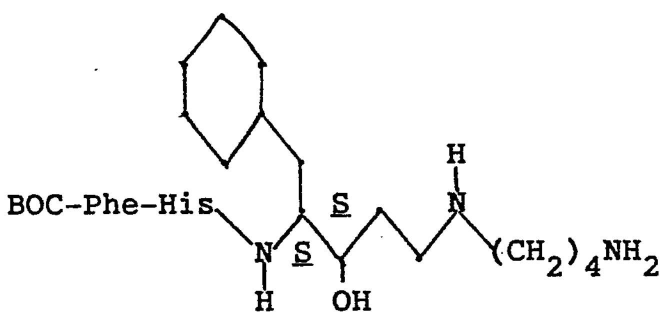

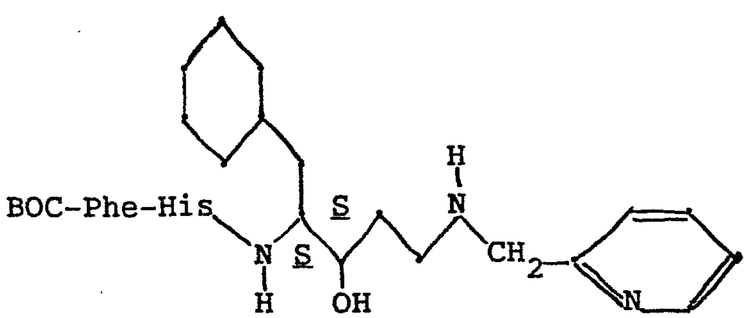

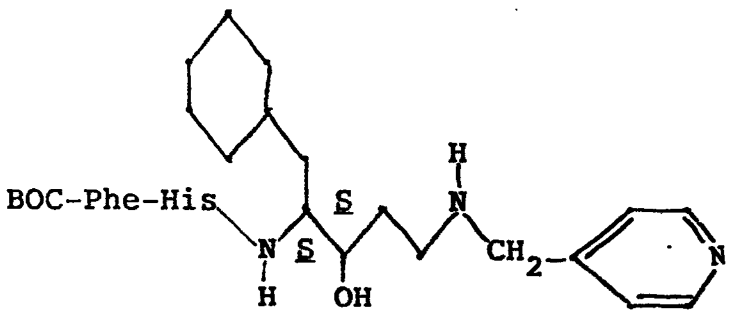

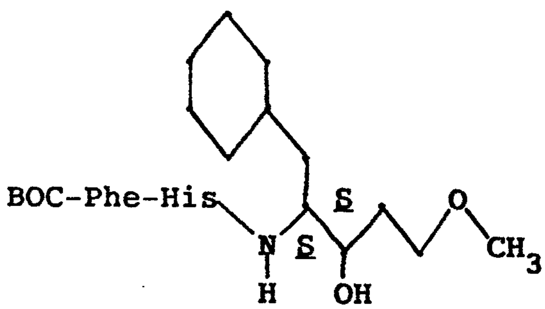

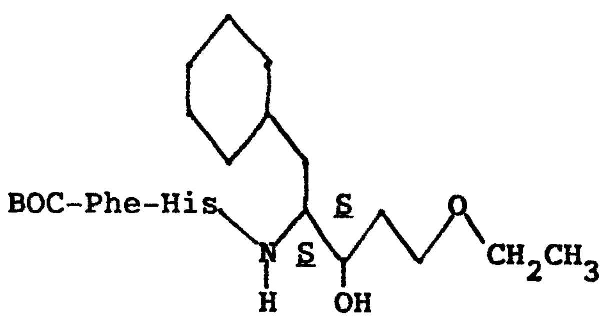

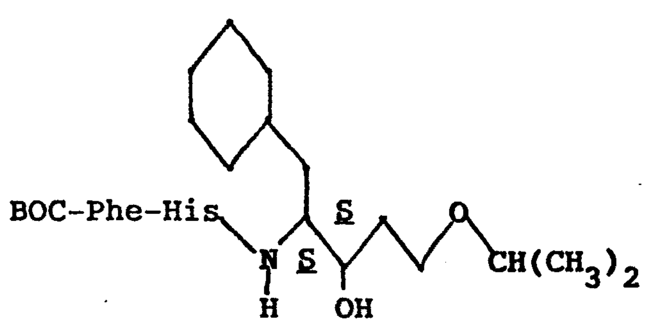

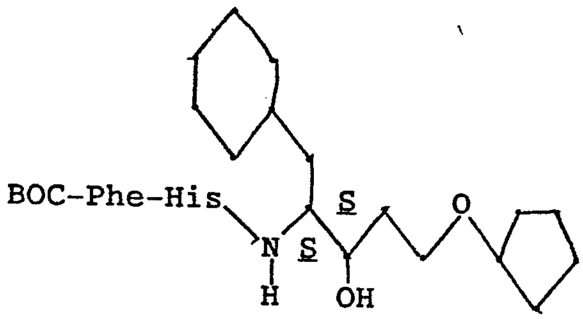

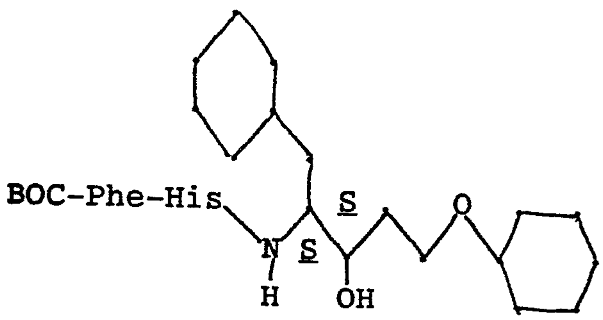

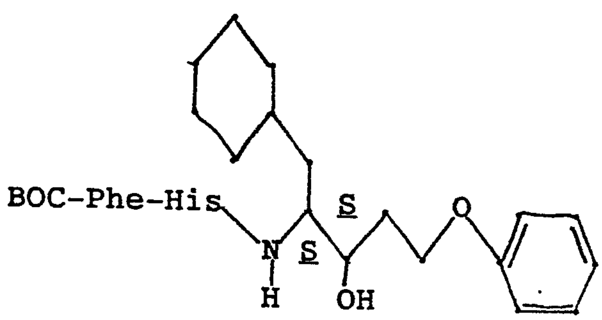

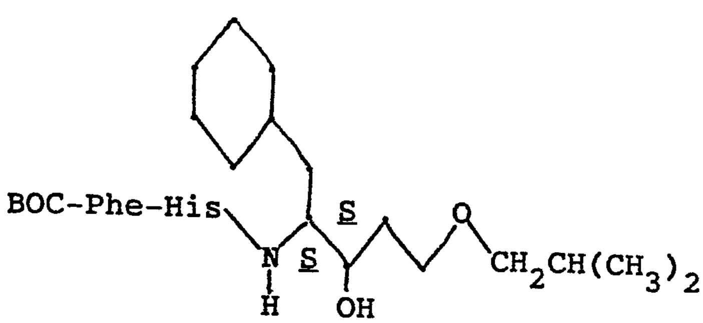

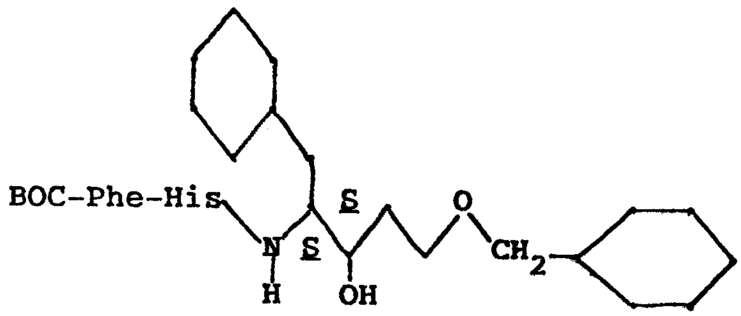

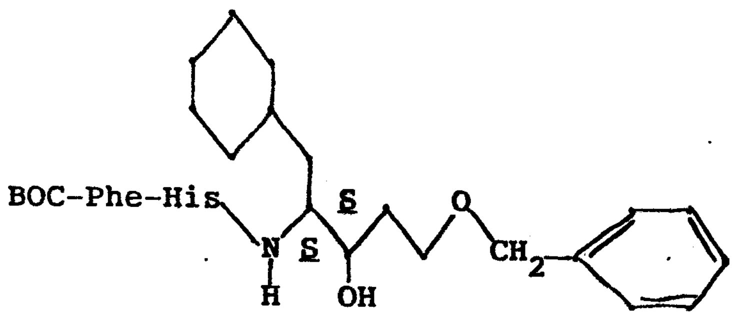

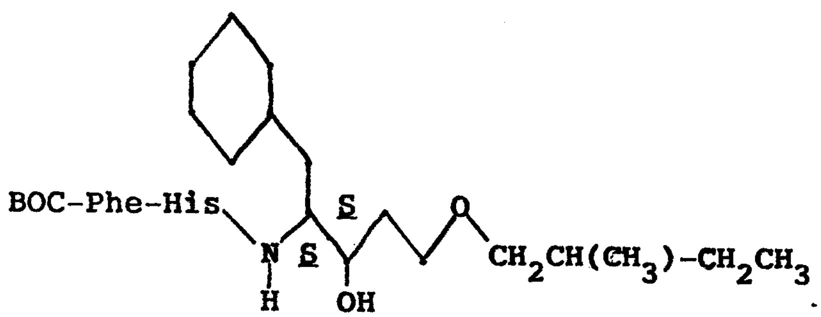

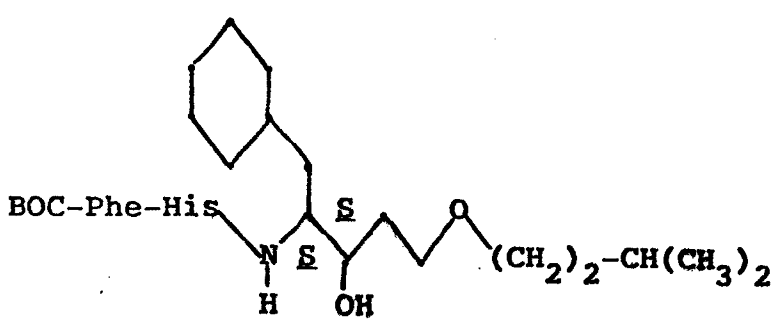

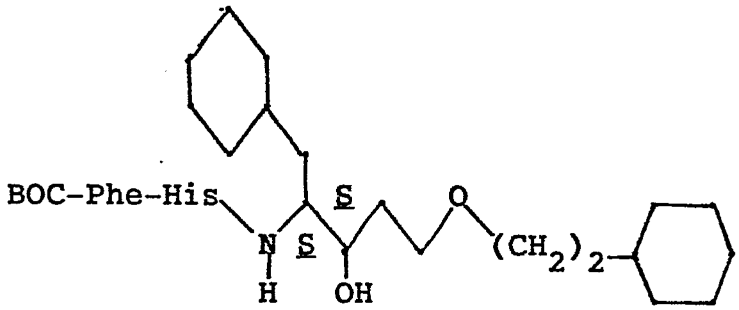

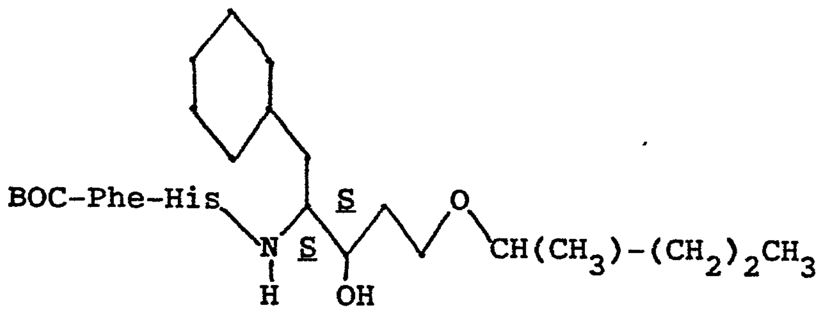

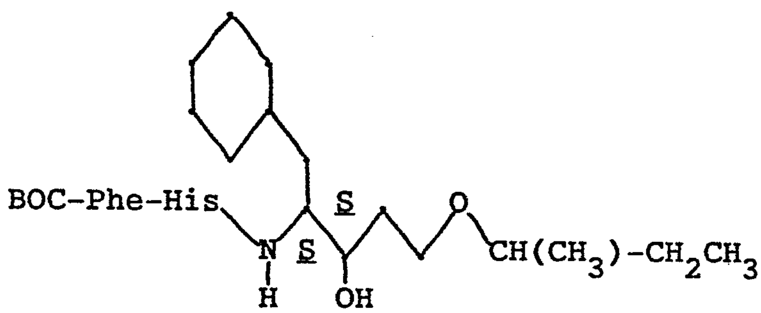

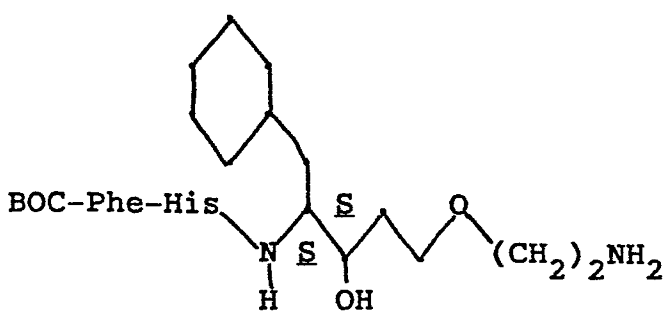

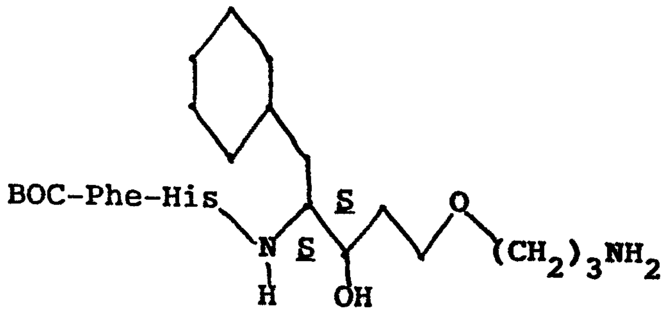

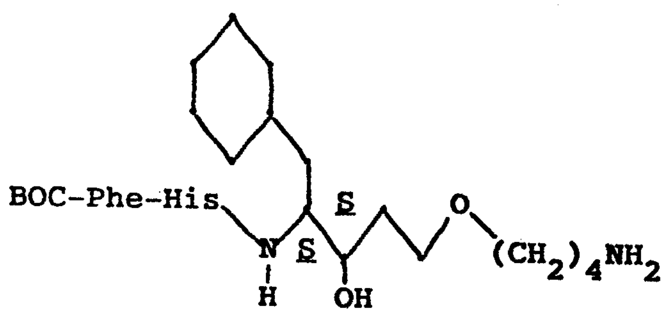

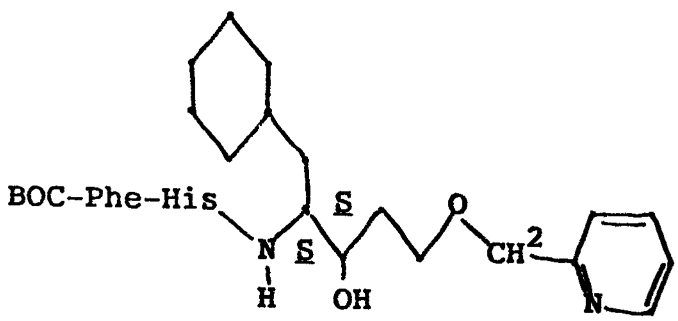

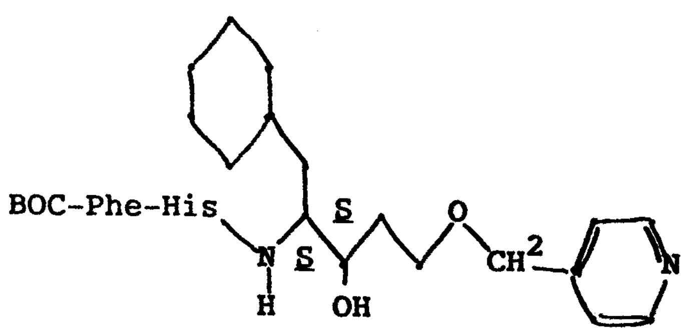

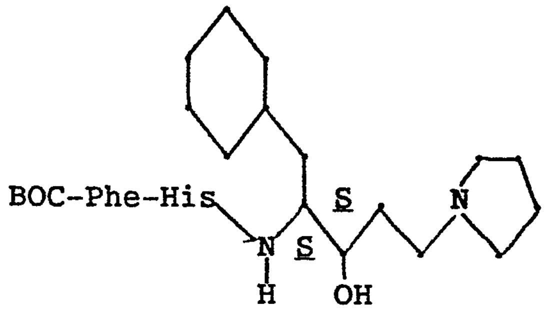

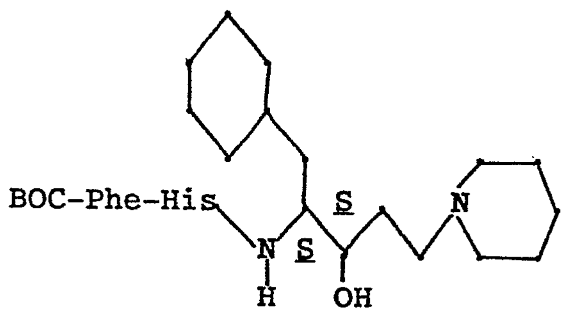

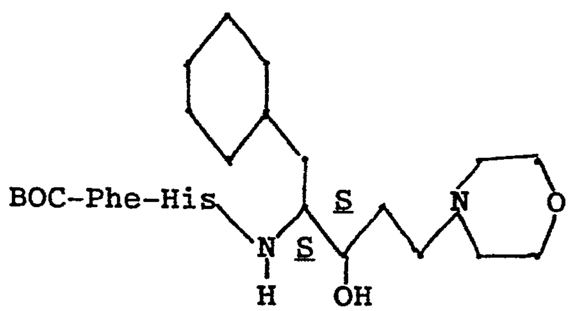

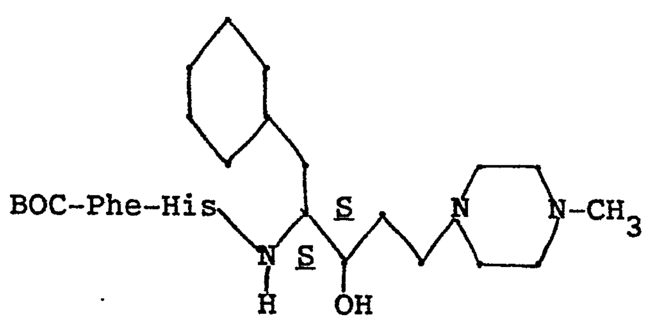

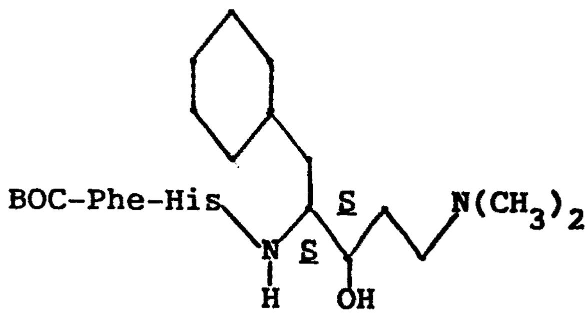

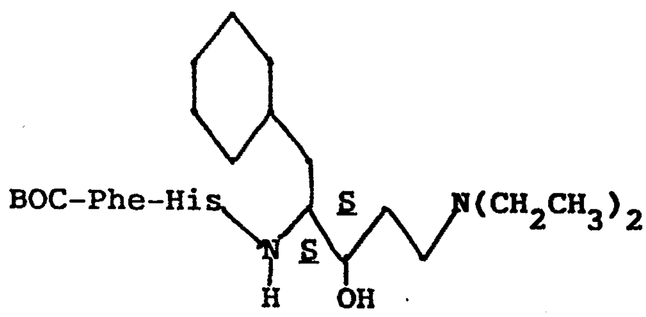

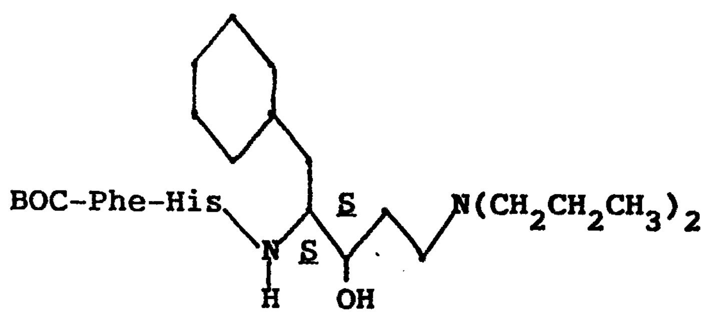

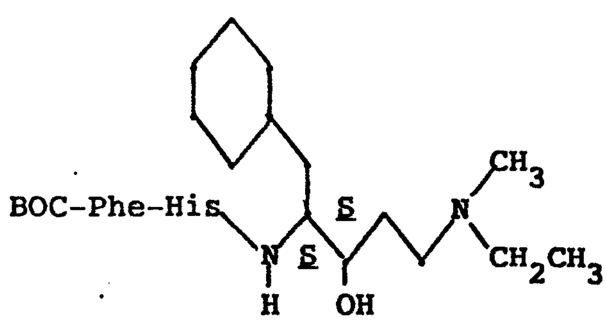

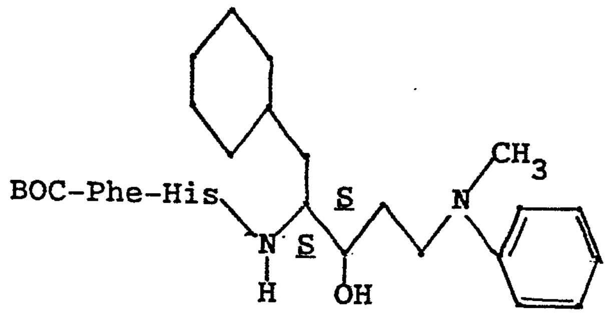

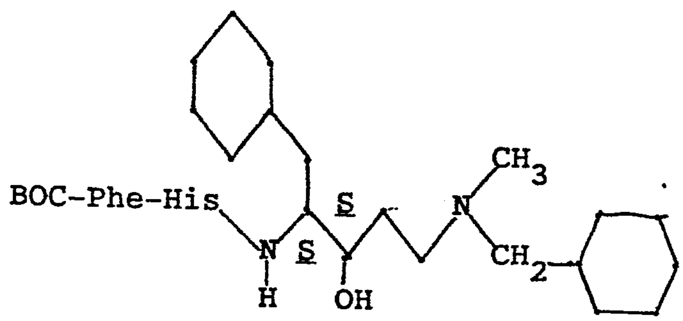

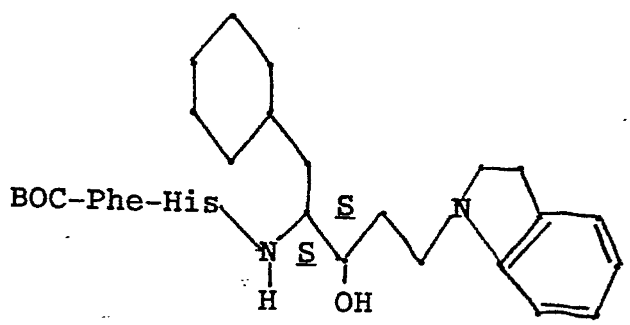

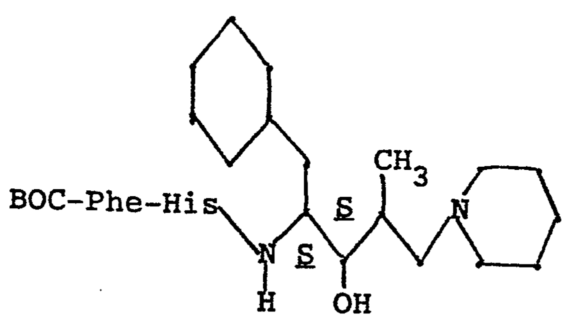

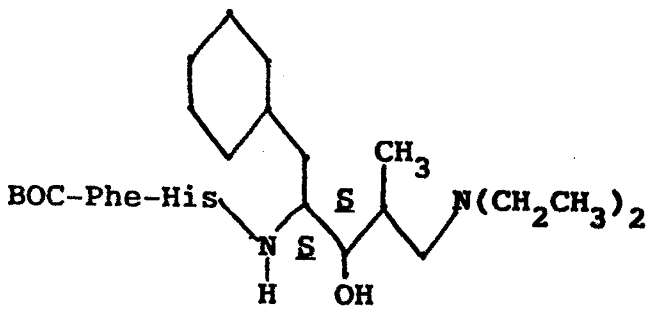

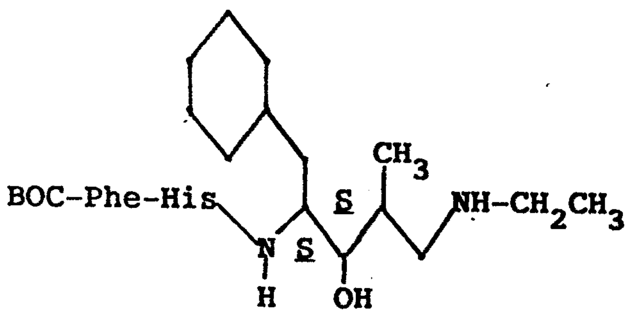

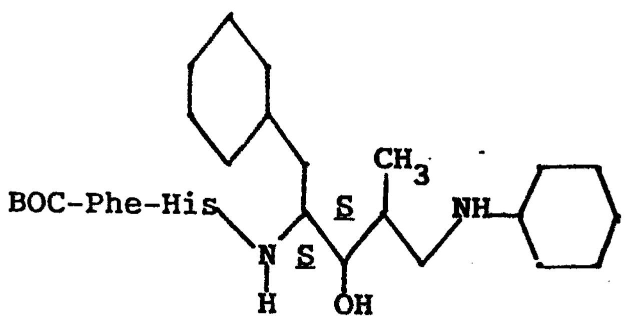

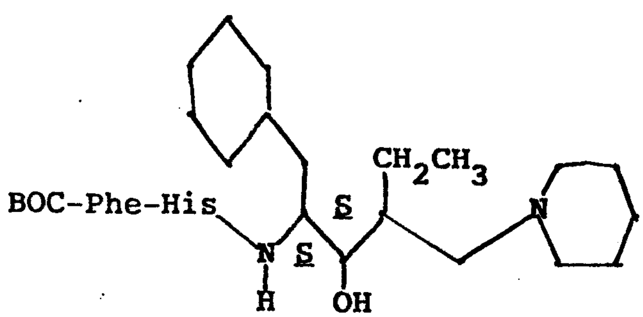

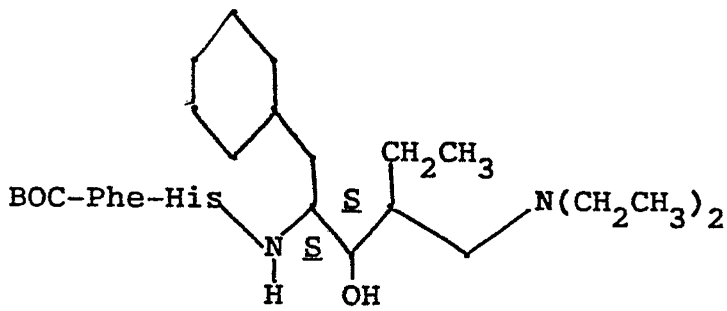

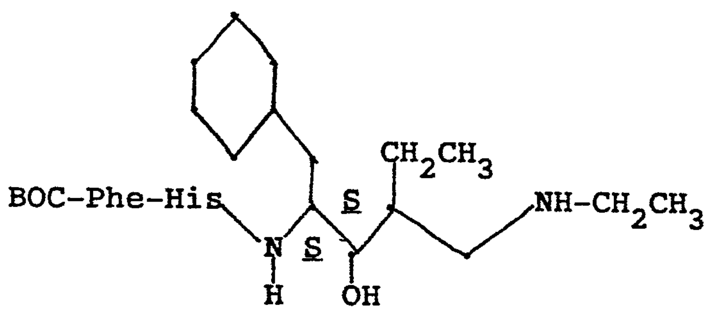

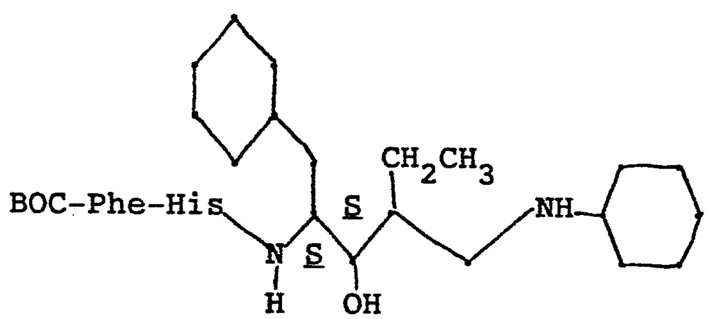

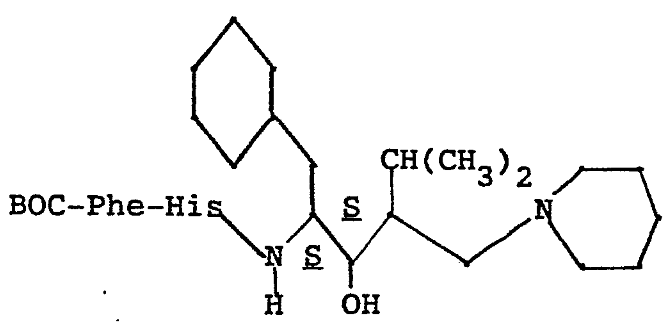

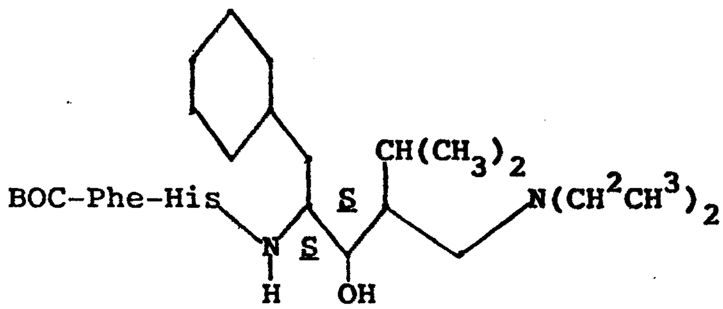

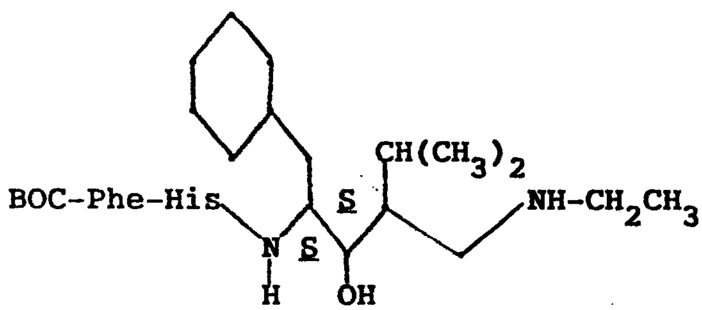

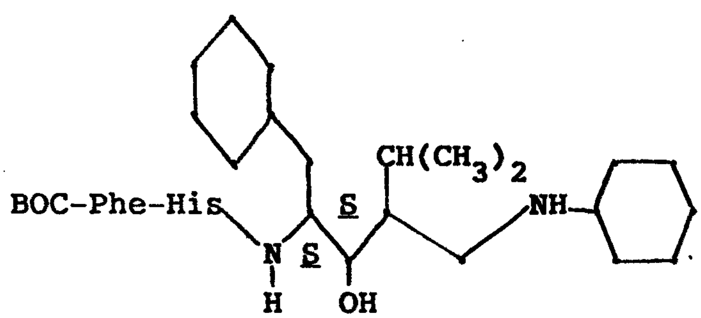

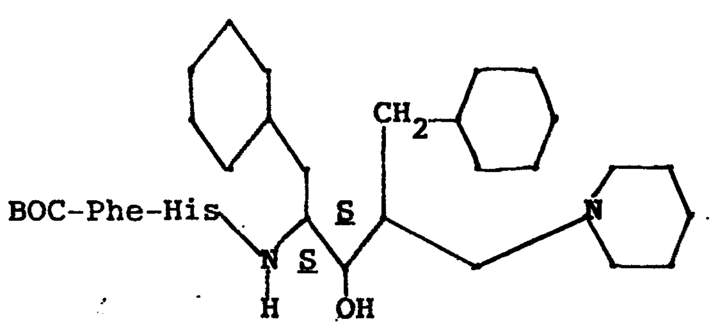

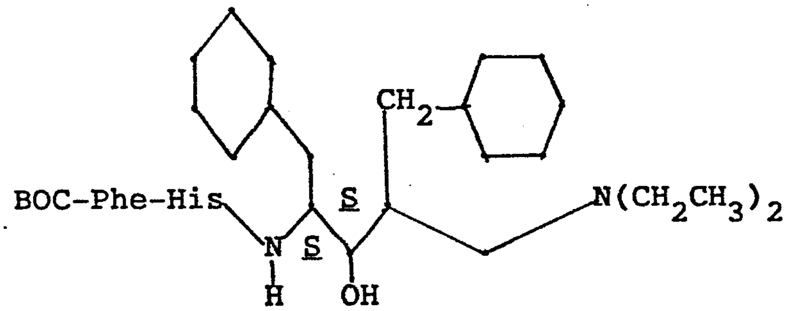

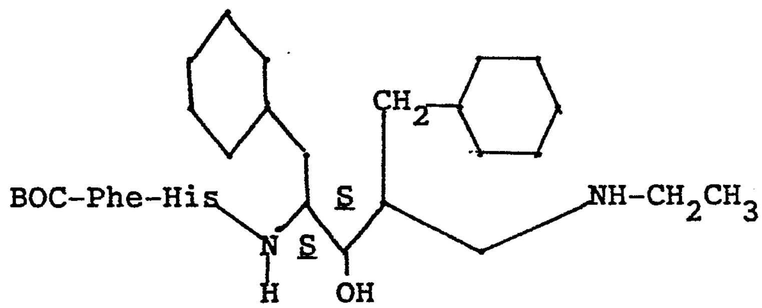

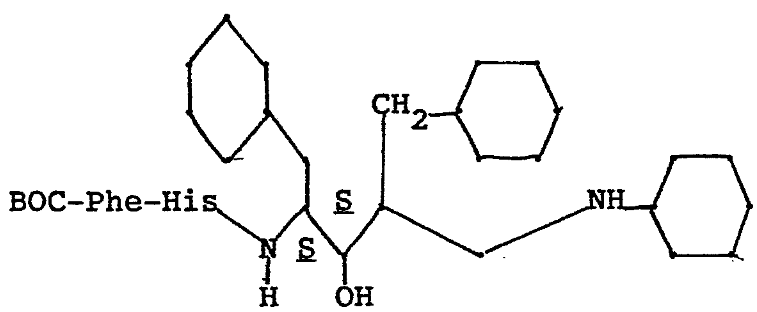

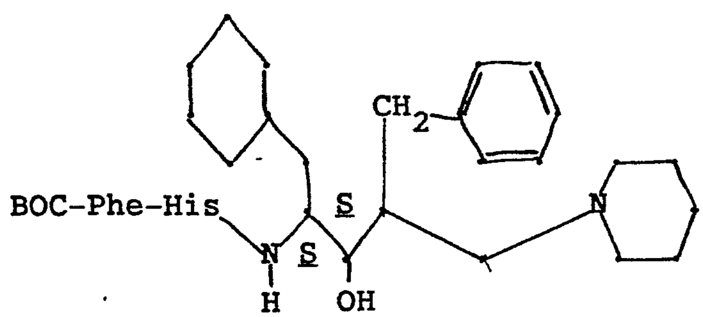

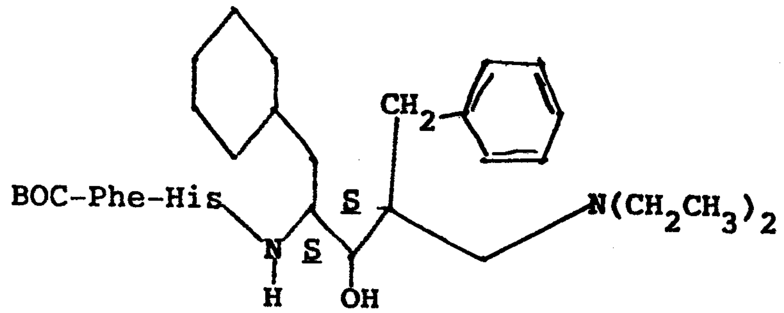

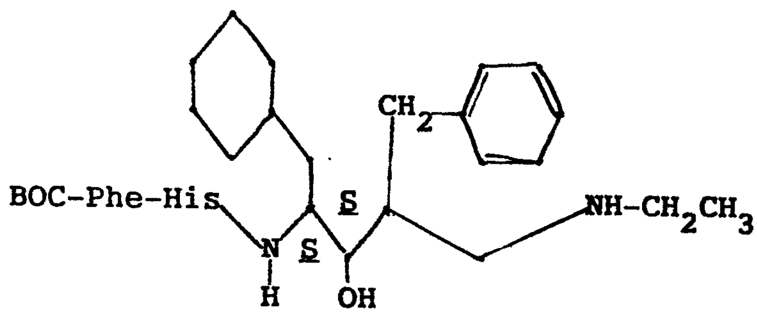

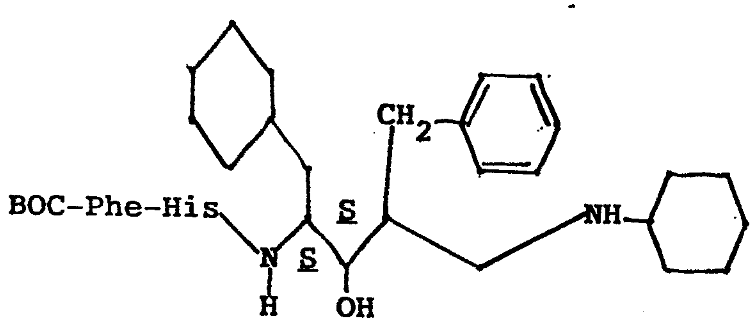

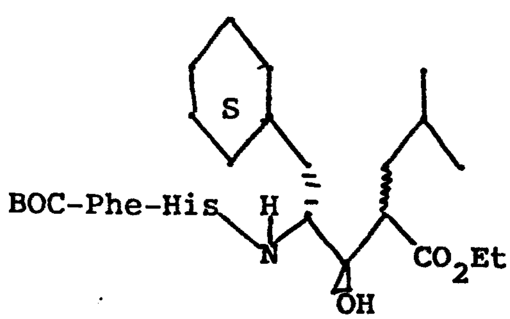

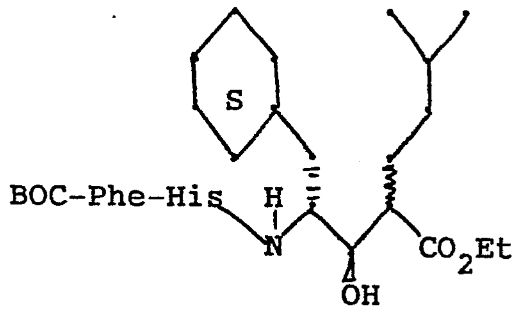

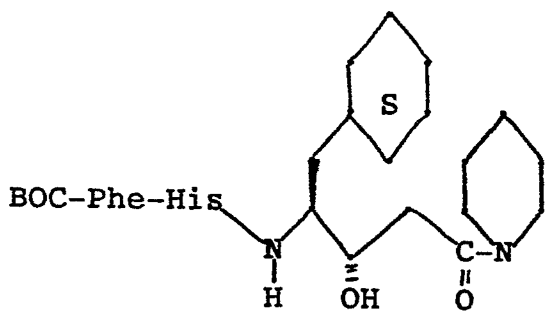

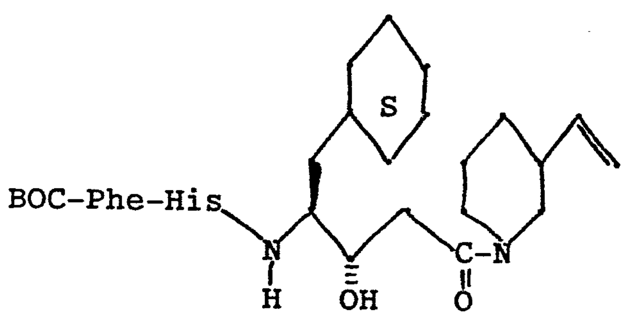

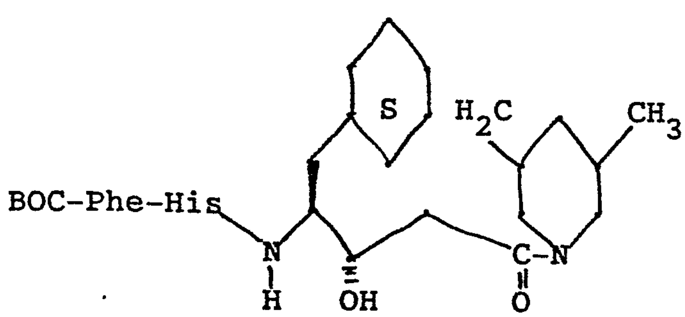

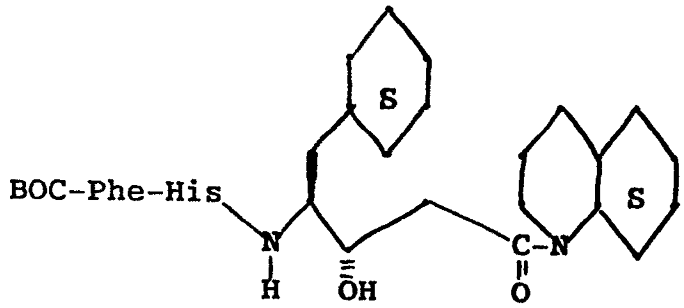

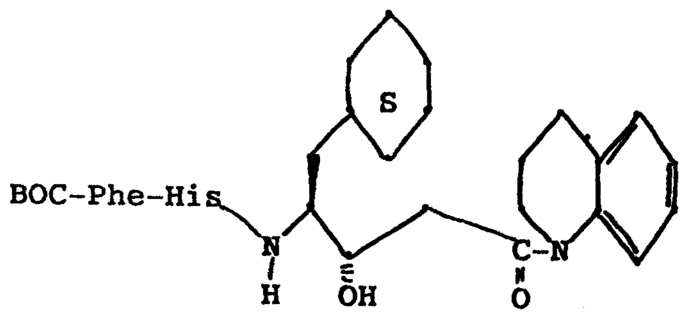

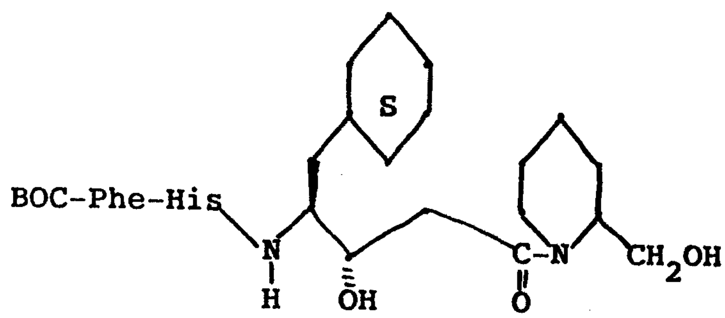

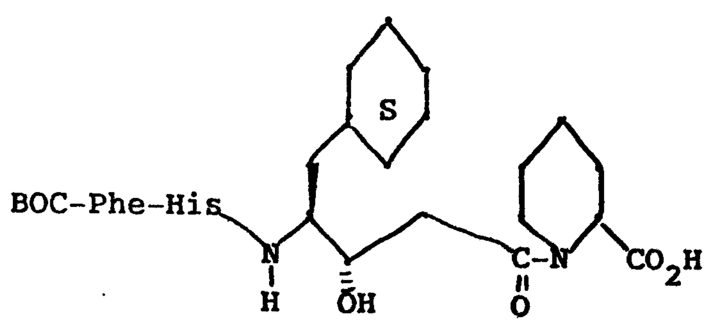

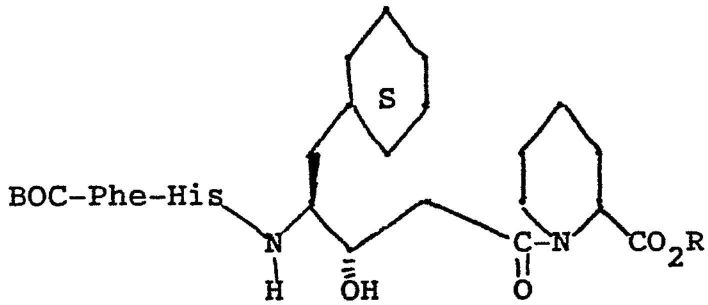

where R=alkyl, aryl, $CH_2OH$

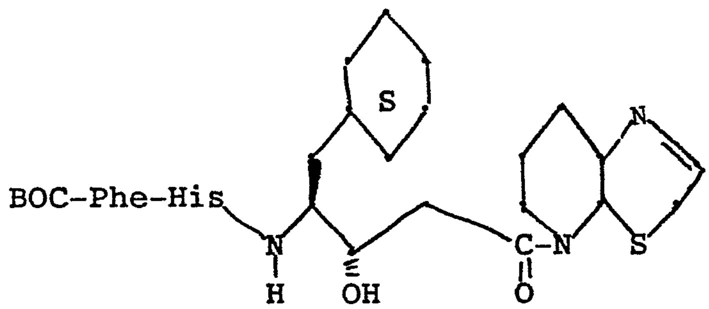

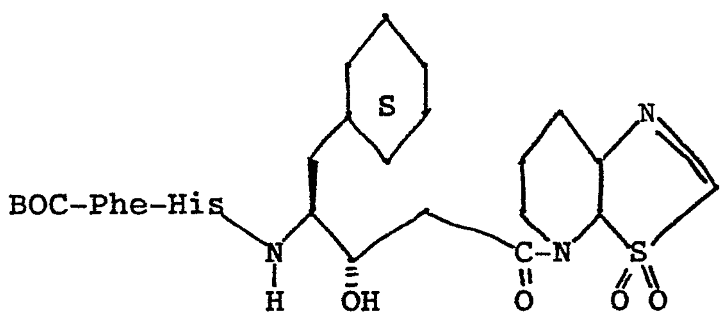

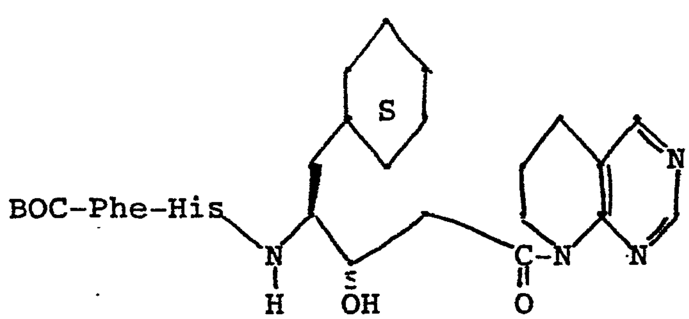

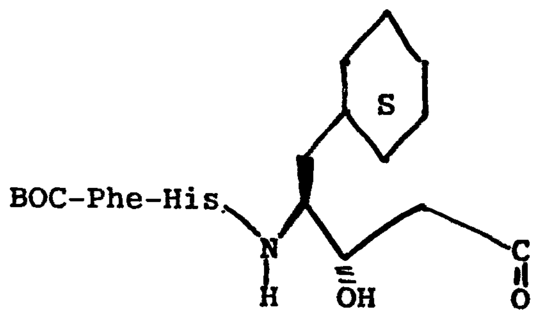

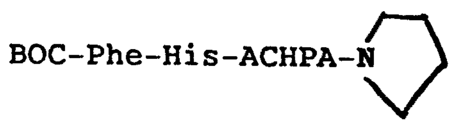

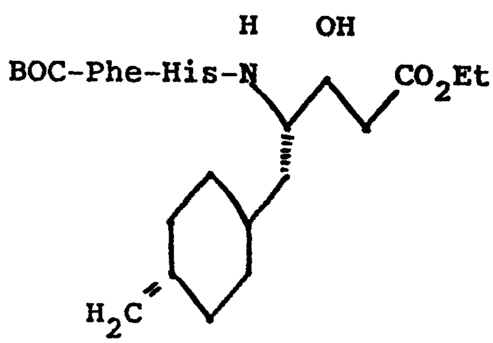

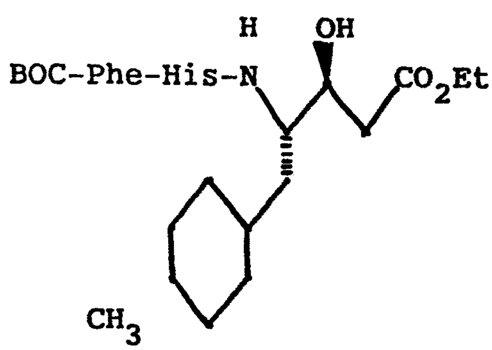

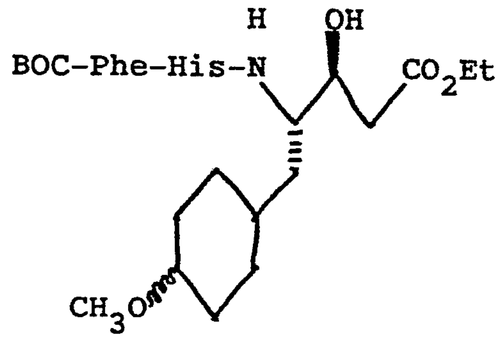

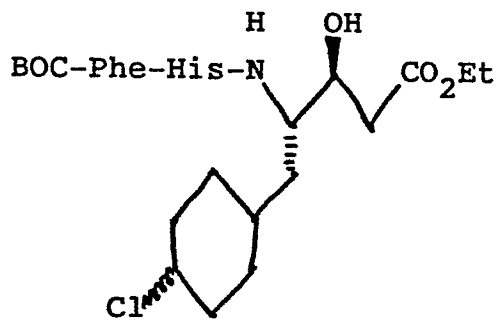

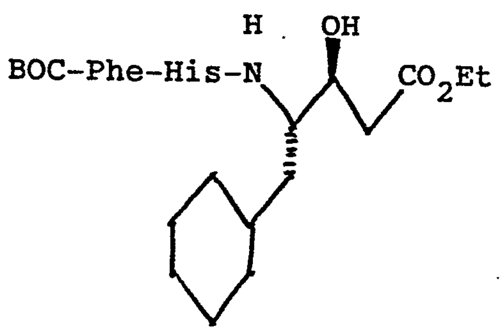

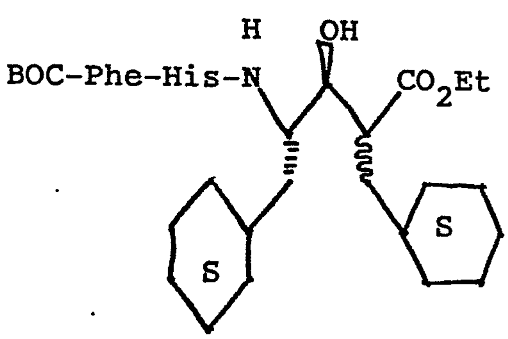

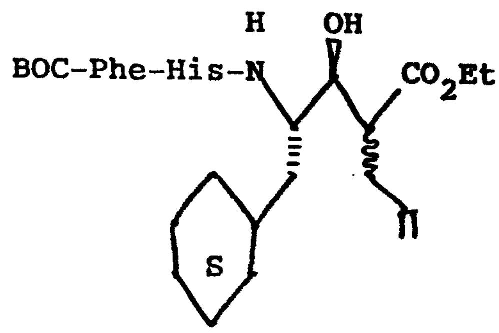

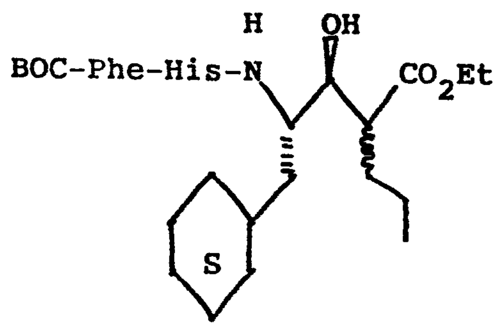

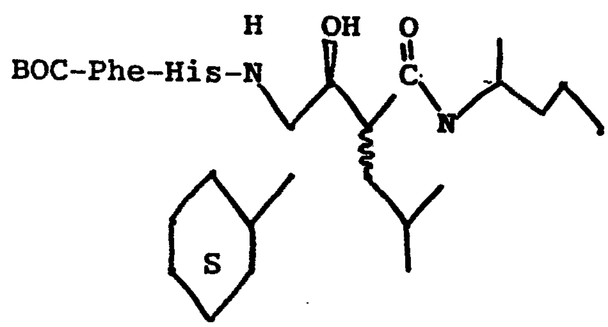

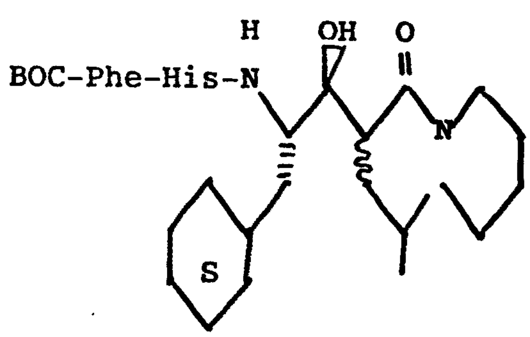

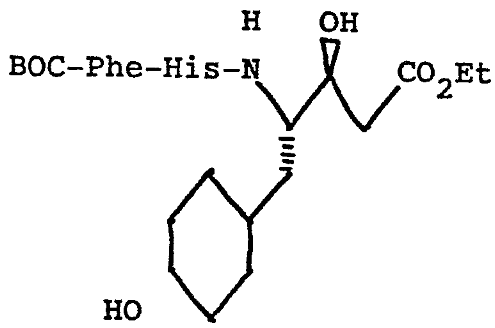

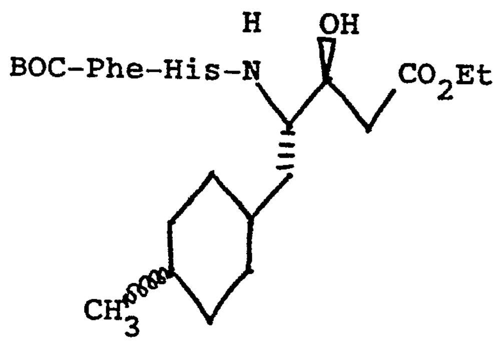

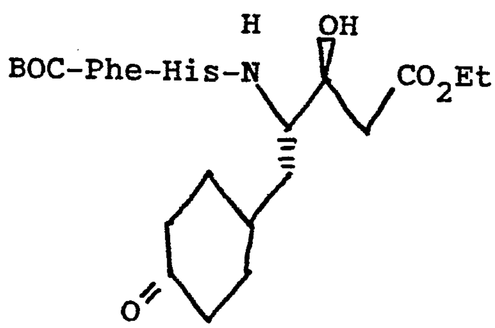

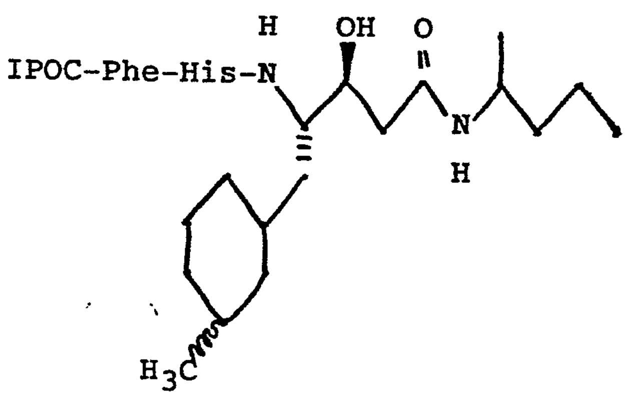

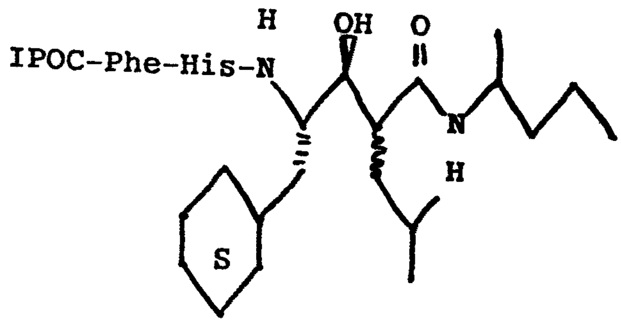

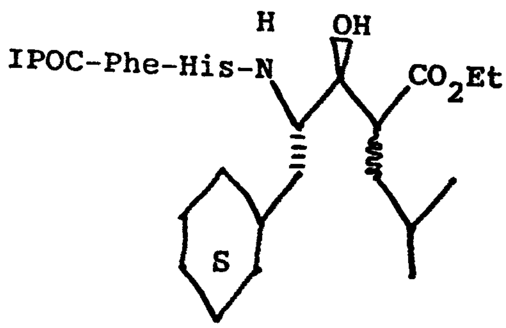

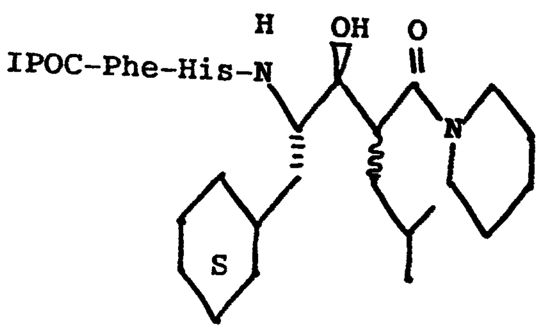

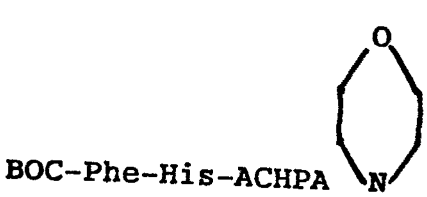

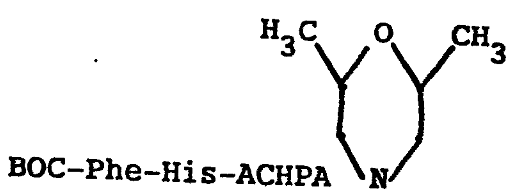

BOC-Phe-His-ACHPA 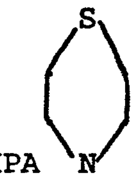

BOC-Phe-His-ACHPA 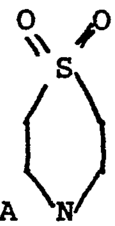

BOC-Phe-His-ACHPA 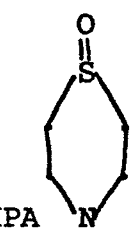

BOC-Phe-His-ACHPA 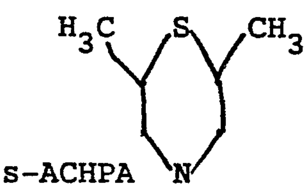

BOC-Phe-His-ACHPA 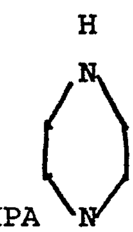

BOC-Phe-His-ACHPA 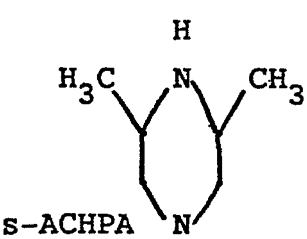

BOC-Phe-His-ACHPA 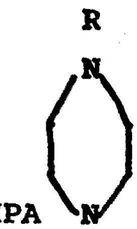 where R=alkyl, aryl, $-CO_2H$, $-CH_2OH$

BOC-Phe-His-ACHPA- 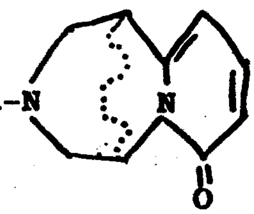

BOC-Phe-His-ACHPA- 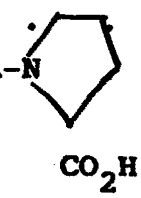

BOC-Phe-His-ACHPA- 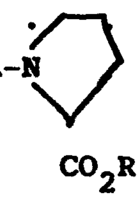 where R=alkyl, aryl, $-CH_2OH$

BOC-Phe-His-ACHPA- 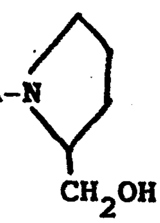

BOC-Phe-His-ACHPA- 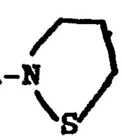

BOC-Phe-His-ACHPA- 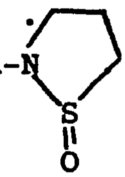

BOC-Phe-His-ACHPA- 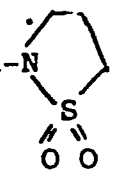

BOC-Phe-His-ACHPA- 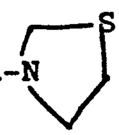

BOC-Phe-His-ACHPA- 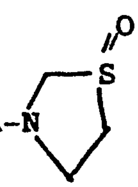

BOC-Phe-His-ACHPA- 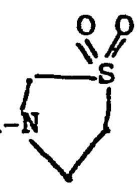

BOC-Phe-His-ACHPA- 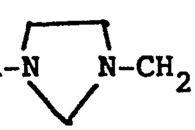

BOC-Phe-His-ACHPA- 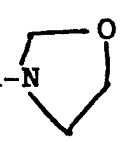

BOC-Phe-His-ACHPA- 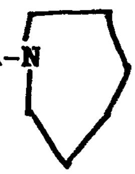

BOC-Phe-His-ACHPA- 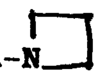

BOC-Phe-His-ACHPA- 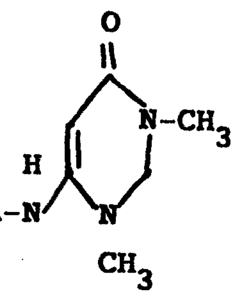

BOC-Phe-His-ACHPA- 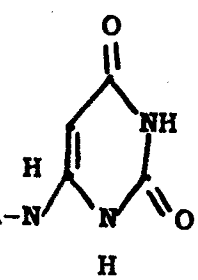

BOC-Phe-His-ACHPA- 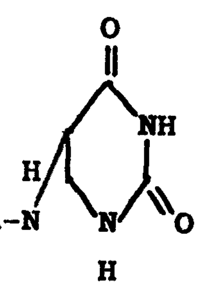

BOC-Phe-His-ACHPA- 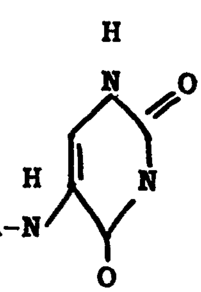

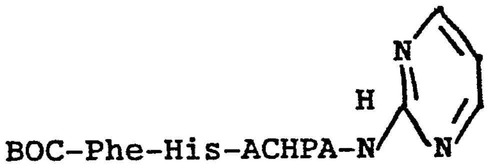

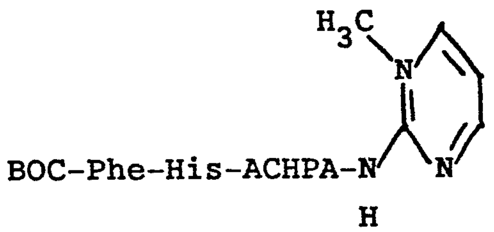

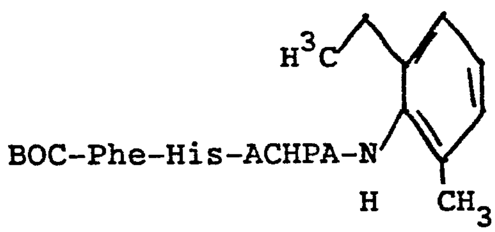

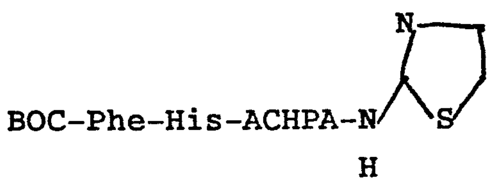

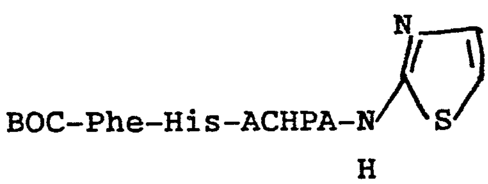

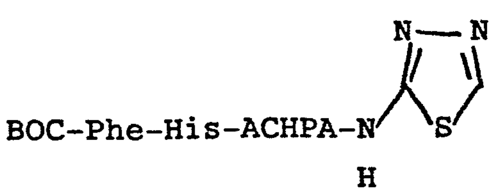

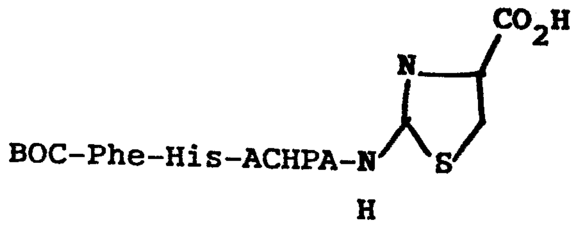

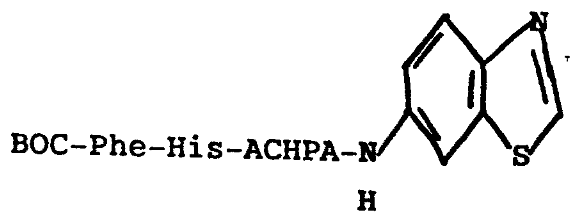

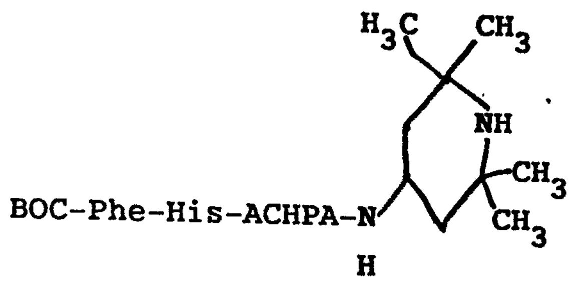

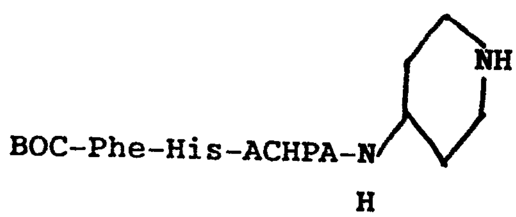

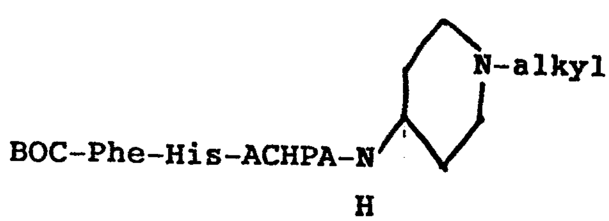

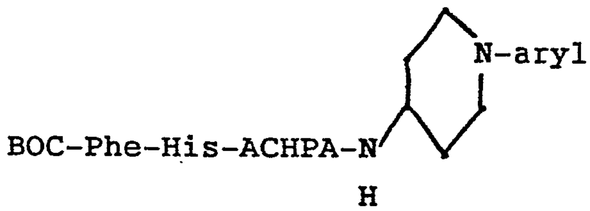

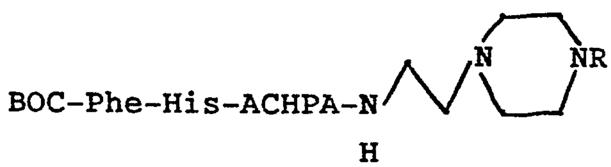

where R=H, alkyl

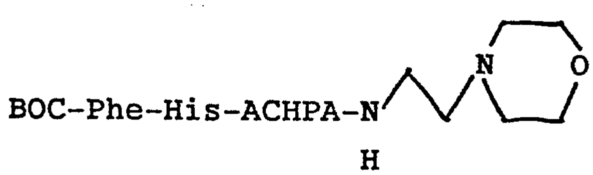

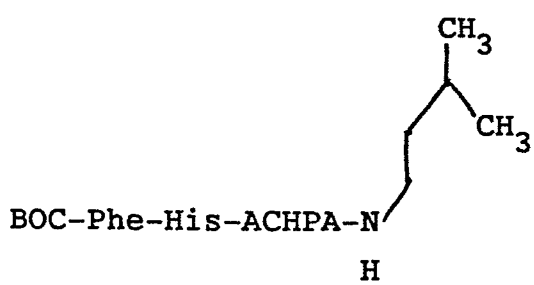

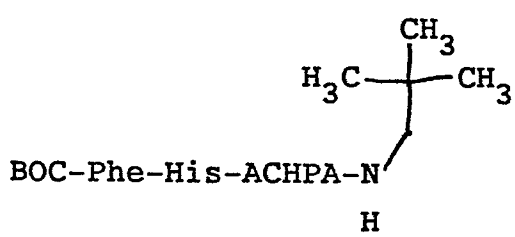

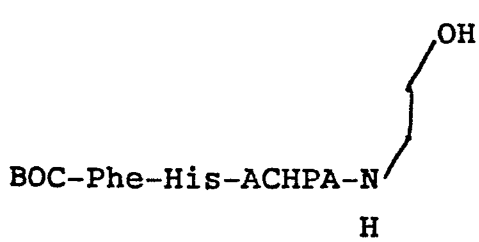

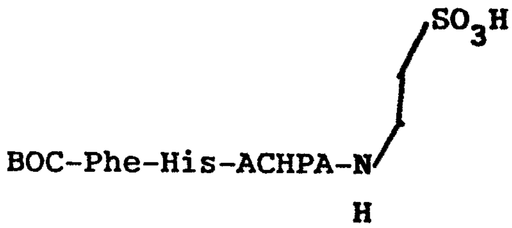

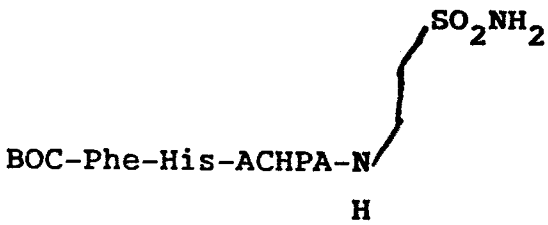

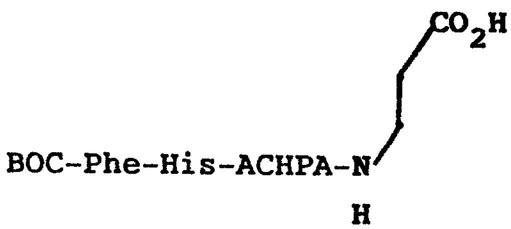

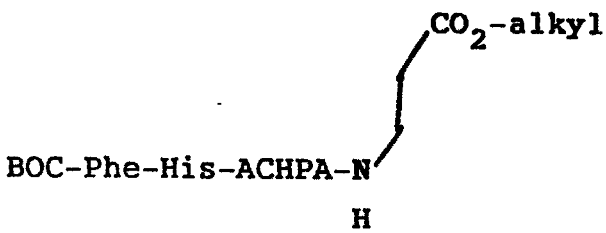

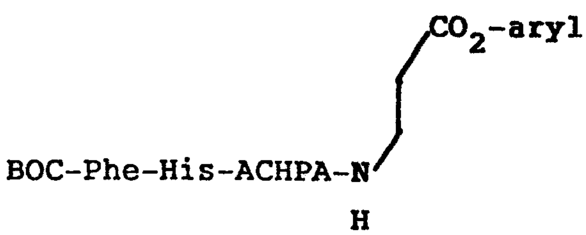

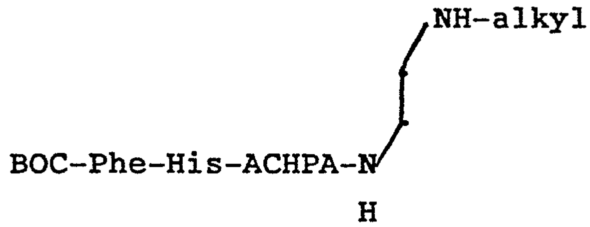

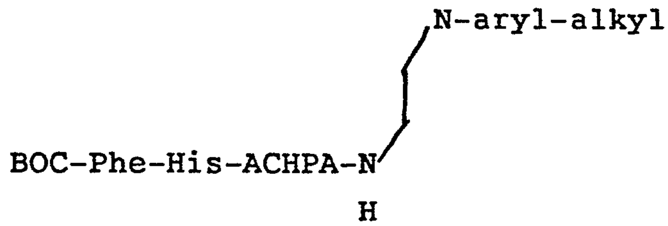

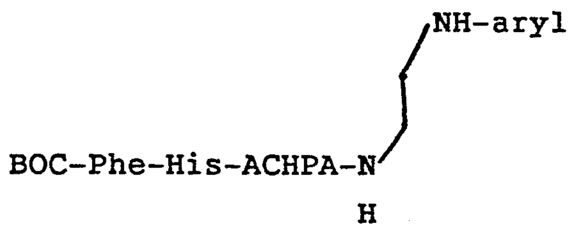

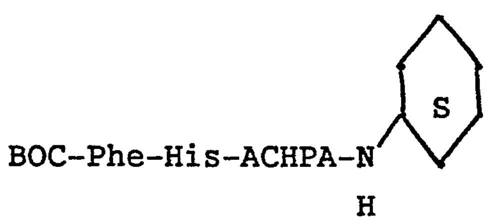

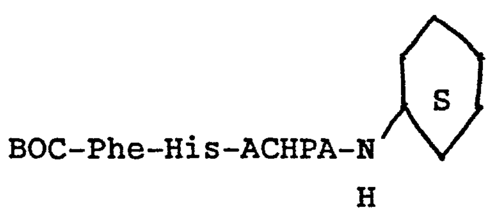

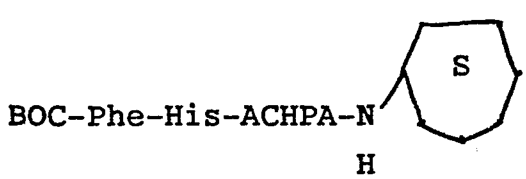

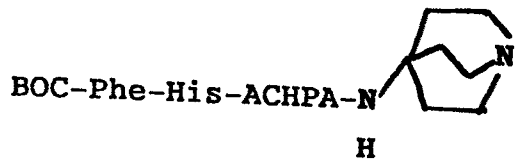

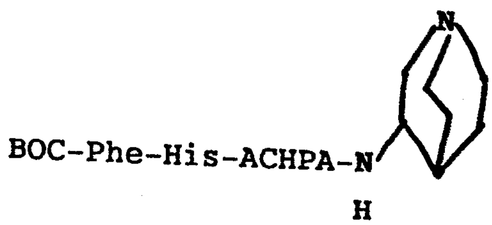

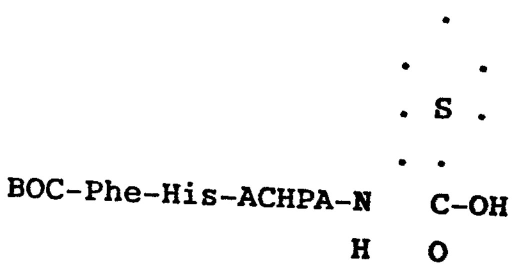

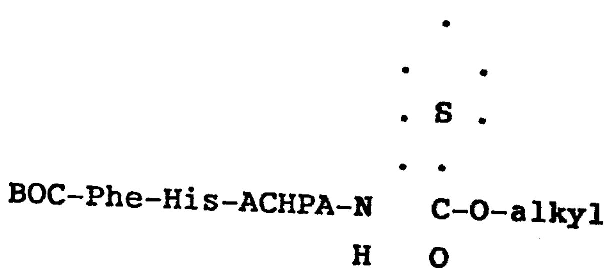

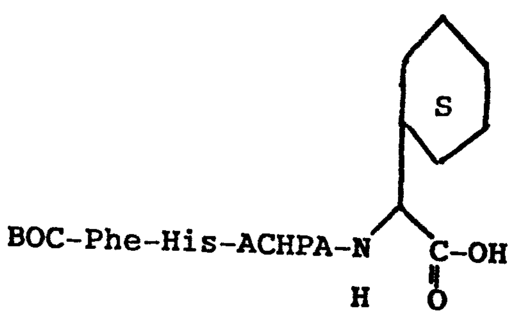

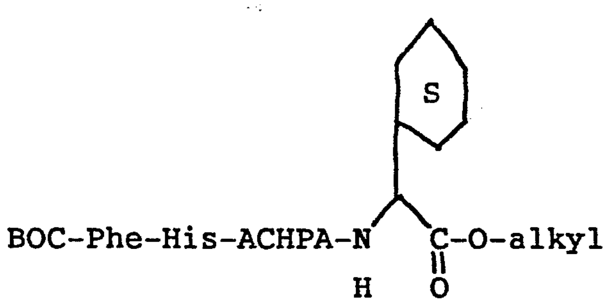

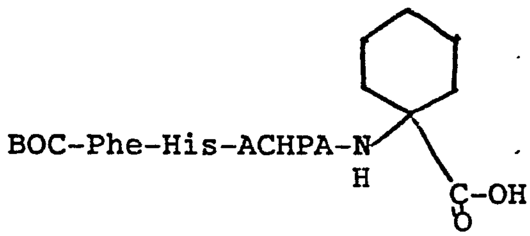

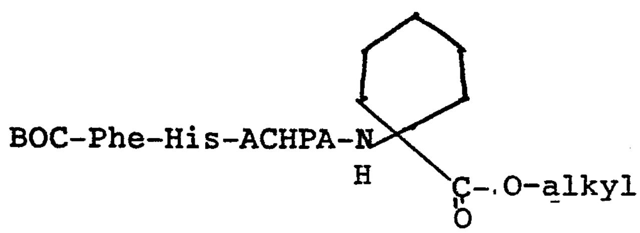

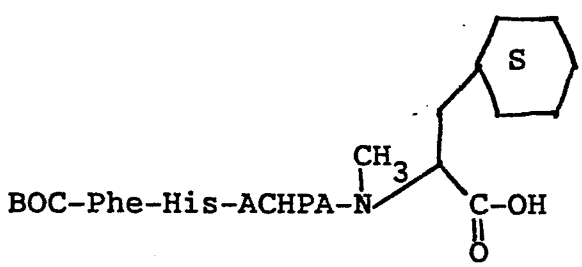

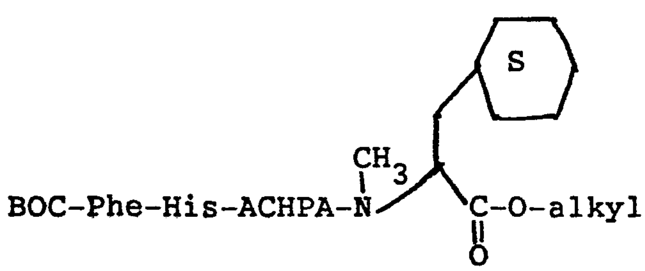

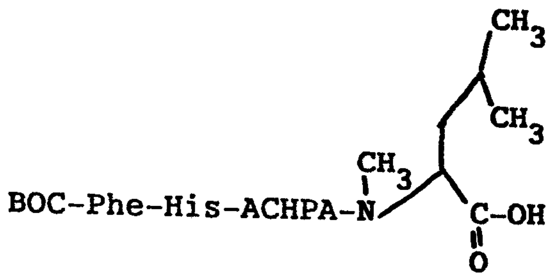

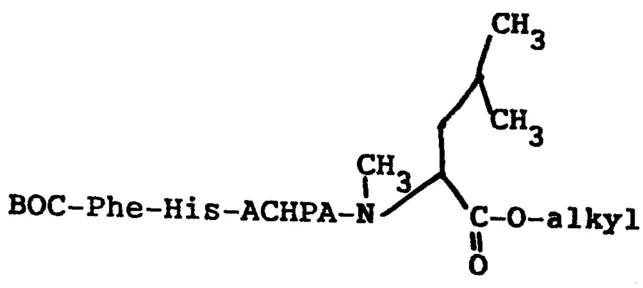

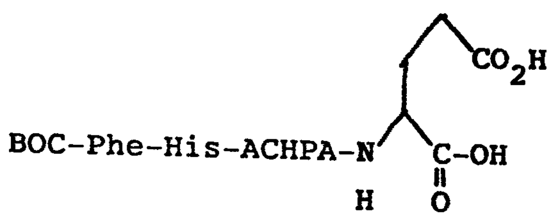

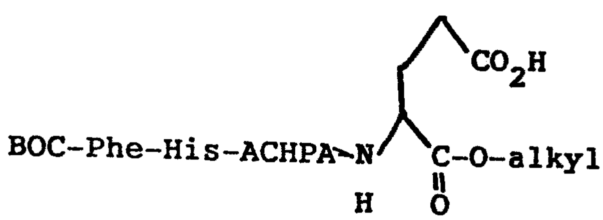

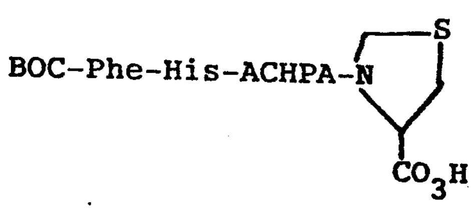

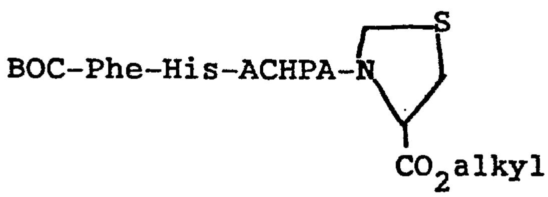

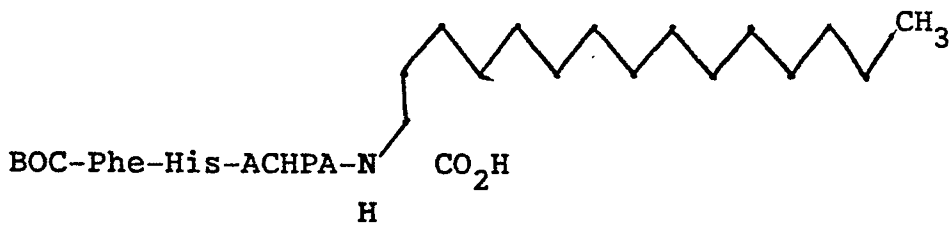

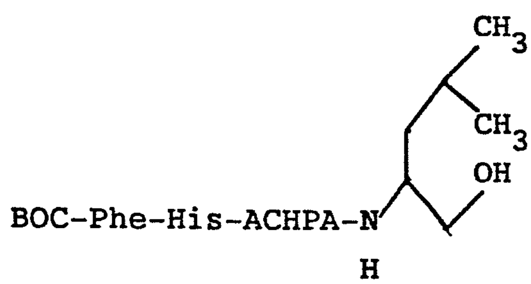

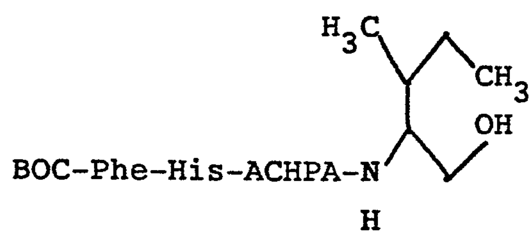

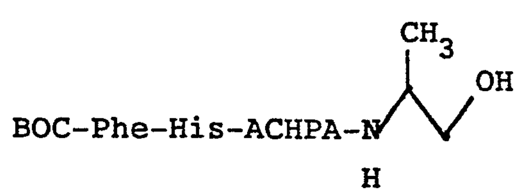

BOC-Phe-His-ACHPA-$C_6H_{12}O_5N$

BOC-Phe-His-ACHPA-$C_5H_{10}O_4N$

BOC-Phe-His-ACHPA-$C_6H_{12}O_4N$

BOC-Phe-His-ACHPA-$C_{14}H_{26}O_{10}N$

BOC-Phe-His-ACHPA-Lys-NH-i-Bu
BOC-Phe-His-ACHPA-Lys(CBZ)-NH-i-Bu
BOC-Phe-His-ACHPA-Lys
BOC-Phe-His-ACHPA-Lys(CBZ)
BOC-Phe-Lys-ACHPA-Lys
BOC-Phe-Lys-ACHPA-Lys(CBZ)
BOC-Phe-Lys-ACHPA-Pro
BOC-Phe-Lys-ACHPA-(4-i-PrO)Pro
BOC-Phe-Lys-ACHPA-NH-[(2S)-methyl]butyl
BOC-Phe-Orn-ACHPA-NH-[(2S)-methyl]butyl
BOC-Phe-Arg-ACHPA-NH[(2S)-methyl]butyl
BOC-Phe-[(S)-4-hydroxybutyl)]Gly-ACHPA-NH-[(2S)-methyl]butyl
BOC-Phe-Arg-ACHPA-NH-[(2S)-methyl]butyl
BOC-Phe-Nva-ACHPA-NH-[(2S)-methyl]butyl
BOC-Phe-Nva-ACHPA-NH-[(2S)-methyl]butyl
BOC-Phe-Nle-ACHPA-NH-[(2S)-methyl]butyl
BOC-Phe-(S-Me)Cys-ACHPA-NH-[(2S)-methyl]butyl
BOC-Tyr-His-ACHPA-NH-[(2S)-methyl]butyl
BOC-(p-$OCH_3$)Phe-His-ACHPA-NH-[(2S)-methyl]butyl
BOC-Trp-His-ACHPA-NH-[(2S)-methyl]butyl
BOC-[3-(1-naphthyl)]Ala-His-ACHPA-NH[(2S)-methyl]butyl
BOC-His-His-ACHPA-NH-[(2S)-methyl]butyl
BOC-(p-n-Pr)Phe-His-ACHPA-NH-[(2S)-methyl]butyl
CBZ-[3-(1-naphthyl)Ala-His-ACHPA-NH-[(2S)-methyl]butyl

i-$PRO_2C$-His-His-ACHPA-NH-[(2S)-methyl]butyl
$Et0_2C$-Phe-His-ACHPA-NH-[(2S)-methyl]butyl
2(S)-hydroxy-3-phenylpropionyl-His-ACHPA-NH-[2S)-methyl]butyl
S-benzylthioacetyl-His-ACHPA-NH-[(2S)-methyl]butyl
Dibenzylacetyl-His-ACHPA-NH-[(2S)-methyl]butyl
Bis-(naphtylmethyl)acetyl-His-ACHPA-NH-[(2S)-methyl]butyl
Bis-(p-hydroxyphenylmethyl)acetyl-His-ACHPA-NH-[(2S)-methyl]butyl
[2-Phenylamino-3-phenylpropionyl-His-ACHPA-NH-[(2S)-methyl]butyl
2-Phenyloxy-3-phenylpropionyl]-His-ACHPA-NH-[(2S)-methyl]butyl
2-Phenylthio-3-phenylpropionyl-His-ACHPA-NH-[(2S)-methyl]butyl
1,3-Diphenylpropyloxcarbonyl-His-ACHPA-NH-](2S)-methyl-butyl
2-(1,3-diphenyl)propyloxcarbonyl-His-ACHPA-NH-[(2S)-methyl]butyl
2-Phenylthio-3-(1-naphthyl)propionyl-His-ACHPA-NH-[(2S)-methyl]butyl
[2-benzyl-2-(3,4-dihydroxy)benzyl]acetyl-His-ACHPA-NH-[(2S-methyl]butyl
[2-benzyl-2-(4-isopropyloxy)benzyl]acetyl-His-ACHPA-NH-[(2S)-methyl]butyl
BOC-Phe-His-[5-amino-6-cyclohexyl-4-hydroxy-2-isopropyl]hexanoyl 2(S)-aminobutane
BOC-Phe His-[5-amino-6-cyclohexyl-4-hydroxy-2-isobutyl]hexanoyl 2(S)-aminobutane
BOC-Phe-His-[5-amino-2-benzyl-6-cyclohexyl-4-hydroxy]hexanoyl 2(S)-aminobutane

BOC-Phe-His-[5-amino-6-cyclohexyl-2-cyclohexlmethyl-4-hydroxy]hexanoyl 2(S)-aminobutane
2(S)-hydroxy-3-phenylpropionyl-His-ACHPA-NH-[(2S)-methyl]butyl
S-benzylthioacetyl-His-ACHPA-NH-[(2S)-methyl]butyl
Dibenzylacetyl-His-ACHPA-Lys-NH[(2S)-methyl]butyl
Dibenzylactyl-His-ACHPA-Ile-NH-[(2S)-methyl]butyl
Dibenzylacetyl-Lys-ACHPA-Ile-NH-[(2S)-methyl]butyl
Dibenzylacetyl-Lys-ACHPA-Lys-NH-[(2S)-methyl]butyl
Bis-(naphtylmethyl)acetyl-Lys-ACHPA-Lys

---

[1] BOC = <u>Tert</u>-butyloxycarbonyl.

[2] ACHPA = (3<u>S</u>, 4<u>S</u>)-4-amino-5-cyclohexyl-3-hydroxy-pentanoyl.

[3] AHPPA = (3<u>S</u>, 4<u>S</u>)-4-amino-3-hydroxy-5-phenyl-pentanoyl.

The inhibitory peptides of the present invention may be better appreciated in terms of substrate analogy from the following illustration of Formula I alongside the octapeptide sequence of a portion of the *pig* renin substrate, which renin cleaves between $Leu^{10}$ and $Leu^{11}$:

| Pro | Phe | His | Leu | Leu | Val | Tyr | |
|---|---|---|---|---|---|---|---|
| 7 | 8 | 9 | 10 | (11) | 12 | 13 | (14) |

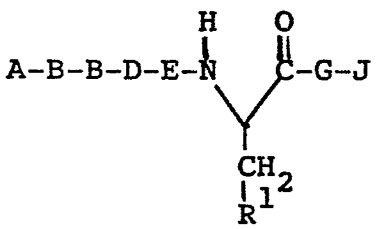

(I.)

As can be seen, a unique aspect and essential feature of the present invention is the substitution of the G component for the double amino acid sequence: $Leu^{10}$-$Leu^{11}$ in the endogenous pig renin substrate. It is believed that substitution of this component for both leucine amino acids rather than just one leucine results in an improved substrate analogy due to the greater linear extent of the component as compared to a single leucine component. Thus, the component more closely approximates Leu-Leu in linear extent, and thereby provides a better "fit" to the renin enzyme.

The inhibitory peptides of the present invention may also be better appreciated in terms of substrate analogy from the following illustration of Formula I alongside the octapeptide sequence of a portion of the <u>human</u> renin substrate, which renin cleaves between $Leu^{10}$ and $Val^{11}$:

| Pro | Phe | His | Leu | Val | Ile | His | |
|---|---|---|---|---|---|---|---|
| 7 | 8 | 9 | 10 | (11) | 12 | 13 | (14) |

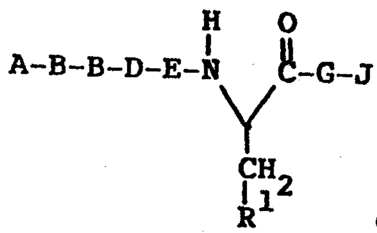

(I.)

As can be seen, a unique aspect and essential feature of the present invention is the substitution of the G component for the double amino acid sequence: $Leu^{10}$-$Val^{11}$ in the endogenous human renin substrate. It is believed that substitution of this component for both the leucine and valine amino acids rather than just the leucine results in an improved substrate analogy due to the greater linear extent of the component as compared to a single leucine component. Thus, the component more closely approximates Leu-Val in linear extent, and thereby provides a better "fit" to the human renin enzyme.

The Formula I compounds can be used in the form of salts derived from inorganic or organic acids and bases. Included among such acid addition salts are the following: acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectinate, persulfate, 3-phenylpropionate, picrate, pivalate, propionate, succinate, tartrate, thiocyanate, tosylate, and undecanoate. Base salts include ammonium salts, alkali metal salts such as sodium and potassium salts, alkaline earth metal salts such as calcium and magnesium salts, salts with organic bases such as dicyclohexylamine salts, N-methyl-D-glucamine, and salts with amino acids such as arginine, lysine, and so forth. Also, the basic nitrogen-containing groups can be quaternized with such agents as lower alkyl halides, such as methyl, ethyl, propyl, and butyl chloride, bromides and iodides; dialkyl sulfates like dimethyl, diethyl, dibutyl; and diamyl sulfates, long chain halides such as decyl, lauryl, myristyl and stearyl chlorides, bromides and iodides, aralkyl halides like benzyl and phenethyl bromides and others. Water or oil-soluble or dispersible products are thereby obtained.

The present invention is also directed to combinations of the novel renin-inhibitory peptides of Formula I with one or more antihypertensive agents selected from the group consisting of diuretics, α and/or ß-adrenergic blocking agents, CNS-acting agents, adrenergic neuron blocking agents, vasodilators, angiotensin I converting enzyme inhibitors, calcium channel blockers, and other antihypertensive agents.

For example, the compounds of this invention can be given in combination with such compounds or salt or other derivative forms thereof as:

Diuretics: acetazolamide; amiloride; bendroflumethiazide; benzthiazide; bumetanide; chlorothiazide; chlorthalidone; cyclothiazide; ethacrynic acid; furosemide; hydrochlorothiazide;

hydroflumethiazide; indacrinone (racemic mixture, or as either the (+) or (-) enantiomer alone, or a manipulated ratio, e.g., 9:1 of said enantiomers, respectively); metolazone; methyclothiazide; muzolimine; polythiazide; quinethazone; sodium ethacrynate; sodium nitroprusside; spironolactone; ticrynafen; triamterene; trichlormethiazide;

α-Adrenergic Blocking Agents: dibenamine; phentolamine; phenoxybenzamine; prazosin; tolazoline;

ß-Adrenergic Blocking Agents: atenolol; metoprolol; nadolol; propranolol; timolol;

((±)-2-[3-(tert-butylamino)-2-hydroxypropoxy]-2-furan-anilide) (ancarolol);

(2-acetyl-7-(2-hydroxy-3-isopropylaminopropoxy)benzofuran HCl) (befunolol);

((±)-1-(isopropylamino)-3-(p-(2-cyclopropylmethoxy-ethyl)-phenoxy)-2-propranol HCl) (betaxolol);

(1-[(3,4-dimethoxyphenethyl)amino]-3-(m-tolyloxy)-2-propanol HCl) (bevantolol);

(((±)-1-(4-((2-isopropoxyethoxy)methyl)phenoxy)-3-iso-propylamino-2-propanol)fumarate) (bisoprolol);

(4-(2-hydroxy-3-[4-(phenoxymethyl)-piperidino]-propoxy)-indole);

(carbazolyl-4-oxy-5,2-(2-methoxyphenoxy)-ethylamino-2-propanol);

(1-((1,1-dimethylethyl)amino)-3-((2-methyl-1H-indol-4-yl)oxy)-2-propanol benzoate) (bopindolol);

(1-(2-exobicyclo[2.2.1]-hept-2-ylphenoxy)-3-[(1-methyl-ethyl)-amino]-2-propanol HCl) (bornaprolol);

(o-[2-hydroxy-3-[(2-indol-3-yl-1,1-dimethylethyl)-amino]propoxy]benzonitrile HCl) (bucindolol);

(α-[(tert.butylamino)methyl]-7-ethyl-2-benzofuranmethanol) (bufuralol);

(3-[3-acetyl-4-[3-(tert.butylamino)-2-hydroxypropyl]-phenyl]-1,1-diethylurea HCl) (celiprolol);

((±)-2-[2-[3-[(1,1-dimethylethyl)amino]-2-hydroxy-propoxy]phenoxy]-N-methylacetamide HCl) (cetamolol);

(2-benzimidazolyl-phenyl(2-isopropylaminopropanol));

((±)-3'-acetyl-4'-(2-hydroxy-3-isopropylaminopropoxy)-acetanilide HCl) (diacetolol);

(methyl-4-[2-hydroxy-3-[(1-methylethyl)aminopropoxy]]-benzenepropanoate HCl) (esmolol);

(erythro-DL-1-(7-methylindan-4-yloxy)-3-isopropylaminobutan-2-ol);

(1-(tert.butylamino)-3-[O-(2-propynyloxy)phenoxy]-2-propanol (pargolol);

(1-(tert.butylamino)-3-[o-(6-hydrazino-3-pyridazinyl)-phenoxy]-2-propanol diHCl) (prizidilol);

((-)-2-hydroxy-5-[(R)-1-hydroxy-2-[(R)-(1-methyl-3-phenylpropyl)amino]ethyl]benzamide);

(4-hydroxy-9-[2-hydroxy-3-(isopropylamino)-propoxy]-7-methyl-5H-furo[3,2-g][1]-benzopyran-5-one) (iprocrolol);

((-)-5-(tert.butylamino)-2-hydroxypropoxy]-3,4-dihydro-1-(2H)-naphthalenone HCl) (levobunolol);

(4-(2-hydroxy-3-isopropylamino-propoxy)-1,2-benziso-thiazole HCl);

(4-[3-(tert.butylamino)-2-hydroxypropoxy]-N-methyliso-carbostyril HCl);

((±)-N-2-[4-(2-hydroxy-3-isopropyl aminopropoxy)-phenyl]ethyl-N'-isopropylurea) (pafenolol);

(3-[[(2-trifluoroacetamido)ethyl]amino]-1-phenoxy-propan-2-ol);

(N-(3-(o-chlorophenoxy)-2-hydroxypropyl)-N'-(4'-chloro-2,3-dihydro-3-oxo-5-pyridazinyl)ethylenediamine);

((±)-N-[3-acetyl-4-[2-hydroxy-3-[(1-methylethyl)amino]-propoxy]phenyl]butanamide) (acebutolol);

((±)-4'-[3-(tert-butylamino)-2-hydroxypropoxy]spiro-[cyclohexane-1,2'-indan]-1'-one) (spirendolol);

(7-[3-[[2-hydroxy-3-[(2-methylindol-4-yl)oxy]propyl]-amino]butyl]thiophylline) (teoprolol);

((±)-1-tert.butylamino-3-(thiochroman-8-yloxy)-2-propanol) (tertatolol);

((±)-1-tert.butylamino-3-(2,3-xylyloxy)-2-propanol HCl) (xibenolol);

(8-[3-(tert.butylamino)-2-hydroxypropoxy]-5-methylcoumarin) (bucumolol);

(2-(3-(tert.butylamino)-2-hydroxy-propoxy)benzonitrile HCl) (bunitrolol);

((±)-2'-[3-(tert-butylamino)-2-hydroxypropoxy-5'-fluorobutyrophenone) (butofilolol);

(1-(carbazol-4-yloxy)-3-(isopropylamino)-2-propanol) (carazolol);

(5-(3-tert.butylamino-2-hydroxy)propoxy-3,4-dihydrocarbostyril HCl) (carteolol);

(1-(tert.butylamino)-3-(2,5-dichlorophenoxy)-2-propanol) (cloranolol);

(1-(inden-4(or 7)-yloxy)-3-(isopropylamino)-2-propanol HCl) (indenolol);

(1-isopropylamino-3-[(2-methylindol-4-yl)oxy]-2-propanol) (mepindolol);

(1-(4-acetoxy-2,3,5-trimethylphenoxy)-3-isopropylaminopropan-2-ol) (metipranolol);

(1-(isopropylamino)-3-(o-methoxyphenoxy)-3-[(1-methylethyl)amino]-2-propanol) (moprolol);

((1-tert.butylamino)-3-[(5,6,7,8-tetrahydro-cis-6,7-dihydroxy-1-naphthyl)oxy]-2-propanol) (nadolol);

((S)-1-(2-cyclopentylphenoxy)-3-[(1,1-dimethylethyl)-amino]-2-propanol sulfate (2:1)) (penbutolol);

(4'-[1-hydroxy-2-(amino)ethyl]methanesulfonanilide) (sotalol);

(2-methyl-3-[4-(2-hydroxy-3-tert.butylaminopropoxy)-phenyl]-7-methoxy-isoquinolin-1-(2H)-one);

(1-(4-(2-(4-fluorophenyloxy)ethoxy)phenoxy)-3-iso-propylamino-2-propanol HCl);

((-)-p-[3-[(3,4-dimethoxyphenethyl)amino]-2-hydroxy-propoxy]-ß-methylcinnamonitrile) (pacrinolol);

((±)-2-(3'-tert.butylamino-2'-hydroxypropylthio)-4-(5'-carbamoyl-2'-thienyl)thiazole HCl) (arotinolol);

((±)-1-[p-[2-(cyclopropylmethoxy)ethoxy]phenoxy]-3-(isopropylamino)-2-propanol) (cicloprolol);

((±)-1-[(3-chloro-2-methylindol-4-yl)oxy]-3-[(2-phenoxyethyl)amino]-2-propanol) (indopanolol);

((±)-6-[[2-[[3-(p-butoxyphenoxy)-2-hydroxypropyl]-amino]ethyl]amino]-1,3-dimethyluracil) (pirepolol);

(4-(cyclohexylamino)-1-(1-naphtholenyloxy)-2-butanol);

(1-phenyl-3-[2-[3-(2-cyanophenoxy)-2-hydroxypropyl]-aminoethyl]hydantoin HCl);

(3,4-dihydro-8-(2-hydroxy-3-isopropylaminopropoxy)-3-nitroxy-2H-1-benzopyran) (nipradolol);

<u>α and ß-Adrenergic Blocking Agents</u>:

((±)-1-tert-butylamino)-3-[o-[2-(3-methyl-5-isoxazolyl)vinyl]phenoxy]-2-propanol) (isoxaprolol);

(1-isopropylamino-3-(4-(2-nitroxyethoxy)phenoxy)-2-propanol HCl);

(4-hydroxy-α-[[3-(4-methoxyphenyl)-1-methylpropyl]-aminomethyl]-3-(methylsulfinyl)-benzmethanol HCl) (sulfinalol);

(5-[1-hydroxy-2-[[2-(o-methoxyphenoxy)ethyl]amino]-ethyl]-2-methylbenzenesulfonamide HCl);

(5-[1-hydroxy-2-[(1-methyl-3-phenylpropyl)amino]ethyl]-salicylamide HCl) (labetalol);

(1-((3-chloro-2-methyl-1H-indol-4-yl)oxy)-3-((2-phenoxyethyl)amino)-2-propanol-hydrogenmalonate) (ifendolol);

(4-(2-hydroxy-3-[(1-methyl-3-phenylpropyl)amino]-propoxy)benzeneacetamide);

(1-[3-[[3-(1-naphthoxy)-2-hydroxypropyl]-amino]-3,3-dimethyl-propyl]-2-benzimidazolinone);

(3-(1-(2-hydroxy-2-(4-chlorophenylethyl)-4-piperidyl)-3,4-dihydroxy)quinoxolin-2(1H)-one);

<u>CNS-Acting Agents</u>: clonidine; methyldopa;

<u>Adrenergic Neuron Blocking Agents</u>: guanethidine; reserpine and other rauwolfia alkaloids such as rescinnamine;

<u>Vasodilators</u>: diazoxide; hydralazine; minoxidil;

<u>Angiotensin I Converting Enzyme Inhibitors</u>:

1-(3-mercapto-2-methyl-1-oxopropyl)-L-proline (captopril);

(1-(4-ethoxycarbonyl-2,4(R,R)-dimethylbutanoyl)-indoline-2(S)-carboxylic acid);

(2-[2-[[1-(ethoxycarbonyl)-3-phenyl-propyl]amino]-1-oxopropyl]-1,2,3,4-tetrahydro-3-isoquinoline carboxylic acid);

((S)-1-[2-[[1-(ethoxycarbonyl)-3-phenylpropyl]amino]-1-oxopropyl]octahydro-1H-indole-2-carboxylic acid HCl);

(N-cyclopentyl-N-(3-(2,2-dimethyl-1-oxopropyl)thiol-2-methyl-1-oxopropyl)glycine) (pivalopril);

((2R,4R)-2-(2-hydroxyphenyl)-3-(3-mercaptopropionyl)-4-thiazolidinecarboxylic acid);

(1-(N-[1(S)-ethoxycarbonyl-3-phenylpropyl]-(S)-alanyl)-cis,syn-octahydroindol-2(S)-carboxylic acid HCl);

((-)-(S)-1-[(S)-3-mercapto-2-methyl-1-oxopropyl]-indoline-2-carboxylic acid);

([1(S),4S]-1-[3-(benzoylthio)-2-methyl-1-oxopropyl]-4-phenylthio-L-proline;

(3-([1-ethoxycarbonyl-3-phenyl-(1S)-propyl]amino)-2,3,4,5-tetrahydro-2-oxo-1-(3S)-benzazepine-1-acetic acid HCl);

(N-(2-benzyl-3-mercaptopropanoyl)-S-ethyl-L-cysteine) and the S-methyl analogue;

(N-(1(S)-ethoxycarbonyl-3-phenylpropyl)-L-alanyl-L-proline maleate) (enalapril);

N-[1-(S)-carboxy-3-phenylpropyl]-L-alanyl-1-proline;

$N^2$-[1-(S)-carboxy-3-phenylpropyl]-L-lysyl-L-proline (lysinopril);

Calcium Channel Blockers:

α-[3-[[2-(3,4-dimethoxyphenyl)ethyl]methylamino]-propyl]-3,4-dimethoxy-α-(1-methylethyl)benzene-acetonitrile (verapamil);

1,4-dihydro-2,6-dimethyl-4-(2-nitrophenyl)-3,5-pyridinedicarboxylic acid dimethyl ester (nifedipine);

2-(2,2-dicyclohexylethyl)piperidine (perhexiline);

N-(1-methyl-2-phenylethyl)- -phenylbenzenepropanamine (prenylamine);

3-(aminosulfonyl)-4-chloro-N-(2,3-dihydro-2-methyl-1H-indol-1-yl)benzamide (indapamide);

(2'-(2-diethylaminoethoxy)-3-phenylpropiophenone (etafenone);

(4-[4,4-bis-(4-fluorophenyl)butyl]-N-(2,6-dimethylphenyl)-1-piperazineacetamide) (lidoflazine);

(2-(N-benzyl-N-methylamino)ethylmethyl-2,6-dimethyl-4-(m-nitrophenyl)-1,4-dihydro-3,5-pyridinedicarboxylate HCl) (nicardipine);

(N-(3,4-dimethoxyphenethyl)-2-(3,4-dimethoxyphenyl)-N-methyl-m-dithiane-2-propylamine-1,1,3,3-tetraoxide) (tiapamil);

(5,6-dimethoxy-2-(3-[(α-(3,4-dimethoxy)phenylethyl)-methylamino]propyl)phthalimidine) (falipamil);

(ß-[(2-methylpropoxy)methyl]-N-phenyl-N-phenylmethyl-1-pyrrolidineethanamine HCl monohydrate) (bepridil);

((+)-cis-3-(acetyloxy)-5-[2-(dimethylamino)ethyl]-2,3-dihydro-2-(4-methoxyphenyl)-1,5-benzothiazepin-4-(5H)-one) (diltiazem);

((E)-1-[bis-(p-fluorophenyl)methyl]-4-cinnamylpiperazine di HCl) (flunarizine);

(5-[(3,4-dimethoxyphenethyl)methylamino]-2-isopropyl-2-(3,4,5-trimethoxyphenyl)valeronitrile (gallopamil);

(ethylmethyl(2,3-dichlorophenyl)-1,4-dihydro-2,6-dimethyl-3,5-pyridinedicarboxylate (felodipine);

(isopropyl-2-methoxyethyl-1,4-dihydro-2,6-dimethyl-4-(3-nitrophenyl)-3,5-pyridinecarboxylate) (nimodipine);

(3-ethyl-5-methyl-1,4-dihydro-2,6-dimethyl-4-(3-nitrophenyl)-3,5-pyridine-dicarboxylate) (nitrendipine);

<u>Other Antihypertensive Agents</u>: aminophylline; cryptenamine acetates and tannates; deserpidine; meremethoxylline procaine; pargyline; trimethaphan camsylate;
and the like, as well as admixtures and combinations thereof.

Typically, the individual daily dosages for these combinations can range from about one-fifth of the minimally recommended clinical dosages to the maximum recommended levels for the entities when they are given singly. Coadministration is most readily accomplished by combining the active ingredients into a suitable unit dosage form containing the proper dosages of each. Other methods of coadministration are, of course, possible.

The novel peptides of the present invention possess an excellent degree of activity in treating renin-associated hypertension and hyperaldosteronism. Some of the peptides also have ACE inhibitor activity which may also make them useful for treating hypertension and congestive heart failure.

For these purposes the compounds of the present invention may be administered parenterally, by inhalation spray, or rectally in dosage unit formulations containing conventional non-toxic pharmaceutically acceptable carriers, adjuvants and vehicles. The term parenteral as used herein includes subcutaneous injections, intravenous, intramuscular, intrasternal injection or infusion techniques. In addition to the treatment of warm-blooded animals such as mice, rats, horses, dogs, cats, etc., the compounds of the invention are effective in the treatment of humans.

The pharmaceutical compositions may be in the form of a sterile injectable preparation, for example as a sterile injectable aqueous or oleagenous suspension. This suspension may be formulated according to the known art using suitable dispersing or wetting agents and suspending agents. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectibles.

The peptides of this invention may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols.

Dosage levels of the order of 0.1 to 4.0 grams per day are useful in the treatment of the above indicated conditions. For example, renin-associated hypertension and hyperaldosteronism are effectively treated by the administration of from 1.0 to 50 milligrams of the compound per kilogram of body weight per day.

The amount of active ingredient that may be combined with the carrier materials to produce a single dosage form will vary depending upon the host treated and the particular mode of administration.

It will be understood, however, that the specific dose level for any particular patient will depend upon a variety of factors including the activity of the specific compound employed, the age, body weight, general health, sex, diet, time of administration, route of administration, rate of excretion, drug combination and the severity of the particular disease undergoing therapy.

Thus, in accordance with the present invention there is further provided a pharmaceutical composition for treating renin-associated hypertension and hyperaldosteronism, or congestive heart failure comprising a pharmaceutical carrier and a therapeutically effective amount of a peptide of the formula:

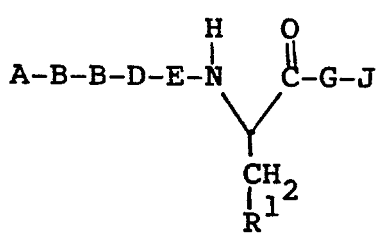

(I.)

wherein A, B, D, E, $R^1$, G, and J have the same meaning as recited further above for Formula I; wherein all of the asymmetric carbon atoms have an S configuration, except for those in the B, D, and G substituents, which may have an S or R configuration; and a pharmaceutically acceptable salt thereof.

Also, in accordance with the present invention there is still further provided a method of treating renin-associated hypertension and hyperaldosteronism, comprising administering to a patient in need of such treatment, a therapeutically effective amount of a peptide of the formula:

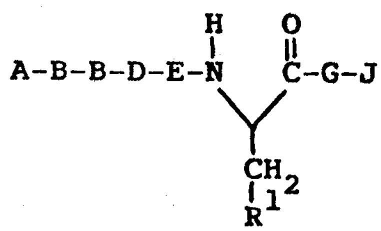

(I.)

wherein A, B, D, E, $R^1$, G, and J have the same meaning as recited further above for Formula I; wherein all of the asymmetric carbon atoms have an S configuration, except for those in the A, D, and G substituents, which may have an S or R configuration; and a pharmaceutically acceptable salt thereof.

The renin inhibitory novel peptides of the present invention may also be utilized in diagnostic methods for the purpose of establishing the significance of renin as a causative or contributory factor in hypertension or hyperaldosteronism in a particular patient. For this purpose the novel peptides of the present invention may be administered in a single dose of from 0.1 to 10 mg per kg of body weight.

Both in vivo and in vitro methods may be employed. In the in vivo method, a novel peptide of the present invention is administered to a patient, preferably by intravenous injection, although parenteral administration is also suitable, at a

hypotensive dosage level and as a single dose, and there may result a transitory fall in blood pressure. This fall in blood pressure, if it occurs, indicates supranormal plasma renin levels.

An in vitro method which may be employed involves incubating a body fluid, preferably plasma, with a novel peptide of the present invention and, after deproteinization, measuring the amount of angiotensin II produced in nephrectomized, pentolinium-treated rats. Another in vitro method involves mixing the plasma or other body fluid with a novel peptide of the present invention and injecting the mixture into a test animal. The difference in pressor response with and without added peptide is a measure of the renin content of the plasma.

Pepstatin may be employed in the methods described above as an active control. See, e.g., U.S. Patent Nos. 3,784,686 and 3,873,681 for a description of the use of pepstatin in diagnostic methods of this type.

The novel peptides of the present invention may be prepared in accordance with well-known procedures for preparing peptides from their constituent amino acids, which will be described in more detail below.

A general method of preparation may be described in the following terms:

A method of preparing a peptide of Formula I, said peptide being comprised of from two to six amino acids identified as I through VI, amino acid (AA) I being the component G at the C-terminus of said peptide, and amino acid (AA) VI being at the

N-terminus of said peptide, to which substituent A is attached, comprising the steps of:

(A) treating the desired ester or amide of the C-terminus amino acid (AA I) with the next adjacent amino acid (AA II) of said peptide, the amino group of said amino acid being protected by a protecting group, in the presence of a condensing agent, whereby a dipeptide of the two amino acids (AA I and II) is formed;

(B) deprotecting the dipeptide formed in Step (A) by removing the protecting group from the amino group of AA II, to give the peptide of Formula I wherein A is hydrogen;

(C) treating the dipeptide formed in Step (B) where an ester of AAI is employed with hydrazine to give the corresponding hydrazide, followed by treatment of said hydrazide with acidic nitrite to give the corresponding acyl azide, followed by treatment of said acyl azide with the appropriate amine compound to give the desired J substituent in the peptide of Formula I; and optionally

(D) treating the dipeptide formed in Step (C) with

      O

$R^2_a$-X-C-W, where X, $R^2_a$, and $R^2_b$, are as defined

   $R^2_b$

above and W is an acid halide, anhydride, or other carbonyl activating group, to give the peptide of Formula I wherein A is other than hydrogen; and optionally

(E) forming a tripeptide up to a hexapeptide of AA I, through AA VI, by repeating the procedures of Steps (A) and (B) using protected AA III through protected AA VI;

(F) deprotecting the tripeptide through hexapeptide formed in Step (E) to give the peptide of Formula I wherein A is hydrogen; and optionally

(G) treating the ttipeptide through hexapeptide formed in Step (H) with $R_a^2-\underset{R_b^2}{X}-\overset{O}{C}-W$, where X, $R_a^2$, and $R_b^2$ are as defined above and W is an acid halide, anhydride, or other carboxyl activating group, to give the peptide of Formula I wherein A is other than hydrogen;

said method also comprising, where necessary, protection of sidechain substituents of the component amino acids AA I through AA VI, with deprotection being carried out as a final step; said method also comprising any combination of the steps set out above, whereby the amino acids I through VI and substituents A, G, and J, are assembled in any desired order to prepare the peptide of Formula I; and said method also comprising employment of the steps set out above in a solid phase sequential synthesis, whereby in the initial step the carboxyl group of the selected amino acid is bound to a synthetic resin substrate while the amino group of said amino acid is protected, followed by removal of the protecting group, the succeeding steps being as set out above, the peptide as it is assembled being attached to said synthetic resin substrate; followed by a step of removing the peptide of Formula I from said synthetic resin substrate; and after removal of the peptide of Formula I from said synthetic resin substrate, the step of teating said ester thereof in accordance with the procedures described in Step (C)

above to give the desired J substituent in the peptide of Formula I; removal of sidechain protecting groups being accomplished either before or after removal of the peptide of Formula I from said synthetic resin substrate.

Preparation of the peptides of Formula I having the desired J substituent, as described above in Step (C), may be illustrated as follows for the particular case where J=benzylamide (and PEP represents the remaining portion of the peptide of Formula I):

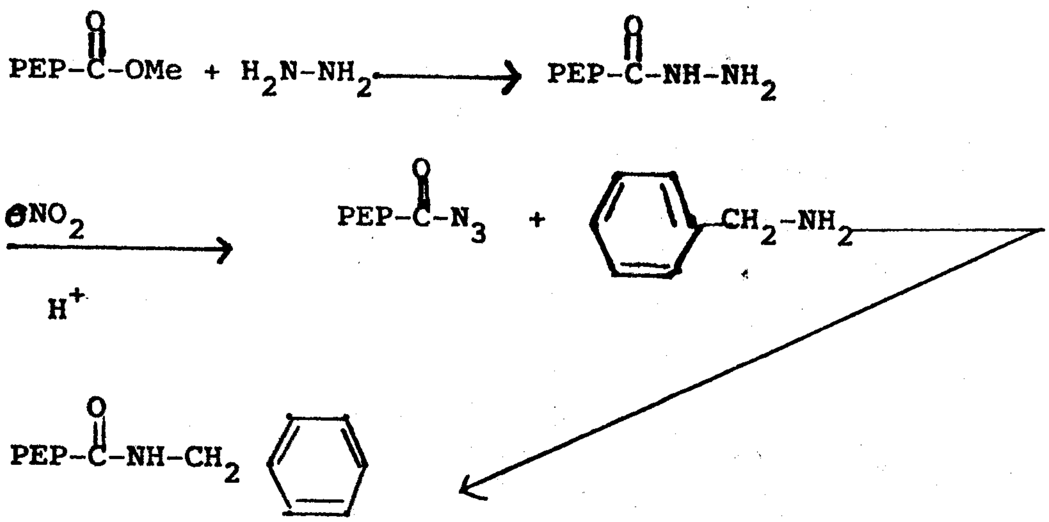


The G Component

I. Where the Component G Is a Peptide Isotere: the novel peptides of the present invention may be prepared in accordance with well-known procedures in synthetic chemistry, as is described in more detail further below. Attachment of the isostere component G to the other components of the novel peptides of the present invention is carried out in the same manner as for any of said other components, and may

involve addition of the isostere component in a protected form. For example, the following reactive groups would require such protection:

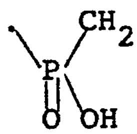 as 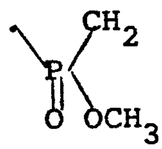 ; and

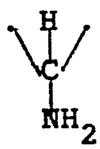 as 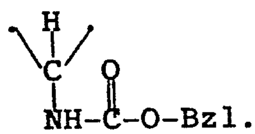

Such protecting groups may be removed as a final or near final step, for example, by base hydrolysis in the former case, or by hydrogenation in the latter.

Preparation of the particular isostere components may be carried out in accordance with procedures described below and in the literature cited particularly as follows:

A. 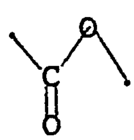 (depsipeptides)

Ondetti et al., Chemistry and Biology of Peptides, ed. J. Meienhofer, Ann. Arbor Science pp. 525-531, 1972.

B. 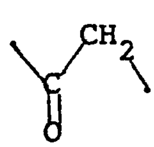 (ketomethylene)

(1) Natarajan et al., Peptides. <u>Synthesis-Structure-Function</u>, ed. D. H. Rich and E. Gross, Pierce Chem. Co., Rockford, Ill., pp. 429-433, 1981.

(2) Van Lommen et al., European Peptide Symposium 16th, <u>Peptides 1980</u>, ed. K. Brunfeldt, Scriptor, Copenhagen, pp. 248-252, 1981.

(3) Almquist et al., <u>J. Med. Chem.</u>, 23:1392-1398, 1980.

C. 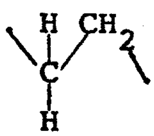 (ethylene)

Kawasaki and Maeda, <u>Biochem. Biophys. Res. Comm.</u> 106:113-116, 1982.

D. 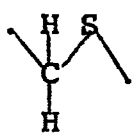 (methylenethio)

(1) Natarajan et al., <u>Id</u>.

(2) Fok and Yankellov, <u>Biochem. Biophys. Res. Comm.</u> 74: 273-278, 1977.

(3) Spatola et al., <u>Peptides. Structure-Function-Biological Function</u>, ed. E. Gross and J. Meienhofer, Pierce Chem. Co., Rockford, Ill., pp. 273-276, 1979.

(4) Spatola and Bettag, <u>J. Org. Chem.</u> 46: 2393-2394, 1981.

E. 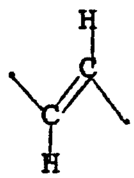 (ethylene)

(1) Natarajan et al., Id.

(2) Hann et al., J. Chem. Soc. Chem. Comm., 234-235, 1980.

(3) Cox et al., J. Chem. Soc. Chem. Comm., 799-800, 1980.

F. 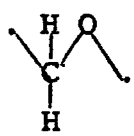 (methylene ether)

Ondetti et al., Id.

G. 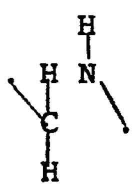 (methylene aza, or reduced isostere)

(1) Van Lommen et al., Id.

(2) Atherton et al., J. Chem. Soc. (C), 3393-3396, 1971.

(3) Parry et al., Chemistry and Biology of Peptides, ed. J. Meienhofer, Ann Arbor Science, pp. 541-544, 1972.

(4) Hudson et al., Int. J. Peptide Protein Res. 15: 122-129, 1979.

(5) Frank and Desiderio, Anal. Biochem. 90: 413-419, 1978.

H. 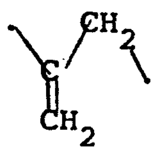 (exomethylene)

Prepared from ketomethylene by Wittig reaction.

I. 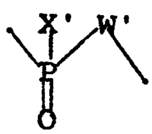

(1) Jacobson and Bartlett, <u>JACS</u> 103: 654-657, 1981.

(2) Jennings-White and Almquist, <u>Tet. Lett.</u>, 23: 2533-2534, 1982.

(3) Morton et al., <u>Tet. Lett.</u>, 23: 4123-4126, 1982.

For example, the compound:

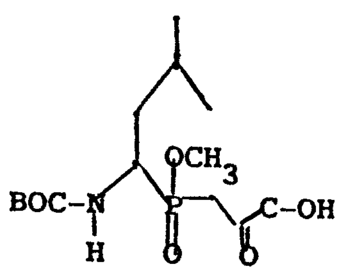

can be prepared in accordance with the following scheme:

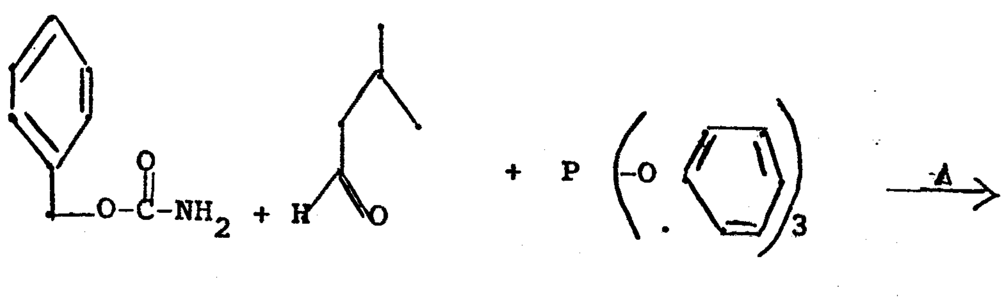

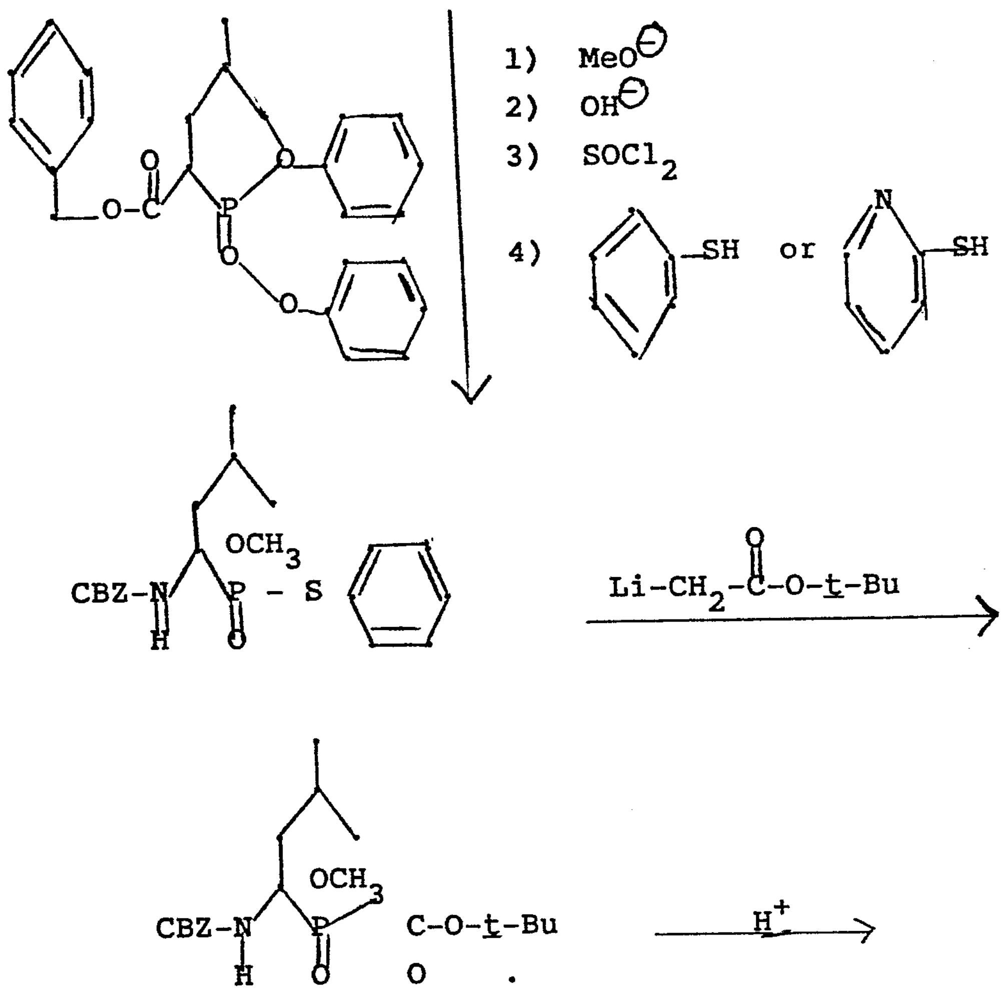


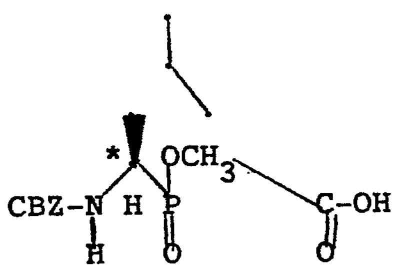

(III.)

(mixture of two pairs of diastereomers; two isomers at *; active isomer indicated).

which can be incorporated into the synthesis for the overall peptide of the present invention, or converted to the α-BOC derivative by hydrogenation over Pd/C catalyst, followed by treatment with

$(BOC)_2O$. Incorporation of (III.) or its BOC analog into a peptide sequence gives, after alkaline hydrolysis of the phosphinate ester, the free phosphinate ( 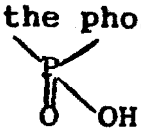 ). The product will contain two isomers at *; the active diastereomer has the relative configuration as an L amino acid, i.e., R-isomer in this case.

Also, the compound: 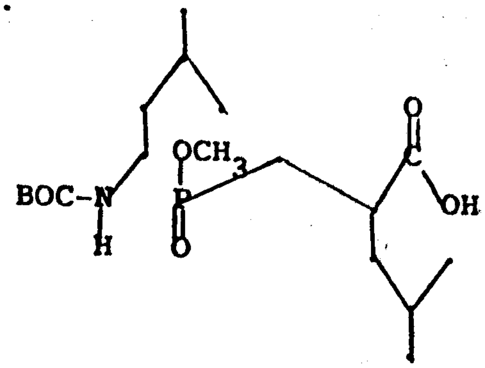

can be prepared in a fashion analgous to that described for (III.) above, for example from:

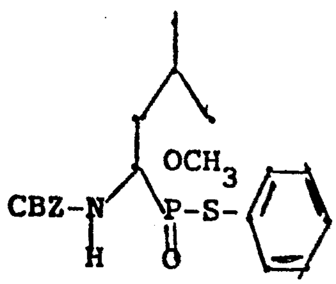

1) 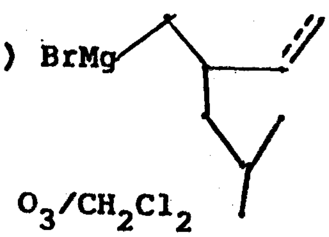

2) $O_3/CH_2Cl_2$ oxidative workup

3) Jones oxidation

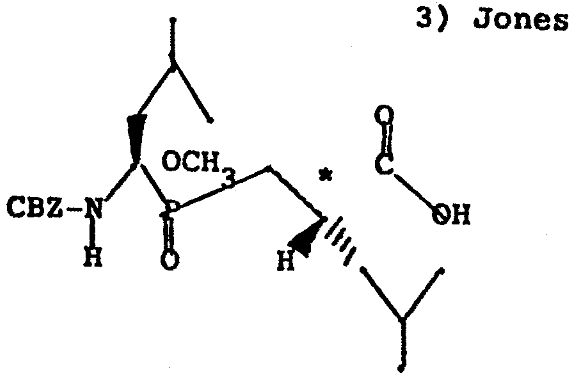

(IV.)

(active isomer shown; other isomers obtained as well)

Incorporation of (IV.) or its N-BOC analog proceeds as for (III.) above, with removal of the methyl phosphinate ester by hydrolysis (alkaline) to give the free phosphinate. The active isomer shown at * has the side chains in the relative configuration of the dipeptide that they mimic. For synthesis of

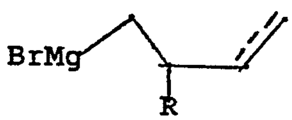 , see Jennings-White and Almquist, Id.

J. 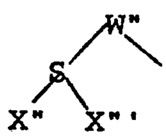

Morton et al., Id.

For example, the compound:

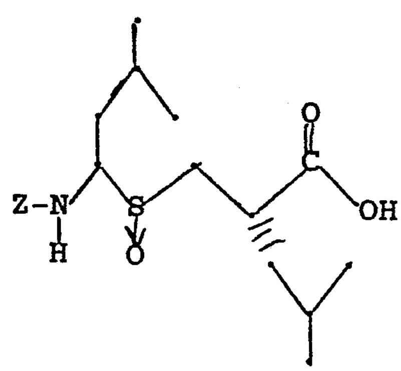

can be prepared in accordance with the following scheme:

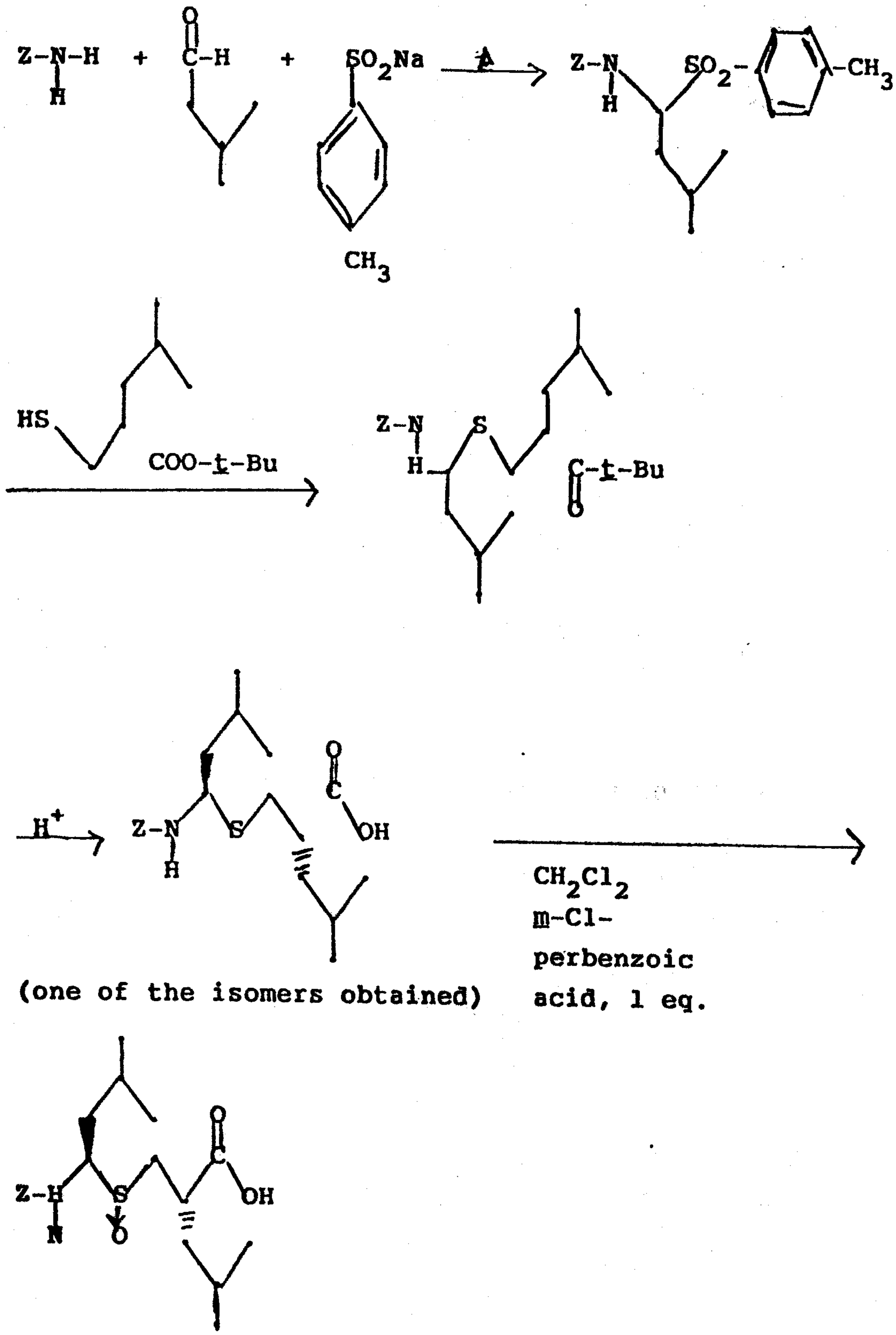
COO-t-Bu
H+
(one of the isomers obtained)
CH2Cl2
m-Cl-
perbenzoic
acid, 1 eq.

The sulfone ( 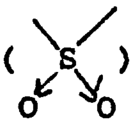 ) can be obtained using excess m-Cl-perbenzoic acid.

Use of 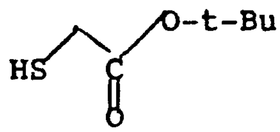 in the second step gives as the final product:

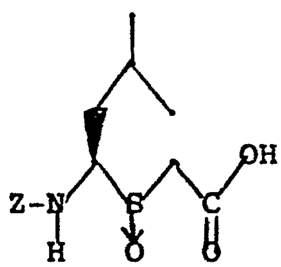

K. 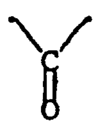 and 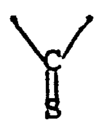

Prepared from the alcohol; see Rich et al., Biochem. Biophys. Res. Comm. 104:1127-1133, 1982.

Conversion of the ketone to the thioketone is with use of:

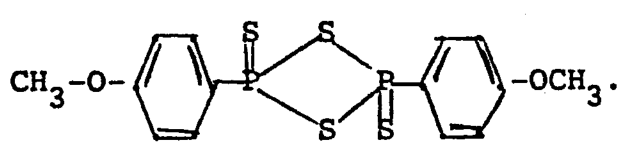

L. 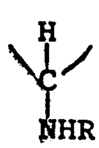

Obtained from 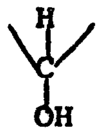 in accordance with the scheme outlined below; the R substituents are attached by conventional methods to the free amine (R=H):

Synthesis of protected 3-amino-3-deoxy-(3S,4S)-Statine:

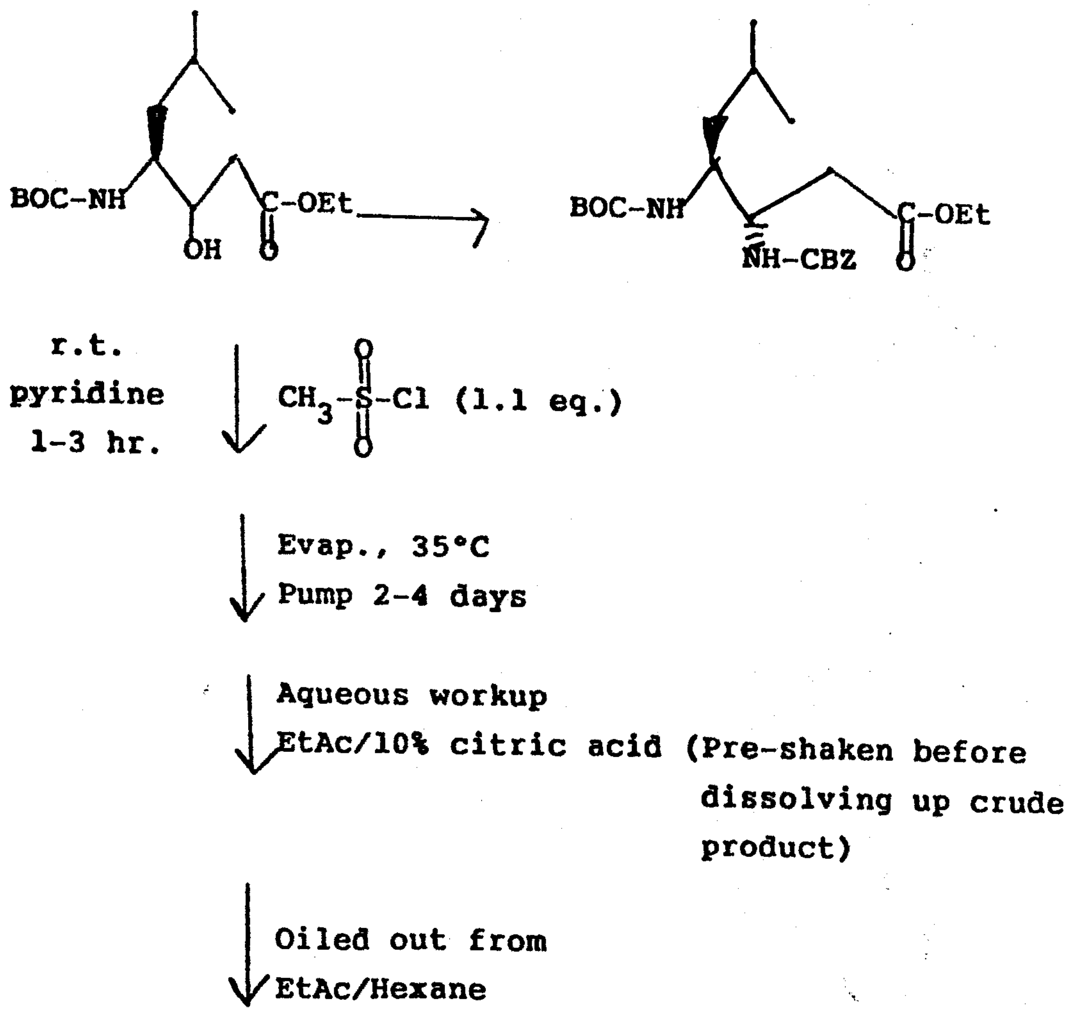

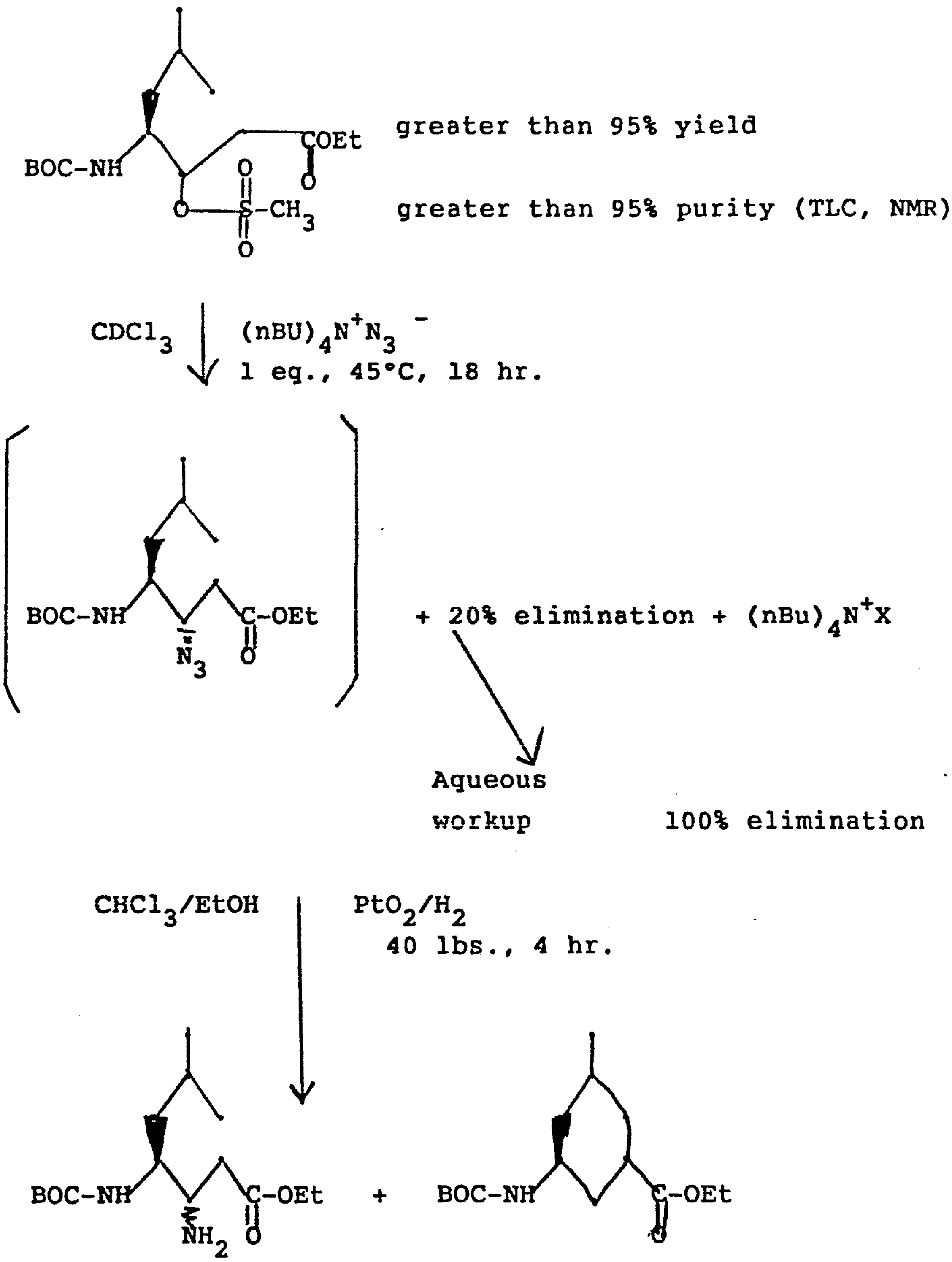
greater than 95% yield
greater than 95% purity (TLC, NMR)
CDCl3
(nBU)4N+N3 -
1 eq., 45°C, 18 hr.
+ 20% elimination + (nBu)4N+X
Aqueous workup
100% elimination
CHCl3/EtOH
PtO2/H2
40 lbs., 4 hr.
+

isolated by extraction into weak acid.

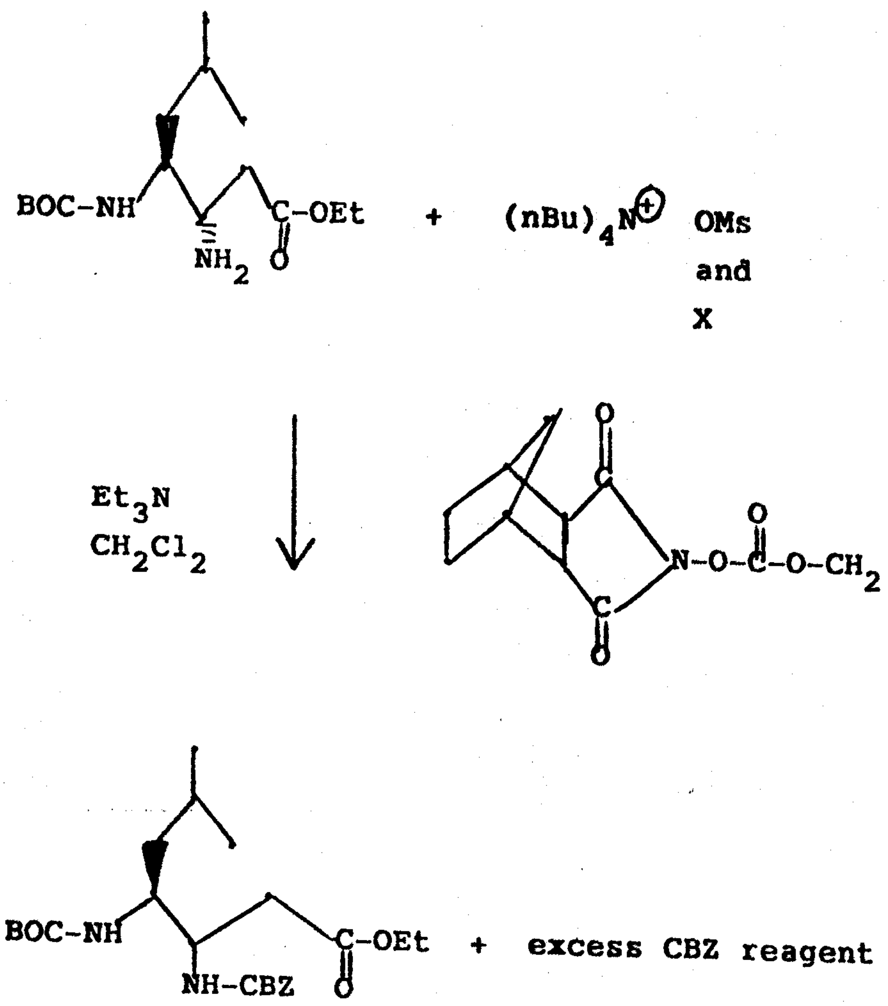


predominantly at
as shown

Base hydrolysis gives the free acid for incorporation into the synthesis of the overall peptides of the present invention.

M.

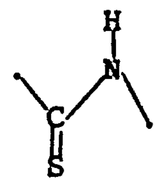

Obtained from the amide according to the method described by Clausen *et al.*, Seventh American Peptide Symposium, 1981:

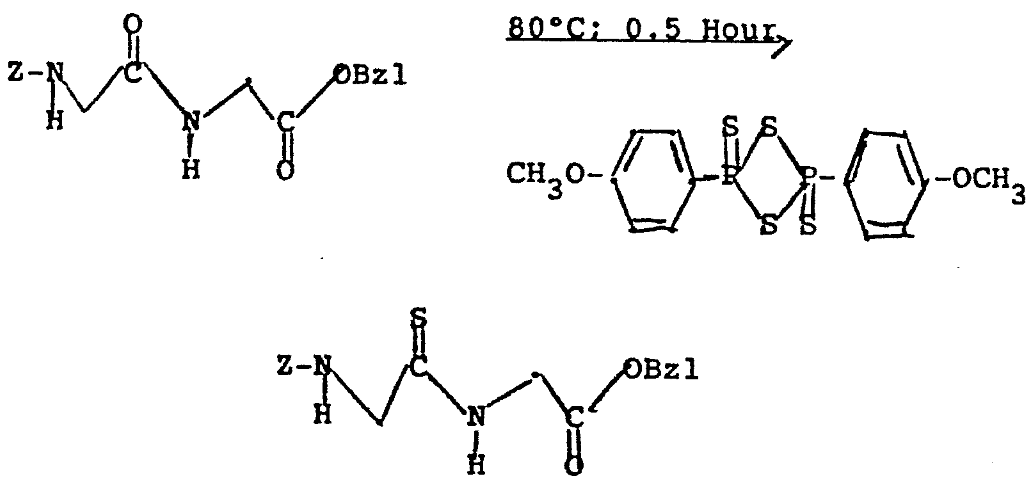


N.

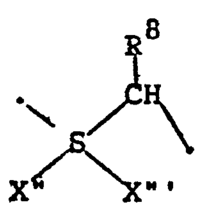

(1) Natarajan *et al.*, *Id.*

(2) Fok and Yankellov, *Id.*

(3) Spatola *et al.*, *Id.*

(4) Spatola and Bettag, *Id.*

(5) Spatola *et al*, *Proceedings of the Seventh American Peptide Symposium*, ed. E. Gross and D. H. Rich, pp. 613-616, 1981.

II. Where the Component G Is a 2-Substituted Statine: an efficient method of preparing the 2-substituted statine component G in the required 2R,3S,4S configuration begins with the preparation of protected phenylalanine aldehyde 1 in three steps beginning from phenylalanine, illustrated as follows:

$$\text{Phe-OH} \longrightarrow \text{Phth-Phe-OH} \xrightarrow{SOCl_2} \xrightarrow{H_2} \text{Phth-Phe-CHO}$$

1

This aldehyde 1 can then be reacted with the ketone silylacetal 2 in a titanium mediated aldol condensation to afford an approximately 1:1 mixture of 3a and 3b, illustrated as follows:

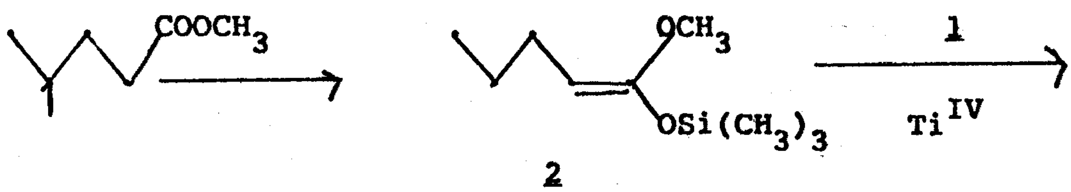


2

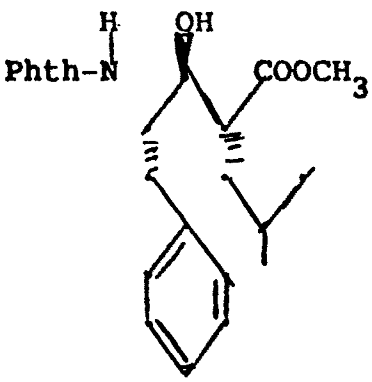

+ isomer

2R,3S,4S 2S,3R,4S

3a 3b

Diasterioselectivity favors by 95% formation of the 3a isomer, and the two diastereomers are thus readily separated by chromatography.

The configurations of the chiral centers can be established as follows: treatment of the phthalimido methyl esters 3a and 3b with excess hydrazine gives the respective amino acyl hydrazides 4a and 4b, which are then converted in a two step/one pot procedure to the corresponding lactams 5a and 5b, to which stereochemical assignments can be made based on PMR analysis. These reactions may be illustrated as follows:

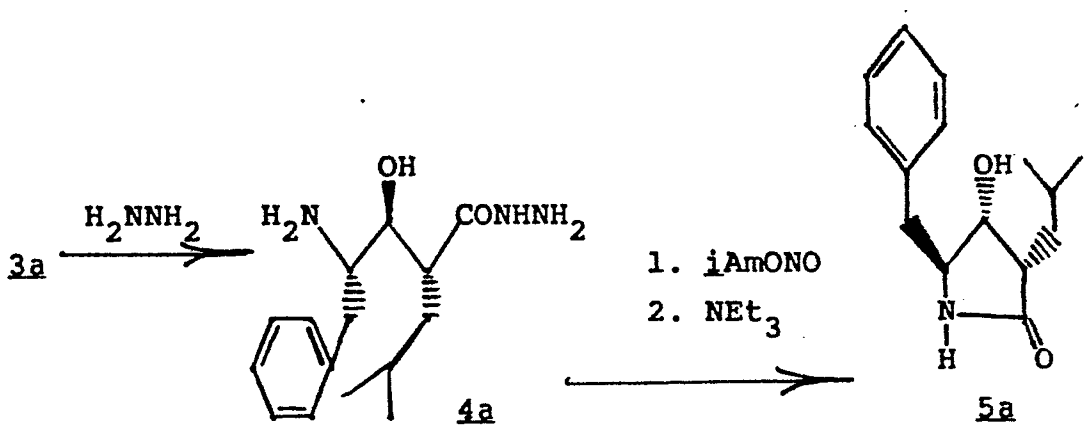


3a 4a 5a

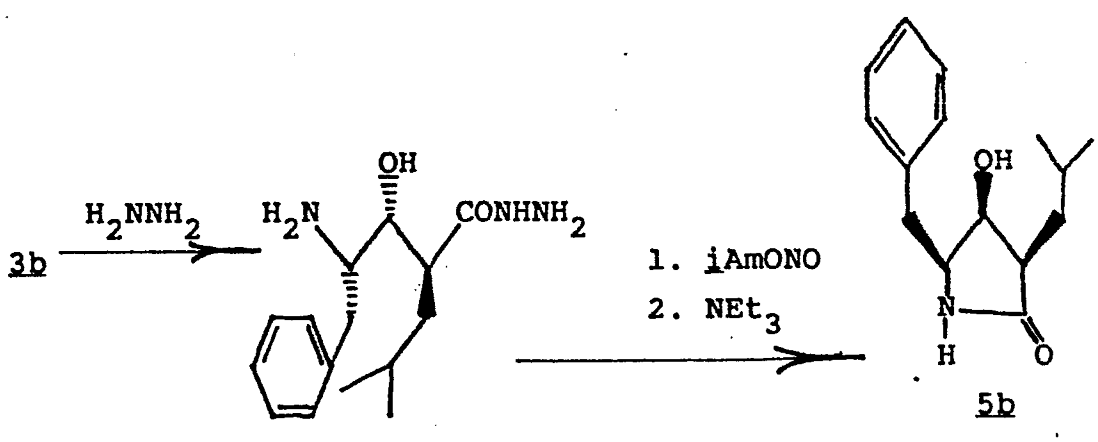


3b 4b 5b

Alternatively, the benzyl ester 6, rather than the methyl ester, may be used to form the ketone silylacetal 7, which can then be reacted with phthalyl phenylalanine aldehyde and phthalyl leucine

aldehyde, for example, to give 8a and 8b, illustrated as follows:

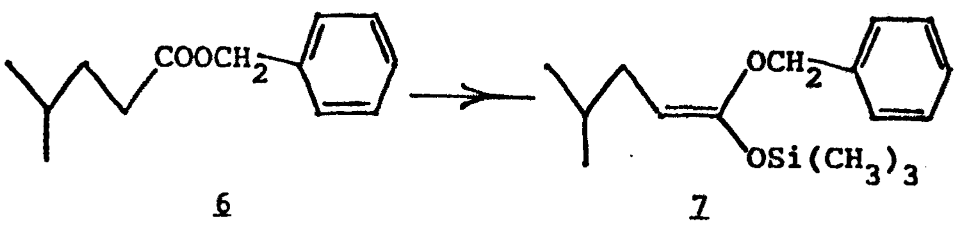

6 7

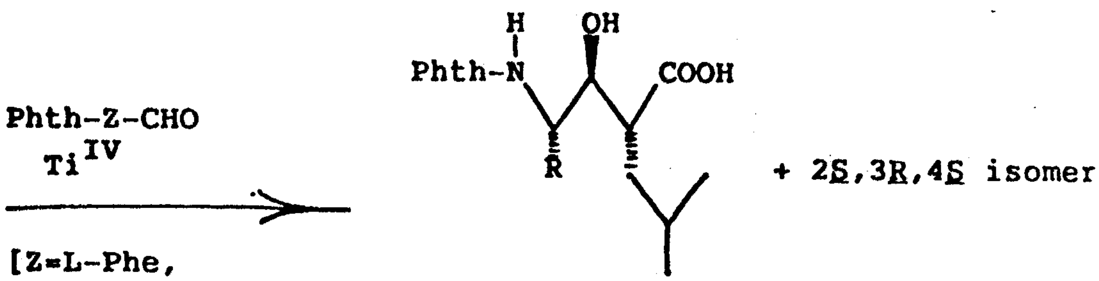


8a, R=benzyl
8b, R=iso-butyl

Separation of the isomers followed by hydrogenation gives a protected 2-substituted statine component which can be used to prepare peptides of Formula I in accordance with well-known methods of peptide synthesis.

Preparation of a renin inhibitory peptide of the present invention containing (2-i-Bu)-Sta may be schematically represented as follows:

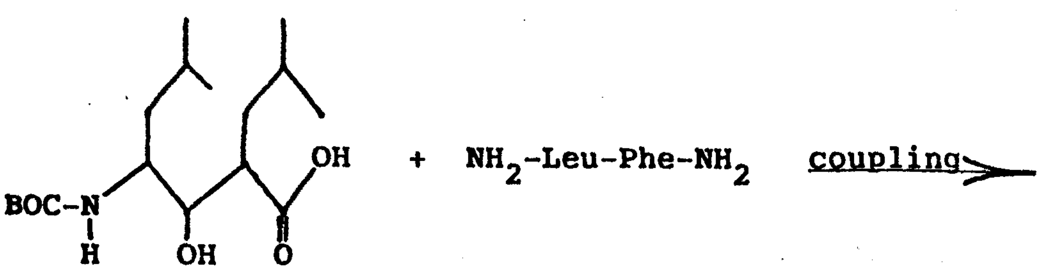

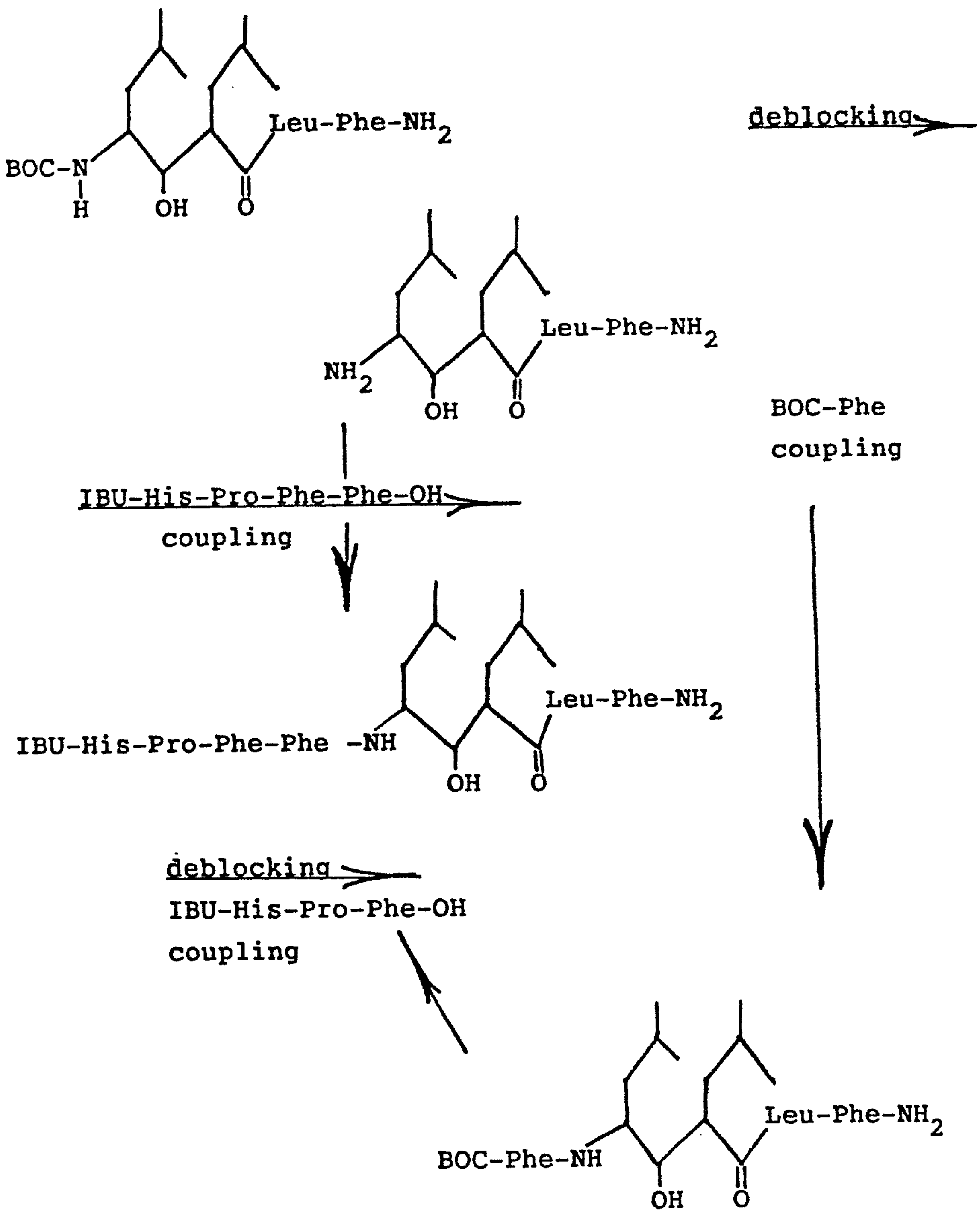


III. <u>Where the Component G Is the Amino Acid Statine Itself:</u> it may be prepared in accordance with the procedure described by Rich et al., <u>J. Org. Chem.</u> 43: 3624, 1978.

The phenyl analog of statine (3S,4S)-4-amino-3-hydroxy-5-phenylpentanoic acid (AHPPA) can be prepared in accordance with the procedure described by Rich et al., J. Med. Chem. 23: 27-33 (1980).

The cyclohexylalanine analog of statine, (3S,4S)-4-amino-5-cyclohexyl-3-hydroxypentanoic acid (ACHPA) can be prepared by catalytic hydrogenation (using $H_2$/Rh on alumina, or other sutiable catalyst) of the BOC-AHPPA, prepared as described in the paragraph immediately above. Alternatively, this and similar statine analogs can be prepared in accordance with the procedure described for statine, where the BOC-Leu starting material is replaced with the amino acid containing the desired side chain. Thus, BOC-ACHPA can also be prepared starting from BOC-L-cyclohexylalanine, itself prepared, for example, by catalytic reduction of BOC-Phe, in the same manner as described for BOC-AHPPA.

The novel inhibitory peptides of the present invention are prepared by using the solid phase sequential synthesis technique.

In the following description several abbreviated designations are used for the amino acid components, certain preferred protecting groups, reagents and solvents. The meanings of such abbreviated designations are given below in Table I.

TABLE I

| Abbreviated Designation | Amino Acid |
|---|---|
| Ala | L-alanine |
| Arg | L-arginine |
| Gly | L-glycine |
| His | D or L-histidine |
| Ile | L-isoleucine |
| Leu | L-leucine |
| Lys | L-lysine |
| Met | L-methionine |
| Orn | L-ornithine |
| Phe | L-phenylalanine |
| Ser | L-serine |
| Sar (N-methylglycine) | L-sarcosine |
| Thr | L-threonine |
| Trp | L-tryptophan |
| Tyr | L-tyrosine |
| Val | L-valine |

| Abbreviated Designation | Protecting Groups |
|---|---|
| BOC | tert-butyloxycarbonyl |
| CBZ | benzyloxycarbonyl |
| DNP | dinitrophenyl |
| OMe | methyl ester |

| Abbreviated Designation | Activating Groups |
|---|---|
| HBT | 1-hydroxybenzotriazole |

| Abbreviated Designation | Condensing Agents |
|---|---|
| DCCI | dicyclohexylcarbodiimide |
| DPPA | diphenylphosphorylazide |

| Abbreviated Designation | Reagents |
|---|---|
| TEA | triethylamine |
| TFA | trifluoroacetic acid |

| Abbreviated Designation | Solvents |
|---|---|
| A | ammonium hydroxide (conc.) |
| AcOH | acetic acid |
| C | chloroform |
| DMF | dimethylformamide |
| E | ethyl acetate |
| M | methanol |
| P | pyridine |
| THF | tetrahydrofuran |
| W | water |

The synthesis of the peptides of the present invention by the solid phase technique is conducted in a stepwise manner on chloromethylated resin. The resin is composed of fine beads (20-70 microns in diameter) of a synthetic resin prepared by copolymerization of styrene with 1-2 percent divinylbenzene. The benzene rings in the resin are chloromethylated in a Friedel-Crafts reaction with chloromethyl methyl

ether and stannic chloride. The Friedel-Crafts reaction is continued until the resin contains 0.5 to 5 mmoles of chlorine per gram of resin.

The amino acid selected to be the C-terminal amino acid of the linear peptide is converted to its amino protected derivative. The carboxyl group of the selected C-terminal amino acid is bound covalently to the insoluble polymeric resin support, as for example, as the carboxylic ester of the resin-bonded benzyl chloride present in chloromethyl-substituted polystyrene-divinylbenzene resin. After the amino protecting group is removed, the amino protected derivative of the next amino acid in the sequence is added along with a coupling agent, such as dicyclohexylcarbodiimide. The amino acid reactant may be employed in the form of a carboxyl-activated amino acid such as ONP ester, an amino acid azide, and the like. Deprotection and addition of successive amino acids is performed until the desired linear peptide is formed.

The selection of protecting groups is, in part, dictated by particular coupling conditions, in part by the amino acid and peptide components involved in the reaction.

Amino-protecting groups ordinarily employed include those which are well known in the art, for example, urethane protecting substituents such as benzyloxy-carbonyl (carbobenzoxy), p-methoxycarbobenzoxy, p-nitrocarbobenzoxy, t-butyoxycarbonyl, and the like. It is preferred to utilize t-butyloxycarbonyl (BOC) for protecting the α-amino group in the amino acids undergoing reaction at the carboxyl end of said amino acid. The BOC protecting group is

readily removed following such coupling reaction and prior to the subsequent step by the relatively mild action of acids (i.e. trifluoroacetic acid, or hydrogen chloride in ethyl acetate).

The OH group of Thr and Ser can be protected by the Bzl group and the -amino group of Lys can be protected by the INOC group or the 2-chlorobenzyloxy-carbonyl (2-Cl-CBZ) group. Neither group is affected by TFA, used for removing BOC protecting groups. After the peptide is formed, the protective groups, such as 2-Cl-CBZ and Bzl, can be removed by treatment with HF or by catalytic hydrogenation.

After the peptide has been formed on the solid phase resin, it may be removed from the resin by a variety of methods which are well known in the art. For example the peptide may be cleaved from the resin with hydrazine, by ammonia in methanol, or by methanol plus a suitable base.

Preparation of the novel inhibitory peptides of the present invention utilizing the solid phase technique is illustrated in the following examples, which however, are not intended to be any limitation of the present invention.

## EXAMPLE 1

BOC_Phe-His-Sta-
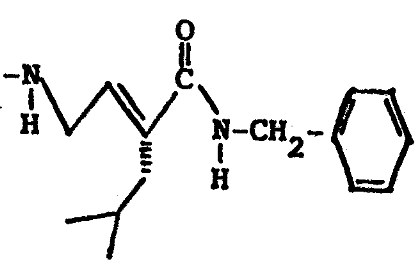

Step A.

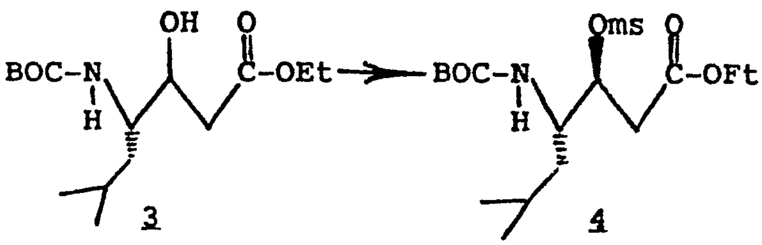

3 4

To an ice cold, stirred solution of BOC-statine ethyl ester 3 (2.60 g, 8.57 mmole) in 10 ml of pyridine is added *via* syringe 0.66 ml (8.57 mmole) of trifluoromethane sulfonyl chloride. Within minutes, pyridinium hydrochloride precipitates from solution. The reaction mixture is protected from moisture and allowed to stand at room temperature overnight. The reaction mixture is filtered and the filtrate concentrated to give a light orange oil. The crude product is filtered through 5-10 g of silica gel (ether elution) to give 3.0 g of a pale yellow oil which is used without further purification. Compound 4 is prone to hydrolysis to 3 and must be used without delay.

Step B.

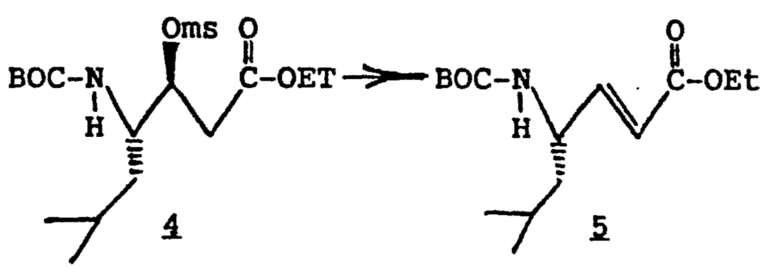

4 5

1,5-Diazabicyclo[4.3.0]non-5-ene (DBN) (0.94 ml, 7.54 mmole) is added in one portion to a

stirred solution of 5 (2.9 g, 6.85 mmole) in 25 ml of dry tetrahydrofuran. The reaction is slightly exothermic and within minutes, a thick white precipitate is formed. The reaction mixture is allowed to stir for several hours more and is then filtered. The filtrate is concentrated *in vacuo*. The residual oil is partitioned between ether and 10% citric acid solution. The organic phase is washed with citric acid (2 x 40 ml) and brine, then dried ($Na_2SO_4$) and concentrated. Flash chromatography on silica gel (7:3 hexane-ethyl acetate elution) provides the analytical sample as an oil (1.6 g). ir; pmr($CDCl_3$): olefinic protons 5.9 (d, J=17), 6.83 (d x d, J=17 and 5). J (coupling constant) is consistent with *trans* double bond.

Step C.

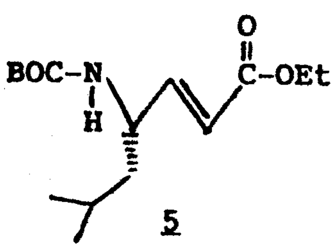
5

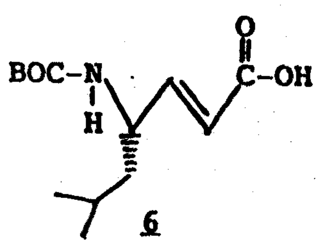
6

The α,β-unsaturataed ester 5 (1.5 g, 5.3 mmole) is dissolved in water/dioxane 20 ml (1:1 v/v) and treated with 7 ml (1.3 equivalents) of 1M sodium hydroxide solution. After 3 hours, 1 ml of 1M sodium hydroxide solution is again added. Dioxane is removed *in vacuo* after a total of 4 hours reaction time. The alkaline aqueous residue is diluted to 25 ml with water and washed with ether (2 x 25 ml).

The aqueous phase is acidified with 10% citric acid and extracted with ether and chloroform. The combined organic extracts are washed with brine and dried ($Na_2SO_4$). Concentration under reduced pressure affords 1.46 g of 6.

Step D.

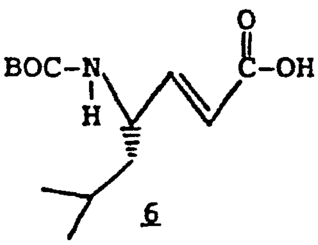

6

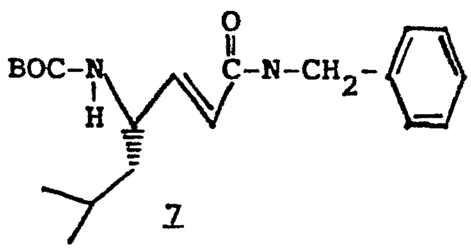

7

The acid 6 (400 mg, 1.6 mmole) is dissolved in 4 ml of methylene chloride under nitrogen. N-methylmorpholine (0.18 ml, 1.6 mmole) is added and the solution is cooled to -5°C. Isobutylchloroformate (0.21 ml, 1.6 mmole) is added and after 15 minutes, 0.22 ml (1.92 mmole) of benzylamine is added to the reaction mixture. After 30 minutes at -5°C the reaction mixture is warmed to room temperature, stirred 1 hour more and diluted with 70 ml of methylene chloride. The organic phase is washed in succession with 10% citric acid (2 x 30 ml), saturated sodium bicarbonate solution (2 x 30 ml), and brine. The organic extracts are dried ($Na_2SO_4$) and concentrated to yield 460 mg of a white solid identified as 7.

Step E.

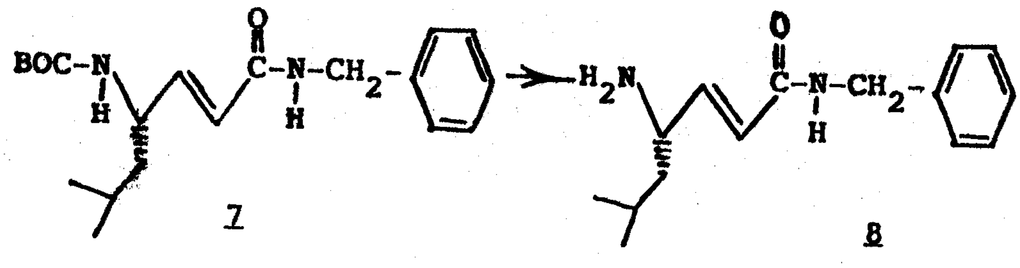

7 8

The BOC-amide 7 (240 mg, 0.69 mmole) is dissolved in 20 ml of ethyl acetate, cooled to 0°C and treated with a stream of hydrogen chloride gas for 1 hour. Solvent and excess reagent are removed under reduced pressure to afford 200 mg of a pale yellow solid.

Step F.

BOC-Phe-His-Sta-OEt → BOC-Phe-His-Sta-$NHNH_2$

1 2

The tripeptide ester BOC Phe-His-Sta-OEt 1 (520 mg, 0.89 mmole) is dissolved in 4 ml of methanol and treated with 2 ml (62 mmole) of 95% hydrazine. After 10 minutes at room temperature the reation mixture is concentrated <u>in vacuo</u> (0.1 Torr) to afford 420 mg of 2 as a tan powder.

Step G.

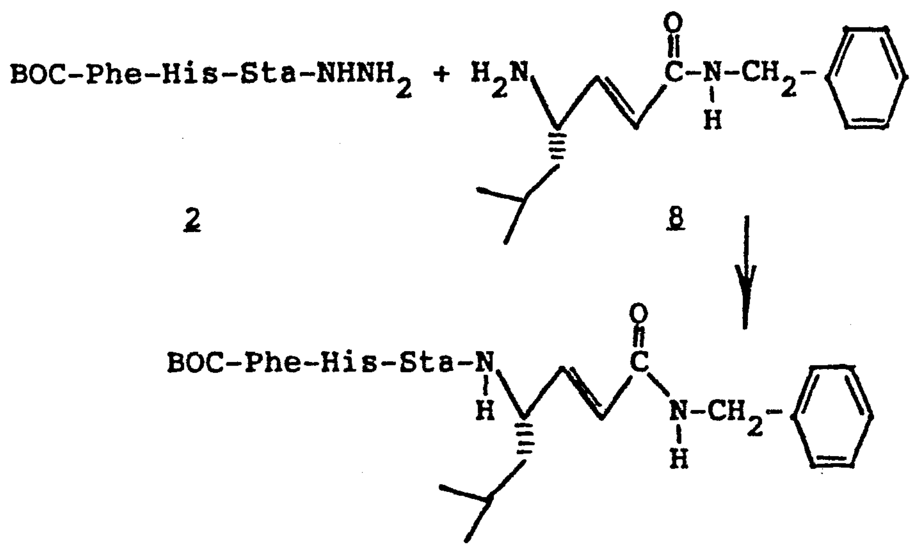

2 8

The hydrazide 2 (240 mg, 0.42 mmole) is dissolved in 2 ml of dry dimethylformamide under nitrogen. The solution is cooled to -20°C and the pH of the reaction mixture adjusted to approximately 0.5-1.0 with tetrahydrofuran saturated with hydrogen chloride. Isoamyl nitrite is then added in 50 µl increments at 15-20 minute intervals until a positive potassium iodide-starch test is obtained (250 µl total). The amine salt 8 (190 mg, 0.67 mmole) is dissolved in 2 ml of dimethylformamide and added to the reaction mixture. After addition is complete, the pH of the reaction mixture is adjusted to 7.5-8.0 with triethylamine and the reaction mixture is allowed to stir at -20°C for 20 hours. The reaction is filtered and the filtrate concentrated. The resulting residue is partitioned between ethyl acetate and water. The organic phase is washed in succession with 10% citric acid solution (2 x 50 ml), 50% sodium bicarbonate solution (2 x 50 ml), and

brine. The organic extracts are dried ($Na_2SO_4$) and rotoevaporated to yield 140 mg of a yellow semi-solid. Chromatography on silica gel (80:10:1 $CHCl_3$-ethanol-ammonia elution) affords the title compound as a pale yellow solid.

## EXAMPLE 2

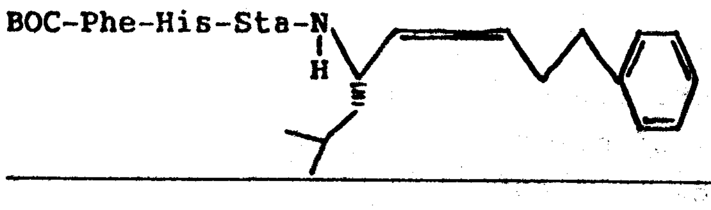

Step A.

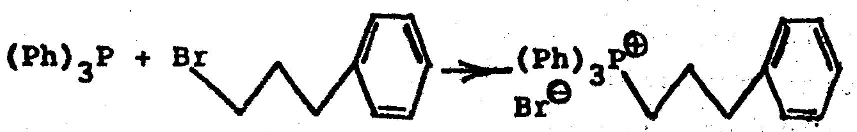

2 3

3-Phenyl-1-bromopropane (19 ml, 72.8 mmole) and triphenyl phosphine (19.11 g, 72.8 mmole) are combined at room temperature and immersed in a preheated oil bath at 150°C. Heating is continued for 1.5 hours at 150-160°C. The dark, brown solution is cooled and diluted with 200 ml of acetone. The acetone is decanted and the residue is triturated with hot ethyl acetate. In this way, an off-white solid is obtained which on further washing with ethyl acetate gives 24.2 g of 3 as a white powder, m.p. 203-207°C.

Step B.

BOC-Leu-CHO + 3 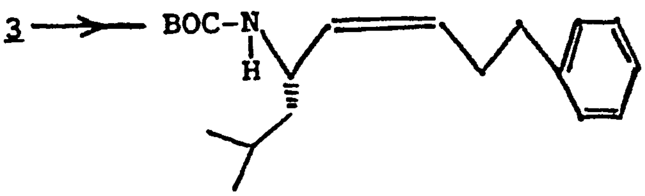

1 4

A rapidly stirred suspension of 3 (1.82 g, 3.95 mmole) in 15 ml of dry tetrahydrofuran is treated dropwise under nitrogen at 0°C with n-butyl lithium (1.4N, 2.82 ml, 3.95 mmole). The reaction mixture, which becomes homogeneous and colors to dark brown, is cooled to -78°C and treated with 5 ml of tetrahydrofuran containing 0.5 g (2.32 mmole) of BOC-leucine aldehyde 1. After four hours at -78°C the reaction mixture is warmed to -10°C for 1 hour and then quenched with saturated ammonium chloride solution. The reaction is partitioned between ether and brine. The organic phase is then washed with 10% citric acid solution (3 x 20 ml), 50% sodium bicarbonate solution (3 x 20 ml), and brine. Rotoevaporation of the dried ($Na_2SO_4$) extracts affords 0.42 g of crude product as a yellow oil. The analytical sample is obtained by chromatography of the crude product on silica gel (hexane-ethyl acetate 9:1 elution).

Step C.

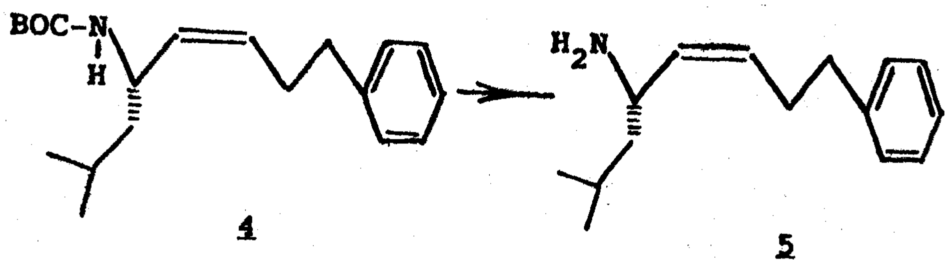

4 5

The BOC-olefin 4 (410 mg, 1.29 mmole) is dissolved in 20 ml of ethyl acetate, cooled to 0°C, and treated with hydrogen chloride gas for 1 hour. Concentration of the reaction mixture *in vacuo* and then under high vacuum gives the HCl salt 5 as a beige solid (310 mg).

Step D.

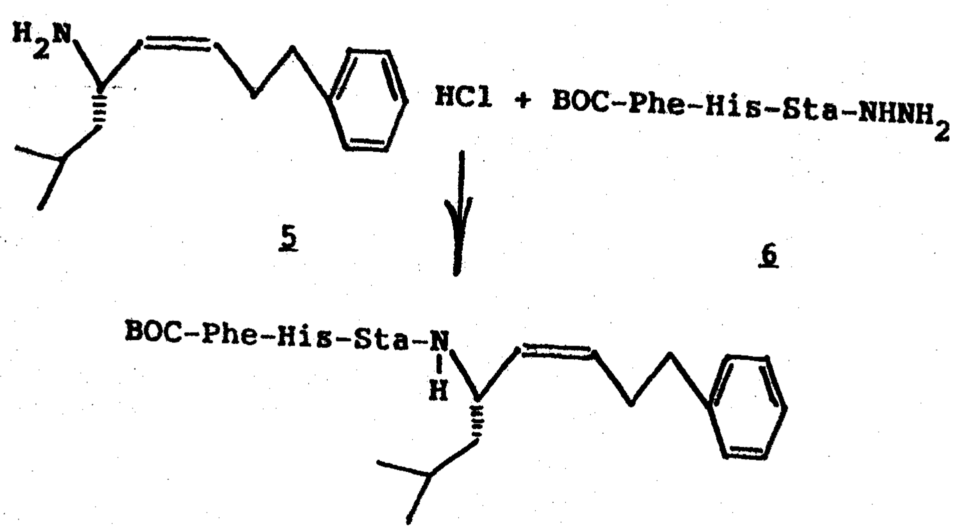


5 6

BOC Phe-His-Sta-NHNH$_2$ (240 mg, 0.42 mmole) [obtained as described in Example 1, Step F] is converted to the corresponding BOC-Phe-His-Sta-N$_3$ (azide) with isoamylnitrite (200 µl) [using

identical reaction conditions as described in Example 1, Step G]. The amine salt 5 (160 mg, 0.63 mmole) is then added [and the reaction is carried out and worked-up as described in Example 1, Step G]. The analytical sample (70 mg) is obtained after silica gel chromatography (80:10:1 $CHCl_3$-ethanol-ammonia elution) as a pale yellow solid.

## EXAMPLE 3

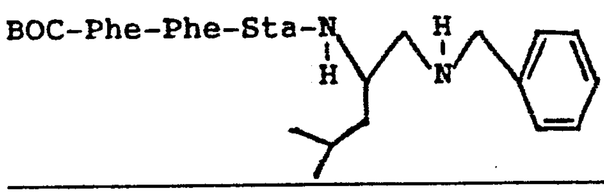

Step A.

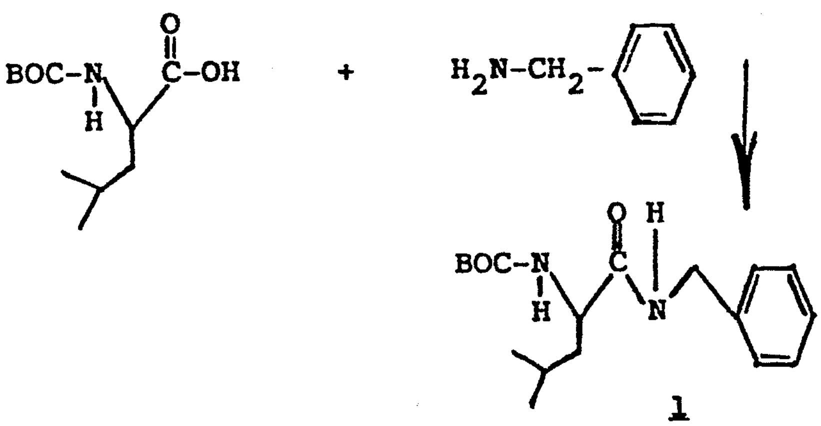

1

N-Methylmorpholine (9.14 ml, 83.2 mmole), and BOC-leucine hydrate (20.0 g, 83.2 mmole) are dissolved in $CH_2Cl_2$ (200 ml) and the solution cooled to -5°C. Isobutylchloroformate (10.8 ml, 83.2 mmole) is added and the solution stirred 15 min. Benzylamine (10.9 ml, 99.8 mmole) is added and the solution stirred 15 min. The solution is warmed to

25°C ( 30 min.) and dichloromethane (300 ml) added. The organic layer is washed with 10% citric acid (2 x 150 ml), water (1 x 150 ml), 10% sodium bocarbonate (2 x 150 ml), and brine (2 x 150 ml); dried over $Na_2SO_4$; and filtered. The filtrate is evaporataed under reduced pressure and the residue dried at 25°C in a vacuum oven to give 24.75 g (93% yield) of 1 as a waxy colorless solid.

Step B.

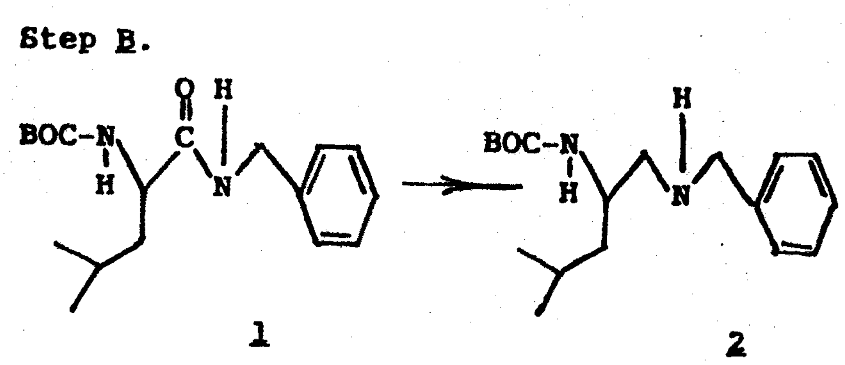

1 2

Compound 1 from Step A. (1.0 g, 3.1 mmole) is dissolved in tetrahydrofuran (6.25 ml) and the solution cooled to -25°C. Diborane (6.25 ml of 1M tetrahydrofuran solution, 6.25 mmole) is added dropwise and the solution stirred 48 hours at -10°C. Methanol (5 ml) is added and the reaction stirred at 25°C for 16 hours. The solvent is removed under reduced pressure and the residue treated with methanol and restripped (3X). Flash chromatography of the final residue using silica gel eluted with 35% ethyl acetate in hexane gives 380 mg (40% yield) of 2 as a light yellow oil.

Step C.

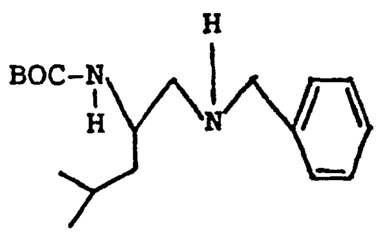

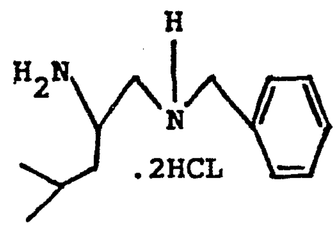

2 3

Protected amine 2 (540 mg, 1.8 mmol) is dissolved in ethyl acetate (10 ml). The solution is cooled to 0°C, saturatad with HCl (g), and stirred 15 min. The solvent is removed *in vacuo*. The residue is treated with ethyl acetate and restripped (4X) to give a quantitative (490 mg) yield of 3 as an off-white solid.

Step D.

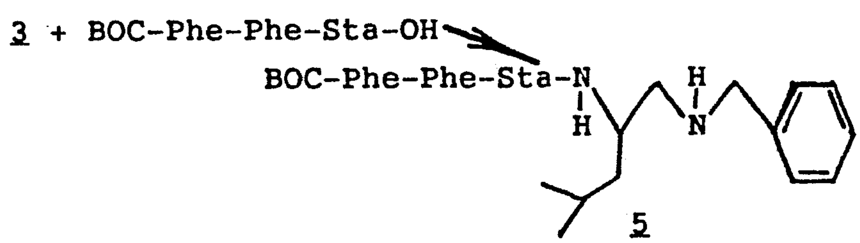

3 + BOC-Phe-Phe-Sta-OH

5

BOC-Phe-Phe-Sta-OH (240 mg, 0.421 mmole), diamine dihydrochloride 3 (130 mg, 0.466 mmole), 1-hydroxybenzotriazole hydrate (HBT) (62.9 mg, 0.466 mmole), and 1-ethyl-3-(3-dimethylaminopropyl) carbodiimide hydrochloride (EDC) (895 mg, 0.466 mmole) are dissolved in degassed dimethylformamide (4 ml) under a nitrogen atmosphere. The pH of the

solution is adjusted to 9.0-9.5 with triethylamine (0.30 ml, 2.16 mmole). After stirring 24 hours, a second portion of HBT (7 mg, .052 mmole) and EDC (9 mg, .047 mmole) is added and the suspension stirred 6 hours. The dimethylformamide is removed *in vacuo*. The residue is treated with 10% citric acid and extracted with ethyl acetate (3X). The organic layers are combined, washed with $H_2O$ (1X), 10% aqueous sodium bicarbonate (2X), and brine (1X), dried over $MgSO_4$, filtered, and stripped under reduced pressure to give 280 mg of a white foam. Flash chromatography on silica gel with 150/10/1/1 of dichloromethane/methanol/water/acetic acid gives the desired product 5 (200 mg, 62.7% yield) as a white foam.

## EXAMPLE 4

BOC-Phe-Phe-Sta-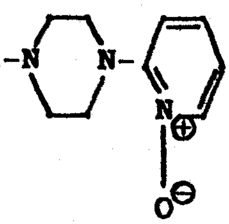

Step A.

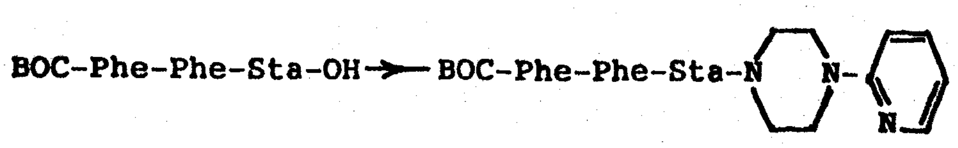


To a solution of 1 ml of dimethylformamide at 0° is added in succession BOC-Phe-Phe-Sta-OH (114

mg, 0.2 mmole), 2-pyridylpiperazine 33.5 μl, 35 mg, 0.22 mmole) diphenylphosphonylazide (47.5 μl, 60.7 mg, 0.22 mmole), and sodium bicarbonate (84 mg, 1 mmole). The resulting suspension is protected from moisture and stirred at 0° for 12 hours. More diphenylphosphonylazide is added (47.5 μl, 0.22 ml) and stirring continued at 0°C. After 2 days the reaction mixture is filtered and the filtrate concentrated *in vacuo*. The residue is chromatographed on silica gel (90:10:1:0.1 chloroform/methanol/water/acetic acid elution) to give 125 mg of the analytical sample as a white solid.

Step B.

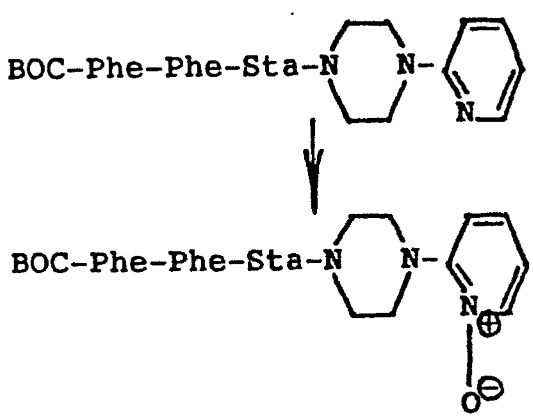

The tri-peptide of Step A. (41 mg, 0.06 mmole) is dissolved in 5 ml of chloroform and the resulting solution is treated with 20 mg of tech. grade m-chloroperbenzoic acid (85%). The reaction mixture is allowed to stand for 19 hours at room temperature and then the solvent is removed under reduced pressure. The residue is chromatographed on silica gel (chloroform-ethanol-ammonia 80:10:1 elution). The material with $R_f$ value of 0.24 is

isolated to provide the analytical sample as a white solid (32 mg).

Another embodiment of the present invention is peptides of the following formula:

$$A^{\circ}-B^{\circ}-D^{\circ}-E^{\circ}-G^{\circ}$$
$$(I^{\circ})$$

wherein:

A° is hydrogen, or $R^{\circ}_{a}$-, $R^{\circ}_{b}CO$ or $R^{\circ}_{b}SO_2$- where $R^{\circ}_{a}$ and $R^{\circ}_{b}$ are alkyl, alkenyl, alkynyl, cycloalkyl, aryl, heterocyclic, aryloxy alkyl, heterocyclic oxy alkyl, aryl alkyl, heterocyclic alkyl, heterocyclic oxyalkyl, and $R^{\circ}_{a}$ and $R^{\circ}_{b}$ may be substituted with up to three members selected from amino, hydroxy, alkyl, halo and alkoxy groups.

B° and D° can independently be absent or can be

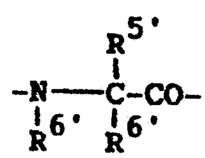

provided that only one of B° or D° is absent.

E° is 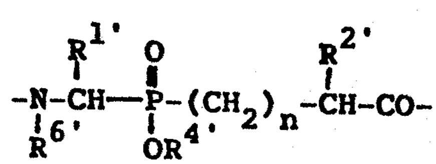

G° is $-R^{3'}$ or is 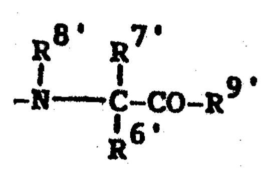

$R^{1'}$ is alkyl, alkenyl, alkynyl, cycloalkyl, cycloalkenyl, cycloalkyl alkyl, aryl alkyl, heterocyclic, heterocyclic each of which may be substituted with up to three members selected from alkyl, halo, amino and alkoxy groups.

n' is 0 or 1.

$R^{2'}$ is hydrogen, alkyl, alkenyl, alkynyl, cycloalkyl, cycloalkenyl, cycloalkyl alkyl, aryl, aryl alkyl, heterocyclic, heterocyclic alkyl, each of which may be substituted with up to three members selected from alkyl, hydroxy, halo, amino, alkylamino, dialkylamino, and alkoxy.

$R^{3'}$ is OH, $NH_2$, $NHR_a^{3'}$, $NR_a^{3'}N_b^{3'}$, $OR_c^{3'}$

where $R_a^{3'}$, $R_b^{3'}$, and $R_c^{3'}$ are separately alkyl, alkenyl, alkynyl, cycloalkyl, cycloalkenyl, cycloalkyl alkyl, aryl, aryl alkyl, heterocyclic, heterocyclic alkyl, each of which may be substituted with up to three members selected from amino, alkyl amino, dialkyl amino, trialkyl ammonium, hydroxy, alkoxy, aryloxy, aryl alkoxyl, or halo.

$R_c^{3'}$ may also be $R_d^{3'}$-CO-V'-$CR_e^{3'}R_f^{3'}$ wherein $R_d^{3'}$ is alkyl or aryl; $R_e^{3'}$ and $R_f^{3'}$ are hydrogen or alkyl; V' is -O- or -NH-.

$R^{4'}$ is hydrogen, alkyl, alkenyl, alkynyl, aryl, arylalkyl, each of which may be substituted with up to three members selected from amino, alkyl amino, dialkyl amino, trialkyl

ammonium, hydroxy, alkoxy, halo or alkyl groups. $R^{4'}$ may also be $R_a^{4'}$-CO-V'-$CR_b^{4'}R_c^{4'}$ wherein $R_a^{4'}$ is alkyl, alkenyl or alkynyl, or aryl; $R_b^{4'}$ and $R_c^{4'}$ are hydrogen, alkyl, alkenyl, or alkynyl; V' is -O- or -NH-.

$R^{5'}$ is hydrogen, alkyl, alkenyl, alkynyl, cycloalkyl, cycloalkenyl, aryl, cycloalkyl alkyl, aryl alkyl, heterocyclic, heterocyclic alkyl, aryloxy alkyl, heterocyclic oxy alkyl, heterocyclic oxy, each of which may be substituted with up to three members selected from amino, alkyl amino, dialkyl amino, trialkyl ammonium, hydroxy, alkoxy, carboxy, alkoxycarbonyl, alkylthio, arylthio, thiol, guanidino, carboxamido and $C_2$-$C_6$ alkanoylamino groups.

$R^{6'}$ is hydrogen, methyl.

$R^{7'}$ can be $R^{5'}$ and taken together with $NR^{8'}$ may be a cyclic amino acid of formulas:

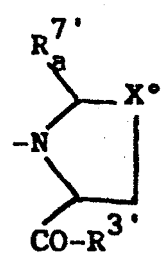
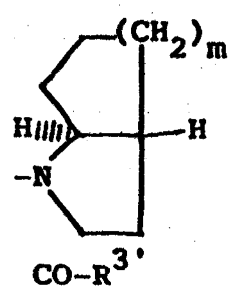
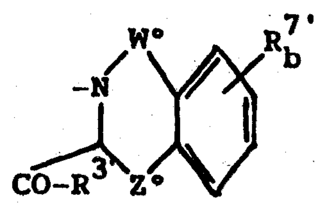

where $R_a^{7'}$ is hydrogen, phenyl, hydroxyphenyl; X is -S- or $-CH_2-$ or $-CH-R_b^7$; m is 1 or 2; and $R_b^7$ is cyclohexyl, phenylthio; W° and Z° are single bonds or $-CH_2$.

$R^{8'}$ is hydrogen, methyl and cycloalkyl including cyclopentyl and indanyl, such that when $R^{8'}$ is cycloalkyl, $R^{6'}$ and $R^{7'}$ are both hydrogen.

$R^{9'}$ is hydroxy, $OR_a^{3'}$, $-NH_2$, $-NHR_a^{3'}$, $NR_a^{3'}R_b^{3'}$, where $R_a^{3'}$ and $R_b^{3'}$ are as defined above, such that when A° and B° are both absent, $R^{9'}$ can be 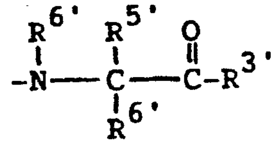

and pharmaceutically acceptable salts thereof.

In the above definitions, the terms alkyl or alk, alkenyl, alkynyl, include hydrocarbon groups of up to 8 carbon atoms which groups may be straight or branched chain. Preferred alkyl or alk groups have 1-4 carbon atoms. Preferred alkenyl and alkynyl groups have 3 to 6 carbon atoms.

The term halo means fluoro, chloro, bromo and iodo.

The term aryl represents hydrocarbon aryl of up to 10 carbon atoms exemplified by phenyl, naphthyl, biphenyl and cycloalkyl-fused derivatives thereof such as indanyl and tetralinyl.

The term heterocyclic represents substituted and unsubstituted 5- or 6-membered ring containing from one to three heteroatoms selected from the group consisting of nitrogen, oxygen and sulfur having varying degrees of unsaturated wherein the nitrogen and sulfur heteroatoms may optionally be oxidized and the nitrogen atom be quaternized, and including any bicyclic group in which any of the above heterocyclic rings is fused to a benzene ring. Heterocyclic

groups in which nitrogen is the heteroatom are preferred; and of these, those containing a single nitrogen atom are more preferred. Fully saturated heterocyclic groups are also preferred. Thus, piperidine is a preferred heterocyclic substituent. Other preferred heterocyclic groups are pyrrolidinyl, imidazolidinyl, morpholinyl, tetrahydrofuranyl, thienyl, pyrimidinyl, imidazolyl, indolyl, quinolinyl, isoquinolinyl, benzothienyl and the like. Where the heterocyclic group is substituted, the preferred substituent is aryl-$C_1$-$C_4$ alkyl.

Cycloalkyl and cycloalkenyl groups contain up to 12 carbon atoms and may be bridged. They include cyclopropyl, cyclopentyl, cyclohexyl, cyclohexenyl, nobornenyl, adamantyl, bicyclo[3.3.0]-octanyl perhydronaphthyl, and the like.

Other embodiments of the present invention are

a) compounds of the formula

$$A°\text{-}D°\text{-}E°\text{-}G°$$
$$(I^a)$$

and pharmaceutically acceptable salts thereof,

b) compounds of the formula

$$A°\text{-}E°\text{-}G°$$
$$(I^a)$$

and pharmaceutically acceptable salts thereof, and

c) compounds of Formula I, I°, $I^a$ and $I^b$ where 1.) X' is $OR_4'$ and $R_4'$ is other than H, and preferably is $C_1$-$C_6$ alkyl or 2.) $R_4'$ in the E° unit is other than H, and preferably is $C_1$-$C_6$ alkyl.

The peptides of the present invention may also be described in terms of common amino acid components and closely related analogs thereof, in accordance with formula (I):

A°-B°-D°-E°-G°

(I)

werein A° has the same meaning as described above: A° and B° can each be, for example, Ala, Leu, Phe, Tyr, Trp, Met, HomoPhe, BishomoPhe, HomoTyr, HomoTrp, 3-(1-Naphthyl)Ala, 3-(2-Naphthyl)Ala, 5-MethoxyTrp, N-MethylPhe, N-Methyl-HomoPhe, α-methylPhe.

It will be understood that closely related analogs of the above common amino acids, for examples, aliphatic amino acids in addition to Ala, Val, Leu, and Ile, such as alpha amino butyric acid (Abu) are included in the broad description of the peptides of the present invention represented by Formula (I) and its definitions.

In the above definitions, when A° is R°CO, and A° is alkyl, aryl, aryl alkyl, heterocyclic or heterocyclic alkyl, these groups being optionally substituted by amino, $C_2$-$C_6$ alkanoylamino, hydroxy, alkyl, alkoxy, alkoxycarbonyl, halo, or nitro; and B° is absent and D° is

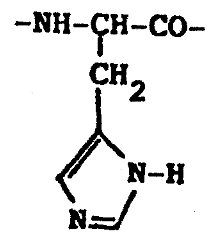

and in E°, $R^{6'}$ is hydrogen, $R^{1'}$ is isobutyl or sec-butyl, $R^{4'}$ is hydrogen, n' is 1, and $R^{2'}$ is hydrogen, then $R^{3'}$ may not be $NH_2$.

Preferred A° units include benzyloxycarbonyl, t-butoxycarbonyl, 1-naphthyloxyacetyl, and 1-naphthylacetyl.

Preferred B° amino acid units include phenylalanine, 3-(1-naphthyl)alanine, tryptophan, and homophenylalanine.

Preferred D° amino acid units include histidine, lysine, and phenylalanine.

Preferred E° units include those with the following formulas:

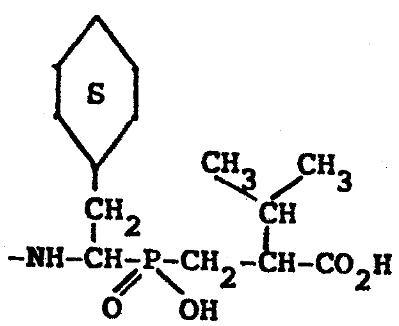 ,

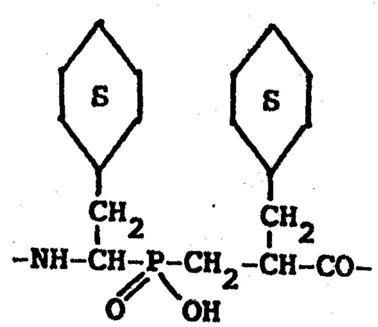

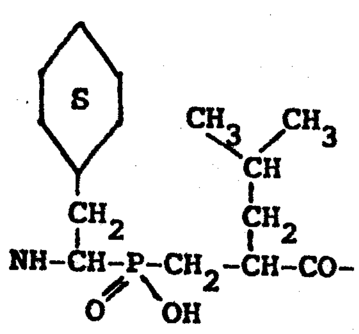 ,

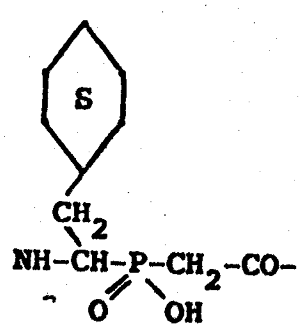

Preferred G° substituents include -OH, -OEt, $-NH_2$.

The amino acid units have asymmetric centers and occur as racemates, racemic mixtures and as individual diastereomers. All isomeric forms are included in the present peptides. In general, the preferred chiral forms of amino acid units B° and D° are the (L) forms. The stereocenters present in the E° unit of the peptides of Formula I° are in general of the chirality which corresponds to the naturally-occurring (L) amino acids. Thus, for example, the unit E° of the formula below possesses the stereochemistry shown in the preferred form:

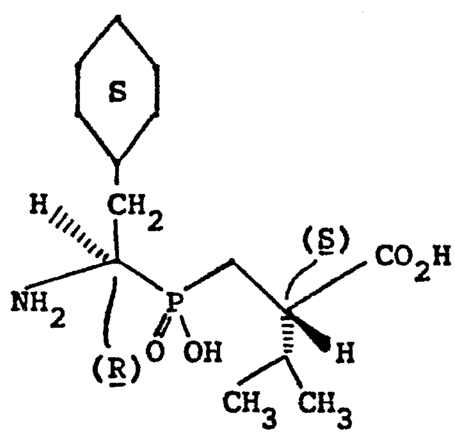

The following are illustrative examples of Formula I° peptides:

1. [N-(N-(N-carbobenzoxy-2-amino-3-(1-naphthyl)-propionyl)histidyl)-1-amino-2-cyclohexylethyl] 2-carboxy-4-methylpentylphosphinic acid
2. [N-(N-(N-carbobenzoxy-2-amino-3-(1-naphthyl)-propionyl)histidyl)-1-amino-2-cyclohexylethyl] 2-carboxy-3-methylbutylphosphinic acid

3. [N-(N-(N-t-butoxycarbonyl-2-amino-3-(1-naphthyl)-propionyl)histidyl)-1-amino-2-cyclohexylethyl] 2-carboxy-3-methylbutylphosphinic acid

4. [N-(N-(N-carbobenzoxy-2-amino-3-(1-naphthyl)-propionyl)histidyl)-1-amino-2-cyclohexylethyl] 2-carbomethoxy-4-methylpentylphosphinic acid

5. [N-(N-(N-carbobenzoxy-2-amino-3-(1-naphthyl)-propionyl)histidyl)-1-amino-2-cyclohexylethyl] 2-carboxamido-4-methylpentylphosphinic acid

6. [N-(N-(N-t-butoxycarbonyl-2-amino-3-(1-naphthyl)-propionyl)histidyl)-1-amino-2-cyclohexylethyl] 2-(N-benzyl)carboxamido-3-methylbutylphosphinic acid

7. [N-(N-carbobenzoxy-phenylalanyl-histidyl)-1-amino-2-cyclohexylethyl] 2-carboxy-4-methylpentyl-phosphinic acid

8. [N-(N-carbobenzoxy-phenylalanyl-histidyl)-1-amino-2-cyclohexylethyl] 2-(N-benzyl)carboxamido-4-methylpentylphosphinic acid

9. [N-(N-carbobenzoxy-phenylalanyl-phenylalanyl)-1-amino-2-cyclohexylethyl] 2-carboxamido-4-methyl-pentylphosphinic acid

10. [N-(N-(N-carbobenzoxy-2-amino-3-(1-naphthyl)-propionyl)histidyl)-1-amino-2-cyclohexylethyl]-carbomethoxymethylphosphinic acid

11. [N-(N-(N-carbobenzoxy-2-amino-3-(1-naphthyl)-propionyl)histidyl)-1-amino-2-cyclohexylethyl]-carboxymethylphosphinic acid

12. [N-(N-(N-carbobenzoxy-2-amino-3-(1-naphthyl)-propionyl)histidyl)-1-amino-2-cyclohexylethyl] carboxamidomethylphosphinic acid

13. [N-(N-(N-carbobenzoxy-2-amino-3-(1-naphthyl)-propionyl)histidyl)-1-amino-2-cyclohexylethyl] 2-carboxy-3-cyclohexylpropylphosphinic acid

14. [N-(N-carbobenzoxy-histidyl)-1-amino-2-cyclohexyl-ethyl] 2-carboxy-4-methylpentylphosphinic acid
15. [N-(phenylalanyl)-1-amino-2-cyclohexylethyl] 2-carboxy-3-methylbutylphosphinic acid
16. [N-(lysyl)-1-amino-2-cyclohexylethyl] 2-carboxy-3-methylbutylphosphinic acid
17. [N-(Nε-1-naphthyloxyacetyl-lysyl)-1-amino-2-cyclohexylethyl] 2-carboxy-3-methylbutylphosphinic acid
18. Methyl [N-(N-(N-carbobenzoxy-2-amino-3-(1-naphthyl)propionyl)histidyl)-1-amino-2-cyclohexyl-ethyl] 2-carboxy-4-methylpentylphosphinate
19. Methyl [N-(N-(N-carbobenzoxy-2-amino-3-(1-naphthyl)propionyl)histidyl)-1-amino-2-cyclohexyl-ethyl]carbomethoxymethylphosphinate
20. Ethyl [N-(N-3-phenylpropionyl-phenylalanyl)-1-amino-2-cyclohexylethyl] 2-carbomethoxy-3-methyl-butylphosphinate

The Formula I° compounds include many which bear acidic and/or basic groups; pharmaceutically acceptable salts of these compounds are included. Among the useful acid addition salts are the following: acetate, adipate, alginate, aspartate, benzoate, benzenesulfonate, bisulfate, butyrate, citrate, camphorate, camphorsulfonate, cyclopentane-propionate, digluconate, dodecylsulfate, ethane-sulfonate, fumarate, glucoheptanoate, glycero-phosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectinate, persulfate, 3-phenylpropionate, picrate, pivalate, propionate, succinate, tartrate, thiocyanate, tosylate, and undecanoate. Among the base salts include ammonium salts, alkali metal salts such as sodium and potassium salts, alkaline earth metal salts such as calcium and magnesium salts, salts with organic bases such as dicyclohexylamine salts, N-methyl-D-glucamine, and salts with amino acids such as arginine, lysine, and the like. Conventional methods for preparing these salts may be used. Also, basic nitrogen-containing groups can be quaternized with such agents as lower alkyl halides, such as methyl, ethyl, propyl, and butyl chloride, bromides and iodides; dialkyl sulfates like dimethyl, diethyl, dibutyl; and diamyl sulfates, long chain halides such as decyl, lauryl, myristyl and stearyl chlorides, bromides and iodides, aralkyl halides like benzyl and phenethyl bromides and others. Water or oil-soluble or dispersible products are thereby obtained.

The Formula I* may also be combined with one or more antihypertensive agents selected from the group consisting of diuretics, α and/or β-adrenergic blocking agents, CNS-acting antihypertensive agents, adrenergic neuron blocking agents, vasodilators, angiotensin I converting enzyme inhibitors, calcium channel blockers and other antihypertensive agents which are described above.

Typically, the individual daily dosages for these combinations can range from about one-fifth of the minimally recommended clinical dosages to the maximum recommended levels for the entities when they are given singly. Coadministration is most readily accomplished by combining the active ingredients into a suitable unit dosage form containing the proper dosages of each. Other methods of coadministration are, of course, possible.

The novel peptides of Formula I° possess an excellent degree of activity in treating hypertension and congestive heart failure. The Formula I° compounds also are expected to be orally active.

For these purposes the compounds of Formula I° may be administered orally, parenterally, by inhalation spray, or rectally in dosage unit formulations containing conventional non-toxic pharmaceutically acceptable carriers, adjuvants and vehicles. The term parenteral as used herein includes subcutaneous injections, intravenous, intramuscular, intrasternal injection or infusion techniques. In addition to the treatment of warm-blooded animals such as mice, rats, horses, dogs, cats, etc., the compounds of the invention are effective in the treatment of humans.

The pharmaceutical compositions containing Formula I° peptides may be provided in oral dosage forms e.g. tablets, capsules, solutions, dispersions, etc., the oral formulations are prepared using conventional procedures and compounding ingredients e.g. carriers, diluents, etc. The compositions may also be in the form of a sterile injectable preparation, for example as a sterile injectable aqueous or oleagenous suspension. This suspension may be formulated according to the known art using suitable dispersing or wetting agents and suspending agents. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution and isotonic

sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectibles.

The peptides of this invention may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols.

Dosage levels of the order of 0.1 to 4.0 grams per day parenterally are useful in the treatment of the above indicated conditions. Oral doses may be 3-10 times higher. For example, renin-associated hypertension and hyperaldosteronism are effectively treated parenterally by the administration of from 1.0 to 50 milligrams of the compound per kilogram of body weight per day.

The amount of active ingredient that may be combined with the carrier materials to produce a single dosage form will vary depending upon the host treated and the particular mode of administration.

It will be understood, however, that the specific dose level for any particular patient will depend upon a variety of factors including the activity of the specific compound employed, the age, body weight, general health, sex, diet, time of administration, route of administration, rate of

excretion, drug combination and the severity of the particular disease undergoing therapy.

Thus, in accordance with the present invention there is further provided a pharmaceutical composition for treating hypertension and congestive heart failure, comprising a pharmaceutical carrier and a therapeutically effective amount of a peptide of the Formula I°.

Also, in accordance with the present invention there is still further provided a method of treating hypertension and congestive heart failure, comprising administering to a patient in need of such treatment, a therapeutically effective amount of a peptide of the Formula I°.

The renin inhibitory peptides of Formula I° may also be utilized in diagnostic methods for the purpose of establishing the significance of renin as a causative or contributory factor in hypertension or congestive heart failure in a particular patient. For this purpose these peptides may be administered in a single dose of from 0.1 to 10 mg per kg of body weight.

Both in vivo and in vitro methods may be employed. In the in vivo method, a novel peptide of the present invention is administered to a patient, preferably by intravenous injection, although other routes of parenteral administration are also suitable, at a hypotensive dosage level and as a single dose, and there may result a transitory fall in blood pressure. This fall in blood pressure, if it occurs, indicates supranormal plasma renin levels.

Some of the peptides of Formula I, particularly those of Formula I° also have

angiotensin converting enzyme (ACE) inhibitor activity. This activity augments renin inhibition in lowering blood pressure and in treating congestive heart failure.

The compounds of the present invention, as characterized by Formula I°, may be viewed as a peptide segment A°-B°-D° linked through an amide bond to a phosphorus-containing component designated as E°. The preparation of these compounds is illustrated in the examples below and in general proceeds as follows:

1. a) Coupling of an amino-protected form of D° to E°, followed by b) amino protecting group removal from D°-E° and c) coupling A-B to the resulting amino group of D°-E°. Or:

2. Coupling of A°-B°-D°, prepared by known techniques as illustrated in the examples which follow, to E by known coupling procedures.

The phosphorus-containing component E°, as well as the amino acid side-chains of B° and D° may contain functionality which requires protection during the coupling reactions. Protecting groups, among those well-known in peptide synthesis, are chosen so as to be compatible with the coupling steps, yet easily removable afterwards. Among those utilized for amino group protection are the *t*-butoxycarbonyl (BOC), benzyloxycarbonyl (CBZ), 9-fluorenylmethyloxycarbonyl (FMOC), and benzyl groups. Carboxylic acids are protected as the methyl, ethyl, benzyl, or t-butyl esters. Phosphonic and phosphinic acids are protected as the methyl or ethyl esters. The peptide coupling procedures referred to above include those brought about by use

of dicyclohexylcarbodiimide/1-hydroxybenzotriazole and of disuccinimido oxallate (K. Takeda *et al.*, *Tetrahedron Lett.*, *24*, 4451-54 (1983)). In many cases, both carboxylic and phosphonic (or phosphinic esters) may be hydrolyzed along with amino protecting groups as the last step in the synthesis. In these cases, treatment of the phosphorus-containing peptide analog with 30% hydrobromic acid in acetic acid, with 6N hydrochloric acid, or with aqueous sodium hydroxide, followed by purification of the resulting deprotected material by ion-exchange chromatography or reverse-phase HPLC, provides the desired product. In instances where the phosphorus-containing component E possesses a carboxyl-terminal amide function, the fully coupled material A°-B°-D°-E° may be treated with 1 equivalent of lithium hydroxide (0.1 N) to hydrolyze selectively an ester function in E°. Standard coupling procedures may then be used to couple the resulting free carboxylic acid to an appropriate amine. This is followed by removal of the remaining protecting groups as described above. Alternatively the amide formation may be carried out prior to coupling of the component E° to the A°-B°-D° unit.

Preparation of the phosphorus-containing components E are carried out as illustrated in the examples to follow.

The 1-aminoalkylphosphonous acids used as starting materials in the examples below are prepared as illustrated in the examples and can be resolved to give optically active materials by the method of Baylis *et al.* (*J. Chem. Soc. Perkin Trans 1*, 2845-53 (1984)). Compounds derived from both the optically

active and racemic materials are claimed in the present invention.

The 1-aminoalkylphosphonic acids used in the examples below are prepared as described and can be resolved to give the optically active materials by the procedure of Kafarski et al., Can. J. Chem., 60, 3081-84 (1982), or can be prepared in optically active form by the method of Huber et al., Tetrahedron Lett., 3049-3052 (1979). Compounds derived from both the optically active and racemic materials are claimed in the present invention.

When the B° or D° units are N-methyl amino acids, or when the amino group of the unit E° which is to be coupled to A°-B°-D° bears an N-methyl group ($R^6$ = $-CH_3$), procedures well known in peptide synthesis are used to prepare the A°-B°-D° segments and to couple this unit to E° or E°-G°. In general, the mixed anhydride procedure (with pivaloyl chloride and N-methylmorpholine) is used, as illustrated by R. M. Wenger, Helv. Chem. Acta., 1984, 67, 502-525. N-methyl amino acids can be prepared by the method of M. J. O'Donnell et al., Tetrahedron Lett., 1984, 25, 3651.

The following schemes and examples illustrate the preparation of peptides of Formula I. In these schemes and examples, the following abbreviations are used: DPPA = diphenylphosphorylazide; DCC = dicyclohexylcarbodiimide; HOBT = 1-hydroxybenzotriazole hydrate; TFA = trifluoroacetic acid; CBZ = carbobenzoxy; BOC = t-butoxycarbonyl; DNP = 2,4-dinitrophenyl; FMOC = 9-fluorenylmethyloxycarbonyl; Bz = benzyl; HOAc = acetic acid; TMS-Cl = trimethylsilyl chloride; AIBN = azobis isobutyronitrile.

It will be understood that the following schemes outline representative examples of the preparation of peptides of Formula I and that similar peptides possessing alternative substituents can equally well be prepared by the routes outlined.

The component E° may be, for example, a phosphinic acid of the Formula II.

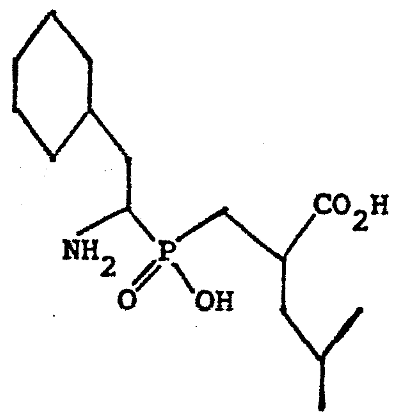

II

Peptide VIII containing II may be prepared as outlined in the scheme below:

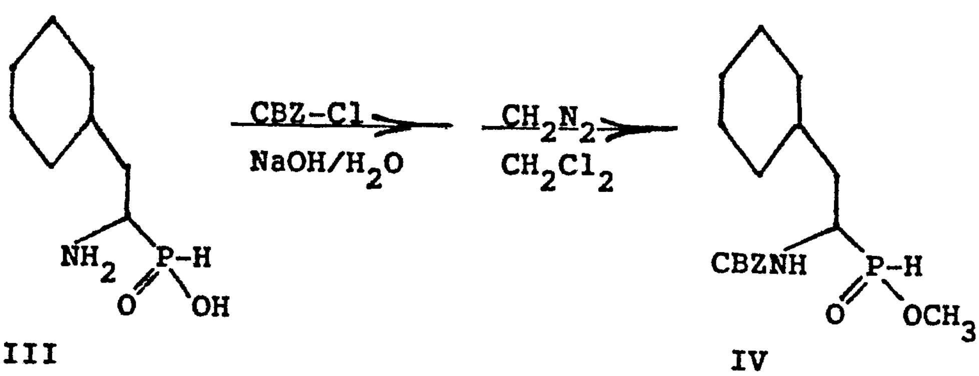


III IV

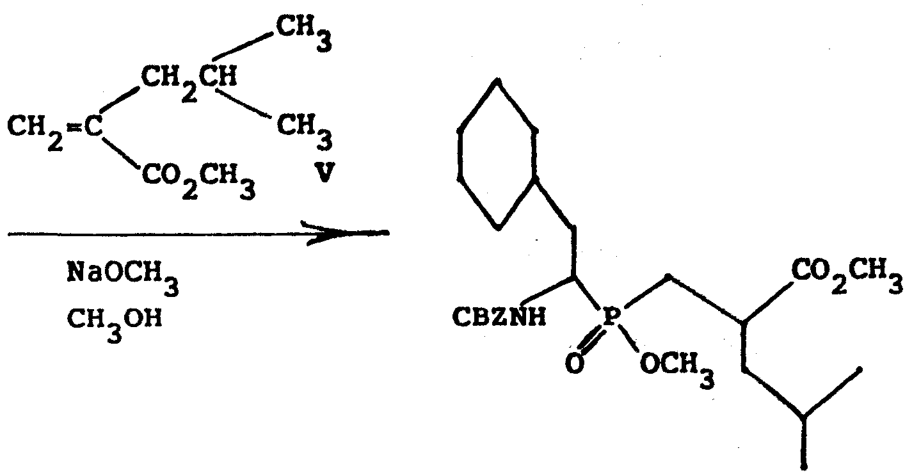


VI

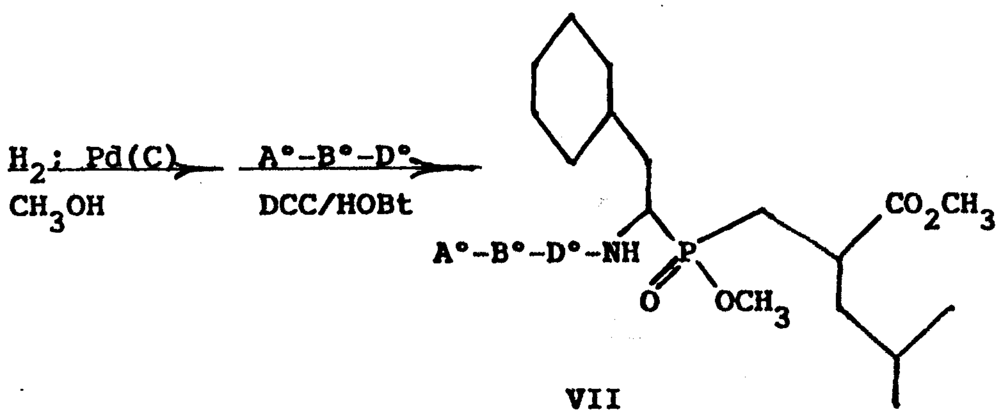


VII

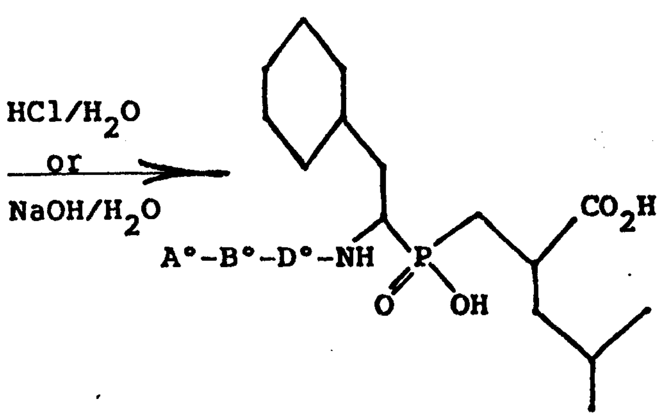


VIII

An alternative procedure for preparation of phosphinate VI is illustrated in the Scheme below:

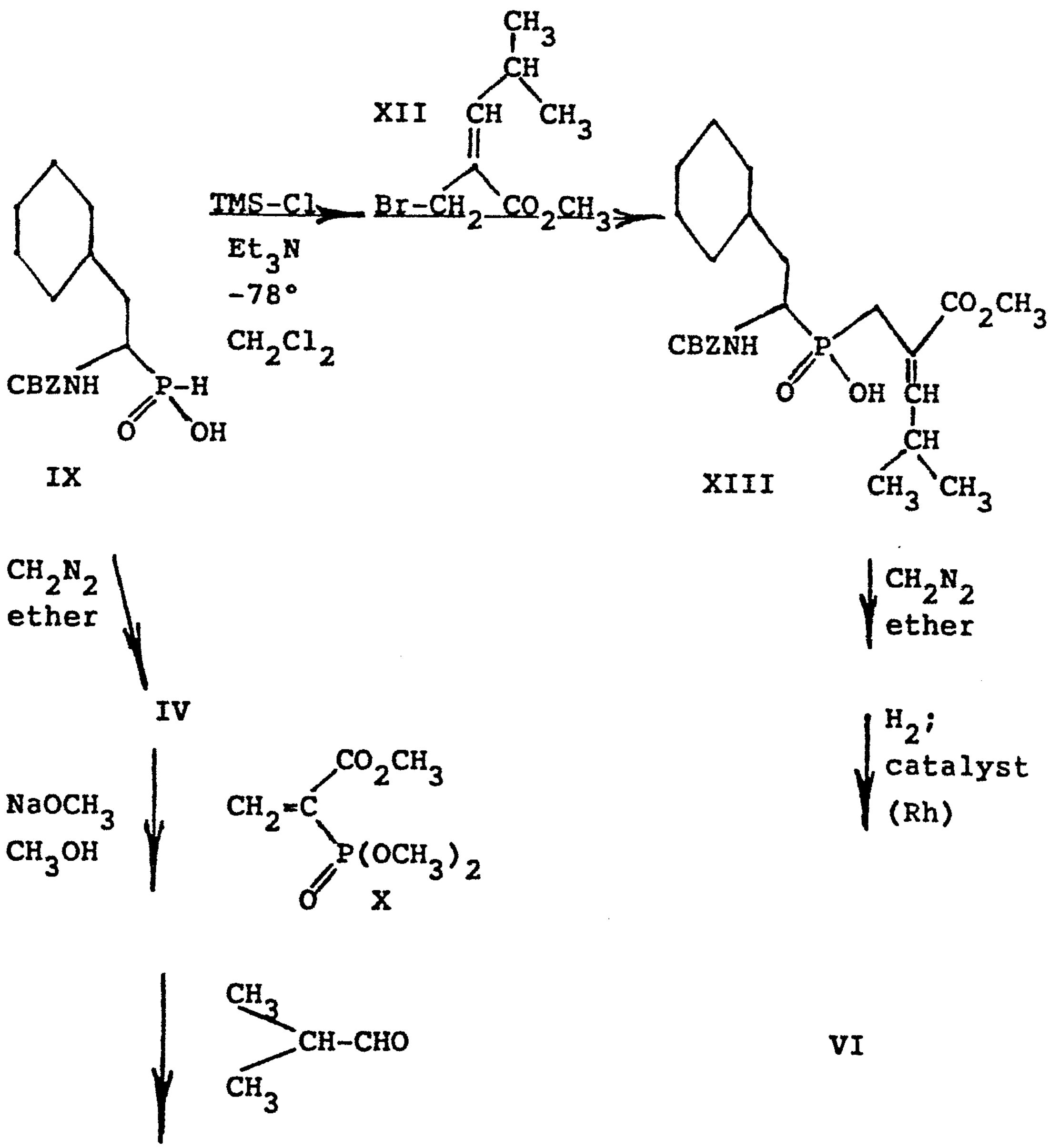


XII

IX

XIII

IV

X

VI

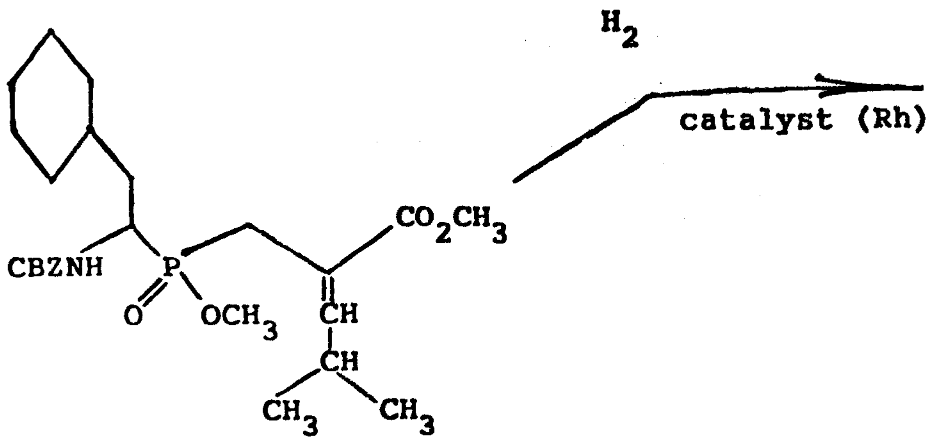


VI

XI

Component E may be for example a phosphinic acid of formula XIV:

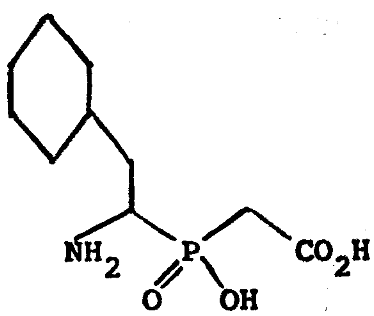

XIV

Preparation of protected forms XVII and XXII of XIV is illustrated in the schemes below:

Route A:

Ketene

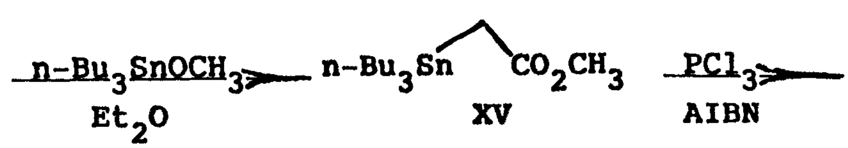


XV

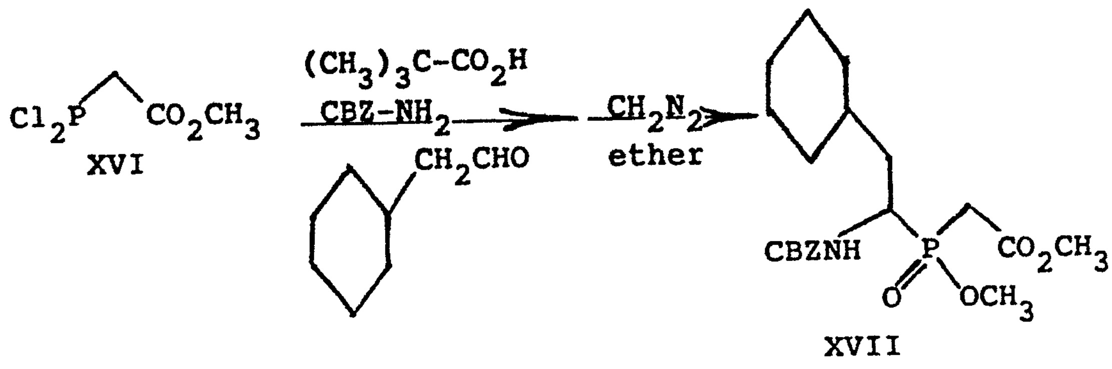


XVI

XVII

Route B:

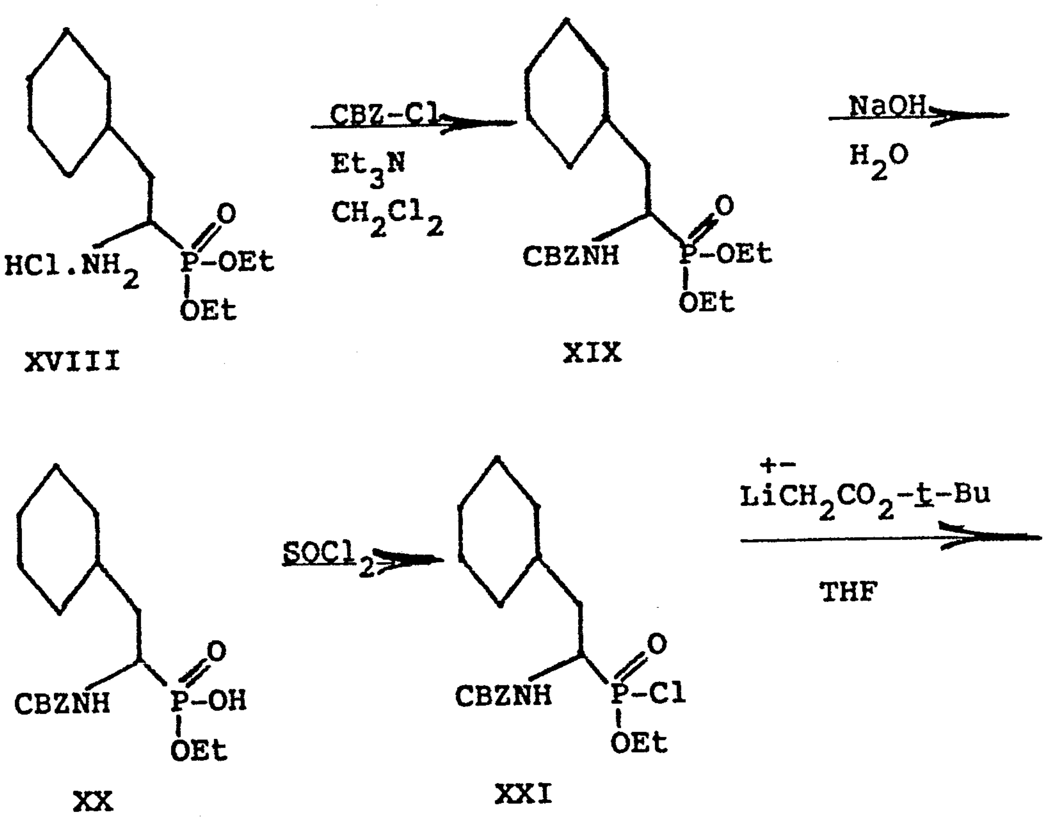


XVIII

XIX

XX

XXI

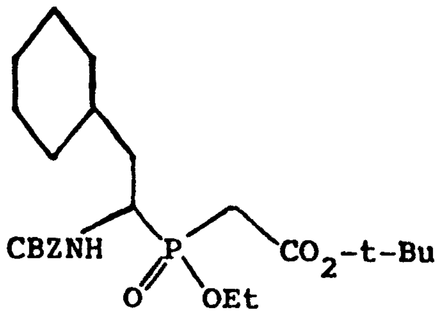

XXII

Route C:

IX

TMS-Cl

$Et_3N$

$CH_2Cl_2$

$BrCH_2CO_2CH_3$

$CH_2N_2$

ether

XVII

Phosphinic esters XVII and XXII may be coupled to an A°-B°-D° unit as illustrated above in preparation of peptide VIII.

The component E° may be a phosphinic ester of the formula XXIII:

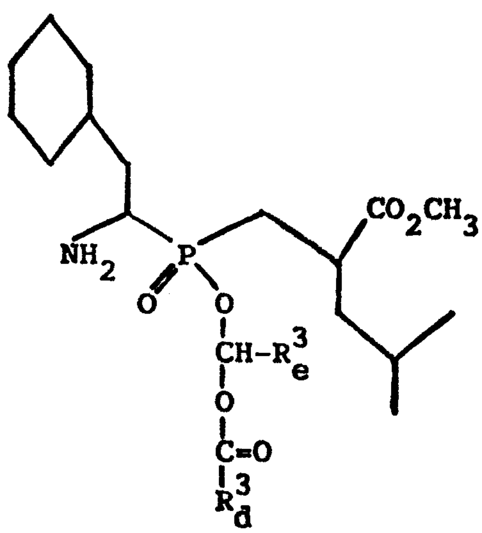

XXIII

Peptide XXVII containing such a component E may be prepared as outlined in the following scheme:

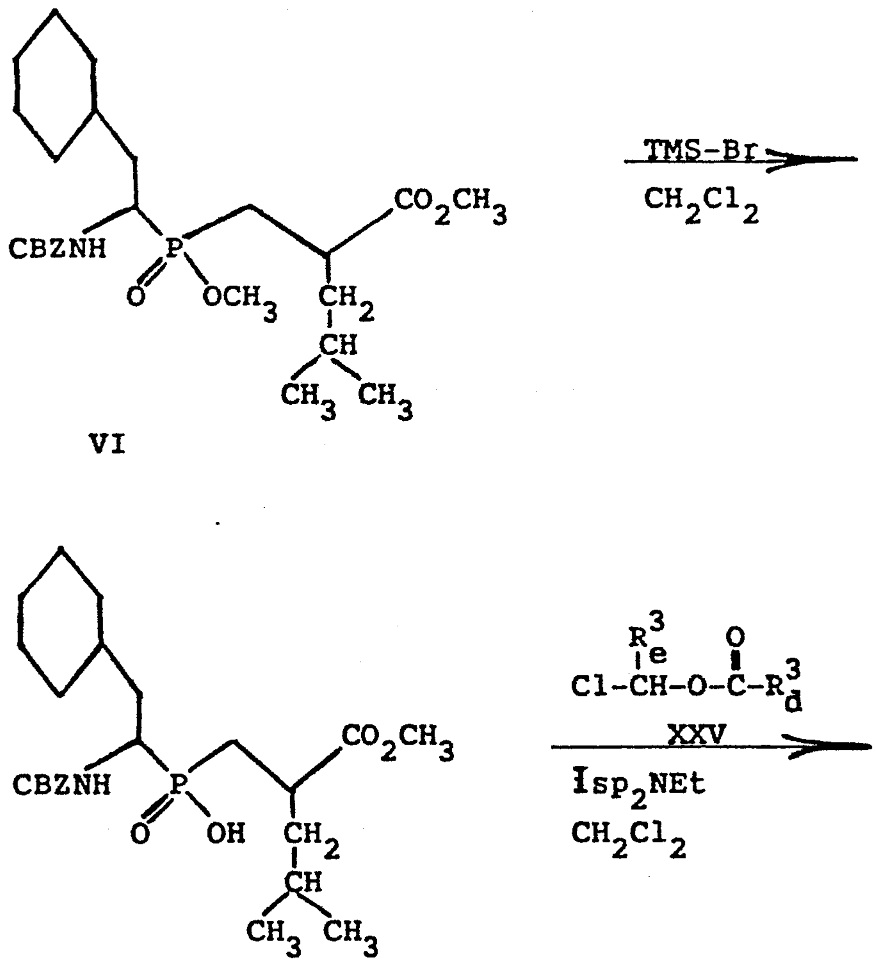


VI

XXIV

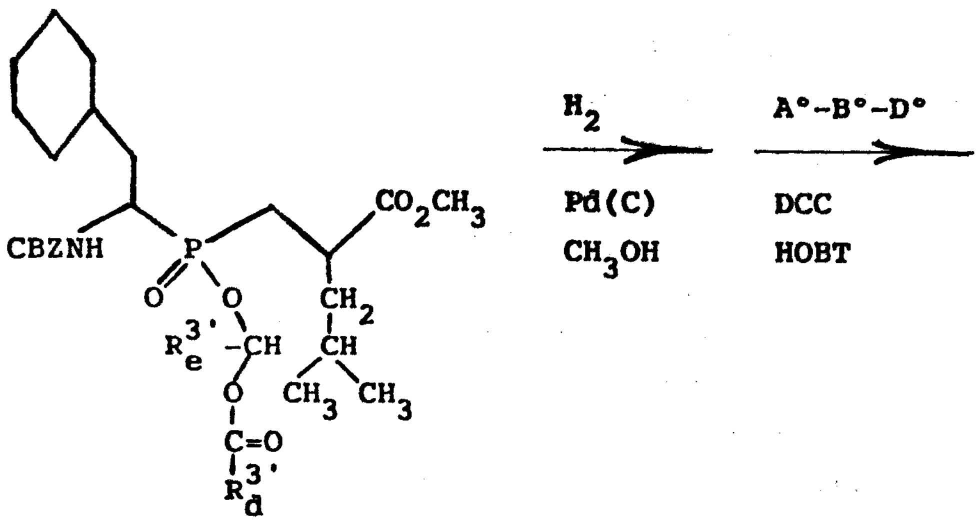


XXVI

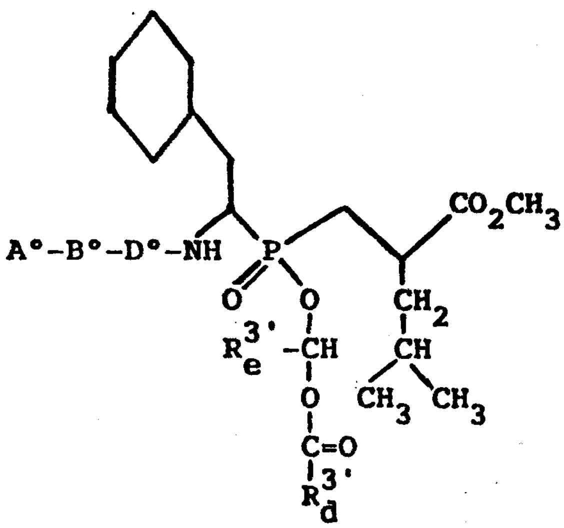

XXVII

Components XXV may be prepared as illustrated below:

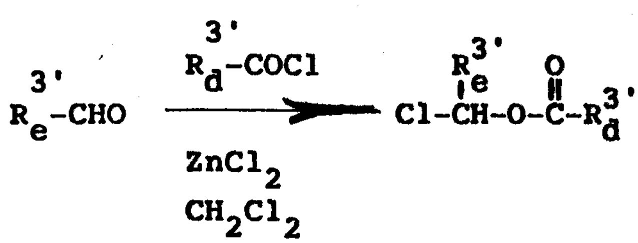

Melting points were recorded on a Thomas-Hoover melting point apparatus and are uncorrected as are all boiling points. $^1H$ NMR spectra were taken on a Varian XL-300 FT spectrometer. Chemical shifts are reported in ppm downfield from tetramethylsilane as internal standard. IR spectra were recorded on a Perkin-Elmer Model 297 spectrometer. Optical rotations were measured with a Perkin Elmer 141 automatic polarimeter in the solvents indicated. Mass spectra (MS) were taken on a Varian 731 spectrometer at 70 eV. Those marked FAB were taken by using the fast atom bombardment method.

The following examples illustrate preparation of representative compounds, particularly those of Formula I°. All temperatures are in °C unless otherwise noted.

## EXAMPLE 1A

Cyclohexylacetaldehyde

A suspension of 100 g (0.46 moles) of pyridinium chlorochromate and 100 g of celite in 800 ml of methylene chloride was stirred vigorously while 38 g (0.3 moles) of 2-cyclohexylethanol in 200 ml of methylene chloride was added all at once. The reaction turned dark immediately and became mildly exothermic. After 1 hour, 1000 ml of ether was added and the reaction mixture was filtered through a bed of silica gel (ca. 250 g) on a fritted glass disk. The pad was rinsed with an additional liter of ether. The combined filtrates were reduced in volume to approximately 200 ml and the solution was washed with 2 x 40 ml of 6N HCl, 1 x 50 ml of saturated

sodium bicarbonate, and 1 x 50 ml of saturated NaCl solution. The organic layer was dried over magnesium sulfate, filtered and evaporated *in vacuo* to give a light green oil. The residue was distilled *in vacuo* to afford 21 g (56%) of a colorless oil (bp 74-76°C at 23 mm of Hg). NMR ($CDCl_3$) (60 MHz): 0.8-2.1 (m, 11H); 2.2-2.4 (m, 2H); 9.6 (t, J=2 Hz, 1H) ppm.

## EXAMPLE 2A

### 1-Amino-2-cyclohexylethylphosphinic acid

A stirred slurry of 26.00 g (0.118 moles) of aminodiphenylmethane-HCl in 100 ml of absolute ethanol was treated with 15.50 g (0.123 moles) of cyclohexylacetaldehyde, immediately followed by 12.8 ml (0.123 moles) of hypophosphorus acid (50% aqueous). The reaction mixture was heated in an oil bath held at 100°C. As the reaction approached reflux it became homogeneous, then heterogeneous again after approximately 5 minutes (white precipitate). After 45 minutes at reflux, an additional 100 ml of ethanol was added to the very thick slurry. Reflux was continued for an additional 3 hours. At this time the reaction mixture was cooled to 0°C in an ice bath, then the solid filtered off. The white solid was washed with 50 ml of ice-cold ethanol and air dried.

The white solid was added to 150 ml of glacial acetic acid and 150 ml of 48% aqueous HBr added. Dissolution occurred over a period of 5 minutes, and the reaction turned a light yellow color. After another 10 minutes a white solid precipitated out of solution. Stirring at room temperature was continued for a total of 2 hours.

The flask was then immersed in an oil bath preheated to 115°C. After 1 hour the reaction was almost homogeneous. After a total of 3 hours at 115°C, the reaction mixture was cooled to 0°C in an ice bath. The solution was washed with 1 x 200 ml and 2 x 100 ml of hexanes. The hexanes wash was discarded and the remaining aqueous acid solution was evaporated to dryness on a rotary evaporator. The resulting semi-crystalline foam was dissolved in 125 ml of absolute ethanol and cooled to 0°C in an ice bath. Propylene oxide (50 ml) was slowly added and a white precipitate was formed. The reaction mixture was allowed to warm to room temperature of its own accord while stirring. After a total of 18 hours, the slurry was cooled to 0°C again, and the solid filtered off. The solid was washed with 100 ml more of ice-cold ethanol and dried to afford 11.98 g (53% overall) of a white solid mp 220-221°C (turns orange and bubbles). NMR ($D_2O$) (60 MHz): 0.8-2.1 (m, 14H); 3.3 (m, 2H); 7.0 (d, J=527 Hz, 1H) ppm.

EXAMPLE 3A

Methyl N-CBZ-1-Amino-2-cyclohexylethylphosphinate

A solution of 7.00 g (0.037 moles) of 1-amino-2-cyclohexylethylphosphonous acid in 105 ml of dioxane and 40 ml of 1N NaOH was cooled to 0°C in an ice bath and stirred vigorously while 10.50 ml (12.53 g; 0.073 moles) of benzyl chloroformate and 80 ml of 1N NaOH were added rapidly and simultaneously over a period of approximately 1 minute. The pH was adjusted to the 8-9 range using 1N NaOH (hydrion paper) added in small increments. The reaction mixture was allowed to come to room temperature, and

vigorous stirring was continued for 48 hours. The dioxane was removed in vacuo and the aqueous washed with 100 ml of ether. The ether layer was discarded and the aqueous solution acidified using 1N $KHSO_4$ to approximately pH = 1-2 (hydrion paper). The solution was extracted with 5 x 100 ml of ethyl acetate. The combined ethyl acetate layers were dried over anhydrous $Na_2SO_4$, filtered, and evaporated in vacuo to afford N-CBZ-1-amino-2-cyclohexylethylphosphonous acid as a white solid.

The solid was redissolved in 200 ml of ethyl acetate and 150 ml of ethereal diazomethane was added all at once. The reaction was stirred at room temperature for 1 hour, at which time the volatiles were removed completely in vacuo to give a viscous oil. This material was crystallized using 200 ml of 1:1 ether:petroleum ether to afford 3.45 g of product. The mother liquors obtained after evaporation of the filtrate were chromatographed on silica gel using 18:1:1 methylene chloride:acetone:methanol to give an additional 5.69 g of product. Total product = 9.14 g (73%). NMR ($CDCl_3$) (60 MHz): 0.8-2.2 (m, 14H); 3.6, 3.8 (s, 3H); 3.8-4.6 (br, 1H); 5.2 (s, 2H); 6.9 (d, J=542 Hz, 1H); 7.2 (s, 5H) ppm.

## EXAMPLE 4A

### Methyl N-BOC-1-amino-2-cyclohexylethylphosphinate

A solution of 0.955 g (0.005 moles) of 1-amino-2-cyclohexylethylphosphonous acid in 10 ml of dioxane and 10 ml of 0.5N NaOH was cooled to 0°C in an ice bath. The reaction mixture was vigorously stirred while 1.26 ml (1.20 g; 0.0055 moles) of di-tert-butyldicarbonate was added all at once. The

reaction mixture was allowed to come to room temperature. After a total reaction time of 17 hours, the dioxane was removed in vacuo and the aqueous was acidified with 1N $KHSO_4$. The mixture was extracted with 3 x 50 ml of ethyl acetate. The combined ethyl acetate solution was dried (anhydrous $Na_2SO_4$) and evaporated in vacuo to a slightly cloudy oil.

The oil was redissolved in 50 ml of ethyl acetate and treated with 50 ml of ethereal diazomethane solution. The reaction mixture was stirred at room temperature for 2 hours, then the volatiles were removed completely in vacuo to give a thick oil. The crude product was chromatographed on silica gel using 18:1:1 methylene chloride:acetone:methanol as the eluant to give 1.17 g (100%) of product as a very thick oil. NMR ($CDCl_3$) (60 MHz): 0.0-2.2 (m containing 9H s at 1.5, 23H (total)); 3.7, 3.9 (s, 3H); 5.2-6.0 (m, 1H); 6.9 (d, J=552 Hz, 1H) ppm.

EXAMPLE 5A

Methyl (N-CBZ-1-amino-2-cyclohexylethyl) 2-carbomethoxy-4-methylpentylphosphinate

A solution of 2.75 g (0.008 moles) of methyl 1-CBZ-amino-2-cyclohexylethylphosphinate in 25 ml of absolute methanol was cooled to 0°C in an ice bath and 4.60 ml of 2M NaOMe in methanol (0.009 moles) was added via syringe. The reaction mixture was stirred at 0°C for 10 minutes, at which time 1.21 g (0.009 moles) of methyl 2-(2-methylpropyl)acrylate was added all at once. The reaction was allowed to proceed at 0°C for 30 minutes, then the ice bath was removed and

the reaction was allowed to proceed at room temperature for 18 hours. The methanol was removed *in vacuo* and the residue treated with 100 ml of 1N HCl. The aqueous was extracted with 3 x 50 ml of ethyl acetate. The combined ethyl acetate was dried over anhydrous $MgSO_4$, filtered, and evaporated *in vacuo* to give a colorless oil. The crude product was chromatographed on silica gel using 18:1:1 methylene chloride:acetone:methanol as the eluant to afford 1.91 g (49%) of the product as a white solid. NMR ($CDCl_3$) (60 MHz): 0.8-2.2 (m, 14H); 3.6, 3.8 (s, 3H); 5.1 (s, 2H); 7.0 (d, J=535 Hz, 1H); 7.3 (s, 5H) ppm.

## EXAMPLE 6A

Methyl (N-BOC-1-amino-2-cyclohexylethyl) 2-carbomethoxy-4-methylpentylphosphinate

A solution of 4.85 g (0.021 moles) of the ester from Example 4A in 35 ml of absolute methanol was cooled to 0°C, whereupon 11.45 ml of 2N NaOMe in methanol (0.023 moles) was added *via* syringe. The reaction was stirred for 10 minutes, at which time 3.10 g (0.022 moles) of methyl 2-(2-methylpropyl)-acrylate was added all at once. Stirring was continued at 0°C for 30 minutes, then the ice bath was removed and stirring was continued at room temperature for 19 hours. At that time the methanol was removed *in vacuo* and the residue treated with 200 ml of 1N HCl. The aqueous was extracted with 3 x 50 ml of ethyl acetate. The combined ethyl acetate washes were dried ($MgSO_4$), filtered and the volatiles evaporated *in vacuo* to give an oil. The

crude product was purified by silica gel chromatography using ethyl acetate as an eluant to afford 4.77 g (61%) of the product as a very viscous oil. NMR ($CDCl_3$) (60 MHz): 0.9 (m, 6H); 0.8-2.2 (m, containing 9H s at 1.5, 29H total); 3.6, 3.8 (s, 3H); 3.7 (s, 3H) ppm.

EXAMPLE 7A

Methyl (1-amino-2-cyclohexylethyl) 2-carbomethoxy-4-methylpentylphosphinate

A mixture of 1.86 g (0.004 moles) of the ester product of Example 5A and 0.95 g of 10% Pd on carbon in 30 ml of absolute methanol was hydrogenated on a Parr type apparatus at 40 psig of hydrogen for 20 hours. The reaction mixture was filtered through a small pad of celite and the pad washed well with methanol. The filtrate was evaporated completely *in vacuo* to afford the pure free amine (1.34 g; 100%) as a viscous oil. NMR ($CDCl_3$) (300 MHz) 0.8-1.0 (m, 6H); 0.8-3.0 (m, 20H); 3.7-4.0 (series of s, total 6H); 8.1 (very br s, 2H) ppm.

EXAMPLE 8A

Methyl (1-amino-2-cyclohexylethyl) 2-carbomethoxy-4-methylpentylphosphinate hydrochloride

A solution of 1.13 g (0.003 moles) of the product of Example 6A in 20 ml of absolute methanol was treated with 12 ml of HCl/methanol (144 g of HCl in 400 ml methanol). The reaction mixture was stirred at room temperature for 4.5 hours. Analysis by thin layer chromatography indicated that no more starting material remained. The volatiles were removed *in vacuo* and replaced several times with more

methanol and re-evaporated. After vacuum drying, this afforded 1.07 g (100%) of the product as a glassy foam. NMR ($CDCl_3$) (60 MHz): 0.9 (m, 6H); 0.8-3.0 (m, 20H); 3.5-4.1 (m, 6H); 8.6 (very br s, 3H) ppm.

## EXAMPLE 9A

Methyl [N-(N-BOC-$N^{im}$-DNP-histidyl)-1-amino-2-cyclohexylethyl] 2-carbomethoxy-4-methylpentylphosphinate

A mixture of 0.694 g (0.002 moles) of the product of Example 7A, 0.88 g (0.002 moles) of N-BOC-$N^{im}$-(2,4-dinitrophenyl)histidine, and 0.297 g (0.0022 moles) of HOBT in 20 ml of dry methylene chloride was stirred at room temperature until the reaction mixture was homogeneous (ca. 1 hour). The reaction mixture was cooled to 0°C in an ice bath and 0.453 g (0.0022 moles) of DCC was added all at once. The reaction was allowed to come to room temperature of its own accord, and slowly became heterogeneous. After stirring at room temperature for 20 hours, the mixture was diluted with 100 ml of ether and filtered through celite. The filtrate was washed with 2 x 20 ml of saturated $NaHCO_3$, dried over anhydrous $Na_2SO_4$, filtered and the volatiles removed *in vacuo* to give a crude yellow foam. The crude material was chromatographed on silica gel using 18:1:1 methylene chloride:acetone:methanol as the eluant to give 0.949 g (63%) of the product as a light yellow glassy foam. NMR ($CDCl_3$) (300 MHz): 0.8-1.0 (m, 6H): 1.0-2.2 (m containing a 9H s at 1.5, total=28H); 2.8 (br s, 1H); 3.1 (m, 2H); 3.6-3.8 (series of s, total=6H); 4.4 (m, 2H); 5,9-6.2 (m, 1H); 6.8-7.4 (m, 2H); 7.5-7.9 (m, 2); 9.6 (dd, 1H); 8.8 (d, 1H) ppm.

EXAMPLE 10A

Methyl [N-($N^{im}$-DNP-histidyl)-1-amino-2-cyclohexyl] 2-carbomethoxy-4-methylpentylphosphinate hydrochloride

A solution of 0.949 g (0.0013 moles) of the product of Example 9A in 10 ml of absolute methanol was treated with 15 ml of HCl in methanol (144 g HCl/400 ml methanol). The reaction mixture was stirred at room temperature for 1 hour. The volatiles were removed completely *in vacuo* to afford 0.795 g (90%) of the crude product as a brown foam. NMR ($CDCl_3$) (300 MHz): 0.8-1.0 (m, 6H); 0.9-2.4 (m, 20H); 2.9 (br s, 2H); 3.2 (m, 1H); 3.7 (br s, 6H); 7.0 (m, 1H); 7.5-7.9 (m, 2H); 8.6 (m, 1H); 8.8 (m, 1H) ppm.

EXAMPLE 11A

N-CBZ-2-Amino-3-(1-naphthyl)propionic acid

A solution of 0.700 g (0.003 moles) of 2-amino-3-(1-naphthyl)propionic acid in 10 ml of dioxane and 3.2 ml of 1N NaOH was cooled to 0°C in an ice bath and 6.40 ml of 1N NaOH and 0.923 ml (1.10 g; 0.0064 moles) of benzyl chloroformate were added simultaneously and rapidly *via* syringe. The reaction mixture was stirred vigorously and the pH was adjusted to the 8-9 range by adding 1N NaOH dropwise. The reaction was allowed to come to room temperature of its own accord while stirring vigorously. After 24 hours at room temperature, the dioxane was removed *in vacuo* and the aqueous washed with 2 x 10 ml of ether. The ether layers were discarded and the aqueous layer was acidified to pH=1-2 (hydrion paper) using 1N $KHSO_4$. The aqueous solution was extracted with 2 x 50 ml of ethyl acetate. The combined ethyl

acetate washings were dried over anhydrous $MgSO_4$, filtered, and the volatiles removed in vacuo to give a clear oil. This was triturated with 5 x 5 ml portions of petroleum ether and dried in vacuo to afford 1.09 g (97%) of the product. NMR ($CDCl_3$) (300 MHz): 3.4 (dd, 1H); 3.8 (dd, 1H); 4.8 (dd, 1H); 5.0 (s, 2H); 5.3 (d, 1H); 7.1-7.6 (m, 9H); 7.8 (d, 1H); 7.9 (d, 1H); 8.1 (d, 1H) ppm.

EXAMPLE 12A

N-BOC-2-Amino-3-(1-naphthyl)propionic acid

A solution of 0.700 g (0.003 moles) of 2-amino-3-(1'-naphthyl)propionic acid in 7 ml of dioxane and 13 ml of 0.5 N NaOH was cooled to 0°C in an ice bath and stirred vigorously while 0.82 ml (0.773 g; 0.0036 moles) of di-tert-butyl dicarbonate was added in one portion. The ice bath was removed and the heterogeneous reaction mixture was stirred vigorously at room temperature. After several hours, the reaction mixture became homogeneous. Vigorous stirring was continued for ca. 24 hours. The dioxane was removed in vacuo and the residue acidified to pH=1-2 with 1N $KHSO_4$. The aqueous was extracted with 2 x 50 ml of ethyl acetate. The combined organic extracts were dried over anhydrous $MgSO_4$, filtered, and the volatiles evaporated in vacuo to give a colorless oil. The oil was triturated 10 ml of petroleum ether to give 0.97 g (94%) of the product as a white powder (crystallization occurs very slowly). NMR ($CDCl_3$) (300 MHz): 0.7 (s, 9H); 3.2 (dd, 1H); 3.9 (dd, 1H); 4.7 (m, 1H); 7.3-7.6 (m, 5H); 7.8 (d, 1H); 7.9 (d, 1H); 8.2 (d, 1H) ppm.

EXAMPLE 13A

Methyl [N-(N-(N-CBZ-2-amino-3-(1-naphthyl)propionyl)-$N^{im}$-DNP-histidyl)-1-amino-2-cyclohexylethyl] 2-carbomethoxy-4-methylpentylphosphinate

A mixture of 0.176 g (0.0005 moles) of N-CBZ-2-amino-3-(1'-naphthyl)-propionic acid and .156 g (0.00055 moles) of disuccinimidyl oxalate in 7 ml of dry acetonitrile was treated with 0.045 ml (0.00055 moles) of dry pyridine. The reaction mixture was stirred at room temperature under nitrogen atmosphere for 20 hours. The mixture became homogeneous during this period. At this time, a solution of 0.300 g (0.00043 moles) of the product of Example 10 in a mixture of 8 ml of dry acetonitrile and 0.28 ml (0.202 g) of triethylamine was added all at once. The reaction turned very dark and stirring at room temperature under nitrogen was continued for 20 hours. The volatiles were evaporated *in vacuo* and the residue added to 30 ml of ethyl acetate, which was washed with 2 x 10 ml of saturated $NaHCO_3$, dried over anhydrous $Na_2SO_4$, filtered, and the volatiles removed *in vacuo*. The residue was chromatographed on silica gel using 18:1:1 methylene chloride:acetone:methanol as the eluant to afford 0.348 g (86%) of the tripeptide as a brownish-yellow glassy foam. NMR ($CDCl_3$) (300 MHz): 0.7-0.9 (m, 6H); 0.9-2.3 (m, 20H); 2.8-3.5 (m, 3H); 3.5-3.8 (series of singlets, total=6H); 4.1-4.8 (m, 2H); 5.0 (s, 2H); 5.3-5.6 (m, 1H); 6.8-7.6 (m, 14H); 7.8 (t, 1H); 7.9 (t, 1H); 8.2 (d, 1H); 8.5 (br, t, 1H); 8.8 (s, 1H) ppm.

## EXAMPLE 14A

Methyl [N-(N-(N-BOC-2-amino-3-(1-naphthyl)propionyl)-$N^{im}$-DNP-histidyl)-1-amino-2-cyclohexylethyl] 2-carbomethoxy-4-methylpentylphosphinate

A suspension of 0.159 g (0.0005 moles) of N-BOC-2-amino-3-(1-naphthyl)propionic acid and 0.156 g (0.00055 moles) of disuccinimidyl oxalate in 7 ml of dry acetonitrile was treated with 0.045 ml (0.044 g; 0.00055 moles) of dry pyridine. After approximately 5 hours, the reaction was homogeneous. The reaction mixture was stirred under nitrogen atmosphere for an additional 15 hours at room temperature. At this time, a solution of 0.300 g (0.00043 moles) of the product of Example 10 in 8 ml of dry acetonitrile containing 0.280 ml (0.202 g; 0.002 moles) of triethylamine was added all at once. The reaction mixture turned very dark, and stirring under nitrogen was continued for 20 hours. The volatiles were removed *in vacuo* and the residue taken up in 30 ml of ethyl acetate. The organic solution was washed with 2 x 10 ml of saturated $NaHCO_3$, dried over anhydrous $Na_2SO_4$, filtered, and the volatiles evaporated *in vacuo*. The residue was chromatographed on silica gel using 18:1:1 methylene chloride:acetone:methanol as the eluant to afford 0.348 g (86%) of the desired product as a yellow glassy foam. NMR ($CDCl_3$) (300 MHz): 0.8-1.0 (m, 6H); 0.9-2.3 (m containing 9H s at 1.4, total=29H); 2.8-3.5 (m, 4H); 3.6-3.8 (series of singlets, total=6H); 4.6 (m, 2H); 4.7 (m, 1H); 5.0 (m, 1H); 6.9-8.0 (m, 10H); 8.2 (d, 1H); 8.6 (dd, 1H); 8.8 (t, 1H) ppm.

## EXAMPLE 15A

Methyl [N-(N-(N-CBZ-2-amino-3-(1-naphthyl)propionyl)-histidyl-1-amino-2-cyclohexylethyl] 2-carbomethoxy-4-methylpentylphosphinate

A solution of 0.100 g (0.0001 moles) of the product of Example 13 in 4 ml of dry DMF was put in a Fisher-Porter tube and approximately 20 ml of ammonia was condensed into the tube also. The reaction turned dark purple. The tube was sealed and stirring was continued for approximately 3 hours. During this time the color went from purple to pink. The tube was opened and the ammonia was allowed to evaporate. The remaining solution was transferred to a flask and the volatiles were removed completely *in vacuo*. The residue was chromatographed on silica gel, giving 0.018 g (22%) of the desired product as a light yellow oil. NMR ($CDCl_3$) (300 MHz): 0.8-1.0 (m, 6H); 1.0-1.8 (m, 23H); 1.9-3.4 (m, 2H); 2.8-3.1 (m, 1H); 3.4 (m, 1H); 3.7-3.8 (series of singlets, total=6H); 4.6 (m, 1H); 5.1 (s, 1H); 5.2 (br s, 2H); 5.6 (m, 1H); 7.2-7.7 (m, 11H); 7.8 (d, 1H); 7.9 (d, 1H); 8.3 (br d, 1H) ppm.

## EXAMPLE 16A

[N-(N-(N-CBZ-2-amino-3-(1-naphthyl)propionyl)histidyl-1-amino-2-cyclohexylethyl] 2-carboxy-4-methylpentyl-phosphinic acid disodium salt

A solution of 0.018 g (0.000022 moles) of the product of Example 15A in 1 ml of ethanol was treated with 0.44 ml of 0.10N NaOH solution (0.000044 moles). The reaction mixture was stirred at room temperature for 16 hours. The volatiles were evaporated completely *in vacuo* and the residue

triturated with anhydrous ether (3 x 2 ml) to give 11 mg (61%) of the disodium salt as a very light yellow amorphous solid. NMR ($CD_3OD$) (300 MHz): 0.8-1.0 (m, 6H); 1.0-1.8 (m, 21H); 2.7 (br s, 1H); 3.0 (dd, 1H); 3.6-3.9 (m, 4H); 4.1 (br s, 1H); 4.2 (dd, 1H); 4.5 (dd, 1H); 5.0 (d, 2H); 7.1-7.6 (m, 11H); 7.8 (dd, 1H); 7.9 (d, 1H); 8.2 (d, 1H) ppm.

EXAMPLE 17A

1,2,3,6-Tetrahydrophenyl vinyl ether

This compound was prepared in 67% yield using the method of Burgstahler (A. W. Burgstahler and I. C. Norton, J. Amer. Chem. Soc., 1961, 83, 198-206).

EXAMPLE 18A

1,2,3,6-Tetrahydrophenylacetaldehyde

This compound was prepared in quantitative yield by the thermal rearrangement of 1,2,3,4-tetrahydrophenyl vinyl ether at 195°C according to the procedure of Burgstahler (A. W. Burgstahler and I. C. Norton, J. Amer. Chem. Soc., 1961, 83, 198-206).

EXAMPLE 19A

1-Amino-2-(1,2,3,6-tetrahydrophenyl)ethylphosphinic acid

A mixture of 11.58 g (0.053 moles) of benzhydrylamine hydrochloride and 6.80 g (0.055 moles) of 1,2,3,6-tetrahydrophenylacetaldehyde in 42 ml of absolute ethanol was treated with 5.75 ml of 50% aqueous hypophosphorus acid. The reaction mixture was placed in an oil bath preheated to 100°C and stirred vigorously. The reaction became homogeneous after about 5 minutes, then heterogeneous

again immediately thereafter. Heating at 100°C was continued for a period of 4 hours. At this time, the reaction mixture was cooled to 0°C in an ice bath, and the solid filtered off and washed with 3 x 10 ml portions of ice-cold ethanol. The solid was dried in vacuo to afford 11.00 g of solid product as a fluffy white powder.

The crude powder was dissolved in 120 ml of trifluoroacetic acid, and the resulting solution was heated to 100°C in an oil bath. After approximately 5 minutes, the reaction had turned dark purple. Heating was continued for 1 hour. The reaction mixture was cooled to room temperature and the residue was partitioned between 250 ml of water and 100 ml of ether. The ether layer was separated and the aqueous washed with another 100 ml of ether. The ether was discarded and the aqueous solution evaporated completely in vacuo. The remaining white solid was treated with 10 x 50 ml of methanol, each time evaporating the methanol on a rotary evaporator. The white solid that remained was triturated with 25 ml of anhydrous ether to afford 3.77 g (36% overall) of the desired product. NMR ($D_2O$) (60 MHz): 1.0-2.4 (m, 9H); 3.2 (m, 1H); 5.4-5.8 (m, 2H); 6.9 (d, J=528Hz, 1H) ppm.

## EXAMPLE 20A

Methyl N-CBZ-1-amino-2-(1,2,3,6-tetrahydrophenyl)-ethylphosphinate

A solution of 2.50 g (0.013 moles) of 1-amino-2-(1,2,3,6-tetrahydrophenyl)ethylphosphonous acid in 15 ml of 1N NaOH and 30 ml of dioxane that had been cooled to 0°C in an ice bath was stirred

vigorously while 3.75 ml (4.45 g; 0.026 moles) of benzyl chloroformate and 30 ml of 1N NaOH were added rapidly and simultaneously. The pH of the solution was adjusted to 8-9 (hydrion paper) by the dropwise addition of additional 1N NaOH. The reaction mixture was allowed to warm to room temperature of its own accord, and the reaction was stirred vigorously for a total of 19 hours. The dioxane was removed *in vacuo* and the remaining aqueous was washed with 50 ml of ether. The ether was discarded and the aqueous was acidified with 1N $KHSO_4$ to approximately pH=1-2 (hydrion paper). The mixture was extracted with 3 x 100 ml of ethyl acetate. The combined ethyl acetate layers were dried over anhydrous $Na_2SO_4$, filtered, and the volatiles evaporated *in vacuo* to yield a viscous oil.

The oil was redissolved in 75 ml of ethyl acetate and 75 ml of diazomethane in ether was added all at once. The reaction mixture was stirred at room temperature for 2 hours, then the volatiles were evaporated completely *in vacuo*. The residual oil was chromatographed on silica gel using 18:1:1 methylene chloride:acetone:methanol as the eluant, to afford 3.29 g (75%) of the product as a waxy solid. NMR ($CDCl_3$) (60 MHz): 1.0-2.2 (m, 9H); 3.4, 3.7 (singlets, total=3H); 3.8-4.2 (m, 1H); 5.0 (br, s, 2H); 5.1-5.7 (m, 2H); 6.2-6.8 (2 multiplets, total=1H); 6.8 (d, J=552Hz, 1H); 7.1 (s, 5H) ppm.

## EXAMPLE 21A

N-BOC-1-amino-2-(1,2,3,6-tetrahydrophenyl)ethylphosphinic acid methyl ester

A solution of 1.50 g (0.008 moles) of 1-amino-2-(1,2,3,6-tetrahydrophenyl)ethylphosphonous

acid in 16 ml of dioxane and 16 ml of 0.5N NaOH was cooled to 0°C in an ice bath, whereupon 2.00 ml (1.90 g; 0.0087 moles) of di-tert-butyl dicarbonate was added all at once. The reaction mixture was stirred vigorously at 0°C for 5 minutes, then removed from the ice bath and stirred at room temperature for 3 hours. The dioxane was removed in vacuo and the aqueous solution acidified to pH=1-2 (hydrion paper) with 1N $KHSO_4$. The mixture was extracted with 2 x 75 ml of ethyl acetate. The combined ethyl acetate was dried over anhydrous $Na_2SO_4$, filtered, and the volatiles evaporated in vacuo to give a viscous oil.

The residual oil was dissolved in 50 ml of ethyl acetate and treated with 40 ml of ethereal diazomethane solution. The reaction mixture was stirred at room temperature for 1 hour and 10 minutes, then the volatiles were evaporated completely in vacuo. The residual oil was chromatographed on silica gel using 25:1:1 methylene chloride:acetone: methanol to afford 1.83 g (95%) of the product as a very thick yellow oil that crystallized very slowly. NMR ($CDCl_3$) (60 MHz): 1.0-2.2 (m containing 9H s at 1.4, total=19H); 3.7, 3.9 (singlets, total=3H); 3.9-4.2 (m, 1H); 5.0-5.7 (m, 2H) ppm.

EXAMPLE 22A

Methyl [N-BOC-1-amino-2-(1,2,3,6-tetrahydro)phenylethyl] 2-carbomethoxy-4-methylpentylphosphinate

A solution of 0.700 g (0.003 moles) of the product of Example 21A in 5 ml of absolute methanol was cooled to 0°C whereupon 1.65 ml (0.0033 moles) of 2N NaOMe in methanol was added over a one minute

period. The reaction mixture was stirred at 0°C for 10 minutes at which time 0.447 g (0.0032 moles) of methyl 2-(2-methylpropyl)acrylate was added all in one portion. The reaction was stirred at 0°C for 30 minutes, then removed from the ice bath and stirred at room temperature for 6.5 hours. The methanol was removed *in vacuo* and the residue treated with 30 ml of 1N HCl. The aqueous mixture was extracted with 3 x 25 ml of ethyl acetate. The combined ethyl acetate layers were dried over anhydrous $MgSO_4$, filtered, and the volatiles evaporated *in vacuo*. The residual oil was purified by chromatography on silica gel using 1% methanol in ethyl acetate as the eluant to afford 0.929 g (82%) of the product as a very thick, colorless oil. NMR ($CDCl_3$) (60 MHz): 0.8-1.0 (m, 6H); 1.0-2.2 (m containing 9H s at 1.5, total=24H); 3.5-3.9 (m, 7H); 4.1-4.5 (v br s, 1H); 5.2-5.7 (m, 2H) ppm.

## EXAMPLE 23A

Methyl [1-amino-2-(1,2,3,6-tetrahydro)phenylethyl] 2-carbomethoxy-4-methylpentylphosphinate hydrochloride

A solution of 0.324 g (0.00087 moles) of the product of Example 22A in 10 ml of absolute methanol was treated with 2.0 ml of HCl in methanol (144 g/400 ml). The reaction mixture was stirred at room temperature for 4 hours and monitored by thin layer chromatography. After all of the starting material had disappeared, the volatiles were evaporated completely *in vacuo*, and the residue evacuated to high vacuum to remove residual hydrogen chloride. This material was used directly in the next reaction. $R_f$=0.64 (5:4:1 methylene chloride: acetone:methanol).

## EXAMPLE 24A

Methyl [N-(N-BOC-glycylglycyl)-1-amino-2-(1,2,3,6-tetrahydro)phenylethyl] 2-carbomethoxy-4-methylpentylphosphinate

A suspension of 0.202 g (0.00087 moles) of N-BOC-glycylglycine and 0.272 g (0.00096 moles) of disuccinimidyl oxalate in 13 ml of dry acetonitrile was treated with 0.078 ml (0.076 g; 0.00096 moles) of dry pyridine. The heterogeneous mixture was stirred at room temperature under nitrogen atmosphere and slowly became homogeneous over a period of several hours. The reaction was stirred at room temperature for a total for 17 hours. At this time, a solution of the product of Example 23 in 14 ml of acetonitrile containing 0.484 ml (0.352 g; 0.0035 moles) of triethylamine was added all in one portion. The reaction mixture was allowed to stir at room temperature under nitrogen atmosphere for 48 hours. At this time the volatiles were evaporated *in vacuo* and the residue dissolved in 30 ml of ethyl acetate. The organic solution was washed with 2 x 20 ml of saturated $NaHCO_3$, dried over anhydrous $Na_2SO_4$, filtered, and the volatiles removed *in vacuo*. The residue was purified by silica gel chromatography using 18:1:1 methylene chloride:acetone:methanol as the eluant to afford the product as a light yellow oil. NMR ($CDCl_3$) (300 MHz): 0.8-1.0 (m, 6H); 1.1-1.9 (m containing 9H s at 1.5, total=22H); 1.9 (br s, 1H); 2.2 (m, 1H); 2.9 (m, 1H); 3.6-3.8 (series of singlets; total=6H); 3.7-4.2 (m, 4H); 4.5 (m, 1H); 5.3-5.8 (m, 2H); 6.7-7.2 (m, 2H) ppm.

## EXAMPLE 25A

Diethyl 2-(N-Benzyl)amino-3-phenylethylphosphonate

A solution of 45.80 g (0.427 moles) of benzylamine in 50 ml of methylene chloride was agitated during the addition of 51.35 g (0.427 moles) of freshly distilled phenylacetaldehyde. After the addition was half-complete, the reaction mixture became cloudy, and after completion of addition the mixture turned a yellow color. At this time, 40 g of anhydrous $Na_2SO_4$ was added and the reaction mixture was stirred at room temperature for 1 hour. The drying agent was filtered off and the filtrate evaporated *in vacuo* to a yellow liquid.

At this time, 56.02 g (0.406 moles) of diethylphosphite was added to the yellow liquid and the mixture was heated in an oil bath maintained at 170°C. The solution was maintained at this temperature for 2.5 hours. The reaction mixture was cooled to room temperature and purified in two approximately equal portions by chromatography on 600 g of silica gel using 2:1 hexanes:ethyl acetate as the eluant. A more mobile impurity came off first, followed by the desired product as a thick yellow liquid. This procedure afforded 59.15 g (42%) of the diester. NMR ($CDCl_3$) (60 MHz): 1.0-1.6 (overlapping t, total=6H); 1.8 (br s, 2H); 2.0 (s, 2H); 2.5-3.5 (m, 2H); 3.7-4.5 (two overlapping q, 4H); 7.0 (m, 10H) ppm.

## EXAMPLE 26A

Diethyl 2-(N-Benzyl)amino-3-phenylethylphosphonate hydrochloride

A solution of 30.00 g (0.086 moles) of the product of Example 25A in 650 ml of anhydrous ether

was treated with HCl gas for 45 minutes. The reaction was allowed to stand overnight and then filtered to remove a small amount of solid material. The remaining 29.15 g (0.084 moles) of starting material was dissolved in 430 ml of anhydrous ether and treated similarly. The combined filtrates from the two reactions was evaporated *in vacuo* and the viscous oil treated with several 200 ml portions of carbon tetrachloride followed by evaporation *in vacuo*. This procedure led to a sticky crystalline mass which was triturated with anhydrous ethyl ether and filtered and then vacuum dried to afford 41.55 g (64%) of the salt as a white crystalline solid. NMR ($CDCl_3$) (60 MHz): 1.0-1.6 (m, 6H); 2.1 (s, 2H); 3.2-4.5 (m, 9H); 7.2 (s, 10H) ppm.

EXAMPLE 27A

Diethyl 2-Amino-3-phenylethylphosphonate hydrochloride

A solution of 2.00 g (0.0052 moles) of the product of Example 26A in 8 ml of absolute ethanol containing 0.200 g of 10% Pd on carbon was hydrogenated in a Parr type apparatus for 7 hours at 40 psig of hydrogen. The reaction mixture was filtered through a celite pad and the pad washed well with ethanol. The combined filtrates were evaporated *in vacuo* to give 1.50 g (99%) of the product as a very viscous, colorless oil. NMR ($d_2$-acetone) (60 MHz): 1.0-1.6 (overlapping t, 6H); 3.3-5.0 (m, 7H); 7.0-7.6 (m, 5H) ppm.

## EXAMPLE 28A

Diethyl 2-Amino-3-cyclohexylethylphosphonate hydrochloride

A solution of 1.50 g (0.0051 moles) of the product of Example 26A from a procedure similar to Example 27 in 20 ml of glacial acetic acid was treated with 1.50 g of $PtO_2$ and hydrogenated at 60°C and 50 psig of hydrogen in a Parr type of apparatus. After 2 hours, the reaction mixture was cooled to room temperature, filtered through a celite pad and the pad washed well with glacial acetic acid. The filtrate was evaporated completely *in vacuo* to give 1.34 g (86%) of essentially pure product. NMR ($CDCl_3$) (60 MHz): 0.8-2.2 (m, 19H); 3.6 (br s, 1H); 3.9-4.5 (m, 4H); 8.6 (v br s, 3H) ppm.

## EXAMPLE 29A

Methyl 2-(2-methylpropyl)acrylate

This compound was prepared by the method of J. Harley-Mason *et al*. (*Tetrahedron* 1980, 36, 1063) in approximately 35% overall yield. NMR ($CDCl_3$) (300 MHz): 0.9 (d, 6H); 1.8 (septet, 1H); 2.2 (d, 2H); 3.7 (s, 3H); 5.5 (d, 1H); 6.1 (d, 1H) ppm.

## EXAMPLE 30A

Methyl (N-CBZ-1-amino-2-cyclohexylethyl) carbomethoxymethylphosphinate

To a mixture of benzylcarbamate (8.66 g, 0.057 mol), pivalic acid (11.72 g, 0.11 mol) and 11 g of predried powdered molecular sieves in 120 ml of dry toluene was added carbomethoxymethyldichlorophosphine (10 g, 0.057 mol). The reaction mixture was cooled to 0°C and to it was added dropwise

cyclohexylacetaldehyde (7.2 g, 0.057 mol). After stirring for 30 minutes at 0°C and 2 hours at room temperature, the reaction was filtered and the solvents removed by evaporation *in vacuo*. The residue was redissolved in 100 ml of dichloromethane cooled to 0°C and esterified with an ether solution of diazomethane. The solvents and excess diazomethane were subsequently removed by evaporation *in vacuo* and the crude product was purified by chromatography to give 11 g of the title compound.

Chromatography: silica, ethyl acetate

TLC (silica, ethyl acetate) $R_f$=0.48

NMR ($CDCl_3$, TMS) 0.9-2.0 (m, 13H), 2.95 (d 16Hz, 2H), 3.64 (s, 3H), 3.7 (d 12Hz, 3H), 3.9-4.5 (m.1H), 5.1 (s,2H), 5.4 and 5.8 (d 10Hz, 1H), 7.2 (s.5H)

mass spectrum: $M^+$

## EXAMPLE 31A

Methyl (N-CBZ-1-amino-3-methylbutyl) carbomethoxy-methylphosphinate

This ester is prepared by the procedure described in Example 33A, using 3-methylbutyraldehyde in place of cyclohexylacetaldehyde. It can also be prepared by the method of P. A. Bartlett *et al.*, *J. Amer. Chem. Soc.*, **106**, 4282-83 (1984).

## EXAMPLE 32A

N-CBZ-1-aminoethylphosphinic acid

The title compound was prepared using the procedure described in Example 2A with paraldehyde replacing hexahydrophenylacetaldehyde.

## EXAMPLE 33A

Methyl (N-BOC-1-amino-2-cyclohexylethyl) 2-carbomethoxy-4-methyl-(E & Z)-2-pentenyl phosphinate

A solution of 0.504 g (1.64 mmol) the phosphinic ester product from Example 4A in dry methanol (2.5 ml) at 0°C was treated dropwise over ten minutes with 0.90 ml of 2.0N methanolic sodium methoxide (1.8 mmol, 1.1 eq.). When addition of the base was complete, 0.38 ml (0.48 g, 2.44 mmol, 1.5 eq.) of trimethyl 2-phosphonoacrylate was added dropwise over 2 minutes. The mixture was warmed to room temperature and stirred for 30 minutes. At this time, tlc analysis (ethyl acetate/acetonitrile/methanol 9:1:.5; E. Merck .25 mm silica plates) indicated complete disappearance of starting phosphinic ester ($R_f$ .8) with formation of a new more polar material with $R_f$ of .3.

The mixture was re-cooled to 0°C and 0.30 ml (0.24 g, 3.3 mmol, 2.0 eq.) of distilled isobutyraldehyde was added dropwise over 2 minutes. The mixture was warmed to room temperature when the addition of the aldehyde was complete. After 1 hour, tlc analysis (as above) indicated complete disappearance of the polar intermediate and formation of a new UV-active material.

The mixture was diluted with ethyl acetate to a total volume of 30 ml and washed with pH 7.0 phosphate buffer (2 x 10 ml). The organic layer was separated and washed once with saturated aqueous sodium chloride (5 ml) and dried ($MgSO_4$). After filtration and removal of volatiles *in vacuo*, the crude product was purified by medium pressure liquid chromatography (10:1 EtOAc:$CH_3CN$). Two major

products were isolated; fraction A (0.190 g, 0.43 mmol, 26%) and fraction B (0.217 g, 0.49 mmol, 30%). Product A, which eluted from the column first, was identified as the E isomer by analysis of its 300 MHz proton NMR spectrum (olefinic proton resonance at 6.68 ppm ($CDCl_3$). Product B is the Z isomer (olefinic proton resonance at 6.03 ppm).

Anal. Calcd for $C_{22}H_{40}NO_6P$:

C, 59.31; H, 9.05; N, 3.14.

Found: (Fraction A) C, 59.07; H, 8.86; N, 3.08;

(Fraction B) C, 59.47; H, 8.82; N, 3.15.

MS (FAB) 446 (both isomers) ($M^+$ +1).

EXAMPLE 34A

Methyl 2-(2-methylpropyl)acrylate

This compound was prepared by the method of J. Harley-Mason et al. (Tetrahedron 1980, 36, 1063) in approximately 35% overall yield. NMR ($CDCl_3$) (300 MHz): 0.9 (d, 6H); 1.8 (septet, 1H); 2.2 (d, 2H); 3.7 (s, 3H); 5.5 (d, 1H); 6.1 (d, 1H) ppm.

EXAMPLE 35A

Methyl 2-(cyclohexylmethyl)acrylate

This compound was prepared as above in approximately 20% overall yield from dimethyl malonate and bromomethylcyclohexane. NMR ($CDCl_3$) (60 MHz): 0.8-2.0 (m, 11H); 2.2 (d, 2H); 3.7 (s, 3H); 5.4 (m, 1H); 6.0 (d, J=2Hz, 1H) ppm.

EXAMPLE 36A

Methyl 2-(n-propyl)acrylate

This compound was prepared as above in approximately 45% overall yield from dimethyl

malonate and n-propyl bromide. bp.=51-53°C at 20 mm of Hg. NMR ($CDCl_3$) (60 MHz): 0-.9 (t, J=7Hz, 3H); 1.2-1.8 (m, 2H); 2.3 (t, J=7Hz, 2H); 3.8 (s, 3H); 5.5 (m, 1H); 6.1 (br s, 1H) ppm.

## EXAMPLE 37A

Methyl 2-(2-propyl)acrylate

This compound was prepared as above in approximately 10% overall yield from dimethyl malonate and isopropyl bromide. bp.=130-132°C at $P_{atm}$; NMR ($CDCl_3$) (60 MHz): 1.1 (d, J=7Hz, 6H); 2.8 (septet, 1H); 3.8 (s, 1H); 5.4 (t, 1H); 6.0 (br s, 1H) ppm.

## EXAMPLE 38A

Methyl [N-(N-(N-BOC-2-amino-3-(1-naphthyl)propionyl)-histidyl)1-amino-2-cyclo-hexylethyl]2-carbomethoxy-4-methylpentylphosphinate

A solution of 0.300 g (0.32 mmol) of the product of Example 14A in 5 ml of dry dimethylformamide was put in a Fisher-Porter tube and treated with ca. 20 ml of anhydrous ammonia. The tube was sealed and the reaction was allowed to stir at room temperature for 8.25 hours. The tube was opened and the ammonia allowed to evaporate. The residue was transferred to a flask and the volatiles removed completely *in vacuo*. The dark residue was triturated several times with 4 ml portions of anhydrous ether. This afforded the desired product as a yellow powder (0.133 g; 54%). The mother liquors were evaporated and the residue chromatographed using mplc and 18:1:1 methylene chloride:acetone:methanol as the eluant to afford an additional 24 mg (10%) of the product as a

crystalline solid. 28H); 2.7-3.2 (m, 6H); 3.5-3.8 (m, 6H); 4.1-5.4 (m, 3H); 6.8-7.6 (m, 6H); 7.6-7.9 (m, 2H); 8.0 (br s, 1H) ppm.

EXAMPLE 39A

Methyl (N-CBZ-1-amino-2-cyclohexyl)2-carbomethoxy-3-cyclohexylpropylphosphinate

A solution of 3.39 g (0.010 moles) of the product of Example 3A in 25 ml of absolute methanol was cooled to 0C under $N_2$. At this time, a solution of 5.50 ml of 2N NaOMe in methanol was added dropwise over a period of ca. 1 min. The reaction mixture was stirred for 10 min. and at this time, 1.91 g (0.0105 moles) of the product of Example 35 was added over a 1 min. period. The reaction mixture was stirred at 0°C for 30 min., then at room temperature for 23 hours. The volatiles were removed *in vacuo* and the residue added to 50 ml of ice-cold 1N HCl. The mixture was extracted with 3 X 50 ml of ethyl acetate. The combined ethyl acetate was dried over anh. $MgSO_4$, filtered, and the volatiles evaporated *in vacuo* to a colorless oil. The residue was chromatographed on silica gel using 18:1:1 methylene chloride:acetone:methanol as the eluant to afford 4.26 g (82%) of the desired product as a very viscous oil. NMR ($CDCl_3$) (300 MHz): 0.7-1.9 (m, 28H); 2.1 (m, 1H); 2.9 (br s, 1H); 3.6-3.8 (m, 6H); 4.1 (br s, 1H); 5.1 (s, 2H); 7.3 (s, 5H) ppm.

EXAMPLE 40A

Methyl (1-amino-2-cyclohexyl)2-carbomethoxy-3-cyclohexylpropylphosphinate

A mixture of 4.00 g (7.7 mmol) of the product of Example 39 in 50 ml of absolute methanol

was treated with 1.00 g of 10% Pd on C and hydrogenated in a Parr apparatus at 50 psig of $H_2$ for 20 hours. The catalyst was filtered off through celite and the pad washed well with methanol. The filtrate was evaporated *in vacuo* and the residue was chromatographed on silica gel using 18:1:1 methylene chloride:acetone:methanol as the eluant. This afforded 2.20 g (74%) of the desired product as a viscous oil. NMR ($CDCl_3$) (300 MHz): 0.7-1.9 (m, 28H); 2.1 (t, 1H); 2.2 (m, 1H); 2.9 (br s, 2H); 3.7 (m, 6H) ppm.

## EXAMPLE 41A

Methyl [N-(N-BOC-$N^{im}$-DNP-histidyl)-1-amino-2-cyclohexylethyl] 2-carbomethoxy-3-cyclohexylpropylphosphinate

A mixture of 2.00 g (5.2 mmol) of the product of Example 40, 2.29 g (5.2 mmol) of N-BOC-$N^{im}$-(DNP)-histidine, and 0.768 g (5.7 mmol) of HOBT in 50 ml of dry methylene chloride was stirred until it was homogeneous. At this time, the reaction mixture was cooled to 0°C in an ice bath and 1.18 g (5.7 mmol) of DCC was added all at once. The reaction mixture was stirred under $N_2$ and allowed to warm to room temperature of its own accord. The mixture slowly became heterogeneous as a white powder precipitated out. After 20 hours the reaction mixture was diluted with 100 ml of anhydrous ether and filtered through celite. The filtrate was evaporated *in vacuo* and the residue was dissolved in 100 ml of methylene chloride. The organic solution was washed with 2 X 50 ml of saturated $NaHCO_3$, dried over anhydrous $Na_2SO_4$, filtered and the

volatiles evaporated *in vacuo*. The residue was chromatographed on silica gel using 18:1:1 methylene chloride:acetone:methanol as the eluant to afford 2.69 g (65%) of the desired product as a viscous dark yellow oil. NMR ($CDCl_3$) (300 MHz): 0.7-1.9 (m containing 9H s at 1.4, 37H); 2.8 (br s, 1H); 3.1 (m, 1H); 3.7 (m, 6H); 4.4 (br s, 1H); 6.0 (m, 1H); 7.0 (m, 1H); 7.5-8.0 (m, 2H); 8.6 (dd, 1H); 8.9 (s, 1H) ppm.

## EXAMPLE 42A

Methyl [N-($N^{im}$-DNP-histidyl)-1-amino-2-cyclohexylethyl] 2-carbomethoxy-3-cyclo-hexylpropylphosphinate

A solution of 2.00 g (2.6 mmol) of the product of Example 41A in 20 ml of methanol was treated all at once with 20 ml of HCl in methanol (134.4 g of HCl in 400 ml of methanol). The reaction mixture was stirred at room temperature for 1 hour 15 minutes. The volatiles were removed completely *in vacuo*, and the residue triturated with 2 X 15 ml of anhydrous ether to afford 1.83 g (99%) of the desired product. NMR ($CDCl_3$) (300 MHz): 0.7-2.0 (m, 28H); 2.2 (m, 1H); 2.8 (m, 1H); 3.4-3.9 (m, 6H); 4.3 (br s, 1H); 4.8 (br s, 1H); 7.4-9.6 (m, 8H) ppm.

## EXAMPLE 43A

Methyl [N-(N-(N-CBZ-2-amino-3-(1-naphthyl)propionyl)-$N^{im}$-DNP-histidyl)-1-amino-2-cyclohexylethyl]2-carbomethoxy-3-cyclohexylpropylphosphinate

A mixture of 0.353 g (1.00 mmol) of the product of Example 11A and 0.312 g (1.1 mmol) of disuccinimidyl oxalate in 10 ml of dry acetonitrile was treated with 0.087 g (0.09 ml; 1.1 mmol) of

pyridine. The reaction mixture was stirred at room temperature under $N_2$ for 16 hours. The mixture turned from heterogeneous to homogeneous during this period. At this time, a mixture of 0.727 g (0.001 moles) of the product of Example 42 and 0.404 g (4.0 mmol; 0.56 ml) of triethylamine in 10 ml of acetonitrile was added all at once. The mixture turned darker and was stirred at room temperature under $N_2$ for 24 hours. At this time, the volatiles were evaporated completely *in vacuo* and the residue dissolved in 50 ml of methylene chloride. The organic solution was washed with 2 X 20 ml of saturated $NaHCO_3$, dried over anhydrous $Na_2SO_4$, filtered, and the volatiles evaporated *in vacuo*. The residue was chromatographed on silica gel using 18:1:1 methylene chloride:acetone:methanol as the eluant to afford 0.631 g (62%) of the desired product as a glassy foam. NMR ($CDCl_3$) (300 MHz): 0.4-1.9 (m, 28H); 2.0-2.2 (m, 1H); 2.3-3.2 (m, 2H); 3.6-3.8 (m, 6H); 4.2-4.8 (m, 2H); 5.1 (d, 2H); 5.3-5.8 (m, 2H); 6.7-8.3 (m, 15H); 8.5 (dt, 2H); 8.8 (q, 1H) ppm.

## EXAMPLE 44A

Methyl [N-(N-(N-BOC-2-amino-3-(1-naphthyl)propionyl)-$N^{im}$-DNP-histidyl)-1-amino-2-cyclohexylethyl] 2-carbomethoxy-3-cyclohexylpropylphosphinate

A mixture of 0.315 g (1.00 mmol) of the product of Example 12 and 0.312 g (0.0011 moles) of disuccinimidyl oxalate in 10 ml of dry acetonitrile was treated with 0.087 g (1.1 mmol; 0.09 ml) of pyridine. The reaction mixture was allowed to stir at room temperature under $N_2$ for 16 hours. During this period the reaction went from heterogeneous to

homogeneous. At this time, a mixture of 0.727 g (1.00 mmol) of the product of Example 42 and 0.404 g (4.00 mmol; 0.56 ml0 of triethylamine in 10 ml of acetonitrile was added all at once. The reaction mixture turned darker and stirring at room temperature under $N_2$ was continued for 24 hours. At this time, the volatiles were removed in vacuo and the residue dissolved in 50 ml of methylene chloride. The organic solution was washed with 2 X 50 ml of saturated $NaHCO_3$, dried over anhydrous $Na_2SO_4$, filtered, and the volatiles removed in vacuo. The residue was chromatographed on silica gel using 18:1:1 methylene chloride:acetone:methanol as the eluant to give 0.571 g (58%) of the desired product as a glassy foam. NMR ($CDCl_3$) (300 MHz)-: 0.7-1.9 (m, containing 9H s at 1.2, 37H); 2.7-3.3 (m, 4H); 3.5-3.8 (m, 6H); 4.4 (m, 3H); 4.7 (m, 1H); 5.0 (m, 1H); 6.8-8.0 (m, 9H); 8.2 (d, 2H); 8.6 (dd, 2H); 8.8 (t, 1H) ppm.

## EXAMPLE 45A

Methyl [N-(N-(N-CBZ-2-amino-4-phenylbutyryl)-$N^{im}$-DNP-histidyl)-1-amino-2-cyclo-hexylethyl] 2-carbomethoxy-4-methylpentylphosphinate

A mixture of 0.313 g (1.00 mmol) of N-CBZ-2-amino-4-phenylbutyric acid and 0.312 g (1.1 mmol) of disuccinimidyl oxalate in 20 ml of dry acetonitrile was treated with 0.087 g (1.1 mmol; 0.90 ml) of pyridine. The reaction mixture was stirred at room temperature under $N_2$ for 24 hours. During this period the reaction went from heterogeneous to homogeneous. At this time a mixture of 0.687 g (0.001 moles) of the product of Example 10 and 404 mg

(4.00 mmol; 0.56 ml) of triethylamine in 10 ml of acetonitrile was added all at once. The reaction mixture was stirred at room temperature under $N_2$ for 30 hours. The volatiles were evaporated completely *in vacuo* and the residue dissolved in 50 ml of methylene chloride. The organic solution was washed with 2 X 15 ml of saturated $NaHCO_3$, dried over anhydrous $Na_2SO_4$, filtered and evaporated *in vacuo*. The residue was chromatographed on silica gel using 18:1:1 methylene chloride:acetone:methanol to give 0.363 g (38%) of the desired product as a glassy foam. NMR ($CDCl_3$) (300 MHz): 0.7-0.9 (m, 6H); 0.9-2.2 (m, 21H); 2.6 (m, 2H); 2.7-3.2 (m, 3H); 3.4-3.7 (m, 6H); 4.1 (m, 1H); 4.3 (m, 1H); 4.7 (m, 1H); 5.0 (q, 2H); 5.3 (m, 1H); 6.8-8.2 (m; 13H); 8.4 (m, 2H); 8.8 (m, 1H) ppm.

## EXAMPLE 46A

Methyl [N-(N-(N-BOC-2-amino-4-phenylbutyryl)-$N^{im}$-DNP-histidyl)-1-amino-2-cyclo-hexylethyl] 2-carbomethoxy-4-methylpentylphosphinate

A mixture of 0.279 g (1.00 mmol) of N-BOC-2-amino-4-phenylbutyric acid and 0.312 g (0.0011 moles) of disuccinimidyl oxalate in 20 ml of dry acetonitrile was treated with 0.087 g (1.1 mmol; 0.90 ml) of pyridine. The reaction mixture was stirred at room temperature under $N_2$ for 24 hours. During this period the reaction mixture went from heterogeneous to homogeneous. At this time, a mixture of 0.687 g (1.00 mmol) of the product of Example 10 and 404 mg (4.00 mmol; 0.56 ml) of triethylamine in 10 ml of acetonitrile was added all at once. The reaction mixture turned darker and was

stirred at room temperature under $N_2$ for 30 hours. The volatiles were removed *in vacuo* and the residue was dissolved in 50 ml of methylene chloride. The organic mixture was washed with 2 X 15 ml of saturated $NaHCO_3$, dried over anhydrous $Na_2SO_4$, filtered, and the volatiles evaporated *in vacuo*. The residue was chromatographed on silica gel using 18:1:1 methylene chloride:acetone:methanol as the eluant. This provided 0.493 g (54%) of the desired product as a glassy foam. NMR ($CDCl_3$) (300 MHz): 0.7-1.0 (m, 6H); 1.0-2.2 (m containing 9H s at 1.5, 28H), 2.6 (m, 2H); 2.7-3.3 (m, 3H); 3.5-3.8 (m, 8H); 4.0 (m, 2H); 4.4 (m, 1H); 4.7 (m, 1H); 5.0 (m, 1H); 6.9-8.0 (m, 7H); 8.5 (m, 2H); 8.8 (m, 1H) ppm.

## EXAMPLE 47A

Methyl [N-(N-(N-CBZ-phenylalanyl)-$N^{im}$-DNP-histidyl)-1-amino-2-cyclohexylethyl]-2-carbomethoxy-4-methylpentylphosphinate

A mixture of 0.156 g (0.55 mmol) of disuccinimidyl oxalate and 0.150 g (0.0005 moles) of N-CBZ-phenylalanine in 10 ml of dry acetonitrile was treated with 0.04 ml (0.5 mmol) of pyridine. The reaction mixture was stirred at room temperature under $N_2$ for 2 hours. At this time, an additional 0.010 g of disuccinimidyl oxalate was added and stirring under $N_2$ continued for 5 hours. During this time the mixture went from heterogeneous to homogeneous. At this time, 0.330 g the product of Example 10 was added, immediately followed by 0.202 g (2.00 mmol; 0.28 ml) of triethylamine. The reaction mixture immediately turned darker. The mixture was stirred at room temperature for 18h. At this time

the volatiles were evaporated completely *in vacuo* and the residue dissolved in 25 ml of ethyl acetate. The organic solution was washed with 3 X 15 ml of saturated $NaHCO_3$, dried over anhydrous $Na_2SO_4$, filtered and the ethyl acetate evaporated *in vacuo*. The residue was chromatographed on silica gel using 18:1:1 methylene chloride:acetone:methanol as the eluant to afford 0.250 g (55%) of the desired product as a glassy foam. NMR ($CDCl_3$) (300 MHz): 0.7-1.0 (m, 6H); 1.0-2.0 (m, 19H); 2.5-3.4 (m, 5H); 3.5-3.7 (m, 6H); 4.0 (br s, 1H); 4.3 (br s, 1H); 5.1 (d, 2H); 5.3 ( m, 1H); 7.1 (m, 2H); 7.2-7.4 (m, 10H); 7.5-8.4 (m, 2H); 8.5 (m, 2H); 8.8 (m, 1H) ppm.

## EXAMPLE 48A

Methyl [N-(N-(N-BOC-phenylalanyl)-$N^{im}$-DNP-histidyl)-1-amino-2-cyclohexylethyl]-2-carbomethoxy-4-methylpentylphosphinate

A mixture of 0.156 g (0.55 mmol) of disuccinimidyl oxalate and 0.133 g (0.50 mmol) of N-BOC-phenylalanine in 10 ml of dry acetonitrile was treated with 0.04 ml (0.50 mmol) of pyridine. The reaction mixture was stirred at room temperature under $N_2$ for 3 hours. At this time, an additional 0.010 g of disuccinimidyl oxalate was added and stirring under $N_2$ continued for 5 hours. During this time the mixture went from heterogeneous to homogeneous. At this time, 0.330 g (0.50 mmol) of the product of Example 10 was added, immediately followed by 0.202 g (2.00 mmol; 0.28 ml) of triethylamine. The reaction mixture immediately turned darker. The mixture was stirred at room temperature for 18.5 hours. At this time the

volatiles were evaporated completely in vacuo and the residue dissolved in 20 ml of ethyl acetate. The organic solution was washed with 3 X 15 ml of saturated $NaHCO_3$, dried over anhydrous $Na_2SO_4$, filtered and the ethyl acetate evaporated in vacuo. The residue was chromatographed on silica gel using 18:1:1 methylene chloride:acetone:methanol as the eluant to afford 0.266 g (61%) of the desired product as a glassy foam. NMR ($CDCl_3$) (300 MHz): 0.7-0.9 (m, 6H); 0.9-2.0 (m containing 3H s at 1.3, 26H); 2.5-3.5 (m, 5H); 3.5-3.7 (m, 6H); 4.0 (br s, 1H); 4.3 (br s, 2H); 4.7 (br s, 1H); 5.0 (m, 1H); 6.8-7.0 (m, 2H); 7.0-7.3 (m, 5H); 7.5-8.0 (m, 2H); 8.6 (m, 2H); 8.9 (m, 1H) ppm.

## EXAMPLE 49A

[N-(N-2-Amino-3-(1-naphthyl)propionyl-histidyl)-1-amino-2-cyclohexylethyl] 2-carboxy-4-methylpentyl-phosphinic acid

A solution of 0.045 g (0.055 mmol) of the product of Example 13A in 3 ml of ethanol was treated with 1.10 ml of 0.100N NaOH (aq.). The reaction mixture was stirred at room temperature for 20 hours, at which time an additional 1.00 ml of 0.100N NaOH was added. Stirring was continued for 1 hour, then the volatiles were evaporated completely in vacuo. The residue was triturated with 3 X 2 ml of anhydrous ether, and the residue was purified by passing it through a column containing ca. 15 g of DOWEX 50W-X4 hydrogen form ion-exchange resin (5.2 meq/dry gram). This afforded 0.007 g (15%) of the desired product as a very light yellow glassy foam. NMR ($CD_3OD$) (300 MHz): 0.6-0.9 (m, 6H); 1.1-2.0 (m, 20H); 2.7 (m, 2H);

3.6 (m, 3H); 4.2 (br s, 1H); 4.4 (m, 1H); 4.6 (m, 1H); 7.2-7.5 (4H); 7.7 (d, 1H); 7.8 (d, 1H); 8.1 (m, 1H) ppm.

## EXAMPLE 50A

Methyl [N-(N-(N-CBZ-2-amino-3-(1-naphthyl)propionyl)-histidyl)-1-amino-2-cyclo-hexylethyl] 2-carbomethoxy-3-cyclohexylpropylphosphinate

A solution of 0.350 g (0.34 mmol) of the product of Example 43A in 4 ml of dry dimethylformamide was treated with ca. 15 ml of anhydrous ammonia in a Fisher-Porter Tube. The tube was sealed and the reaction mixture stirred at room temperature for 21 hours. During this time the reaction color went from dark purple to reddish brown. The tube was opened and the ammonia allowed to evaporate of its own accord. The remaining volatiles were evaporated completely *in vacuo* and the residue triturated with anhydrous ether until a free flowing crystalline solid was obtained.

## EXAMPLE 51A

Methyl [N-(N-(N-BOC-2-amino-3-(1-naphthyl)propionyl)-histidyl)-1-amino-2-cyclo-hexylethyl] 2-carbomethoxy-3-cyclohexylpropylphosphinate

A solution of 0.350 g (0.36 mmol) of the product of Example 44A in 4 ml of dry dimethylformamide was treated with ca. 15 ml of anhydrous ammonia in a Fisher-Porter Tube. The tube was sealed and the reaction mixture stirred at room temperature for 21 hours. During this time the reaction color went from dark purple to reddish brown. The tube was opened and the ammonia allowed to evaporate of its

own accord. The remaining volatiles were evaporated completely in vacuo and the residue triturated with anhydrous ether until a free flowing crystalline solid was obtained.

EXAMPLE 52A

Methyl [N-(N-(N-CBZ-2-amino-4-phenyl)butyryl-histidyl)-1-amino-2-cyclohexyl-ethyl] 2-carbomethoxy-4-methylpentylphosphinate

A solution of 0.200 g (0.21 mmol) of the product of Example 45A in 2 ml of dry dimethylformamide was treated with ca. 12 ml of anhydrous ammonia in a Fisher-Porter Tube. The tube was sealed and the reaction mixture stirred at room temperature for 21 hours. During this period the color of the reaction went from dark purple to reddish-brown. At this time the tube was opened and the ammonia allowed to evaporate of its own accord. The remaining volatiles were evaporated completely in vacuo and the residue was triturated with anhydrous ether until a free flowing solid was obtained.

EXAMPLE 53A

Methyl [N-(N-(N-BOC-2-amino-4-phenyl)butyryl-histidyl)-1-amino-2-cyclohexylethyl] 2-carbomethoxy-4-methylpentylphosphinate

A solution of 0.200 g (0.22 mmol) of the product of Example 46A in 2 ml of dry methylformamide was treated with ca. 12 ml of anhydrous ammonia in a Fisher-Porter Tube. The tube was sealed and the reaction mixture stirred at room temperature for 21 hours. During this period the color of the reaction went from dark purple to reddish-brown. At this time

the tube was opened and the ammonia allowed to evaporate of its own accord. The remaining volatiles were evaporated completely *in vacuo* and the residue was triturated with anhydrous ether until a free flowing solid was obtained.

## EXAMPLE 54A

Methyl (N-histidyl-1-amino-2-cyclohexylethyl) 2-carbomethoxy-4-methylpentylphosphinate

A solution of 0.200 g (0.28 mmol) of the product of Example 10A in 2 ml of dimethylformamide was put into a Fisher-Porter tube and ca. 12 ml of ammonia was condensed in. The tube was sealed and the reaction was stirred at room temperature for 16 hours. The tube was opened and the ammonia was allowed to evaporate of its own accord. The remaining volatiles were evaporated completely *in vacuo* and the residue triturated with anhydrous ether. This afforded the desired product as a yellow solid after purification by chromatography on DOWEX 50W-X4.

## EXAMPLE 55A

Methyl (N-histidyl-1-amino-2-cyclohexylethyl) 2-carbomethoxy-3-cyclohexylpropylphosphinate

A solution of 0.200 g (0.28 mmol) of the product of Example 42A in 2 ml of dimethylformamide was put into a Fisher-Porter tube and ca. 12 ml of ammonia was condensed in. The tube was sealed and the reaction was stirred at room temperature for 16 hours. The tube was opened and the ammonia was allowed to evaporate of its own accord. The

remaining volatiles were evaporated completely *in vacuo* and the residue triturated with anhydrous ether. This afforded the desired product as a yellow solid after purification by chromatography on DOWEX 50W-X4.

EXAMPLE 56A

N-Methyl 1-amino-2-cyclohexylphosphinic acid

This acid is prepared using the procedure described in Example 2A with methylamine hydrochloride replacing aminophenylmethane hydrochloride.

EXAMPLE 57A

Methyl N-CBZ-N-methyl 1-amino-2-cyclohexylethylphosphinate

This ester is prepared from N-methyl-1-amino-2-cyclohexylphosphinic acid using the procedure described in Example 3A.

EXAMPLE 58A

Diethyl N-methyl-1-amino-2-phenylethylphosphonate

This ester is prepared using the procedure described in Example 25A with equimolar amounts of methyl amine hydrochloride and triethylamine substituted for benzylamine.

EXAMPLE 59A

Diethyl N-CBZ-N-methyl-1-amino-2-cyclohexylethylphosphonate

This ester is prepared by treatment of the product of Example 58A with benzyl chloroformate and $Et_3N$ in $CH_2Cl_2$ and is purified by silica gel chromatography.

## EXAMPLE 60A

Diethyl N-CBZ-1-amino-2-cyclohexylethylphosphonate

This ester is prepared by treatment of the product of Example 27A with benzyl chloroformate and $Et_3N$ in $CH_2Cl_2$ and is purified by silica gel chromatography.

## EXAMPLE 61A

Trimethylacetoxymethyl (N-CBZ-1-amino-2-cyclohexylethyl) 2-carbomethoxy-4-methylpentylphosphinate

Treatment of the product of Example 5 with trimethylsilyl bromide in anhydrous $CH_2Cl_2$, followed by dilution of the mixture into brine and extraction with ethyl acetate, affords (N-CBZ-1-amino-2-cyclohexylethyl)-2-carbomethoxy-4-methylpentylphosphinic acid as a light-yellow foam. A solution of this acid (100 mg) in DMF (0.5 ml) was stirred under nitrogen as $isp_2NEt$ (82 µl) and then a solution of chloromethyl trimethylacetate (71 mg) in DMF (0.5 ml) were added. After 3 days, additional $isp_2NEt$ (82 µl) and chloromethyl trimethylacetate (75 mg) were added. After a total of 4 days, the mixture was diluted with ethyl acetate and washed with 1M citric acid and brine and dried ($MgSO_4$), giving a colorless oil. The product (62 mg, 50%) was isolated by preparative tlc (silica gel) in 1:1 ethyl acetate:hexanes. Mass spectrum (FAB) m/e 582 ($M^+$ +1). 300 MHz NMR ($CDCl_3$) 0.8-2.3 (33H, m); 2.9 (1H, m); 3.7 (3H, s); 4.2 (1H, m); 4.8-5.2 (1H, m); 5.15 (2H, m); 7.35 (5H, s).

## EXAMPLE 62A

(1-Amino-2-cyclohexylethyl) 2-carbomethoxy-4-methylpentylphosphinic acid

To the product of Example 8 (60 mg; 0.158 mmol) was added HBr-HOAc solution (5 equivalents) and an equivalent volume of HOAc. The solution was stirred for 24 hours and then concentrated to dryness, giving the product (40 mg; 61%). MS: m/e 334 ($M^+$ +1). NMR ($D_2O$): 0.6-2.3 (22H, m); 2.8 (1H, br s); 3.7 (3H, m); 3.3-3.6 (1H, m); 4.2 (1H, m); 8.0 (3H, br s).

## EXAMPLE 63A

Methyl [N-(N-t-butoxycarbonyl-phenylalanyl)-1-amino-2-cyclohexylethyl] 2-carbomethoxy-4-methylpentylphosphinate

The product of Example 8A (0.26 g) was dissolved in dry $CH_2Cl_2$ (3 ml) and neutralized with $Et_3N$ (0.095 ml) at 0°C. To the above solution were added sequentially N-Boc-phenylalanine (0.22 g), HOBT (0.18 g) and DCC (0.19 g). The mixture after stirring for 4 hours at 0°C was stirred at 25°C for an additional 15 hours. The mixture was filtered, and the $CH_2Cl_2$ phase was washed with $NaHCO_3$ (saturated), water, saturated NaCl and dried ($MgSO_4$). Removal of solvent *in vacuo* gave a foam which was purified by medium pressure liquid chromatography (mplc) using ethyl acetate-hexane (3:1) on a silica gel column yield 0.28 g (70%) (foam). NMR ($CDCl_3$): 7.23 (5H, s), 6.62 (1H, m), 5.15 (1H, m), 4.1-4.41 (2H, m), 3.63 (6H, m), 3.11 (2H, m), 2.85 (2H, m), 0.85-2.05 (32H, m). MS (FAB): m/e 595 ($M^+$ +1).

EXAMPLE 64A

[N-(Phenylalanyl)-1-amino-2-cyclohexylethyl] 2-carbomethoxy-4-methylpentylphosphinic acid hydrochloride

The product of Example 63A (39 mg) was treated with trimethylsilylbromide (0.2 ml) in $CH_2Cl_2$ (0.2 ml) at 25°C for 6 hours. Removal of excess reagent and the solvent *in vacuo* gave an oil which was dissolved in MeOH (5 ml), stirred for 15 minutes at 25°C and evaporation. The foam, thus obtained, was treated with HCl in dioxane (2N) (0.15 ml) at 25°C for 45 minutes. Removal of excess reagent *in vacuo* and addition of dry ether gave the product as a white solid (hygroscopic) (25 mg). NMR ($CDCl_3$): 8.95 (1H, bm), 8.32 (1H, m), 7.85 (1H, m), 7.32 (5H, s), 4.05-4.55 (2H, m), 3.62 (3H, m), 3.28 (2H, m), 2.81 (2H, m), 0.80-2.1 (23H, m). MS (FAB): m/e 481 ($M^+$ +1).

EXAMPLE 65A

Methyl [N-(Nα-BOC-N -CBZ-lysyl)-1-amino-2-cyclohexylethyl] 2-carbomethoxy-4-methylpentylphosphinate

The free amine obtained from the product of Example 8 (0.39 g) and $Et_3N$ (0.141 ml), was coupled with $N^{\alpha}$-BOC-N -CBZ-lysine (0.44 g) in presence of HOBT (0.27 g) and DCC (0.25 g) in dry THF at 0°C. The crude product was obtained, as a foam, which was purified by mplc using ethyl acetate-hexane (3:2). Yield 0.45 g (63%). NMR ($CDCl_3$): 7.34 (5H, s), 6.75 (1H, m), 5.36 (1H, m), 5.12 (3H, m), 4.42 (1H, m), 4.10 (1H, m), 3.66 (6H, m), 3.16 (2H, q), 2.88 (2H, m), 0.88-2.15 (38H, m). MS (FAB): m/e 710 ($M^+$ +1); m/e 610 ($M^+$ +1-100).

EXAMPLE 66A

[N-(Lysyl)-1-amino-2-cyclohexylethyl] 2- carboxy-4-methylpentylphosphinic acid

The product of Example 65A (60 mg) was stirred with aqueous 1N NaOH (0.18 ml) in acetone (1 ml) for 24 hours. Removal of acetone *in vacuo* and acidification with cold 1N HCl gave the free acid as an oil. The oil was then treated with HBr-AcOH (33%) (1 ml) for 12 hours at 25°C. Excess HBr was removed *in vacuo*, and the product was precipitated with dry ether. The hygroscopic solid was filtered and dissolved in MeOH (1 ml), and treated with propylene oxide (0.2 ml). The product was finally precipitated with dry ether. The solid was filtered, washed with dry ether and dried to give white powder (39 mg). NMR ($CD_3OD$): 4.18 (2H, m), 2.85 (2H, m), 0.85-2.15 (29H, m). MS (FAB): m/e 498 ($M^+$ +1).

EXAMPLE 67A

Methyl [N-(Nα-(N-BOC-2-amino-3-(1-naphthyl)propionyl)-N-CBZ-lysyl)-1-amino-2-cyclohexylethyl] 2-carbomethoxy-4-methylpentylphosphinate

The product of Example 65A (0.25 g) was dissolved in 50% TFA in $CH_2Cl_2$ (2 ml), and the mixture was stirred at 25°C for 1-1/2 hours. Removal of excess reagent and $CH_2Cl_2$ *in vacuo* gave an oil which upon drying over $P_2O_5$ and NaOH *in vacuo* gave the amine salt as a foam.

The above foam was dissolved in EtOAc (10 ml) and neutralized with saturated $NaHCO_3$ (aqueous). The EtOAc phase was washed with brine and dried over ($Na_2SO_4$). Removal of solvent *in vacuo* gave the free amine as an oil. The free amine was then coupled

with the product of Example 12A (0.18 g) in the presence of HOBT (0.1 g) and DCC (0.11 g) at 0°C in $CH_2Cl_2$ (5 ml). The crude product was purified by flash chromatography using EtOAc-hexane (3:1). Yield 0.25 g (foam). NMR ($CDCl_3$): 6.85-8.1 (13H, m), 6.56 (1H, m), 5.05-5.40 (4H, m), 4.25-4.55 (3H, m), 3.68 (6H, m), 3.12 (2H), 2.85 (2H, m), 0.80-2.20 (39H, m). MS (FAB): m/e 907 ($M^+$ +1).

EXAMPLE 68A

Methyl [N-(Nα-(2-amino-3-(1-naphthyl)propionyl)-N -CBZ-lysyl)-1-amino-2-cyclohexylethyl] 2-carbomethoxy-4-methylpentylphosphinate hydrochloride

The product of Example 67A (60 mg) was dissolved in 50% TFA in $CH_2Cl_2$ (1 ml) at 25°C for 2 hours. Removal of solvent *in vacuo* gave a foam which upon drying *in vacuo* over $P_2O_5$ and NaOH gave a glass-like hygroscopic solid (45 mg). The solid was then treated with 1N HCl in MeOH, and the solvent was removed *in vacuo* to give the desired product as hydrochloride salt (40 mg). NMR ($CDCl_3$): 6.8-8.3 (15H, m), 5.35-5.55 (2H, m), 5.12 (2H, s), 4.45 (2H, m), 4.15 (1H, m), 3.65 (6H, m), 3.10 (2H, m), 2.80 (2H, m), 0.80-2.1 (30H, m). MS (FAB): m/e 807 ($M^+$ +1). FAB-MS: $(M+H)^+$ 807.

EXAMPLE 69A

Methyl [N-(Nα-(N-BOC-2-amino-3-(1-naphthyl)propionyl)-lysyl-1-amino-2-cyclohexylethyl] 2-carbomethoxy-4-methylpentylphosphinate

The product of Example 68A (50 mg) was treated with $H_2$ in the presence of Pd-C (10%) (10 mg) in MeOH (5 ml) (containing AcOH (7 μl)) for 3

hours at 40 psi. The catalyst was filtered off and the filtrate upon evaporation *in vacuo* gave the pure product as the acetate salt (40 mg). NMR ($CDCl_3$): 6.95-8.1 (11H, m), 5.60 (1H, d), 5.22 (1H, m), 4.51 (2H, m), 4.12 (1H, m), 3.68 (6H, m), 2.6-2.95 (4H, m), 2.05 (3H, s), 0.85-2.15 (39H, m). MS (FAB): m/e 773 ($M^+$ +1).

## EXAMPLE 70A

Methyl (1-amino-2-cyclohexylethyl)carbomethoxymethylphosphinate hydrochloride

The product of Example 30A (3.58 g) was hydrogenated in methanol (30 ml) [containing concentrated HCl (0.72 ml)] over Pd-C (10%) (0.35 g) at 40 psi overnight at 25°C. The catalyst was filtered off and the filtrate was evaporated to give the product as glass-like solid foam (2.67 g) (94%). MS (FAB): m/e 278 ($M^+$ +1).

## EXAMPLE 71A

Methyl [(N-BOC-phenylalanyl)-1-amino-2-cyclohexylethyl] carbomethoxymethylphosphinate

The product of Example 70A (0.78 g) was treated with $Et_3N$ (0.35 ml) in a mixture of $THF-CH_2Cl_2$ (1:1) (6 ml) at 0°C. The free amine, thus obtained, was coupled with N-BOC-phenylalanine (0.66 g) in presence of DCC (0.56 g) and HOBT (0.5 g) under standard condition. Filtration and processing of the filtrate gave the crude product as a foam which was purified by flash chromatography on silica gel using 10% hexane in ethyl acetate. Yield (1.18 g) (91%) (foam). MS: M+H = 525, M-99 = 425 (-BOC). $^1H$ NMR: 7.35-7.15 (5H, m), 5.15 (1H, br s), 4.90

(1H, br s), 4.6 (1H, m), 4.4 (1H, m), 3.85-3.65 (6H, m), 3.20-3.0 (2H, m), 3.0 (2H, d, J=15Hz), 2.0-1.7 (13H, m), 1.4 (9H, m).

EXAMPLE 72A

(N-Phenylalanyl-1-amino-2-cyclohexylethyl) 2-carbomethoxymethylphosphinic acid hydrobromide

The product of Example 71A (43 mg) was treated with HBr-AcOH (33%) (0.15 ml) at 25°C for 16 hours. Excess HBr was removed *in vacuo*, and the residue was dissolved in ethyl acetate (2 ml) and treated with excess propylene oxide. Removal of solvent *in vacuo* and drying over $P_2O_5$ and NaOH gave the titled compound as a glass like solid (24 mg). NMR ($CDCl_3$): 7.9-7.5 (1H, m), 7.35 (5H, m), 4.4-4.0 (3H, m), 3.74 (3H, m), 3.31-2.9 (1H, m), 2.9-2.6 (2H, dd), 2.0-0.8 (13H, m). MS (FAB): m/e 411 ($M^+$ +1).

EXAMPLE 73A

Methyl [N-(Nα-BOC-N -CBZ-lysyl)-1-amino-2-cyclohexylethyl] carbomethoxymethylphosphinate

The product of Example 70A (0.75 g) was dissolved in a mixture of dry THF (3 ml) and $CH_2Cl_2$ (3 ml) and neutralized with $Et_3N$ (0.33 ml). $N^{\alpha}$-BOC-N -CBZ-lysine N-hydroxysuccinimide ester (0.39 g) was added to the above solution, and the mixture was stirred at 25°C for 24 hours. The crude product obtained, after concentration of the reaction mixture, was purified by flash chromatography on silica gel using ethyl acetate as the eluent. Yield (0.5 g) (foam). NMR ($CDCl_3$): 7.36 (5H, s), 6.45 (1H, brs), 5.4-4.7 (2H, m), 5.1

(2H, s), 4.6 (1H, m), 4.05 (1H, m), 3.85-3.60 (6H, m), 3.2 (2H, brs), (2H, d, J=10), 1.90-.8 (19H, m), 1.60, 1.40 (9H, s). MS (FAB): m/e 640 ($M^+$ +1).

EXAMPLE 74A

Methyl [N-(Nα-BOC-lysyl)-1-amino-2-cyclohexylethyl] carbomethoxymethylphosphinate

The product of Example 73A (60 mg) was hydrogenated over Pd/C (10%) in MeOH (5 ml) containing 1 equivalent of acetic acid under standard conditions to give the titled compound as a foam (51 mg). NMR ($CDCl_3$): 8.5 (3H, br s), 8.2-7.6 (1H, m), 5.9-5.5 (1H, m), 4.5 (1H, m), 4.1 (1H, br s), 3.7 (6H, m), 3.2-2.8 (4H, m), 2.0 (3H, s), 1.90-.7 (19H, m), 1.4 (9H, s). MS: m/e 506 ($M^+$ +1).

EXAMPLE 75A

Methyl [N-(N-(2-naphthyloxy)acetyl-phenylalanyl)-1-amino-2-cyclohexylethyl] carbomethoxymethylphosphinate

The product of Example 71A (0.395 g) was treated with trifluoroacetic acid $-CH_2Cl_2$ (1:1) (5 ml) at 25°C for 2 hours. Removal of excess reagent *in vacuo* gave the trifluoroacetate salt of the free amine as a foam, which was dissolved in dry THF (5 ml) and neutralized with $Et_3N$ (0.12 ml). To the free amine, thus obtained, (2-naphthoxy)-acetic acid N-hydroxysuccinimide ester (0.31 g) was added, and the mixture was stirred at 25°C for 24 hours. The crude product obtained, after removal of solvent *in vacuo*, was purified by mplc using 25% hexane in ethyl acetate. Yield 0.34 g (65%) (foam). MS: M+H = 609. $^1H$ NMR: 7.8-7.0 (12H, m), 6.9 (1H,

d, J=15Hz), 4.90 (1H, m), 4.7-4.4 (3H, m), 3.8-3.6 (6H, m), 3.25-2.9 (4H, m), 3.0-2.8 (2H, d, J=15Hz), 1.9-0.7 (13H, m).

EXAMPLE 76A

Methyl [N-(N-(N-CBZ-2-amino-3-(2-naphthyl)propionyl-phenylalanyl)-1-amino-2-cyclohexylethyl] carbomethoxy-methylphosphinate

The product of Example 71A (0.43 g) was reacted with 50% TFA in $CH_2Cl_2$ (5 ml) at 25°C for 2.5 hours. Removal of excess reagent in vacuo gave the corresponding TFA salt as a foam. The above salt was dissolved in dry THF (5 ml) and treated with $Et_3N$ (0.13 ml) followed by the addition of N-CBZ-2-amino-3-(1-naphthyl)propionic acid (0.33 g), HOBT (0.19 g) and DCC (0.21 g). The mixture was stirred at 0°C for 2 hours and at 25°C for 16 hours. The reaction mixture was filtered, and the filtrate was diluted with EtOAc (20 ml) washed with saturated $NaHCO_3$, water and dried ($MgSO_4$). Removal of the solvent gave the crude product as an oil, which was purified by flash chromatography on silica gel using 20% hexane in ethyl acetate. Yield 0.49 g (69%) (foam). $^1$NMR ($CDCl_3$): 8.2-7.0 (17H, m), 6.9-6.2 (2H, m), 5.9-5.5 (1H, m), 5.25-4.90 (4H, m), 4.85-4.6 (1H, m, CH), 4.6-4.2 (3H, m), 3.8-3.3 (6H), 3.0-2.6 (2H, m), 1.9-0.7 (11H, m).

EXAMPLE 77A

Methyl [N-(Nα-(N-CBZ-2-amino-3-(2-naphthyl)propionyl)-N -CBZ-lysyl)-1-amino-2-cyclohexylethyl] carbomethoxy-methylphosphinate

The product of Example 73A (0.23 g) was treated with 50% TFA-$CH_2Cl_2$ (2.5 ml) at 25°C and

the corresponding trifluoroacetate salt of the amine was obtained as described before. The salt was dissolved in dry THF (4 ml), neutralized with $Et_3N$ (0.05 ml) and coupled with N-CBZ-ß-naphthylalanine (0.125 g) in the presence of HOBT (0.072 g) and DCC (0.081 g) under standard conditions. The reaction was filtered, and the filtrate was washed with saturated $NaHCO_3$, water and dried ($MgSO_4$). Removal of the solvent gave an oil which was purified by mplc using 20% hexane in ethyl acetate. Yield 0.21 (68%). $^1$H NMR: 8.3-7.1 (17H, m), 7.0-5.2 (4H, m), 5.1 (4H, m), 4.4-4.0 (2H, m), 4.5 (2H, m), 3.8-3.4 (6H, m), 3.1-2.8 (4H, m), 1.9-.6 (19H, m).

Claims to the invention follow.

WHAT IS CLAIMED IS:

1. A peptide of the formula:

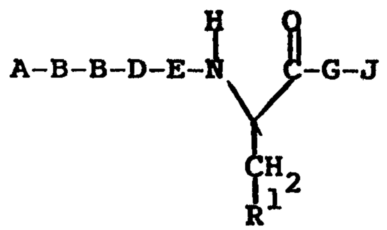

(I.)

wherein:

A is hydrogen; or 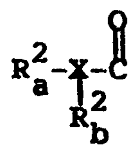

where

X is -O-; -O-CH-; -CH-O-; -CH-; -NH-CH-; or -S-CH-; and

$R^2_a$ and $R^2_b$ may be the same or different and are hydrogen; W-$(CH_2)_n$- or W-$(CH_2)_m$-CH=CH-$(CH_2)_p$, where W is hydrogen; $C_{1-4}$alkyl; aryl; $C_{3-7}$cycloalkyl; or $C_{3-7}$cycloalkyl or aryl substituted with up to five members independently selected from the group consisting of $C_{1-8}$alkyl, trifluoromethyl, hydroxy, $C_{1-4}$alkoxy, and halo; n is 0 to 5; m is 0 to 2; and p is 0 to 2; except that where X is -O-, only one of $R^2_a$ or $R^2_b$ is present;

B is absent; glycyl; sarcosyl; or 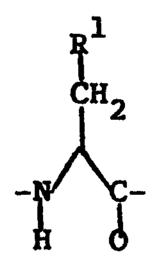 ,

where $R^1$ is as defined further below;

D is absent; or 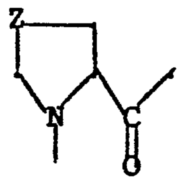 , where Z is $-(CH_2)_1-$ and 1 is 1 or 2; or -S-;

E is absent; or 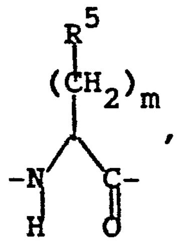 , where m is 1 to 4; and

$R^5$ is hydrogen; $C_{1-4}$ alkyl; aryl; aryl-$C_{1-4}$ alkyl; aryl $C_{1-4}$ alkyl or aryl where the aryl portion is substituted with up to three members selected from the group consisting of $C_{1-4}$ alkyl, trifluoromethyl, hydroxy, $C_{1-4}$ alkoxy, and halo; or indolyl;

G is (1) 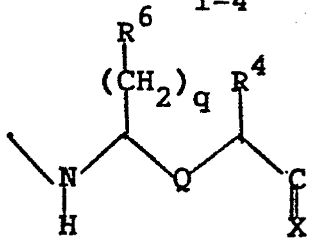 . where q is 1 to 4; X is O, or H, H;

$R^4$ is hydrogen; or 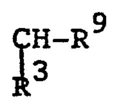 ,

where $R^9$ is hydrogen; $C_{1-4}$alkyl; hydroxy, or $C_{3-7}$cycloalkyl; and $R^3$ is hydrogen; $C_{1-4}$alkyl; aryl; aryl $C_{1-4}$alkyl; aryl $C_{1-4}$alkyl or aryl substituted with up to three members selected from the group consisting of $C_{1-4}$alkyl, trifluoromethyl, hydroxy, $C_{1-4}$alkoxy, and halo; or indolyl;

$R^6$ is $C_{3-6}$ alkyl; $C_{3-7}$ cycloalkyl; aryl; or $C_{3-7}$cycloalkyl or aryl substituted with up to three members selected from the group consisting of $C_{1-4}$alkyl, trifluoromethyl, hydroxy, $C_{1-4}$alkoxy, and halo; and

Q is 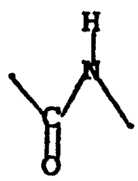 ; 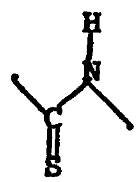 ; 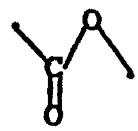 ; 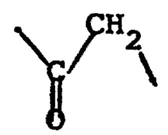 ;

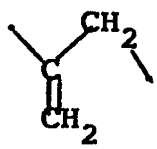 ; 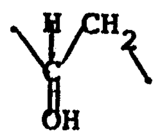 ; 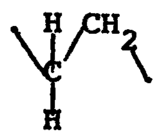 ; 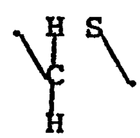 ;

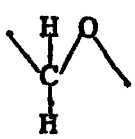 ; 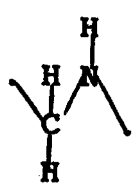 ; 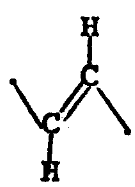 ; 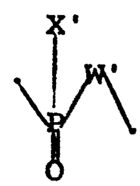 ;

wherein X' is hydroxy; $OR_4'$ wherein $R_4'$ is hydrogen, alkyl, alkenyl, alkynyl, aryl, or arylalkyl, each of which may be optionally substituted with up to three members selected from amino, alkylamino, dialkylamino, trialkylammonium, hydroxy, alkoxy, alkyl, halo or $R_a^4$-CO-V'-$CR_b^4R_c^4$ wherein $R_a^4$ is alkyl, alkenyl, alkynyl or aryl, $R_b^4$ and $R_c^4$ are hydrogen, alkyl, alkenyl or alkynyl

and V' is -O- or -NH-; amino; or mono- or di-$C_{1-4}$alkyl amino; and W' is absent; -O-; -NH-; or $-CH_2-$;

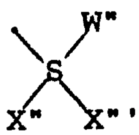

where X" and X"' are independently

absent; or 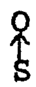; and

W" is absent; $-CH_2-$; or 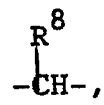, where $R^8$ is hydrogen or $C_{1-3}$ alkyl;

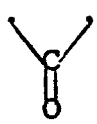 ; 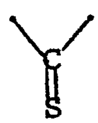 ; or

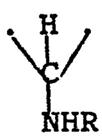 ; where R is hydrogen; $C_{1-4}$ alkyl; formyl; $C_{1-4}$ alkanoyl; aroyl; carboxy; $C_{1-4}$ alkoxycarbonyl; aryloxycarbonyl; or aryl $C_{1-4}$ alkoxycarbonyl; or

(2) 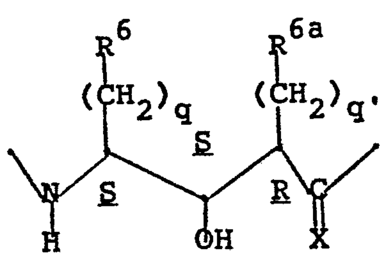 where q is 1 to 4; q' is 0 to 4; X is O or H, H;

$R^6$ is as defined above; and

$R^{6a}$ is hydrogen; $C_{1-8}$alkyl; $C_{2-8}$alkyl substituted with one or two members independently selected from the group consisting of hydroxy, carboxy, carboxy ester or amide, amino, mono-, di-, or tri-$C_{1-4}$alkylamino, and guanidyl; wherein said substitution occurs on the last 1 or 2 carbon atoms of the alkyl chain; aryl; $C_{3-7}$cycloalkyl; or aryl or $C_{3-7}$cycloalkyl substituted with up to three members selected from the group consisting of $C_{1-4}$alkyl, trifluoromethyl, hydroxy, $C_{1-4}$alkoxy, and halo; wherein the substituent of the above formula has 2<u>R</u>, 3<u>S</u>, 4<u>S</u> configuration;

J is (1) $-Y-(CH_2)_n-R^7$

where

Y is -NH-, -O- or $N(CH_2)_n-R^7$;

n is 0 to 5; and

$R^7$ is hydrogen, <u>provided</u> that where n is 0 and $R^7$ is hydrogen, that G is other than Sta and E is other than Phe; hydroxy; $C_{1-4}$alkyl; $C_{3-7}$cycloalkyl; aryl; aryl substituted with up to five members independently selected from the group consisting of $C_{1-6}$alkyl, trifluoromethyl, hydroxy, $C_{1-4}$alkoxy, amino, mono- or di- $C_{1-4}$ alkylamino, and halo; $N(R')_2$, where R' may be the same or different and is hydrogen, $C_{1-4}$alkyl, aryl, aryl $C_{1-4}$alkyl, heterocyclic, or

heterocyclic $C_{1-4}$alkyl;
$N(R')^{+}{}_{3}A^{-}$, where R' is as defined above, and $A^{-}$ is a counterion;
guanidyl; heterocyclic; heterocyclic substituted with up to five members independently selected from the group consisting of $C_{1-6}$alkyl, hydroxy, trifluoromethyl, $C_{1-4}$alkoxy, halo, aryl, aryl $C_{1-4}$alkyl, amino, and mono- or di-$C_{1-4}$alkylamino; or heterocyclic substituted with another, the same or different, heterocyclic;

(2) 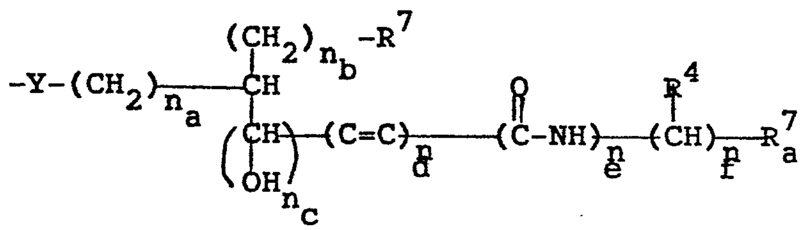

where
Y is as defined above;
$n_a$ is 0 or 1;
$n_b$ is 1 to 4;
$n_c$ is 0 or 1;
$n_d$ is 0 or 1;
$n_e$ is 0 or 1, provided that $n_e$ cannot be 1 when $n_d$ is 0;
$n_f$ is 1 to 4;

$R^4$ is hydrogen; or $-\underset{R^3}{\underset{|}{C}}H-R^9$, where

$R^9$ is hydrogen; $C_{1-4}$alkyl; hydroxy; or

$C_{3-7}$cycloalkyl; and $R^3$ is hydrogen; $C_{1-4}$alkyl; aryl; aryl $C_{1-4}$alkyl; aryl $C_{1-4}$alkyl or aryl substituted with up to three members selected from the group consisting of $C_{1-4}$alkyl, trifluoromethyl, hydroxy, $C_{1-4}$alkoxy, and halo; or indolyl; and

$R^7$ and $R^7_a$ may be the same or different and have the same meaning as $R^7$ above and $R^7_a$ may additionally be

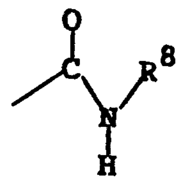 or 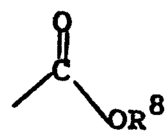,

where $R^8$ is hydrogen or $C_{1-3}$alkyl;

(3) 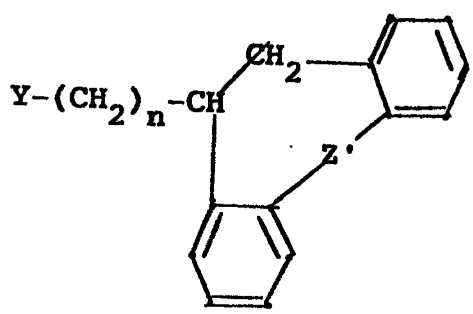

where

Y is as defined above;

n is 0 or 1; and

Z' is

(a) - 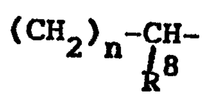

where

n is 0 or 1; and

$R^8$ is as defined above; or

(b) 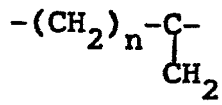

where

n is 0 or 1; or

(4) (a) 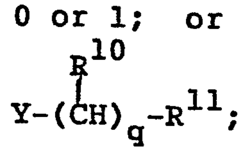; (b) 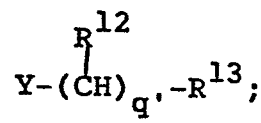; or

(c) 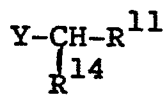

where

Y is -NH- or -O-;

q is 1-5;

q' is 0-5;

$R^{10}$ is hydrogen; hydroxy; $N(R")_2$, where R" may be the same or different and is hydrogen or $C_{1-4}$alkyl; guanidyl; or $N^+(R")_3A^-$, where R" is as defined above, and $A^-$ is a counterion; provided that at least one $R^{10}$ is not hydrogen;

$R^{11}$ is $C_{1-4}$alkyl; $C_{3-7}$cycloalkyl; aryl; aryl substituted with up to three members independently selected from the group consisting of $C_{1-6}$alkyl, trifluoromethyl, hydroxy, $C_{1-4}$alkoxy, amino, mono- or di- $C_{1-4}$alkylamino, amino

$C_{1-4}$alkyl, mono-, di-, or tri-$C_{1-4}$alkylamino-$C_{1-4}$alkyl, halo, carboxy, carboxy ester or amide, carboxy-$C_{1-4}$alkoxy, carboxy-$C_{1-4}$-alkoxy ester or amide, α-aminocarboxy-$C_{1-4}$alkyl, α-aminocarboxy-$C_{1-4}$alkyl ester or amide, carboxy-$C_{1-4}$alkyl, carboxy-$C_{1-4}$alkyl ester or amide, guanidyl, and guanidyl-$C_{1-4}$alkyl; carboxy, ester or amide; sulfo; heterocyclic; or heterocyclic substituted with up to five members independently selected from the group consisting of $C_{1-6}$alkyl, hydroxy, trifluoromethyl, $C_{1-4}$alkoxy, halo, aryl, aryl $C_{1-4}$alkyl, amino, and mono- or di-$C_{1-4}$alkylamino;

$R^{12}$ is hydrogen; or carboxy, ester or amide;

$R^{13}$ is carboxy, ester or amide; sulfo; or aryl substituted with up to three members selected from the group consisting of amino-$C_{1-4}$alkyl, mono-, di-, or tri-$C_{1-4}$-alkylamino-$C_{1-4}$-alkyl, halo, carboxy, carboxy ester or amide, carboxy-$C_{1-4}$alkoxy, carboxy-$C_{1-4}$alkoxy ester or amide, α-amino-carboxy-$C_{1-4}$alkyl, α-aminocarboxy-$C_{1-4}$alkyl ester or amide, carboxy-$C_{1-4}$alkyl, carboxy-$C_{1-4}$alkyl ester or amide,

guanidyl, and guanidyl-$C_{1-4}$alkyl; and

$R^{14}$ is carboxy, ester or amide;

(d) 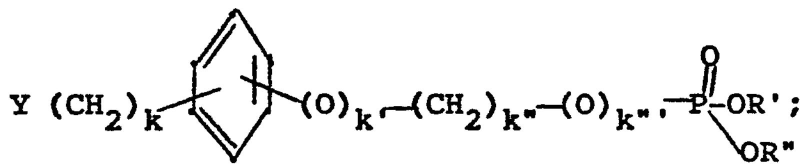 ; or

(e) 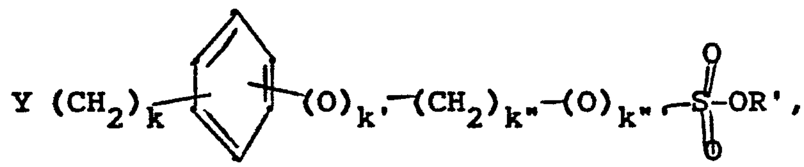 ,

where

Y is -NH- or -O-;

k is 0-4;

k' is 0 or 1;

k" is 0-4;

k"'is 0 or 1;

R' is hydrogen or $C_{1-4}$alkyl; and

R" is hydrogen or $C_{1-4}$alkyl;

(5) 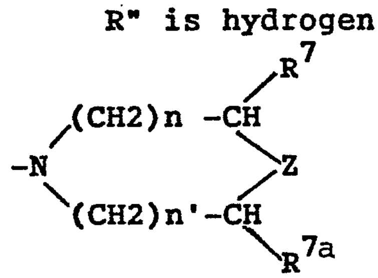

where Z is NH, N-$R^7$,O,S or $CHR^7$;

n' is 0 to 5; and

$R^{7a}$ is hydrogen, hydroxy, $C_{1-4}$-alkyl,$C_{3-7}$-cydoalkyl, aryl,ary 1 substituted with from one to five members independently selected from the group consisting of $C_{1-6}$-alkyl trifluoromethyl, hydroxy, $C_{1-4}$alkoxy, amino, mono- or di- $C_{1-4}$ alkylamino,

and halo; $N(R')_2$, where R' may be the same or different and is hydrogen, $C_{1-4}$alkyl, aryl, aryl $C_{1-4}$alkyl, heterocycli, or heterocyclic $C_{1-4}$alkyl; $N(R')_3^+A^-$, where R' is as defined above, and $A^-$ is a counterion; guanidyl; heterocyclic; heterocyclic substitutued with up to five members independently selected from the group consisting of $C_{1-6}$alkyl, hydroxy, trifluoromethyl, $C_{1-4}$alkoxy, halo, aryl, aryl $C_{1-4}$alkyl, amino, and mono- or di-$C_{1-4}$alkylamino; or heterocyclic substituted with another, the same or different, heterocyclic;

$R^1$ is hydrogen; $C_{1-4}$ alkyl; hydroxy $C_{1-4}$alkyl; aryl; aryl substituted with up to three members selected from the group consisting of $C_{1-4}$ alkyl, trifluoromethyl, hydroxy, $C_{1-4}$ alkoxy, and halo; indolyl; 4-imidazolyl; amino $C_{2-4}$ alkyl; acyl $C_{2-4}$ alkyl wherein the acyl is $R^9-\overset{O}{\overset{\|}{C}}-$ and $R^9$ is as defined above; guanidyl $C_{2-3}$ alkyl; or methylthiomethyl;

wherein all of the asymmetric carbon atoms have an S configuration, except for those in the B, D, and G substituents, which may have an S or R configuration;

and a pharmaceutically acceptable salt thereof.

2. A peptide according to Claim 1 wherein the peptide is a member selected from the group consisting essentially of:

BOC-His-Pro-Phe-His-Sta-OEt

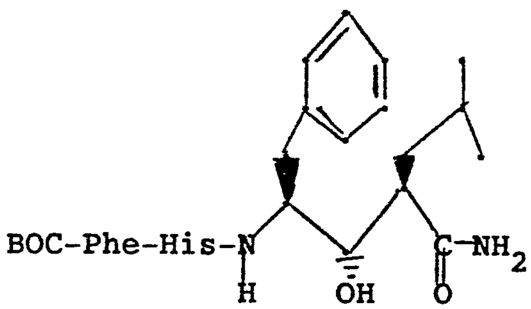

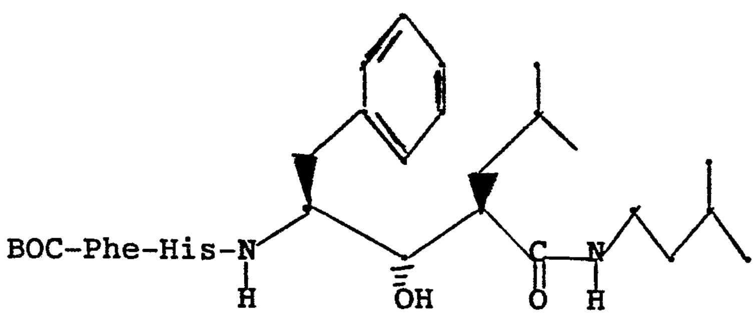

BOC-Phe-His-ACHPA-$NH_2$

BOC-HomoPhe-His-Sta-$NH_2$

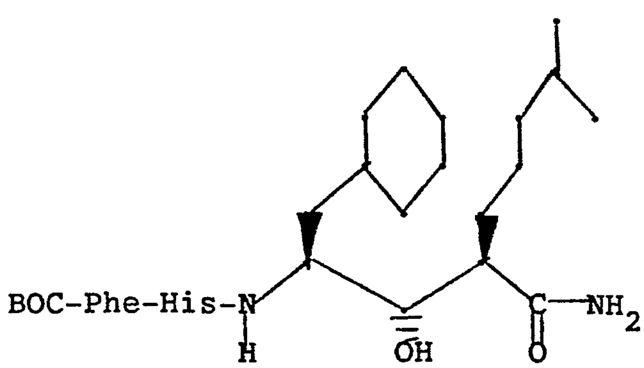

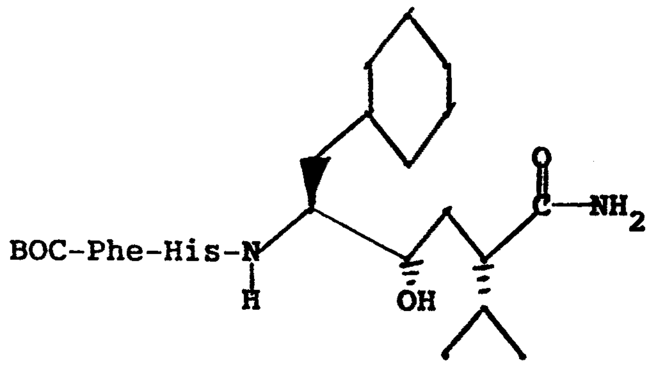

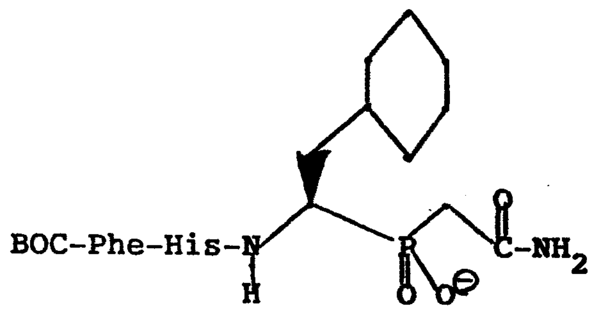

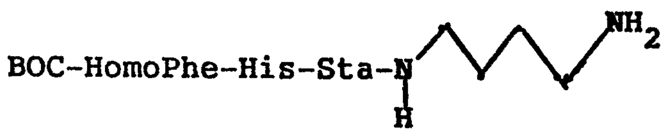

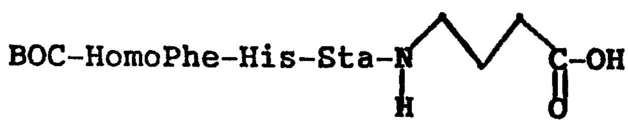

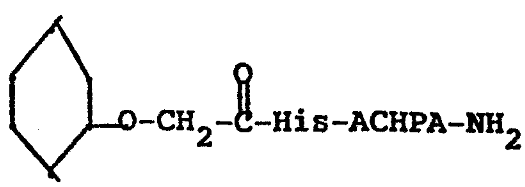

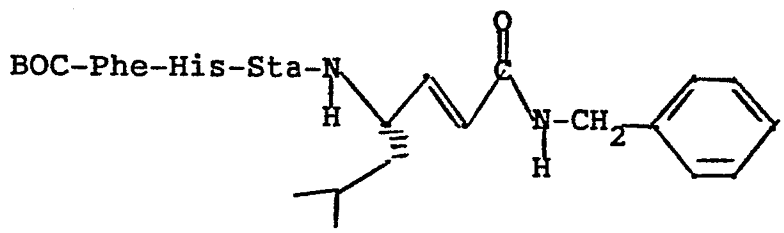

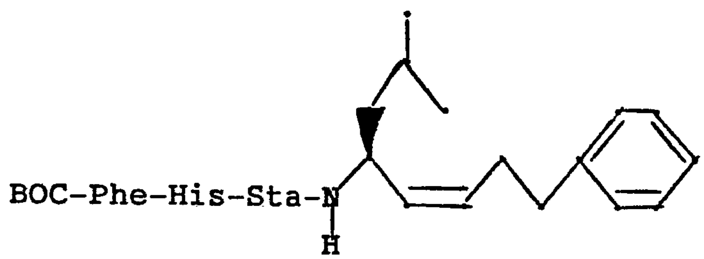

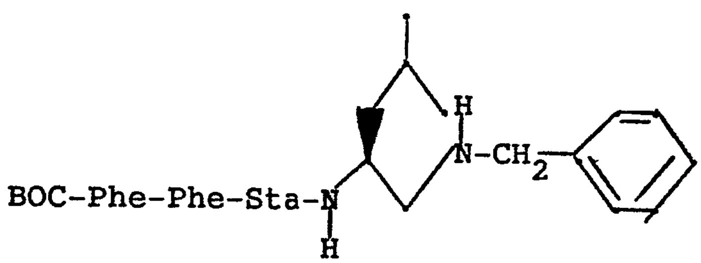

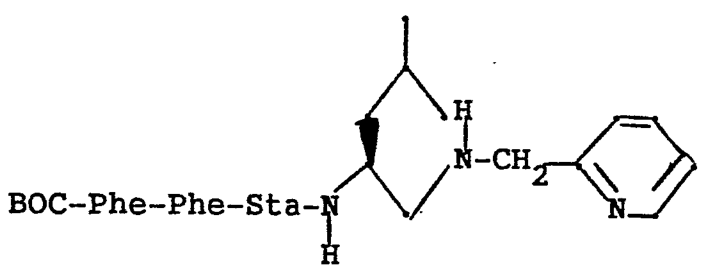

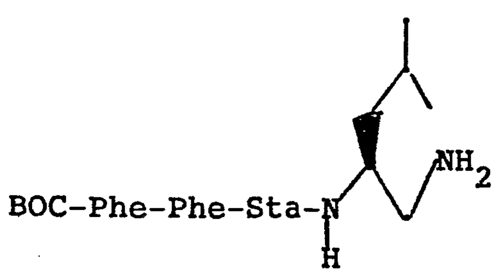

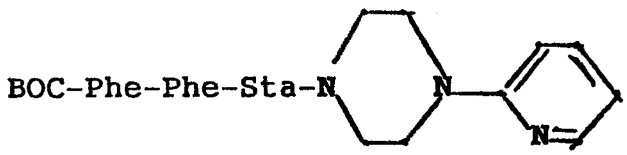

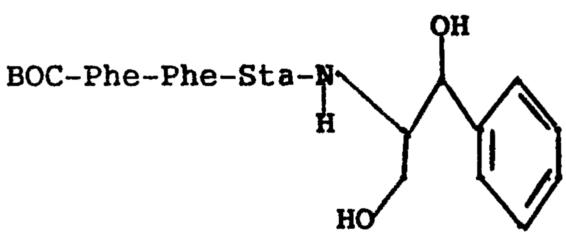

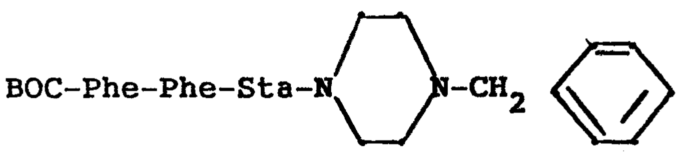

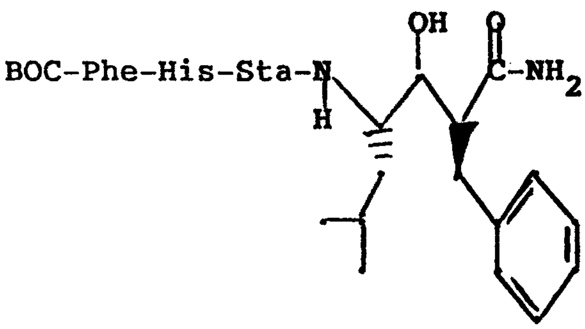

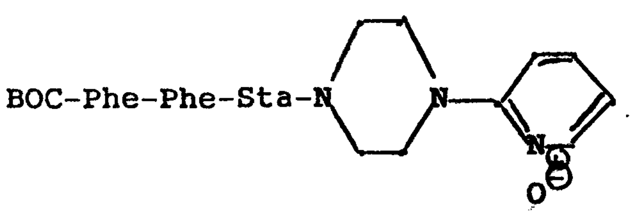

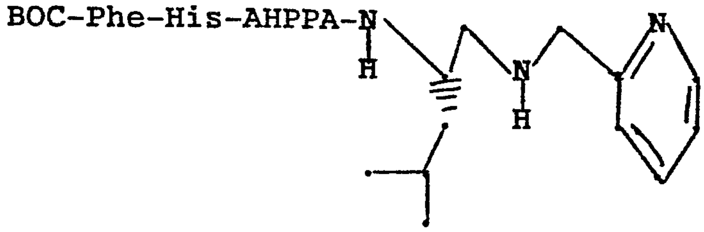

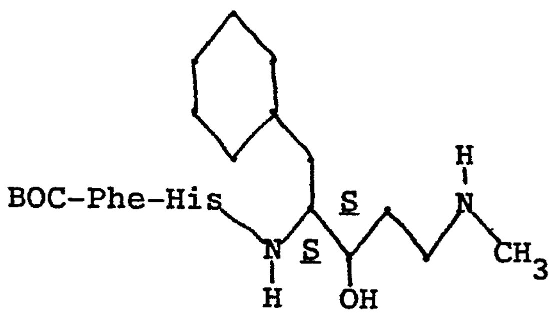

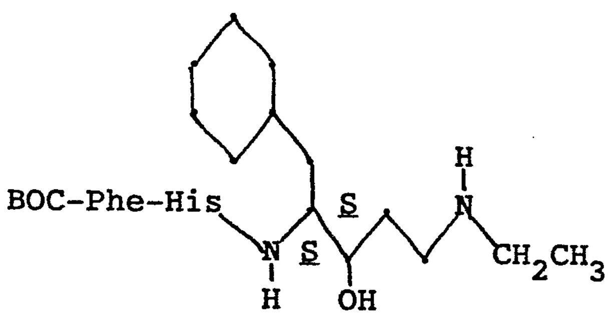

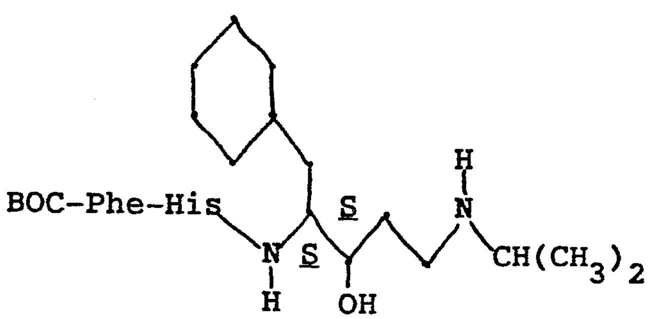

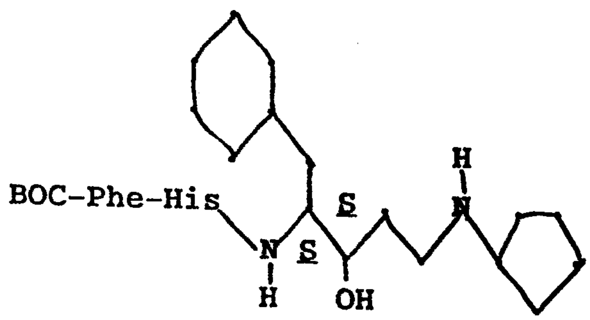

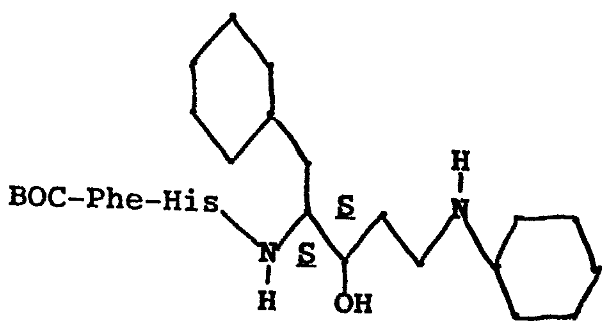

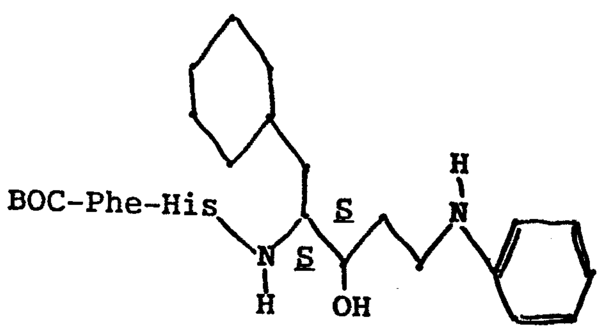

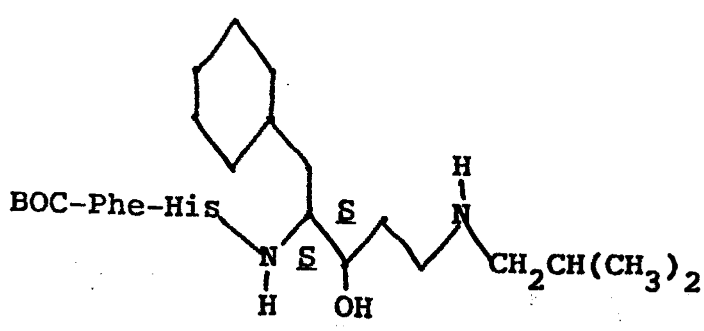

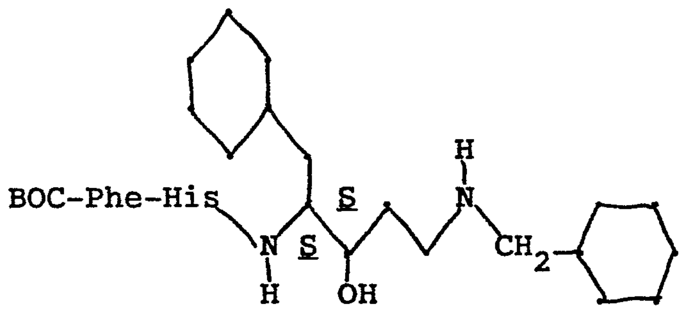

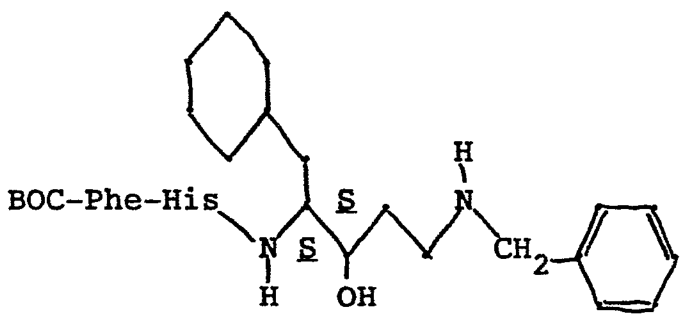

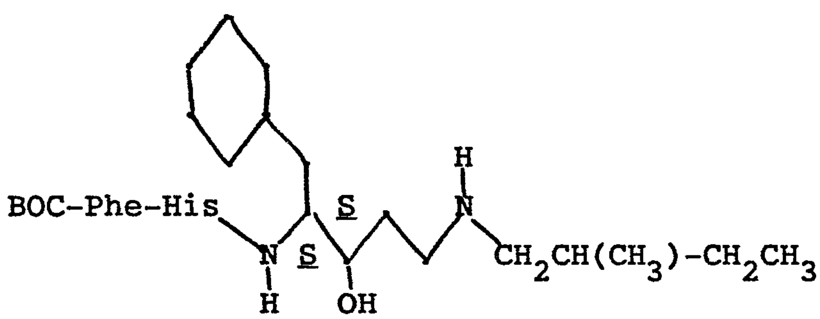

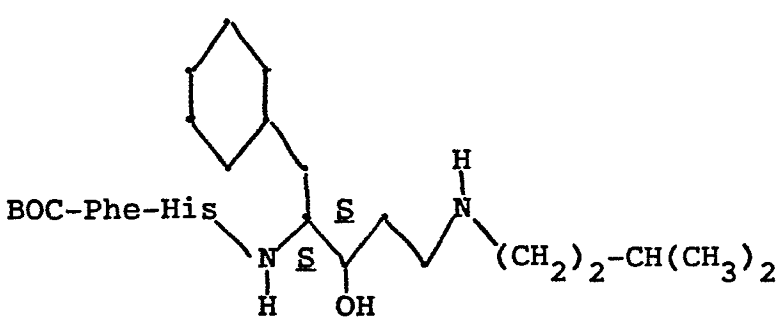

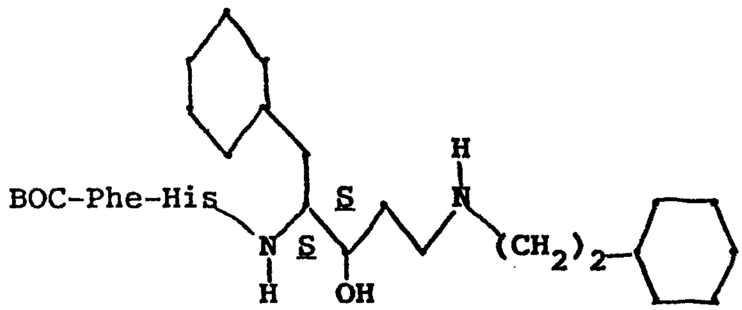

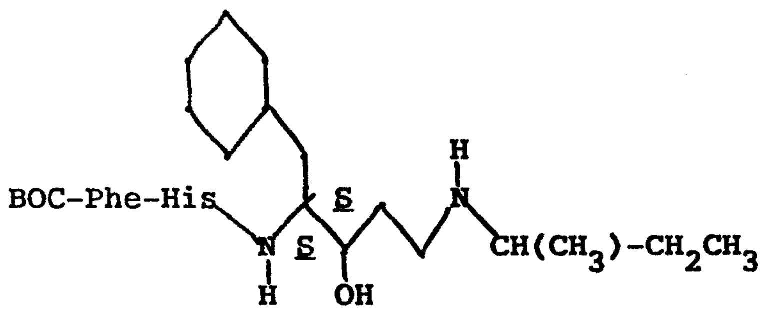

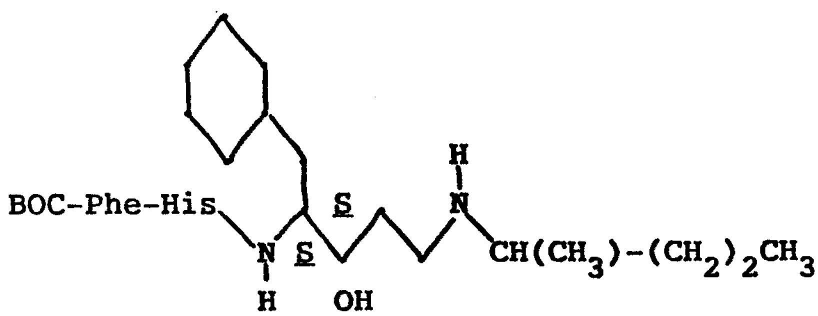

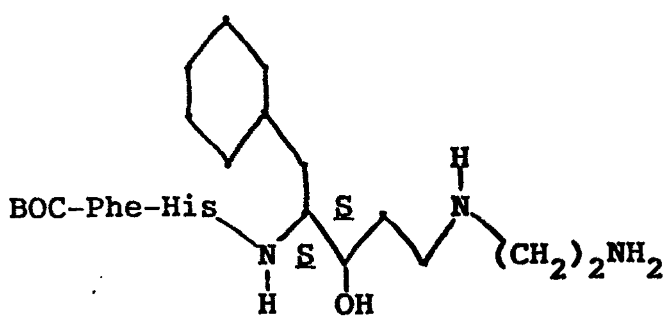

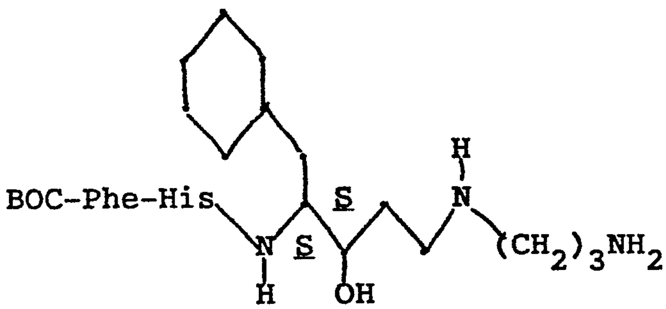

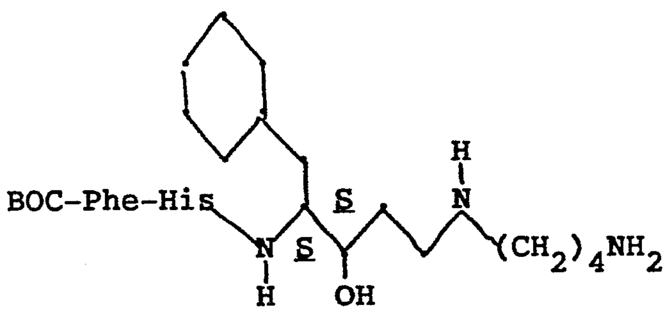

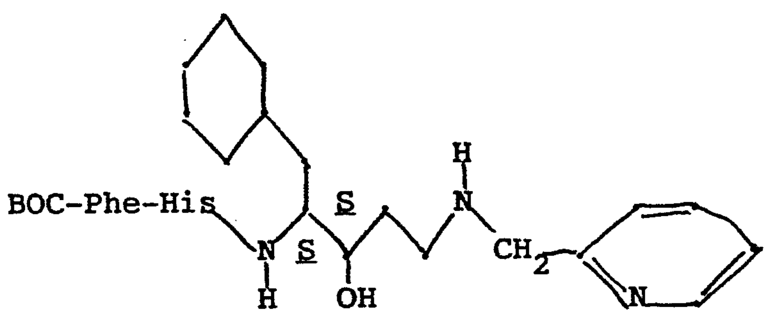

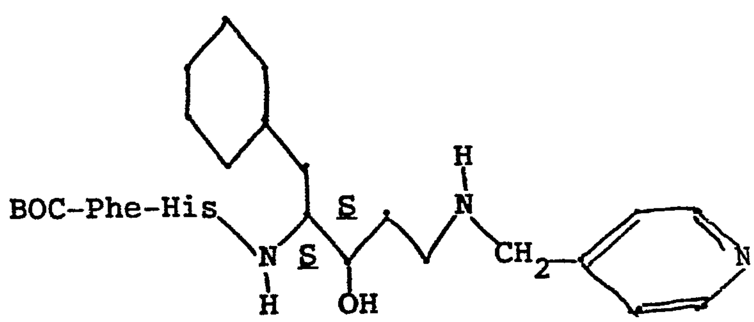

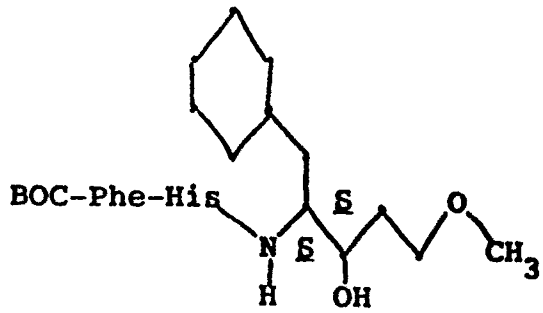

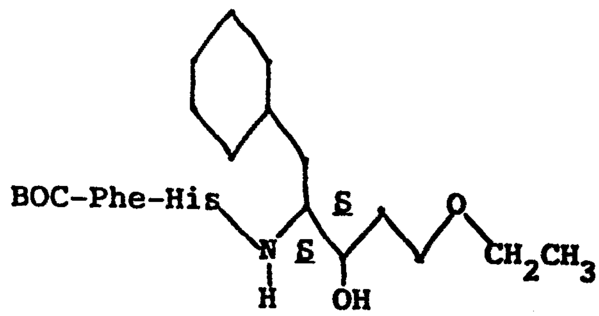

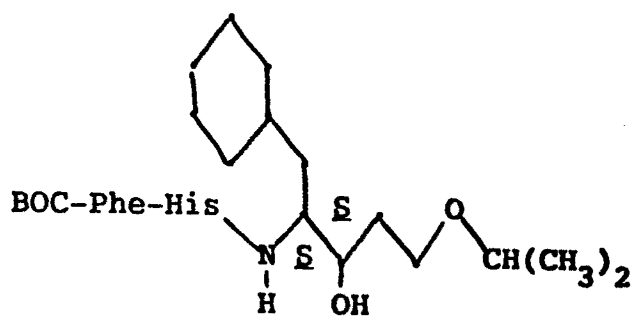

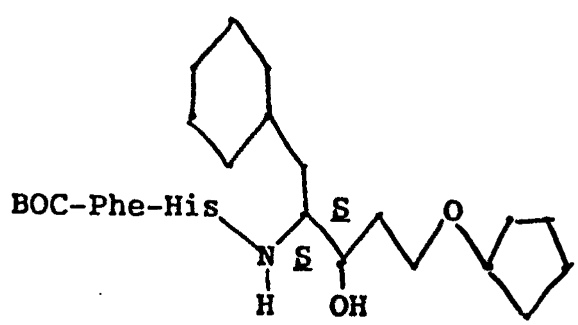

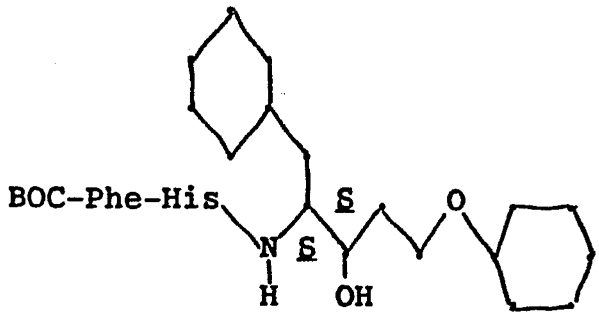

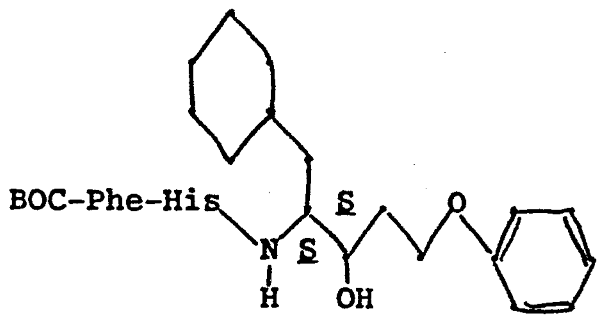

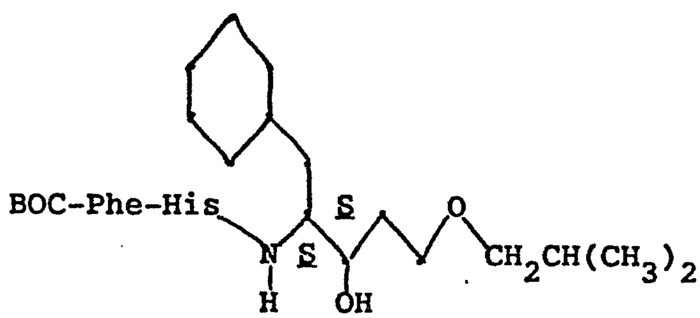

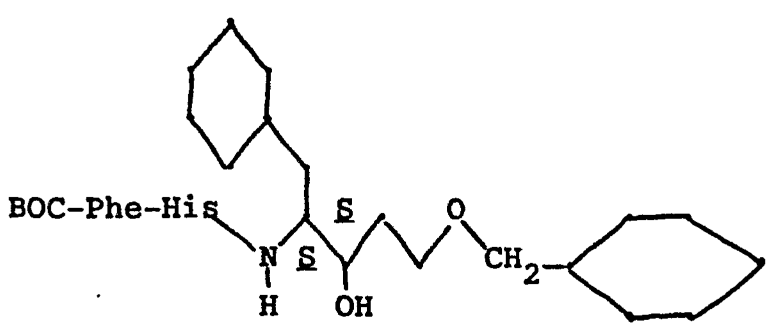

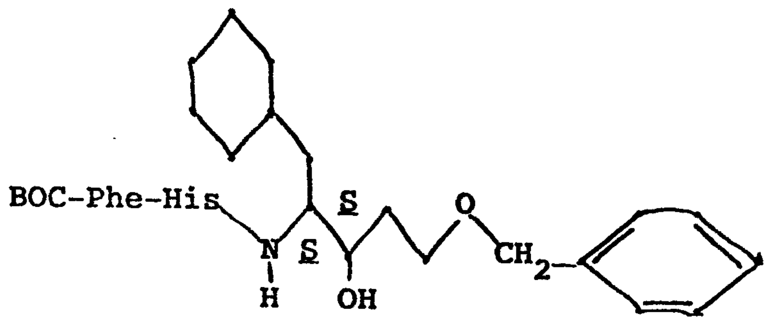

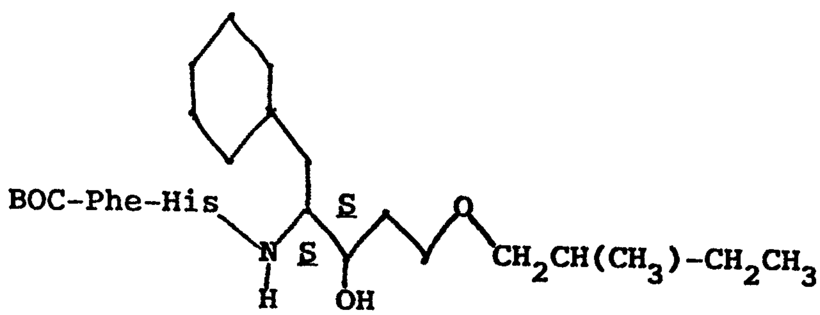

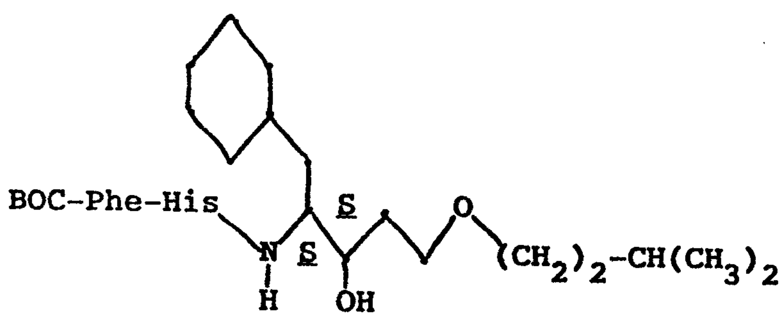

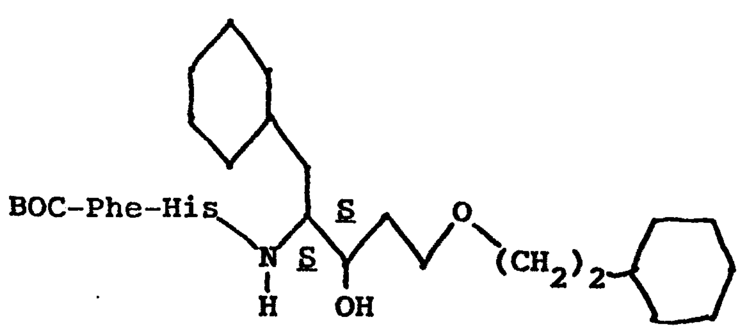

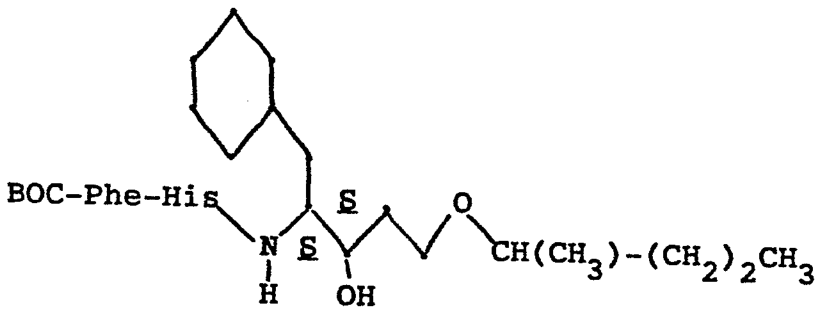

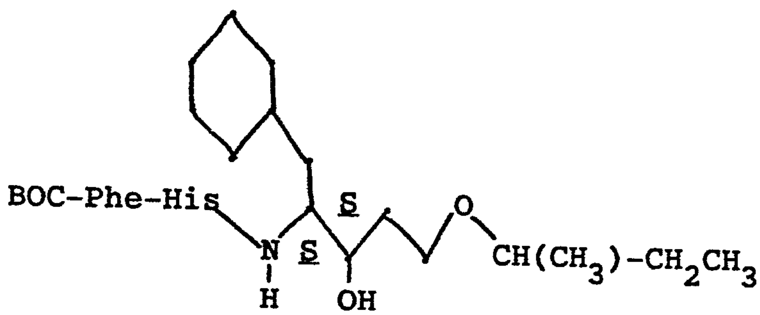

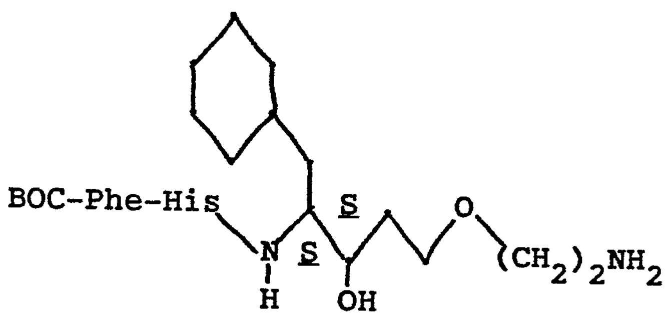

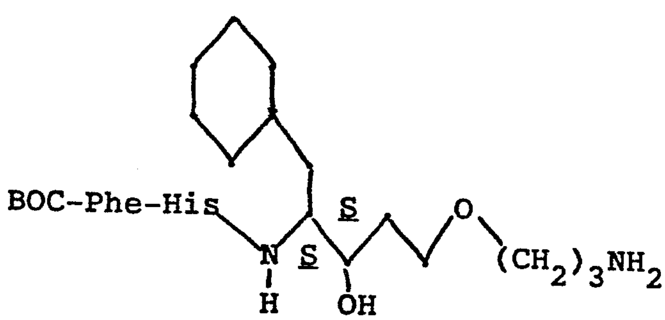

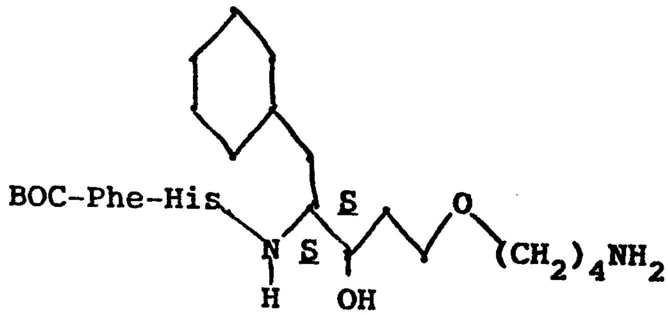

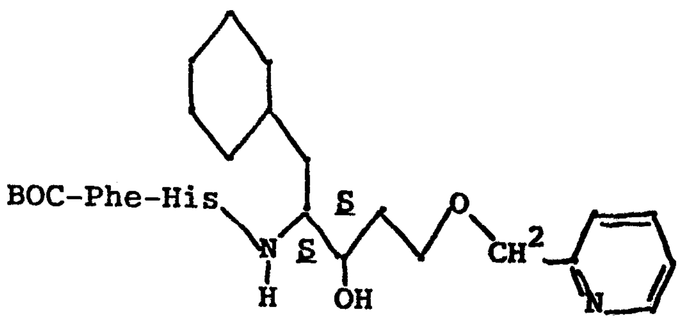

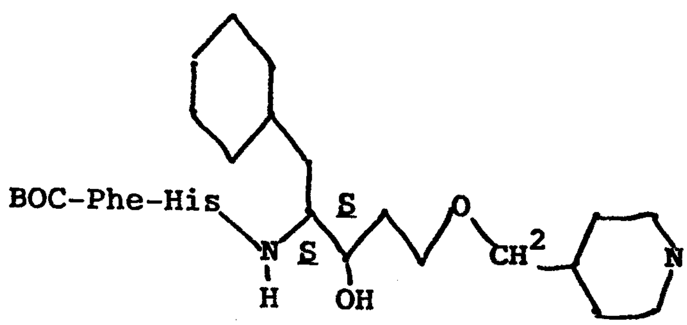

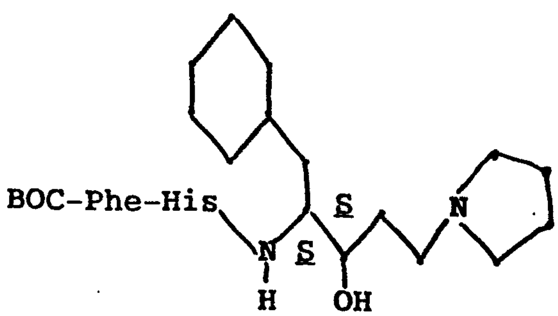

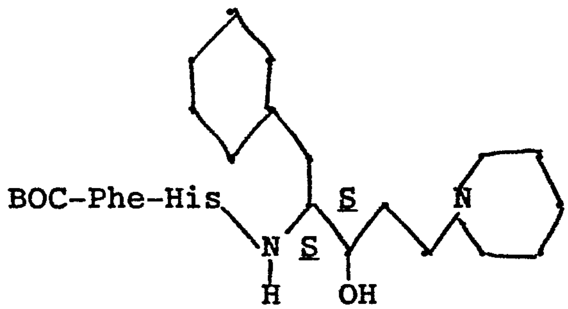

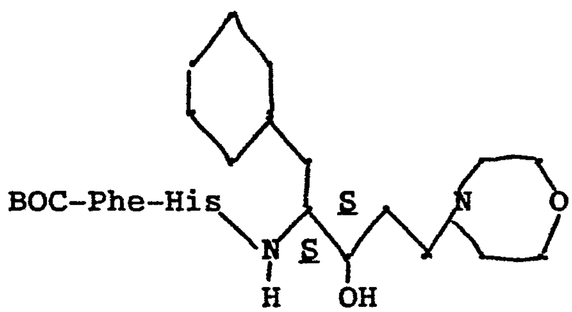

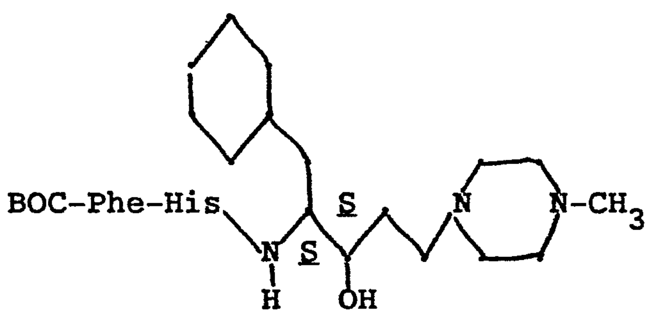

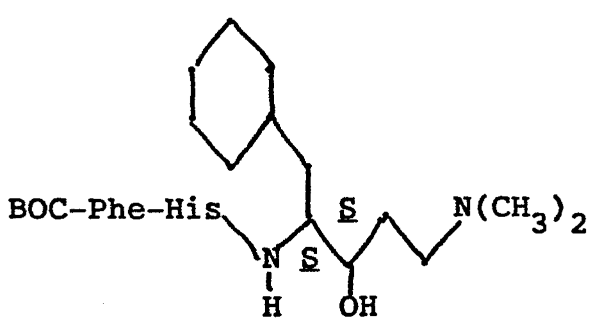

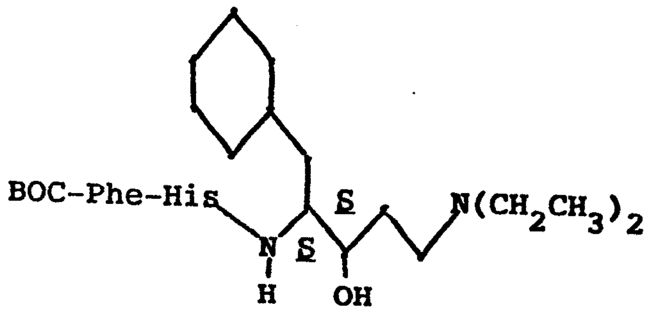

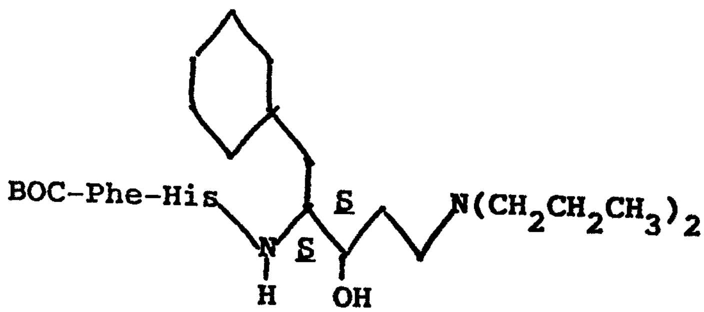

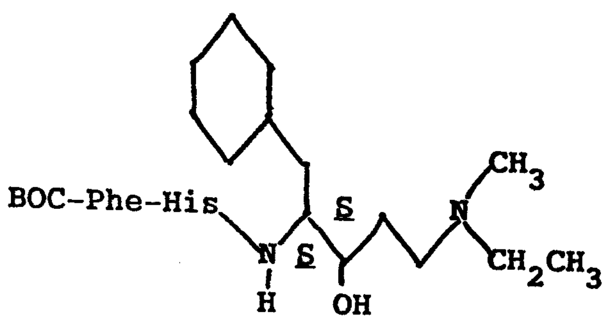

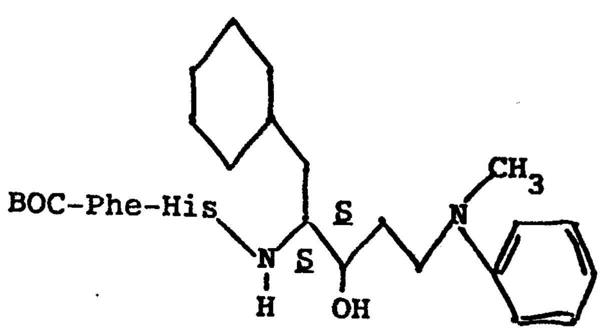

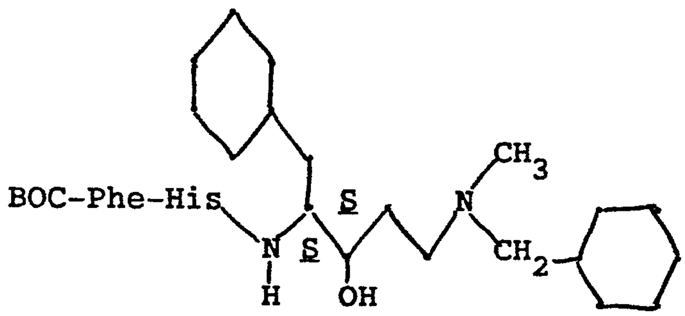

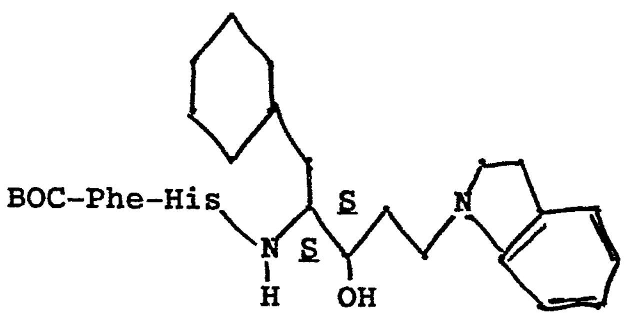

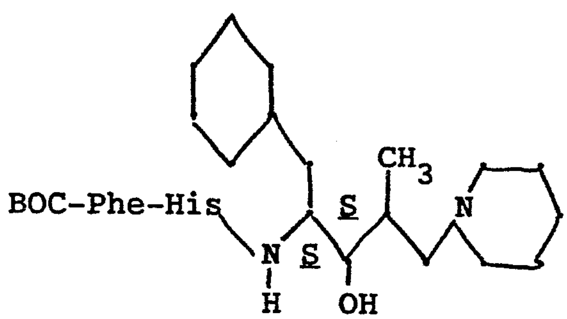

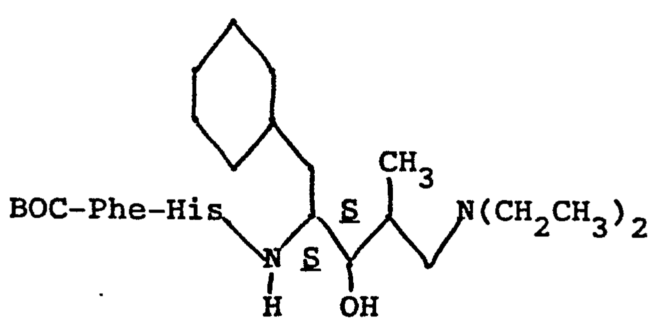

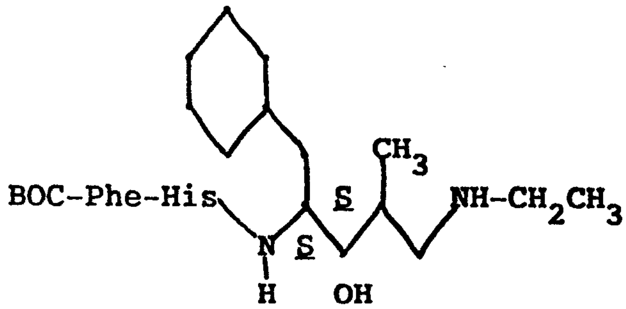

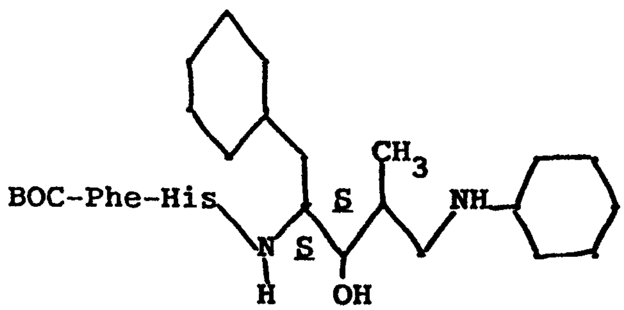

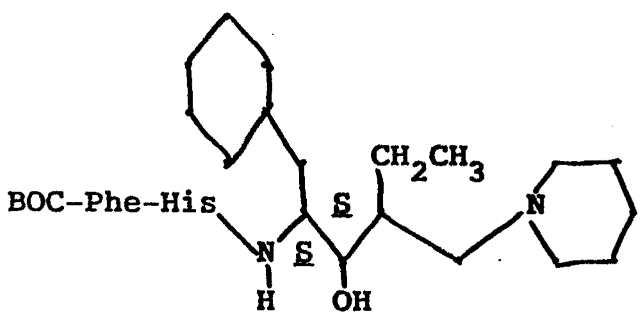

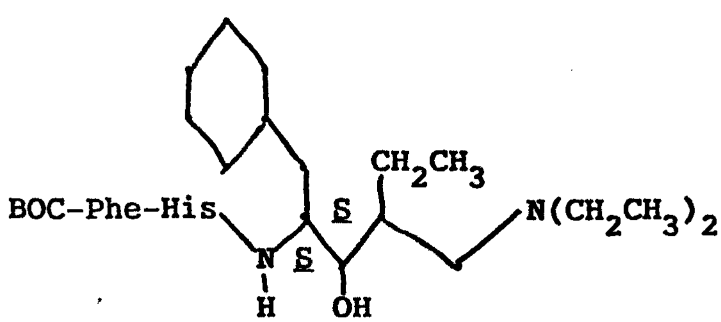

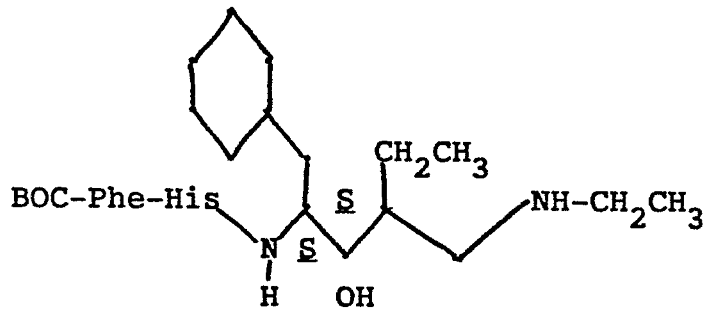

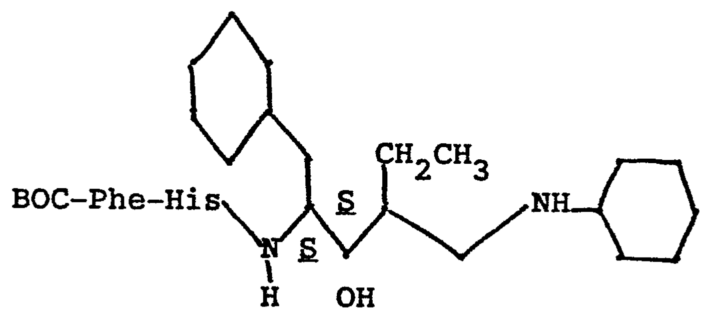

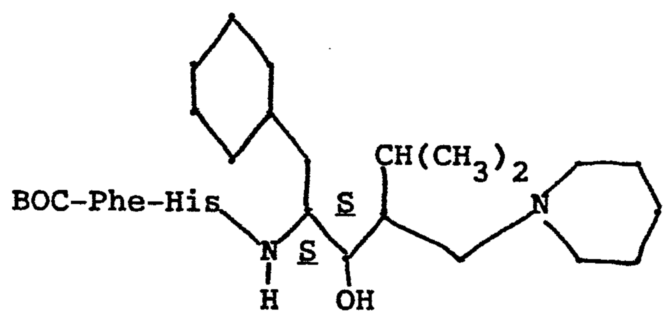

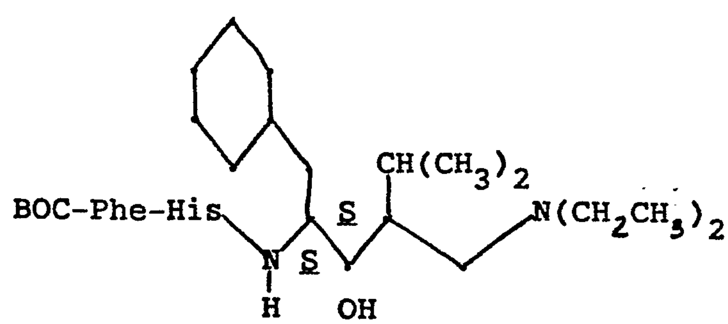

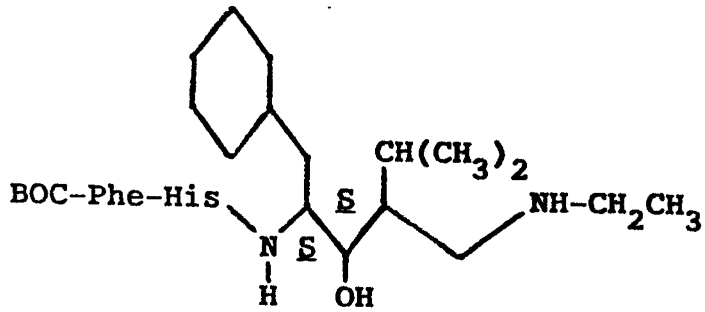

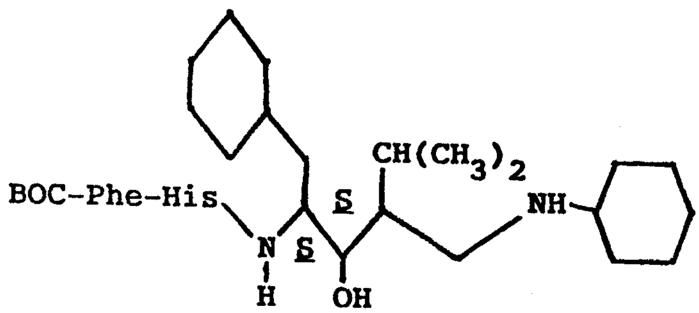

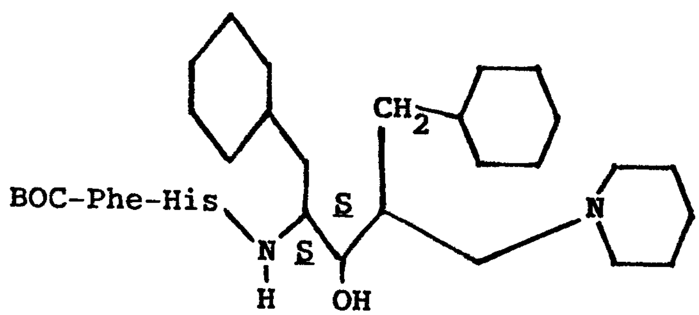

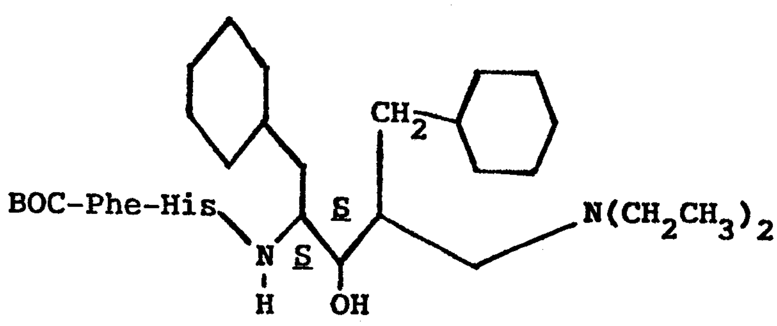

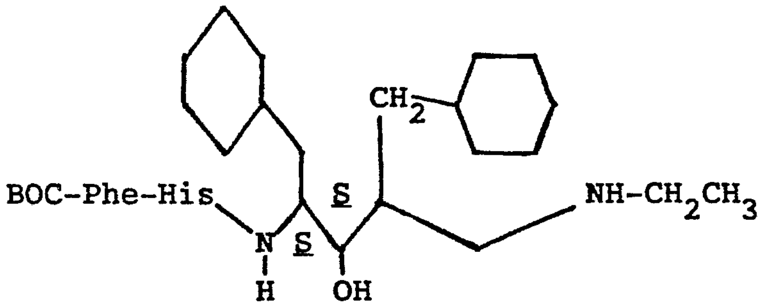

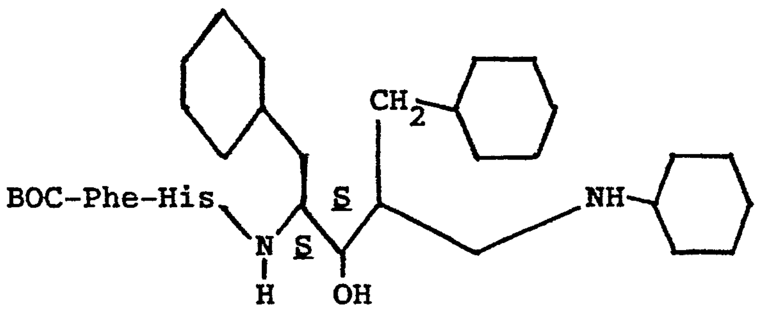

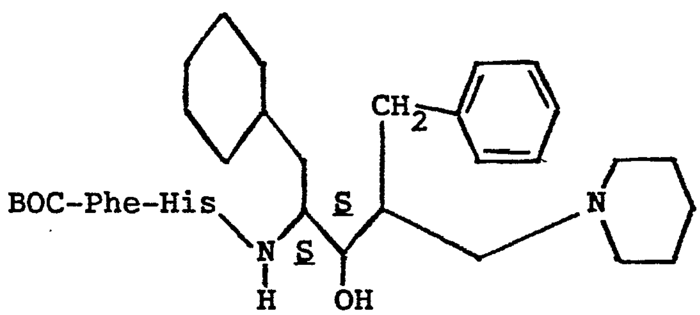

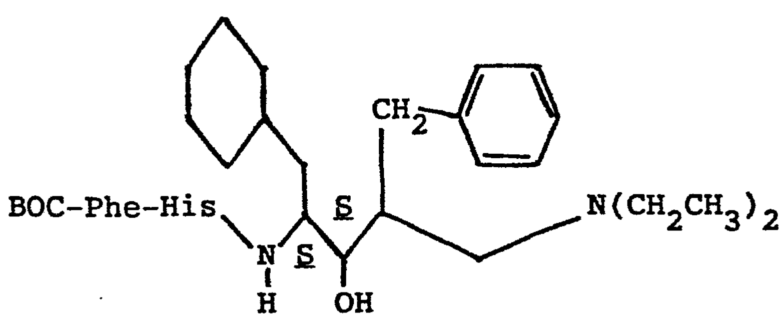

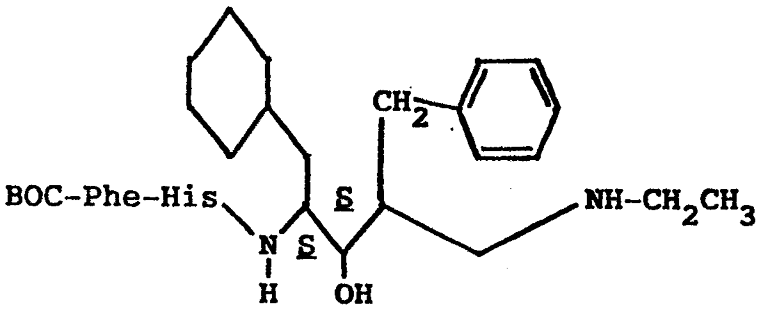

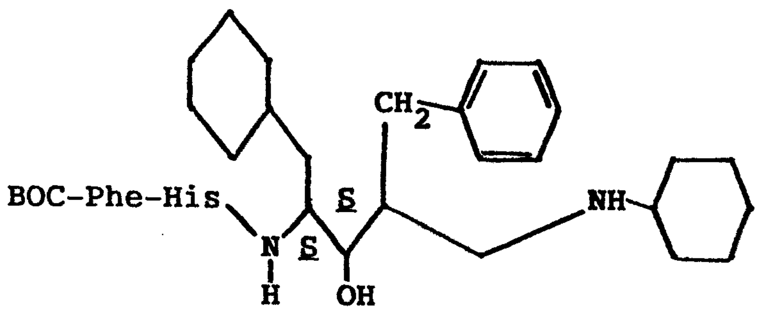

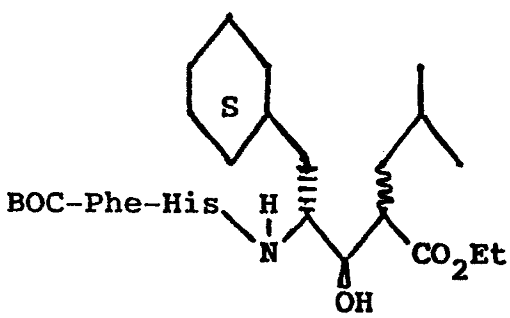

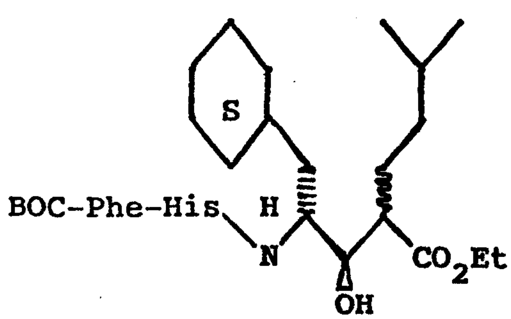

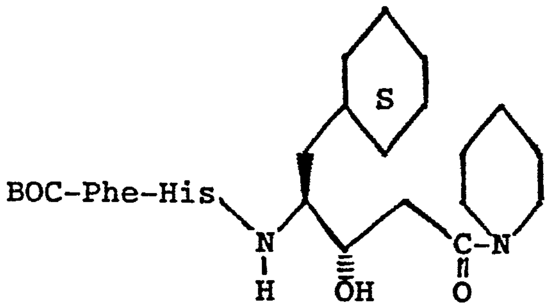

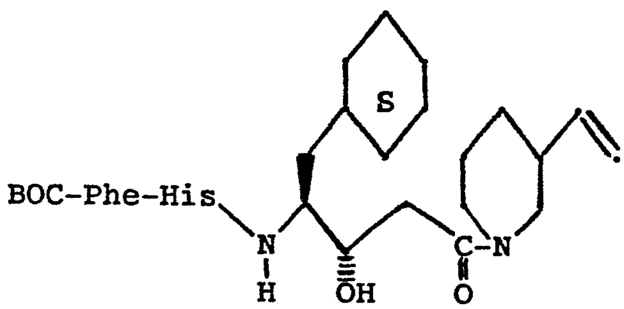

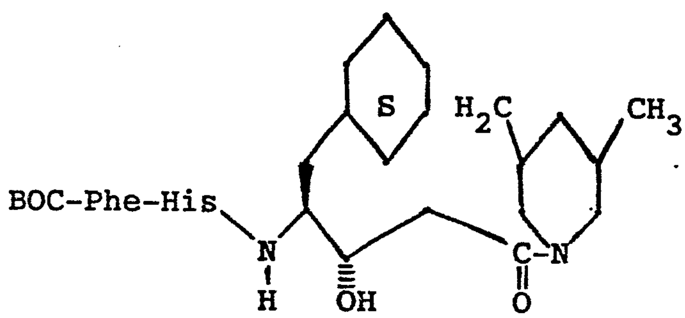

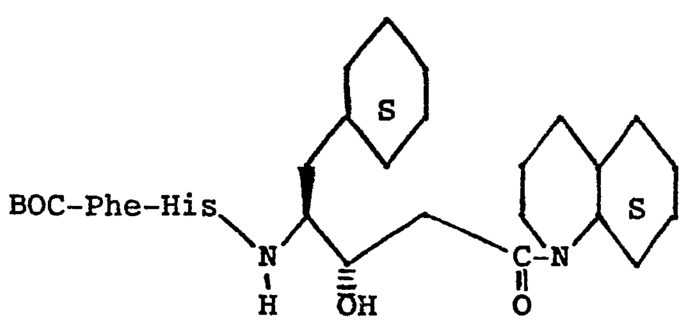

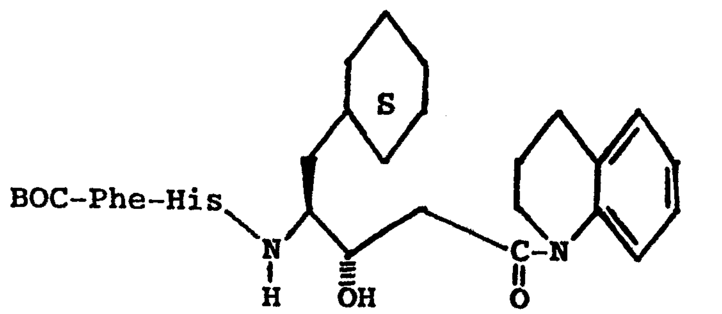

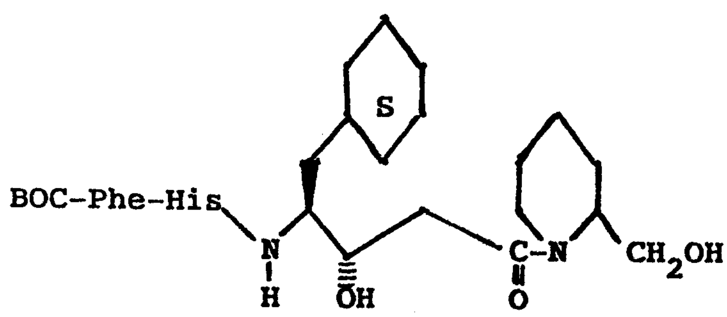

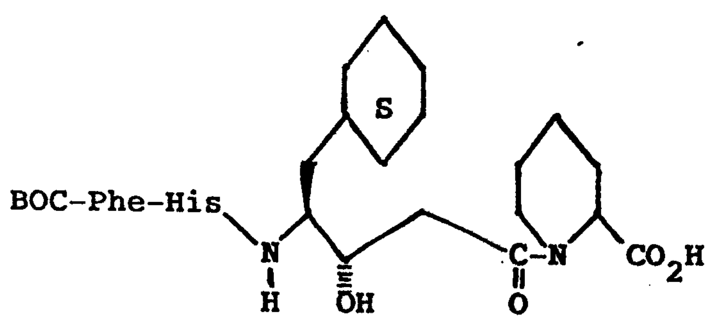

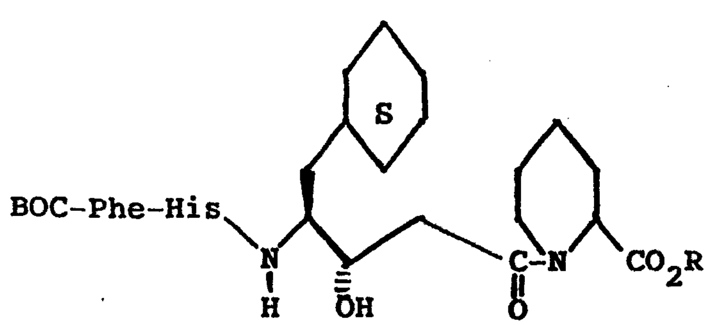

where R=alkyl, aryl, $CH_2OH$

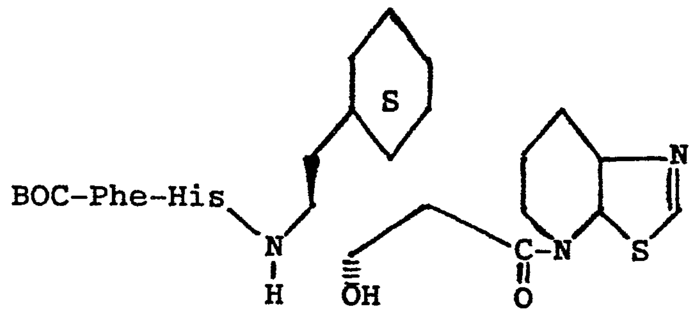

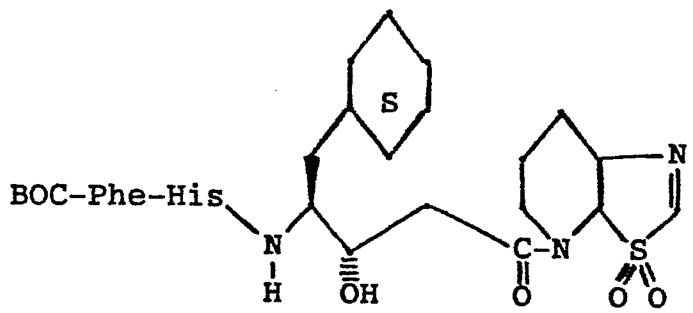

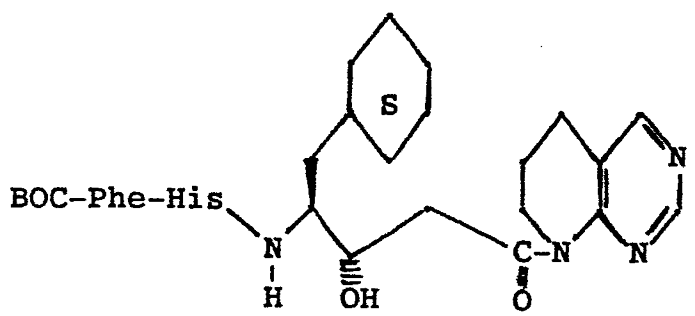

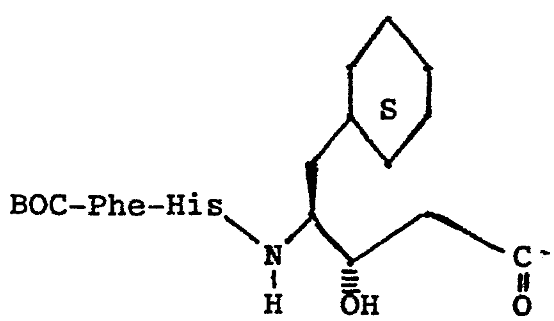

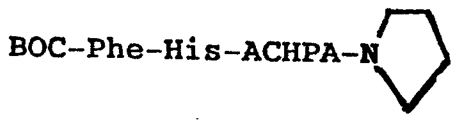

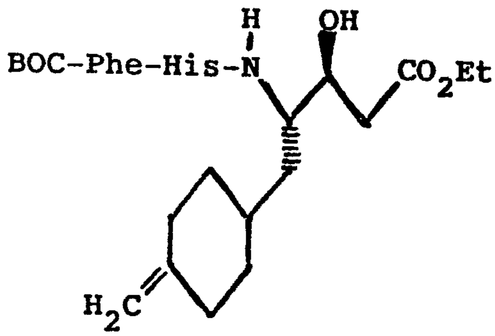

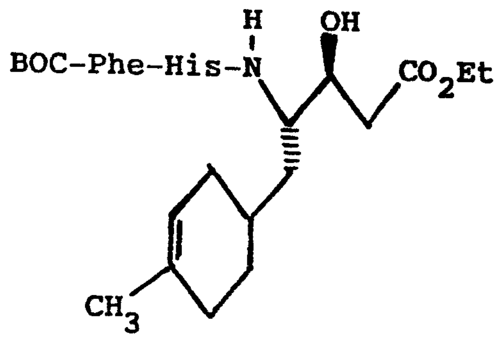

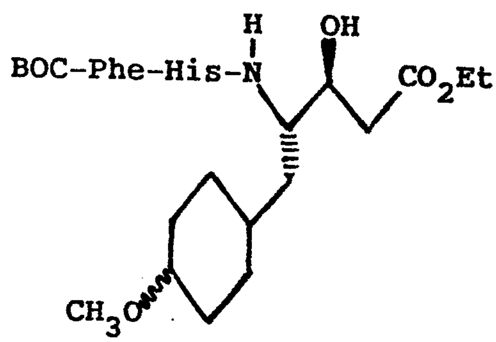

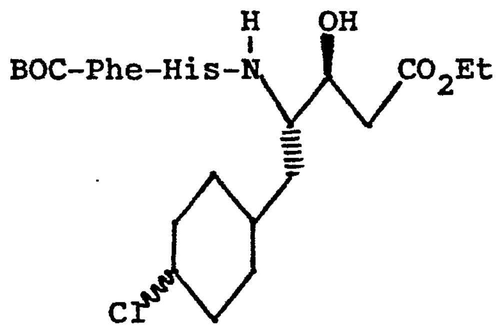

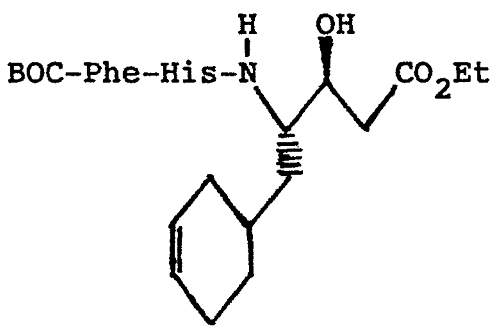

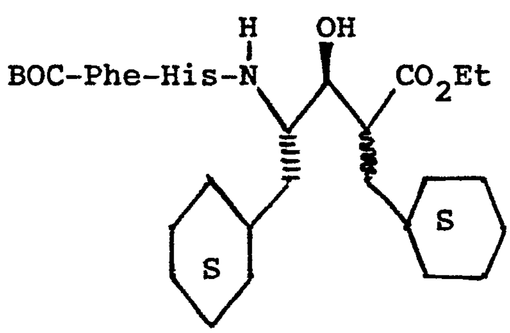

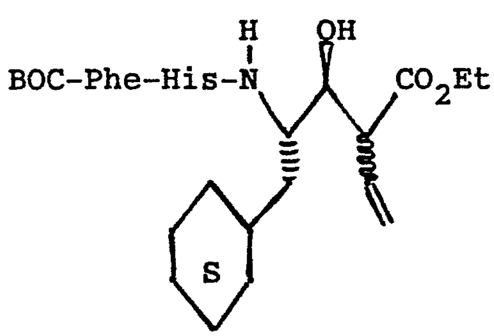

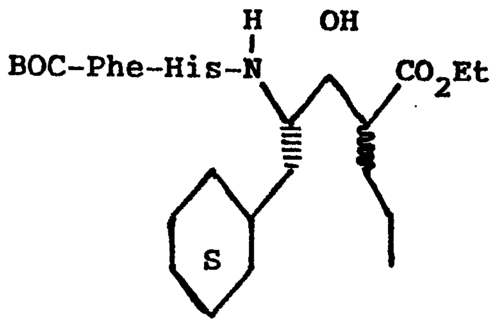

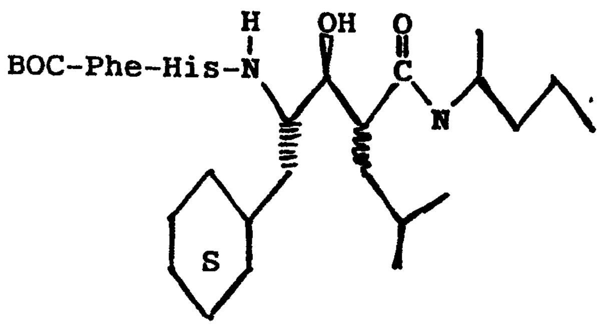

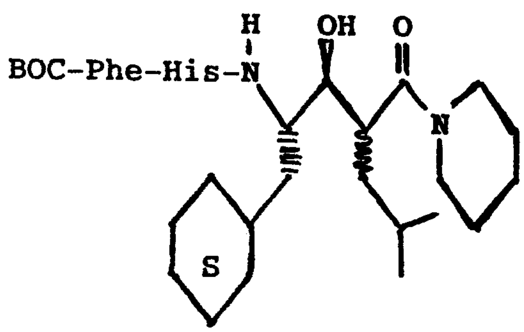

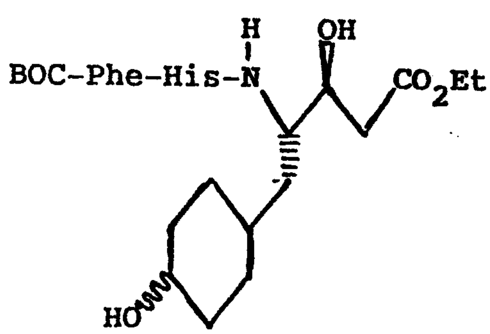

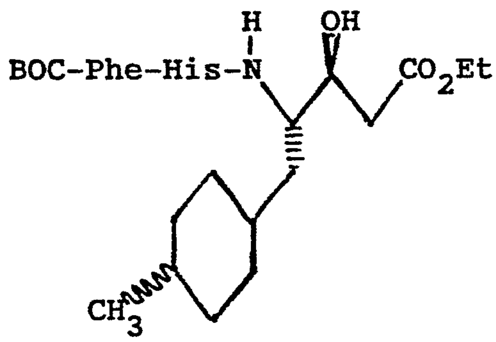

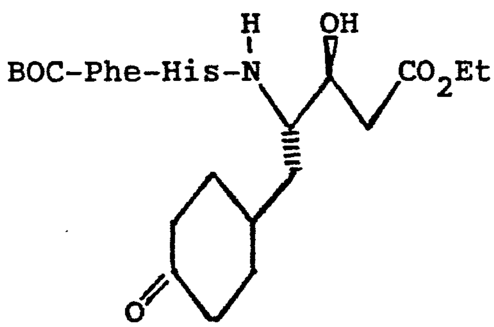

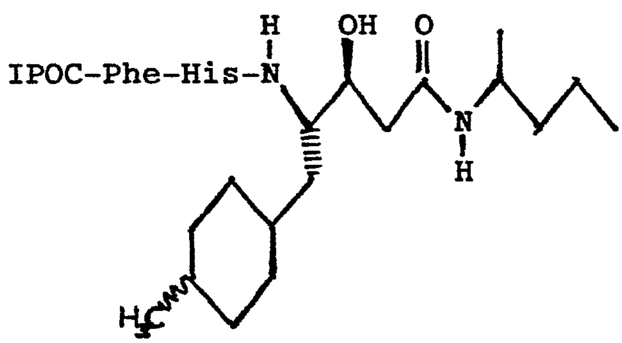

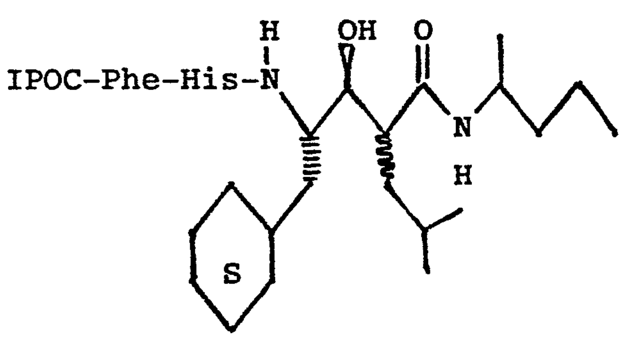

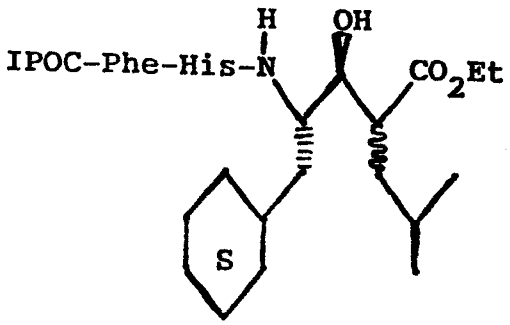

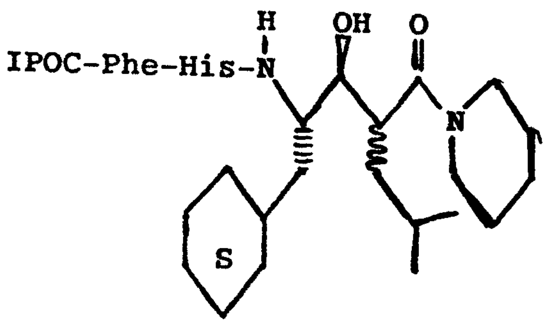

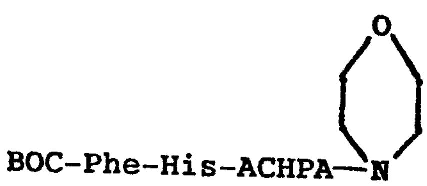

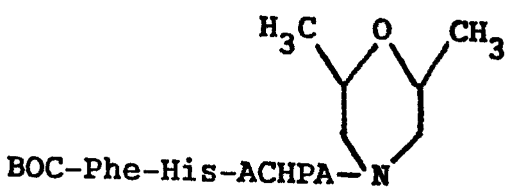

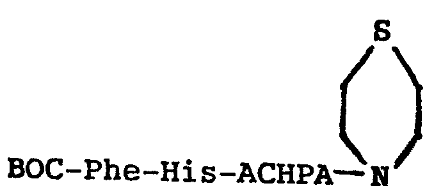

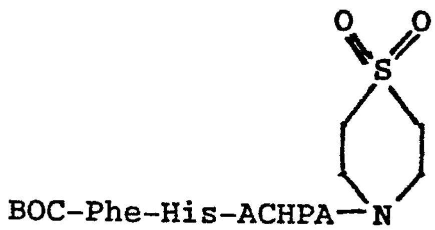

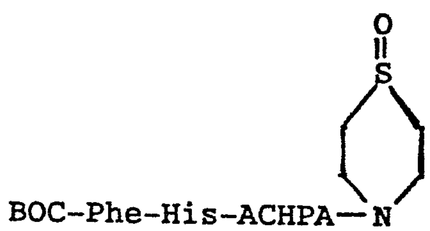

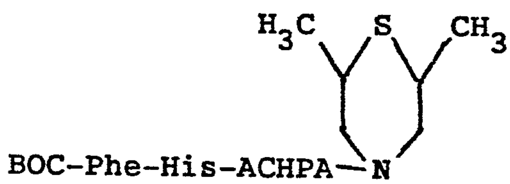

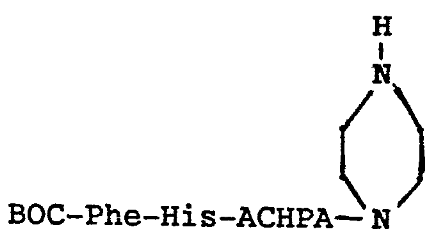

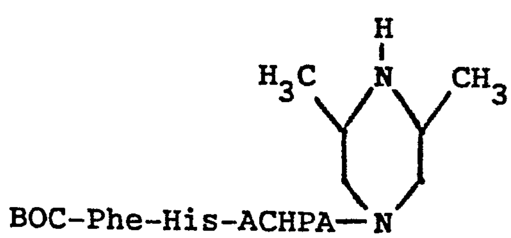

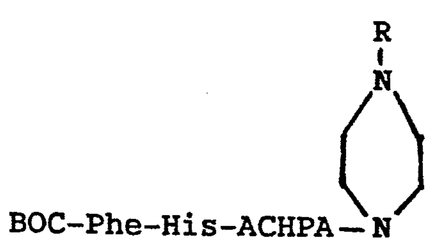

where R=alkyl, aryl, $-CO_2H$, $-CH_2OH$

BOC-Phe-His-ACHPA- 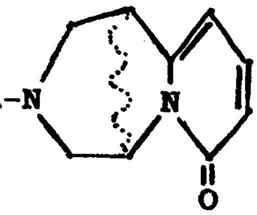

BOC-Phe-His-ACHPA- 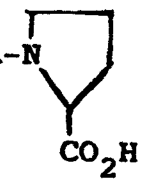

BOC-Phe-His-ACHPA- 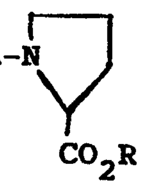 where R=alkyl, aryl, $-CH_2OH$

BOC-Phe-His-ACHPA- 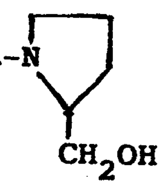

BOC-Phe-His-ACHPA- 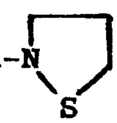

BOC-Phe-His-ACHPA- 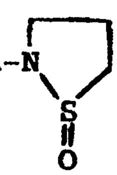

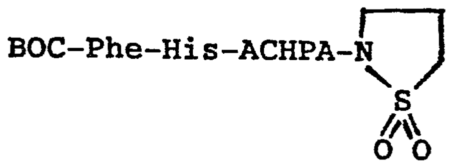

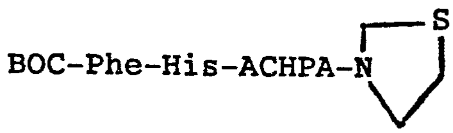

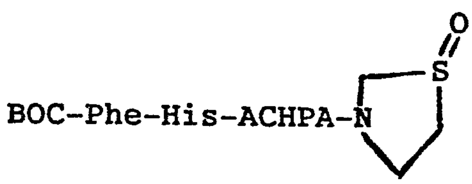

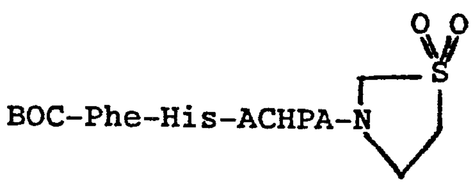

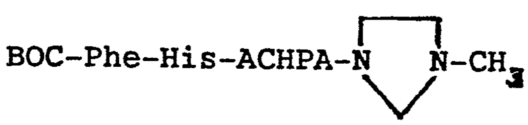

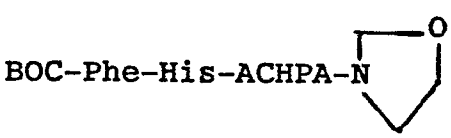

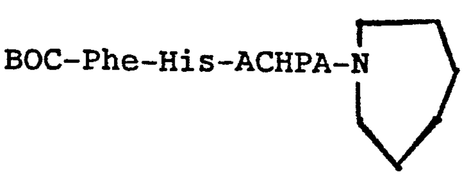

BOC-Phe-His-ACHPA- 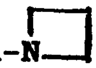

BOC-Phe-His-ACHPA- 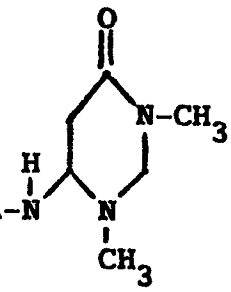

BOC-Phe-His-ACHPA- 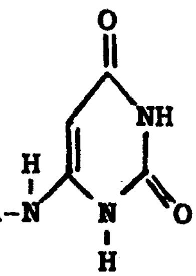

BOC-Phe-His-ACHPA- 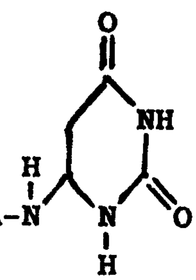

BOC-Phe-His-ACHPA- 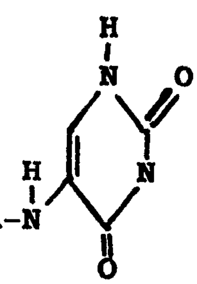

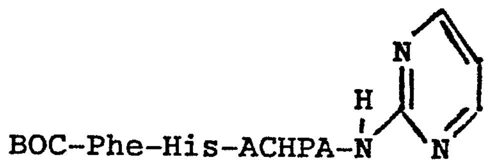

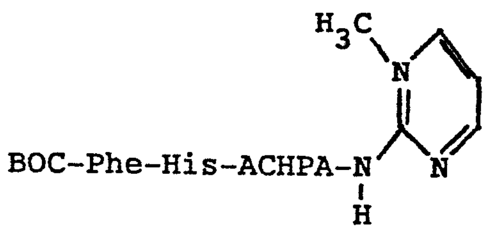

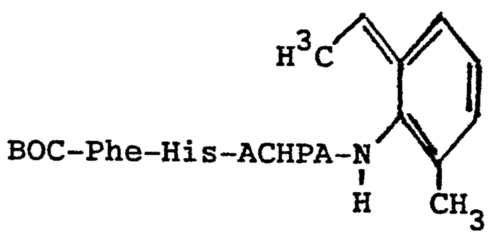

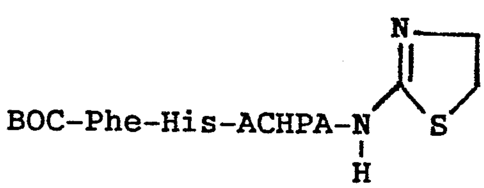

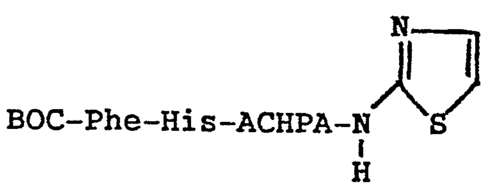

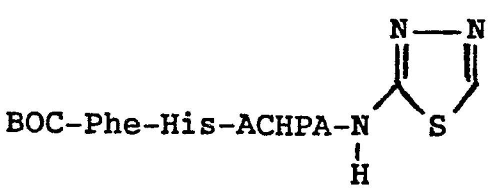

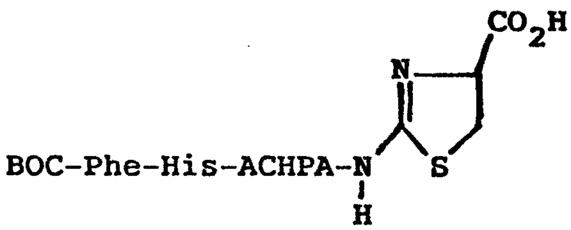

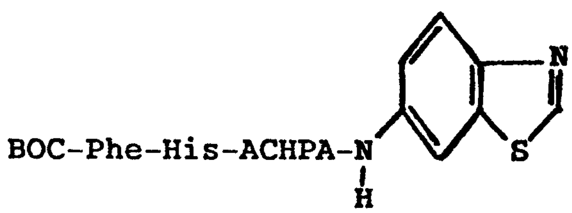

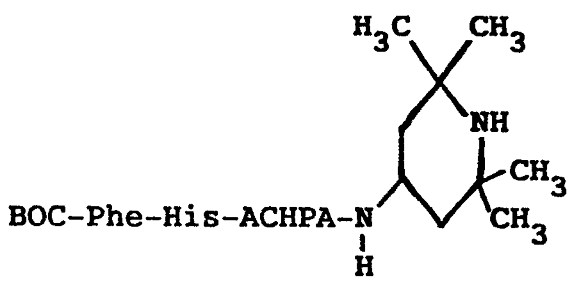

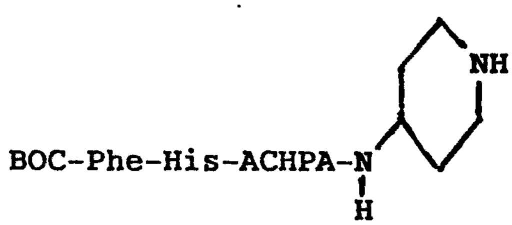

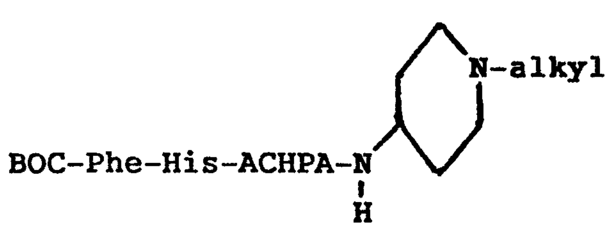

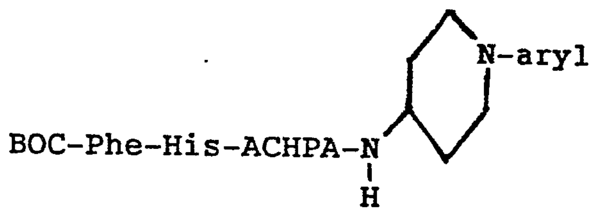

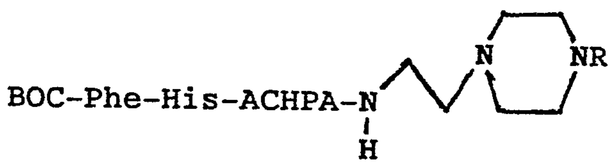 where R=H, alkyl

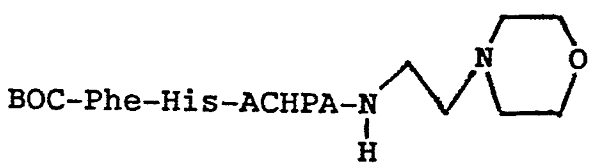

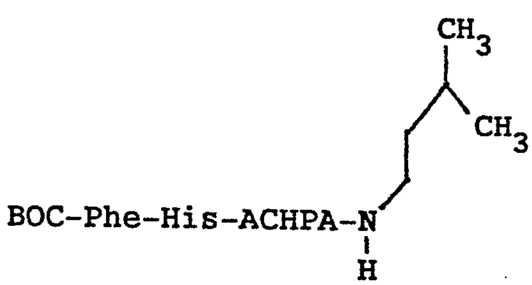

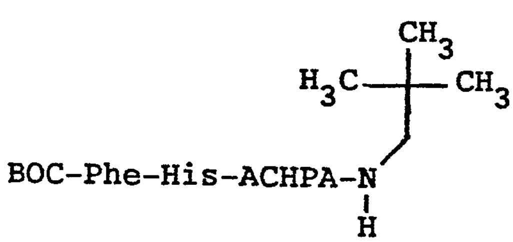

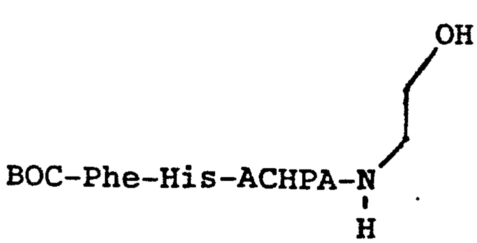

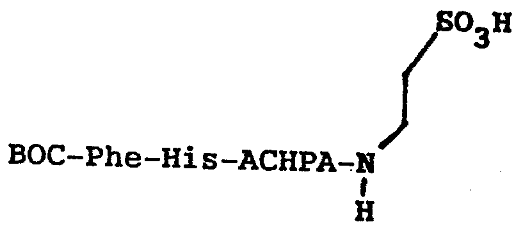

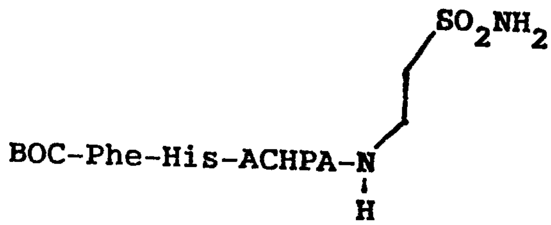

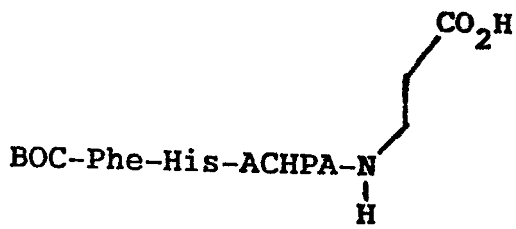

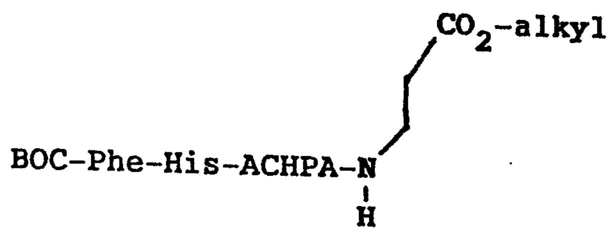

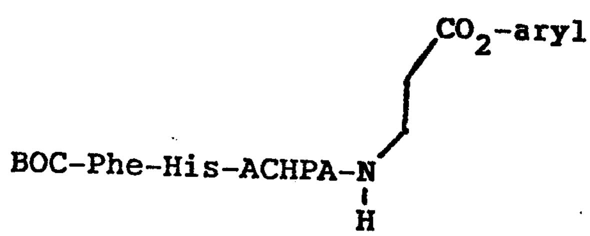

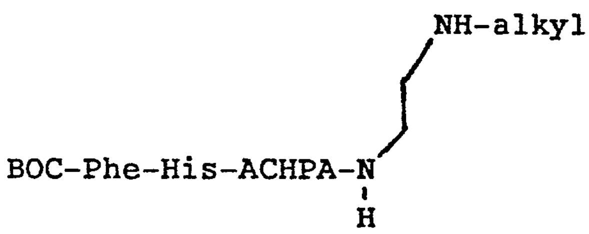

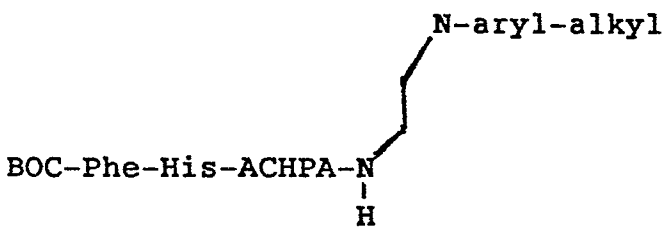

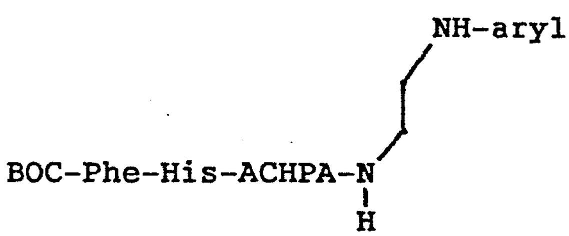

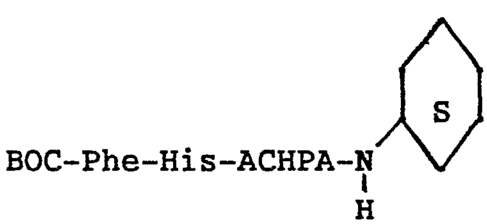

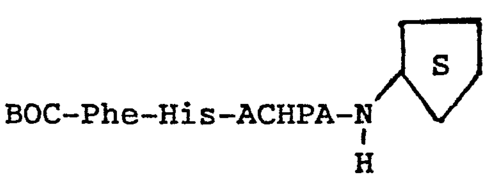

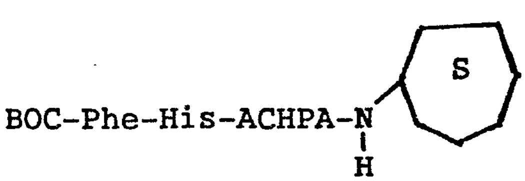

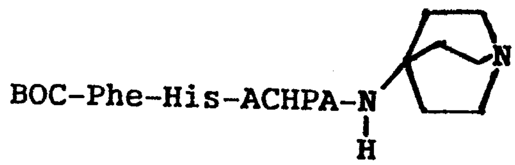

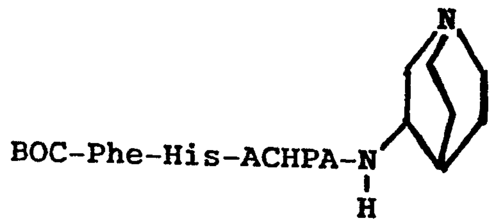

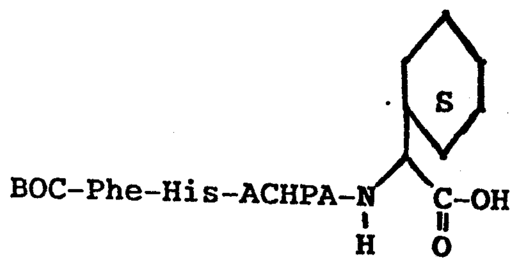

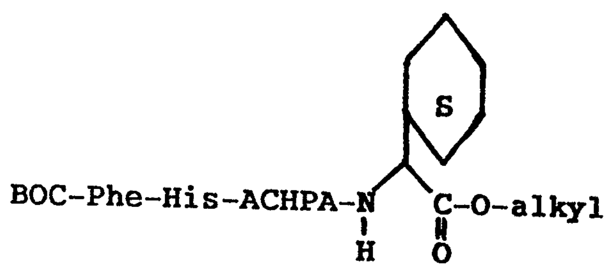

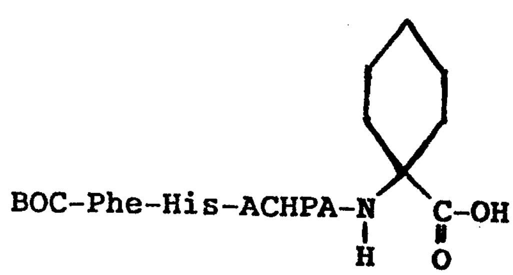

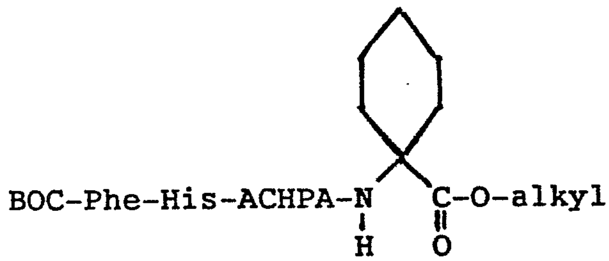

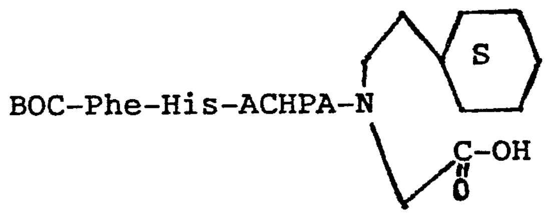

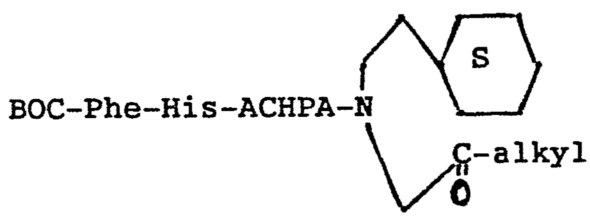

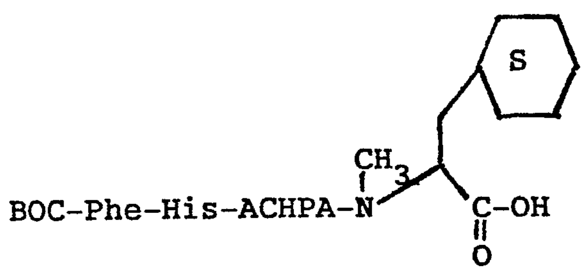

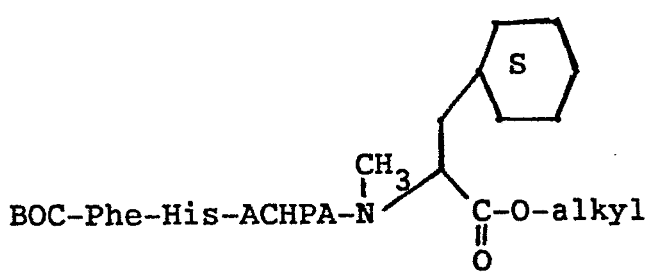

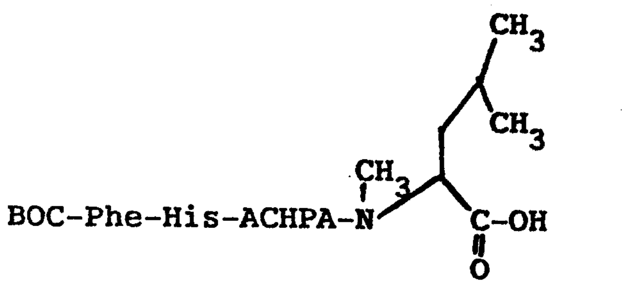

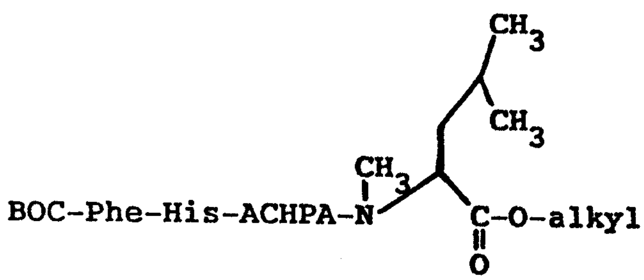

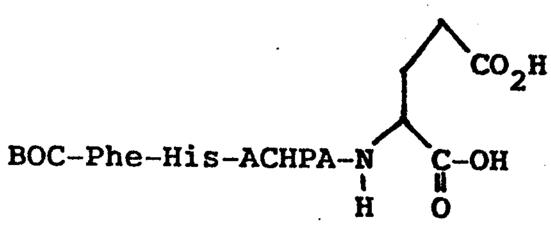

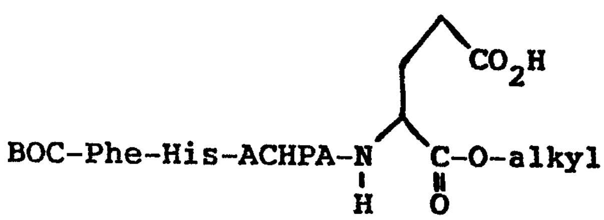

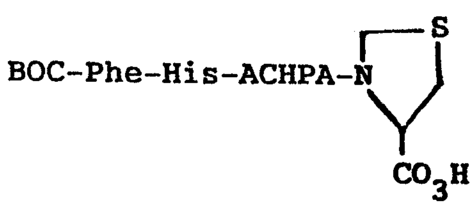

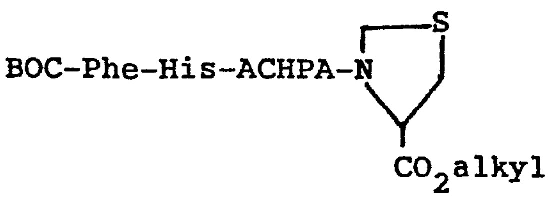

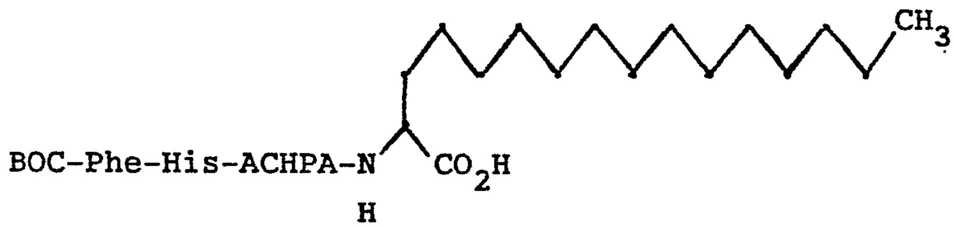

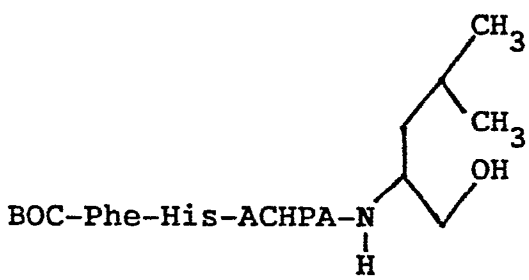

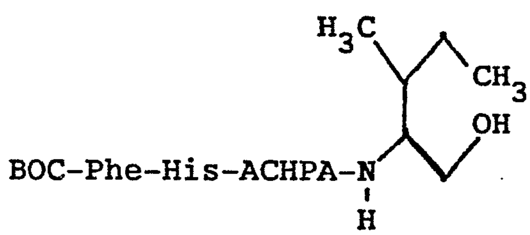

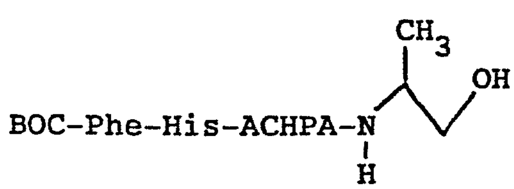

BOC-Phe-His-ACHPA-$C_6H_{12}O_5N$

BOC-Phe-His-ACHPA-$C_5H_{10}O_4N$

BOC-Phe-His-ACHPA-$C_6H_{12}O_4N$

BOC-Phe-His-ACHPA-$C_{14}H_{26}O_{10}N$

BOC-Phe-His-ACHPA-Lys-NH-i-Bu
BOC-Phe-His-ACHPA-Lys(CBZ)-NH-i-Bu
BOC-Phe-His-ACHPA-Lys
BOC-Phe-His-ACHPA-Lys(CBZ)
BOC-Phe-Lys-ACHPA-Lys
BOC-Phe-Lys-ACHPA-Lys(CBZ)
BOC-Phe-Lys-ACHPA-Pro
BOC-Phe-Lys-ACHPA-(4-i-Pr0)Pro
BOC-Phe-Lys- ACHPA-NH-[(2S)-methyl]butyl
BOC-Phe-Orn-ACHPA-NH-[(2S)-methyl]butyl
BOC-Phe-Arg-ACHPA-NH[(2S)-methyl]butyl
BOC-Phe-[(S)-4-hydroxybutyl)]Gly-ACHPA-NH-
BOC-Phe-Arg-ACHPA-NH-[(2S)-methyl]butyl
BOC-Phe-Nva-ACHPA-NH-[(2S)-methyl]butyl
BOC-Phe-Nva-ACHPA-NH-[(2S)-methyl]butyl
BOC-Phe-Nle-ACHPA-NH-[(2S)-methyl]butyl
BOC-Phe-(S-Me)Cys-ACHPA-NH-[(2S)-methyl]butyl
BOC-Tyr-His-ACHPA-NH-[(2S)-methyl]butyl
BOC-(p-$OCH_3$)Phe-His-ACHPA-NH-[(2S)-methyl] butyltyl
BOC-Trp-His-ACHPA-NH-[(2S)-methyl]butyl
BOC-[3-(1-naphthyl)]Ala-His-ACHPA-NH[(2S)-methyl]butyl
BOC-His-His-ACHPA-NH-[(2S)-methyl]butyl
BOC-(p-n-Pr)Phe-His-ACHPA-NH-[(2S)-methyl] butyl
CBZ-[3-(1-naphthy)Ala-His-ACHPA-NH-[(2S)-methyl]butyl
i-$PRO_2C$-His-His-ACHPA-NH-[(2S)-methyl]butyl

$EtO_2C$-Phe-His-ACHPA-NH-[(2S)-methyl]butyl
2(S)-hydroxy-3-phenylpropionyl-His-ACHPA-NH-[92S)-methyl]butyl
S-benzylthioacetyl-His-Achpa-NH-[(2S)-methyl]butyl
Dibenzylacety-His-ACHPA-NH-[(2S0-methyl]butyl
Bis-(naphtylmethyl)acetyl-His-ACHPA-NH-[(2S)-methyl]butyl
Bis-(p-hydrocyphenylmethyl)acetyl-His-ACHPA-NH-[(2S)-methyl]butyl
2-Phenylamino-3-phenylpropionyl-His-ACHPA-NH-[(2S)-methyl]butyl
2-Phenyloxy-3-phenylpropionyl-His-ACHPA-NH-[(2S)-methyl-butyl[(2S)-me
2-Phenylthio-3-phenylpropionyl-His-ACHPA-NH-[(2S)-methyl]butyl
1,3-Diphenylpropyloxycarbon-His-ACHPA-NH-[(2S)-methyl]butyl
2-(1,3-diphenyl)propyloxycarbonyl-His-ACHPA-NH-[(2S)-methyl]butyl
2-Phenylthio-3-(1-naphthyl)propionyl-His-ACHPA-NH-[(2S)-methyl]butyl
[2-benzyl-2(3,4-dihydroxy)benzyl]acetyl-His-ACHPA-NH-[(2S)-methyl]butyl
[2-benzyl-2-(4-isopropyloxy)benzyl]acetyl-His-ACHPA-NH-[(2S)-methyl]butyl
BOC-Phe-His-[5-amino-66-cyclohexyl-4-hydroxy-2-isopropyl]hexanoyl 2(S)-aminobutane
BOC-Phe-His-[5-amino-6-cyclohexyl-4-hydroxy-2-isobutyl]hexanoyl 2(S)-aminobutane
BOC-Phe-His-[5-amino-2-benzyl-6-cyclohexyl-4-hydroxy]hexanoyl 2(S)-aminobutane

BOC-Phe-His-]5-amino-6-cyclohexyl-2-cyclohexyl-4-hydroxy]hexanoyl 2(S)-aminobutane

2(s)-hydroxy-3-phenylpropionyl-His-ACHPA-NH-[(2S)-methyl]butyl

S-benzylthioacetyl-His-ACHPA-NH-[(2S)-methyl]butyl

Dibenzylacetyl-His-ACHPA-Lys-NH[(2S)-methyl]butyl

Dibenzylactyl-His-ACHPA-Ile-NH-[(2S)-methyl]butyl

Dibenzylacetyl-His-ACHPA-Ile-Nh-[(2S)-methyl]butyl

Dibenzylacetyl-Lys-ACHPA-Ile-NH-[(2S)-methyl]butyl

Bis-(naphtylmethyl)acetyl-Lys-ACHPA-Lys

3. A pharmaceutical composition for treating renin-associated hypertension, comprising a pharmaceutical carrier and a therapeutically effective amount of a peptide according to Claim 1.

4. A pharmaceutical composition for treating renin-associated hypertension, comprising a pharmaceutical carrier and a therapeutically effective amount of a peptide according to Claim 2.

5 . A pharmaceutical composition for treating renin-associated hyperaldosteronism, comprising a pharmaceutical carrier and a therapeutically effective amount of a peptide according to Claim 1.

6 . A peptide of the formula:

$$A^{\circ}-B^{\circ}-D^{\circ}-E^{\circ}-G^{\circ}$$
$$(I^{\circ})$$

wherein:

$A^{\circ}$ is hydrogen, or $R^{\circ}_{a}$-, $R^{\circ}_{b}CO$ or $R^{\circ}_{b}SO_2$- where $R^{\circ}_{a}$ and $R^{\circ}_{b}$ are alkyl, alkenyl, alkynyl, cycloalkyl, aryl, heterocyclic, aryloxy alkyl, heterocyclic oxy alkyl, aryl alkyl, heterocyclic alkyl, heterocyclic oxyalkyl, and $R^{\circ}_{a}$ and $R^{\circ}_{b}$ may be

substituted with up to three members selected from amino, hydroxy, alkyl, halo and alkoxy groups.

B° and D° can independently be absent or can be

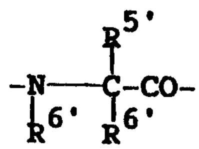

provided that only one of B° or D° may be absent.

E° is 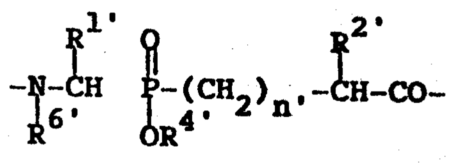

G° is $-R^{3'}$ or is 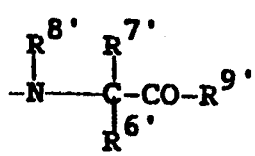 .

$R^{1'}$ is alkyl, alkenyl, alkynyl, cycloalkyl, cycloalkenyl, cycloalkyl alkyl, aryl alkyl, heterocyclic, heterocyclic each of which may be substituted with up to three members selected from alkyl, halo, amino and alkoxy groups.

n' is 0 or 1.

$R^{2'}$ is hydrogen, alkyl, alkenyl, alkynyl, cycloalkyl, cycloalkenyl, cycloalkyl alkyl, aryl, aryl alkyl, heterocyclic, heterocyclic alkyl, each of which may be substituted with up to three members selected from alkyl, hydroxy, halo, amino, alkylamino, dialkylamino, and alkoxy.

$R^{3'}$ is OH, $NH_2$, $NHR_a^{3'}$, $NR_a^{3'}N_b^{3'}$, $OR_c^{3'}$

where $R_a^{3'}$, $R_b^{3'}$, and $R_c^{3'}$ are separately alkyl, alkenyl, alkynyl, cycloalkyl, cycloalkenyl, cycloalkyl alkyl, aryl, aryl alkyl, heterocyclic, heterocyclic alkyl, each of which may be substituted with up to three members selected from amino, alkyl amino, dialkyl amino, trialkyl ammonium, hydroxy, alkoxy, aryloxy, aryl alkoxyl, or halo.

$R_c^{3'}$ may also be $R_d^{3'}$-CO-V'-$CR_e^{3'}R_f^{3'}$ wherein $R_d^{3'}$ is alkyl or aryl; $R_e^{3'}$ and $R_f^{3'}$ are hydrogen or alkyl; V' is -O- or -NH-.

$R^{4'}$ is hydrogen, alkyl, alkenyl, alkynyl, aryl, arylalkyl, each of which may be substituted with up to three members selected from amino, alkyl amino, dialkyl amino, trialkyl ammonium, hydroxy, alkoxy, halo or alkyl groups. $R^{4'}$ may also be $R_a^{4'}$-CO-V'-$CR_b^{4'}R_c^{4'}$ wherein $R_a^{4'}$ is alkyl, alkenyl or alkynyl, or aryl; $R_b^{4'}$ and $R_c^{4'}$ are hydrogen, alkyl, alkenyl, or alkynyl; V' is -O- or -NH-.

$R^{5'}$ is hydrogen, alkyl, alkenyl, alkynyl, cycloalkyl, cycloalkenyl, aryl, cycloalkyl alkyl, aryl alkyl, heterocyclic, heterocyclic alkyl, aryloxy alkyl, heterocyclic oxy alkyl, heterocyclic oxy, each of which may be substituted with up to three members selected from amino, alkyl amino, dialkyl amino, trialkyl ammonium,

hydroxy, alkoxy, carboxy, alkoxycarbonyl, alkylthio, arylthio, thiol, guanidino, carboxamido and $C_2$-$C_6$ alkanoylamino groups.

$R^{6'}$ is hydrogen, methyl.

$R^{7'}$ can be $R^{5'}$ and taken together with $NR^{8'}$ may be a cyclic amino acid of formulas:

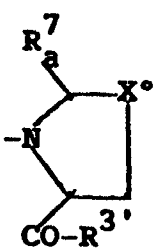

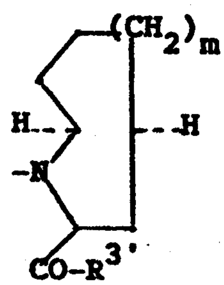

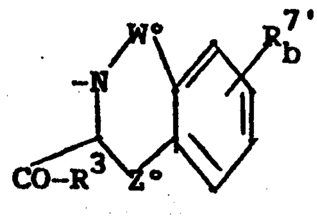

where $R_a^{7'}$ is hydrogen, phenyl, hydroxyphenyl; X is -S- or $-CH_2-$ or $-CH-R_b^7$; m is 1 or 2; and $R_b^7$ is cyclohexyl, phenylthio; W° and Z° are single bonds or $-CH_2$.

$R^{8'}$ is hydrogen, methyl and cycloalkyl including cyclopentyl and indanyl, such that when $R^{8'}$ is cycloalkyl, $R^{6'}$ and $R^{7'}$ are both hydrogen.

$R^{9'}$ is hydroxy, $OR_a^{3'}$, $-NH_2$, $-NHR_a^{3'}$, $NR_a^{3'}R_b^{3'}$, where $R_a^{3'}$ and $R_b^{3'}$ are as defined above, such that when A° and B° are both absent, $R^{9'}$ can be 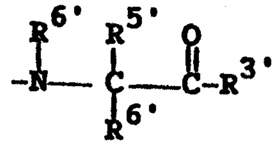

and pharmaceutically acceptable salts thereof.

7. A peptide of Claim 6 wherein B° is absent.

8. A peptide of Claim 6 wherein $R^{1'}$ is $-CH_2$-cyclohexyl.

9. A peptide of Claim 8 wherein $R^{6'}$ is H, and n' is 1.

10. A peptide of Claim 9 wherein $R^{2'}$ is $-CH(CH_3)_2$, $-CH_2-CH(CH_3)_2$, $-CH_2$-cyclohexyl and $R^{4'}$ is H.

11. A peptide of Claim 9 wherein $R^{6'}$ is H, $R^{2'}$ is H, n is 0, and $R^{4'}$ is H.

12. A compound of Claim 6 selected from

[N-(N-(N-carbobenzoxy-2-amino-3-(1-naphthyl)-propionyl)histidyl)-1-amino-2-cyclohexylethyl] 2-carboxy-4-methylpentylphosphinic acid;

[N-(N-(N-carbobenzoxy-2-amino-3-(1-naphthyl)-propionyl)histidyl)-1-amino-2-cyclohexylethyl] 2-carboxy-3-methylbutylphosphinic acid;

[N-(N-(N-t-butoxycarbonyl-2-amino-3-(1-naphthyl)-propionyl)histidyl)-1-amino-2-cyclohexylethyl] 2-carboxy-3-methylbutylphosphinic acid;

[N-(N-(N-carbobenzoxy-2-amino-3-(1-naphthyl)-propionyl)histidyl)-1-amino-2-cyclohexylethyl] 2-carbomethoxy-4-methylpentylphosphinic acid;

[N-(N-(N-carbobenzoxy-2-amino-3-(1-naphthyl)-propionyl)histidyl)-1-amino-2-cyclohexylethyl] 2-carboxamido-4-methylpentylphosphinic acid;

[N-(N-(N-t-butoxycarbonyl-2-amino-3-(1-naphthyl)-propionyl)histidyl)-1-amino-2-cyclohexylethyl] 2-(N-benzyl)carboxamido-3-methylbutylphosphinic acid;

[N-(N-carbobenzoxy-phenylalanyl-histidyl)-1-amino-2-cyclohexylethyl] 2-carboxy-4-methylpentylphosphinic acid;

[N-(N-carbobenzoxy-phenylalanyl-histidyl)-1-amino-2-cyclohexylethyl] 2-(N-benzyl)carboxamido-4-methylpentylphosphinic acid;

[N-(N-carbobenzoxy-phenylalanyl-phenylalanyl)-1-amino-2-cyclohexylethyl] 2-carboxamido-4-methylpentylphosphinic acid;

[N-(N-(N-carbobenzoxy-2-amino-3-(1-naphthyl)-propionyl)histidyl)-1-amino-2-cyclohexylethyl]-carbomethoxymethylphosphinic acid;

[N-(N-(N-carbobenzoxy-2-amino-3-(1-naphthyl)-propionyl)histidyl)-1-amino-2-cyclohexylethyl]-carboxymethylphosphinic acid;

[N-(N-(N-carbobenzoxy-2-amino-3-(1-naphthyl)-propionyl)histidyl)-1-amino-2-cyclohexylethyl] carboxamidomethylphosphinic acid;

[N-(N-(N-carbobenzoxy-2-amino-3-(1-naphthyl)-propionyl)histidyl)-1-amino-2-cyclohexylethyl] 2-carboxy-3-cyclohexylpropylphosphinic acid;

[N-(N-carbobenzoxy-histidyl)-1-amino-2-cyclohexylethyl] 2-carboxy-4-methylpentylphosphinic acid;

[N-(phenylalanyl)-1-amino-2-cyclohexylethyl] 2-carboxy-3-methylbutylphosphinic acid;

[N-(lysyl)-1-amino-2-cyclohexylethyl] 2-carboxy-3-methylbutylphosphinic acid;

[N-(Nα-1-naphthyloxyacetyl-lysyl)-1-amino-2-cyclohexylethyl] 2-carboxy-3-methylbutylphosphinic acid;

Methyl [N-(N-(N-carbobenzoxy-2-amino-3-(1-naphthyl)propionyl)histidyl)-1-amino-2-cyclohexylethyl] 2-carboxy-4-methylpentylphosphinate;

Methyl [N-(N-(N-carbobenzoxy-2-amino-3-(1-naphthyl)propionyl)histidyl)-1-amino-2-cyclohexyl-ethyl]carbomethoxymethylphosphinate; and

Ethyl [N-(N-3-phenylpropionyl-phenylalanyl)-1-amino-2-cyclohexylethyl] 2-carbomethoxy-3-methyl-butylphosphinate.

13. A pharmaceutical composition for treating hypertension or congestive heart failure containing a compound of Claim 6.

14. A pharmaceutical composition for treating hypertension containing a compound of Claim 1 or 6 and one or more antihypertensive agents selected from the group consisting essentially of:

Diuretics: acetazolamide; amiloride; bendroflumethiazide; benzthiazide; bumetanide; chlorothiazide; chlorthalidone; cyclothiazide; ethacrynic acid; furosemide; hydrochlorothiazide; hydroflumethiazide; indacrinone (racemic mixture, or as either the (+) or (-) enantiomer alone, or a manipulated ratio, e.g., 9:1 of said enantiomers, respectively); metolazone; methyclothiazide; muzolimine; polythiazide; quinethazone; sodium ethacrynate; sodium nitroprusside; spironolactone; ticrynafen; triamterene; trichlormethiazide;

α-Adrenergic Blocking Agents: dibenamine; phentolamine; phenoxybenzamine; prazosin; tolazoline;

β-Adrenergic Blocking Agents: atenolol; metoprolol; nadolol; propranolol; timolol;

((±)-2-[3-(tert-butylamino)-2-hydroxypropoxy]-2-furananilide) (ancarolol);

(2-acetyl-7-(2-hydroxy-3-isopropylaminopropoxy)benzofuran HCl) (befunolol);

((±)-1-(isopropylamino)-3-(p-(2-cyclopropylmethoxyethyl)-phenoxy)-2-propranol HCl) (betaxolol);

(1-[(3,4-dimethoxyphenethyl)amino]-3-(m-tolyloxy)-2-propanol HCl) (bevantolol);

(((±)-1-(4-((2-isopropoxyethoxy)methyl)phenoxy)-3-isopropylamino-2-propanol)fumarate) (bisoprolol);

(4-(2-hydroxy-3-[4-(phenoxymethyl)-piperidino]-propoxy)-indole);

(carbazolyl-4-oxy-5,2-(2-methoxyphenoxy)-ethylamino-2-propanol);

(1-((1,1-dimethylethyl)amino)-3-((2-methyl-1H-indol-4-yl)oxy)-2-propanol benzoate) (bopindolol);

(1-(2-exobicyclo[2.2.1]-hept-2-ylphenoxy)-3-[(1-methylethyl)-amino]-2-propanol HCl) (bornaprolol);

(o-[2-hydroxy-3-[(2-indol-3-yl-1,1-dimethylethyl)-amino]propoxy]benzonitrile HCl) (bucindolol);

(α-[(tert.butylamino)methyl]-7-ethyl-2-benzofuranmethanol) (bufuralol);

(3-[3-acetyl-4-[3-(tert.butylamino)-2-hydroxypropyl]-phenyl]-1,1-diethylurea HCl) (celiprolol);

((±)-2-[2-[3-[(1,1-dimethylethyl)amino]-2-hydroxypropoxy]phenoxy]-N-methylacetamide HCl) (cetamolol);

(2-benzimidazolyl-phenyl(2-isopropylaminopropanol));

((±)-3'-acetyl-4'-(2-hydroxy-3-isopropylaminopropoxy)-acetanilide HCl) (diacetolol);

(methyl-4-[2-hydroxy-3-[(1-methylethyl)aminopropoxy]]-benzenepropanoate HCl) (esmolol);

(erythro-DL-1-(7-methylindan-4-yloxy)-3-isopropylaminobutan-2-ol);

(1-(tert.butylamino)-3-[O-(2-propynyloxy)phenoxy]-2-propanol (pargolol);

(1-(tert.butylamino)-3-[o-(6-hydrazino-3-pyridazinyl)-phenoxy]-2-propanol diHCl) (prizidilol);

((-)-2-hydroxy-5-[(R)-1-hydroxy-2-[(R)-(1-methyl-3-phenylpropyl)amino]ethyl]benzamide);

(4-hydroxy-9-[2-hydroxy-3-(isopropylamino)-propoxy]-7-methyl-5H-furo[3,2-g][1]-benzopyran-5-one) (iprocrolol);

((-)-5-(tert.butylamino)-2-hydroxypropoxy]-3,4-dihydro-1-(2H)-naphthalenone HCl) (levobunolol);

(4-(2-hydroxy-3-isopropylamino-propoxy)-1,2-benziso-thiazole HCl);

(4-[3-(tert.butylamino)-2-hydroxypropoxy]-N-methyliso-carbostyril HCl);

((±)-N-2-[4-(2-hydroxy-3-isopropyl aminopropoxy)-phenyl]ethyl-N'-isopropylurea) (pafenolol);

(3-[[(2-trifluoroacetamido)ethyl]amino]-1-phenoxy-propan-2-ol);

(N-(3-(o-chlorophenoxy)-2-hydroxypropyl)-N'-(4'-chloro-2,3-dihydro-3-oxo-5-pyridazinyl)ethylenediamine);

((±)-N-[3-acetyl-4-[2-hydroxy-3-[(1-methylethyl)amino]-propoxy]phenyl]butanamide) (acebutolol);

((±)-4'-[3-(tert-butylamino)-2-hydroxypropoxy]spiro-[cyclohexane-1,2'-indan]-1'-one) (spirendolol);

(7-[3-[[2-hydroxy-3-[(2-methylindol-4-yl)oxy]propyl]-amino]butyl]thiophylline) (teoprolol);

((±)-1-tert.butylamino-3-(thiochroman-8-yloxy)-2-propanol) (tertatolol);

((±)-1-tert.butylamino-3-(2,3-xylyloxy)-2-propanol HCl) (xibenolol);

(8-[3-(tert.butylamino)-2-hydroxypropoxy]-5-methylcoumarin) (bucumolol);

(2-(3-(tert.butylamino)-2-hydroxy-propoxy)benzonitrile HCl) (bunitrolol);

((±)-2'-[3-(tert-butylamino)-2-hydroxypropoxy-5'-fluorobutyrophenone) (butofilolol);

(1-(carbazol-4-yloxy)-3-(isopropylamino)-2-propanol) (carazolol);

(5-(3-tert.butylamino-2-hydroxy)propoxy-3,4-dihydrocarbostyril HCl) (carteolol);

(1-(tert.butylamino)-3-(2,5-dichlorophenoxy)-2-propanol) (cloranolol);

(1-(inden-4(or 7)-yloxy)-3-(isopropylamino)-2-propanol HCl) (indenolol);

(1-isopropylamino-3-[(2-methylindol-4-yl)oxy]-2-propanol) (mepindolol);

(1-(4-acetoxy-2,3,5-trimethylphenoxy)-3-isopropylaminopropan-2-ol) (metipranolol);

(1-(isopropylamino)-3-(o-methoxyphenoxy)-3-[(1-methylethyl)amino]-2-propanol) (moprolol);

((1-tert.butylamino)-3-[(5,6,7,8-tetrahydro-cis-6,7-dihydroxy-1-naphthyl)oxy]-2-propanol) (nadolol);

((S)-1-(2-cyclopentylphenoxy)-3-[(1,1-dimethylethyl)-amino]-2-propanol sulfate (2:1)) (penbutolol);

(4'-[1-hydroxy-2-(amino)ethyl]methanesulfonanilide) (sotalol);

(2-methyl-3-[4-(2-hydroxy-3-tert.butylaminopropoxy)-phenyl]-7-methoxy-isoquinolin-1-(2H)-one);

(1-(4-(2-(4-fluorophenyloxy)ethoxy)phenoxy)-3-isopropylamino-2-propanol HCl);

((-)-p-[3-[(3,4-dimethoxyphenethyl)amino]-2-hydroxypropoxy]-ß-methylcinnamonitrile) (pacrinolol);

((±)-2-(3'-tert.butylamino-2'-hydroxypropylthio)-4-(5'-carbamoyl-2'-thienyl)thiazole HCl) (arotinolol);

((±)-1-[p-[2-(cyclopropylmethoxy)ethoxy]phenoxy]-3-(isopropylamino)-2-propanol) (cicloprolol);

((±)-1-[(3-chloro-2-methylindol-4-yl)oxy]-3-[(2-phenoxyethyl)amino]-2-propanol) (indopanolol);

((±)-6-[[2-[[3-(p-butoxyphenoxy)-2-hydroxypropyl]-amino]ethyl]amino]-1,3-dimethyluracil) (pirepolol);

(4-(cyclohexylamino)-1-(1-naphtholenyloxy)-2-butanol);

(1-phenyl-3-[2-[3-(2-cyanophenoxy)-2-hydroxypropyl]-aminoethyl]hydantoin HCl);

(3,4-dihydro-8-(2-hydroxy-3-isopropylaminopropoxy)-3-nitroxy-2H-1-benzopyran) (nipradolol);

@ and ß-Adrenergic Blocking Agents:

((±)-1-tert-butylamino)-3-[o-[2-(3-methyl-5-isoxazolyl)vinyl]phenoxy]-2-propanol) (isoxaprolol);

(1-isopropylamino-3-(4-(2-nitroxyethoxy)phenoxy)-2-propanol HCl);

(4-hydroxy-α-[[3-(4-methoxyphenyl)-1-methylpropyl]-aminomethyl]-3-(methylsulfinyl)-benzmethanol HCl) (sulfinalol);

(5-[1-hydroxy-2-[[2-(o-methoxyphenoxy)ethyl]amino]-ethyl]-2-methylbenzenesulfonamide HCl);

(5-[1-hydroxy-2-[(1-methyl-3-phenylpropyl)amino]ethyl]-salicylamide HCl) (labetalol);

(1-((3-chloro-2-methyl-1H-indol-4-yl)oxy)-3-((2-phenoxyethyl)amino)-2-propanol-hydrogenmalonate) (ifendolol);

(4-(2-hydroxy-3-[(1-methyl-3-phenylpropyl)amino]-propoxy)benzeneacetamide);

(1-[3-[[3-(1-naphthoxy)-2-hydroxypropyl]-amino]-3,3-dimethyl-propyl]-2-benzimidazolinone);

(3-(1-(2-hydroxy-2-(4-chlorophenylethyl)-4-piperidyl)-3,4-dihydroxy)quinoxolin-2(1H)-one);

<u>CNS-Acting Agents</u>: clonidine; methyldopa;

<u>Adrenergic Neuron Blocking Agents</u>: guanethidine; reserpine and other rauwolfia alkaloids such as rescinnamine;

<u>Vasodilators</u>: diazoxide; hydralazine; minoxidil;

<u>Calcium Channel Blockers</u>:

α-[3-[[2-(3,4-dimethoxyphenyl)ethyl]methylamino]-propyl]-3,4-dimethoxy-α-(1-methylethyl)benzene-acetonitrile (verapamil);

1,4-dihydro-2,6-dimethyl-4-(2-nitrophenyl)-3,5-pyridinedicarboxylic acid dimethyl ester (nifedipine);

2-(2,2-dicyclohexylethyl)piperidine (perhexiline);

N-(1-methyl-2-phenylethyl)- -phenylbenzenepropanamine (prenylamine);

3-(aminosulfonyl)-4-chloro-N-(2,3-dihydro-2-methyl-1H-indol-1-yl)benzamide (indapamide);

(2'-(2-diethylaminoethoxy)-3-phenylpropiophenone (etafenone);

(4-[4,4-bis-(4-fluorophenyl)butyl]-N-(2,6-dimethylphenyl)-1-piperazineacetamide) (lidoflazine);

(2-(N-benzyl-N-methylamino)ethylmethyl-2,6-dimethyl-4-(m-nitrophenyl)-1,4-dihydro-3,5-pyridinedicarboxylate HCl) (nicardipine);

(N-(3,4-dimethoxyphenethyl)-2-(3,4-dimethoxyphenyl)-N-methyl-m-dithiane-2-propylamine-1,1,3,3-tetraoxide) (tiapamil);

(5,6-dimethoxy-2-(3-[(α-(3,4-dimethoxy)phenylethyl)-methylamino]propyl)phthalimidine) (falipamil);

(β-[(2-methylpropoxy)methyl]-N-phenyl-N-phenylmethyl-1-pyrrolidineethanamine HCl monohydrate) (bepridil);

((+)-cis-3-(acetyloxy)-5-[2-(dimethylamino)ethyl]-2,3-dihydro-2-(4-methoxyphenyl)-1,5-benzothiazepin-4-(5H)-one) (diltiazem);

((E)-1-[bis-(p-fluorophenyl)methyl]-4-cinnamylpiperazine di HCl) (flunarizine);

(5-[(3,4-dimethoxyphenethyl)methylamino]-2-isopropyl-2-(3,4,5-trimethoxyphenyl)valeronitrile (gallopamil);

(ethylmethyl(2,3-dichlorophenyl)-1,4-dihydro-2,6-dimethyl-3,5-pyridinedicarboxylate (felodipine);

(isopropyl-2-methoxyethyl-1,4-dihydro-2,6-dimethyl-4-(3-nitrophenyl)-3,5-pyridinecarboxylate) (nimodipine);

(3-ethyl-5-methyl-1,4-dihydro-2,6-dimethyl-4-(3-nitrophenyl)-3,5-pyridine-dicarboxylate) (nitrendipine);

<u>Angiotensin I Converting Enzyme Inhibitors</u>:

1-(3-mercapto-2-methyl-1-oxopropyl)-L-proline (captopril);

(1-(4-ethoxycarbonyl-2,4(R,R)-dimethylbutanoyl)-indoline-2(S)-carboxylic acid);

(2-[2-[[1-(ethoxycarbonyl)-3-phenyl-propyl]amino]-1-oxopropyl]-1,2,3,4-tetrahydro-3-isoquinoline carboxylic acid);

((S)-1-[2-[[1-(ethoxycarbonyl)-3-phenylpropyl]amino]-1-oxopropyl]octahydro-1H-indole-2-carboxylic acid HCl);

(N-cyclopentyl-N-(3-(2,2-dimethyl-1-oxopropyl)thiol-2-methyl-1-oxopropyl)glycine) (pivalopril);

((2R,4R)-2-(2-hydroxyphenyl)-3-(3-mercaptopropionyl)-4-thiazolidinecarboxylic acid);

(1-(N-[1(S)-ethoxycarbonyl-3-phenylpropyl]-(S)-alanyl)-cis,syn-octahydroindol-2(S)-carboxylic acid HCl);

((-)-(S)-1-[(S)-3-mercapto-2-methyl-1-oxopropyl]-indoline-2-carboxylic acid);

([1(S),4S]-1-[3-(benzoylthio)-2-methyl-1-oxopropyl]-4-phenylthio-L-proline;

(3-([1-ethoxycarbonyl-3-phenyl-(1S)-propyl]amino)-2,3,4,5-tetrahydro-2-oxo-1-(3S)-benzazepine-1-acetic acid HCl);

(N-(2-benzyl-3-mercaptopropanoyl)-S-ethyl-L-cysteine) and the S-methyl analogue;

(N-(1(S)-ethoxycarbonyl-3-phenylpropyl)-L-alanyl-L-proline maleate) (enalapril);

N-[1-(S)-carboxy-3-phenylpropyl]-L-alanyl-1-proline;

$N^2$-[1-(S)-carboxy-3-phenylpropyl]-L-lysyl-L-proline (lysinopril);

<u>Other Antihypertensive Agents</u>: aminophylline; cryptenamine acetates and tannates; deserpidine; meremethoxylline procaine; pargyline; trimethaphan camsylate;

including pharmaceutical salt and ester forms thereof.